# EUROPEAN PATENT APPLICATION

(11) **EP 4 339 181 A1**
(43) Date of publication of application: **20.03.2024**
(21) Application number: 22806866.4
(22) Date of filing: 13.05.2022
(51) Int. Cl.: C07C 35/36, C07C 233/64, C07C 309/29, A61K 31/165, A61P 35/00

(54) **NAPHTHALENE RING-CONTAINING COMPOUND, PHARMACEUTICAL COMPOSITION CONTAINING SAME, AND USE THEREOF**

(30) Priority: 14.05.2021 CN 202110533784; 05.05.2022 CN 202210506641
(71) Applicant: Jiangsu Yahong Meditech Co., Ltd., Taizhou, Jiangsu 225316 (CN); Asieris Pharmaceuticals (Shanghai) Co., Ltd., Pudong, Shanghai 201203 (CN)
(72) Inventor: WU, Liang, Shanghai 201203 (CN); DENG, Yijun, Shanghai 201203 (CN); HE, Zhenhua, Shanghai 201203 (CN); SUN, Qiaoling, Shanghai 201203 (CN); ZHOU, Chen, Shanghai 201203 (CN); WANG, Tielin, Shanghai 201203 (CN)
(74) Representative: Haseltine Lake Kempner LLP
(86) International application number: PCT/CN2022/092865
(87) International publication number: WO 2022/237904

(57) **Abstract**

A naphthalene ring-containing compound, a pharmaceutical composition containing same, and the use thereof. A naphthalene ring-containing compound as represented by formula III, a pharmaceutically acceptable salt thereof, a solvate thereof, or a solvate of the pharmaceutically acceptable salt thereof. The compound is new in structure and has a better activity on cancers.

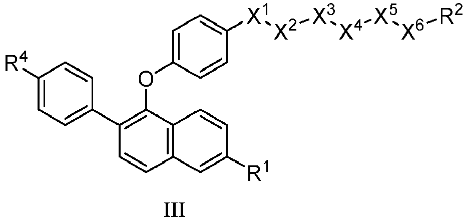

## Description

The present application claims the right of the priorities of Chinese patent application 2021105337844 filed on May 14, 2021 and Chinese patent application 2022105066419 filed on May 5, 2022. The contents of the above Chinese patent applications are incorporated herein by reference in their entireties.

### TECHNICAL FIELD

The present disclosure relates to a naphthalene ring-containing compound, a pharmaceutical composition comprising the same, and a use thereof.

### BACKGROUND

Estrogen receptor (ER), one of the key members of the nuclear hormone receptor family, acts as a ligand-activated transcription factor involved in both up-regulation and downregulation of gene expression. Natural agonists of the estrogen receptor include 17-β-estradiol (E2) and its closely related metabolites. Natural agonists bind to the estrogen receptor to cause receptor dimerization, and then bind to the corresponding estrogen response element (ERE) on DNA. The ER-DNA complex recruits other transcription factors responsible for transcribing DNA downstream of the ERE into mRNA. Since the expression of a large number of genes is regulated by the estrogen receptor, and since the estrogen receptor is expressed in many cell types, estrogen receptor regulation by binding natural hormones or synthetic ER ligands can have a profound effect on the physiology and pathophysiology of organisms.

There are two distinct forms of human estrogen receptors, commonly referred to as estrogen receptor α (abbreviated as ERα) and estrogen receptor β (abbreviated as EPβ), which are encoded by their respective corresponding genes. The two ERs are distributed differently in different tissues. ERα is found in endometrium, breast cancer cells, ovarian stromal cells, and hypothalamus. ERβ is mainly found in kidney, brain, bone, heart, prostate, and endothelial cells.

Estrogen plays an important role in maintaining bone density, regulating blood lipids, neuroprotection, etc. Clinical trials have shown that the estrogen receptor mediates a variety of diseases, which are collectively referred to as estrogen-dependent diseases. The estrogen receptor is overexpressed in certain types of proliferative diseases, such as breast cancer, uterine cancer, and endometriosis, and plays a key role in the proliferation of breast cancer cells, as evidenced by the favorable efficacy of estrogen receptor antagonists in the treatment of breast cancer.

As the most common malignant tumor in women, the incidence of breast cancer is increasing year by year all over the world, and estrogen receptor-positive breast cancer patients account for one third of all breast cancer patients. Currently, treatments for estrogen receptor-positive breast cancer include ablation of ovarian function through surgery, radiation therapy, or medical treatment in premenopausal women with advanced breast cancer. In postmenopausal patients, treatment is achieved by inhibiting estrogen synthesis with aromatase inhibitors.

In addition, estrogen receptor antagonists have been widely used in the treatment of estrogen receptor-positive breast cancer, for example, tamoxifen, a competitive ER antagonist, but its effectiveness is often limited by its demonstrated partial agonist effect, which leads to incomplete blockade of estrogen receptor-mediated pathways. In contrast, fulvestrant is a novel estrogen receptor antagonist, as well as an estrogen receptor protein degrader, which is completely free of the partial agonist side effects associated with currently available estrogen receptor antagonists such as tamoxifen.

Although fulvestrant has shown a good effect in the treatment of breast cancer due to its unique mechanism of action, the maximum protein degradation rate is about 61% due to the non-oral administration method and its incomplete estrogen receptor protein degradation rate, which makes the development of an oral estrogen degrader with a high protein degradation rate significant.

Proteolysis targeting chimera (PROTAC) is a cutting-edge technology that utilizes endogenous proteins to recruit E3 ubiquitin ligases for the degradation of specific target proteins, which utilizes the ubiquitin proteasome system (UPS) and autophagy/lysosome pathway essential for human normal physiology to realize the degradation of disease-related target proteins, thus achieving therapeutic effects for the corresponding diseases. This technology not only overcomes the shortcomings of macromolecular antibodies and kinases targeting small molecules, but also has the potential advantage of breaking through the bottleneck of "undruggable targets" and overcoming drug resistance in tumors, which is very likely to bring about a revolutionary change in the research and development of new drugs as well as improvement of tumor treatment.

The proteolysis targeting chimeric compound comprises three components: a target protein ligand, an E3 ubiquitin ligase ligand, and a linker. The E3 ubiquitin ligase is directed to the target protein through the target protein ligand, thereby selectively leading to the polyubiquitination of the target protein, resulting in recognition and degradation of the target protein by the proteasome. To date, PROTAC technology can be used to target a variety of proteins, including transcription factors, scaffold proteins, enzymes, and regulatory factors. This technology has already received a great deal of attention from pharmaceutical companies around the world. PROTAC technology provides a new technical platform for drug development through the above mechanism.

### CONTENT OF THE PRESENT INVENTION

The present disclosure provides a naphthalene ring-containing compound, a pharmaceutical composition comprising the same, and a use thereof. The compound of the present disclosure has a novel structure and shows better activity on cancer. The compound of the present disclosure is expected to show comparable or even better efficacy than existing drugs for the treatment of breast cancer. Preferred compounds among the compounds of the present disclosure have comparable or even better inhibitory activity on the proliferation of drug-resistant mutant cells than existing antagonists on estrogen receptors.

The present disclosure provides a naphthalene ring-containing compound of formula **III**, a pharmaceutically acceptable salt thereof, a solvate thereof, or a solvate of the pharmaceutically acceptable salt thereof; 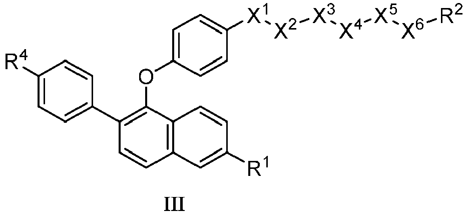
wherein R¹ is hydrogen, cyano, hydroxyl, or C₁-C₄ alkoxy;
X¹ is -O-, 5- to 6-membered heterocycloalkylene, or 5- to 6-membered heterocycloalkylene substituted by R^{x1-1}, in the 5- to 6-membered heterocycloalkylene and the 5- to 6-membered heterocycloalkylene substituted by R^{x1-1}, the heteroatom is one or more than one of O, S, and N, and the number of heteroatoms is 1, 2, or 3; for example, the case where the number of heteroatoms is 2 comprises: both are O, both are N, both are S, one is O and one is N, one is O and one is S, and one is S and one is N;
R^{x1-1} is independently C₁-C₄ alkyl;
X² is absent, -C(=O)-, C₁-C₆ alkylene, C₂-C₁₅ heteroalkylene, or C₂-C₁₅ heteroalkylene substituted by R^{x2-1}; in the C₂-C₁₅ heteroalkylene, the heteroatom is one or more than one of O, S, and N, and the number of heteroatoms is 1, 2, 3, 4, 5, 6, 7, 8, or 9;
R^{x2-1} is independently C₁-C₄ alkyl;
X³ is absent, -C(=O)-Ph-, 5- to 6-membered heterocycloalkylene, 5- to 6-membered heteroarylene, 5- to 6-membered heterocycloalkylene substituted by R^{x3-1}, or 5- to 6-membered heteroarylene substituted by R^{x3-2}; in the 5- to 6-membered heterocycloalkylene and the 5- to 6-membered heterocycloalkylene substituted by R^{x3-1}, the heteroatom is one or more than one of O, S, and N, and the number of heteroatoms is 1, 2, or 3; in the 5- to 6-membered heteroarylene and the 5- to 6-membered heteroarylene substituted by R^{x3-2}, the heteroatom is one or more than one of O, S, and N, and the number of heteroatoms is 1, 2, or 3;
R^{x3-1} and R^{x3-2} are independently C₁-C₄ alkyl;
X⁴ is absent, C₁-C₁₀ alkylene, or C₂-C₁₅ heteroalkylene; in the C₂-C₁₅ heteroalkylene, the heteroatom is one or more than one of O, S, and N, and the number of heteroatoms is 1, 2, 3, 4, 5, 6, 7, 8, or 9;
X⁵ is absent, 5- to 6-membered heterocycloalkylene, or 5- to 6-membered heterocycloalkylene substituted by R^{x5-1}; in the 5- to 6-membered heterocycloalkylene and the 5- to 6-membered heterocycloalkylene substituted by R^{x5-1}, the heteroatom is one or more than one of O, S, and N, and the number of heteroatoms is 1, 2, or 3;
R^{x5-1} is independently C₁-C₄ alkyl;
X⁶ is absent, -O-, -NH-, -C(=O)-NH-, 5- to 6-membered heterocycloalkylene, -C(=O)-5- to 6-membered heterocycloalkylene, or 5- to 6-membered heterocycloalkylene substituted by R^{x6-1}; in the 5- to 6-membered heterocycloalkylene, the -C(=O)-5- to 6-membered heterocycloalkylene, and the 5- to 6-membered heterocycloalkylene substituted by R^{x1-1}, the heteroatom is one or more than one of O, S, and N, and the number of heteroatoms is 1, 2, or 3;
R^{x6-1} is independently C₁-C₄ alkyl;
R² is 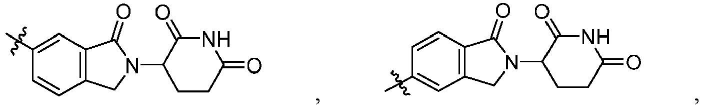 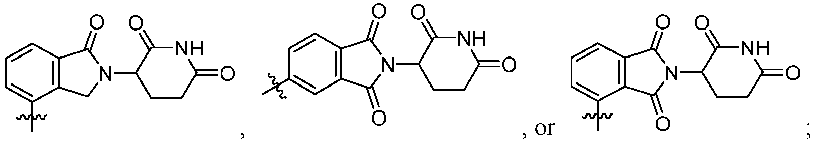 ;
R⁴ is -SO₂-C₁-C₄ alkyl or 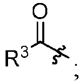
R³ is 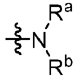 or 3- to 6-membered heterocycloalkyl, R^{a} and R^{b} are independently hydrogen or C₁-C₄ alkyl; the 3- to 6-membered heterocycloalkyl is linked to carbonyl through N; in the 3- to 6-membered heterocycloalkyl, the heteroatom is N, and the number of heteroatoms is 1 or 2.

In a certain embodiment, in the naphthalene ring-containing compound of formula **III**, the pharmaceutically acceptable salt thereof, the solvate thereof, or the solvate of the pharmaceutically acceptable salt thereof, some of the groups or moieties in the naphthalene ring-containing compound of formula **III** have the meanings set forth below, and the rest have the meanings set forth in any one of the preceding embodiments (hereinafter referred to as "in a certain embodiment"): the naphthalene ring-containing compound of formula **III** is a naphthalene ring-containing compound of formula **I** or **II**:

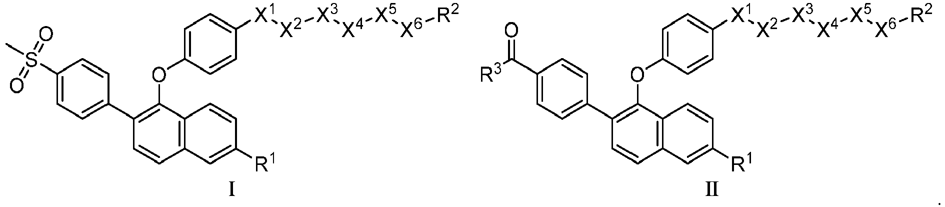

In a certain embodiment, in the naphthalene ring-containing compound of formula **I**,
wherein R¹ is hydrogen, cyano, hydroxyl, or C₁-C₄ alkoxy;
X¹ is -O-, 5- to 6-membered heterocycloalkylene, or 5- to 6-membered heterocycloalkylene substituted by R^{x1-1}, in the 5- to 6-membered heterocycloalkylene and the 5- to 6-membered heterocycloalkylene substituted by R^{x1-1}, the heteroatom is one or more than one of O, S, and N, and the number of heteroatoms is 1, 2, or 3;
R^{x1-1} is independently C₁-C₄ alkyl;
X² is C₁-C₆ alkylene or C₂-C₁₅ heteroalkylene; in the C₂-C₁₅ heteroalkylene, the heteroatom is one or more than one of O, S, and N, and the number of heteroatoms is 1, 2, 3, 4, 5, 6, 7, 8, or 9;
X³ is absent, -C(=O)-Ph-, 5- to 6-membered heterocycloalkylene, 5- to 6-membered heteroarylene, 5- to 6-membered heterocycloalkylene substituted by R^{x3-1}, or 5- to 6-membered heteroarylene substituted by R^{x3-2}; in the 5- to 6-membered heterocycloalkylene and the 5- to 6-membered heterocycloalkylene substituted by R^{x3-1}, the heteroatom is one or more than one of O, S, and N, and the number of heteroatoms is 1, 2, or 3; in the 5- to 6-membered heteroarylene and the 5- to 6-membered heteroarylene substituted by R^{x3-2}, the heteroatom is one or more than one of O, S, and N, and the number of heteroatoms is 1, 2, or 3;
R^{x3-1} and R^{x3-2} are independently C₁-C₄ alkyl;
X⁴ is absent, C₁-C₁₀ alkylene, or C₂-C₁₅ heteroalkylene; in the C₂-C₁₅ heteroalkylene, the heteroatom is one or more than one of O, S, and N, and the number of heteroatoms is 1, 2, 3, 4, 5, 6, 7, 8, or 9;
X⁵ is absent, 5- to 6-membered heterocycloalkylene, or 5- to 6-membered heterocycloalkylene substituted by R^{x5-1}; in the 5- to 6-membered heterocycloalkylene and the 5- to 6-membered heterocycloalkylene substituted by R^{x5-1}, the heteroatom is one or more than one of O, S, and N, and the number of heteroatoms is 1, 2, or 3;
R^{x5-1} is independently C₁-C₄ alkyl;
X⁶ is absent, -O-, -NH-, -C(=O)-NH-, 5- to 6-membered heterocycloalkylene, -C(=O)-5- to 6-membered heterocycloalkylene, or 5- to 6-membered heterocycloalkylene substituted by R^{x6-1}, in the 5- to 6-membered heterocycloalkylene, the -C(=O)-5- to 6-membered heterocycloalkylene, and the 5- to 6-membered heterocycloalkylene substituted by R^{x1-1}, the heteroatom is one or more than one of O, S, and N, and the number of heteroatoms is 1, 2, or 3;
R^{x6-1} is independently C₁-C₄ alkyl;
R² is

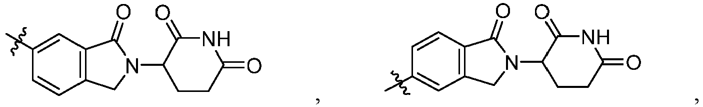

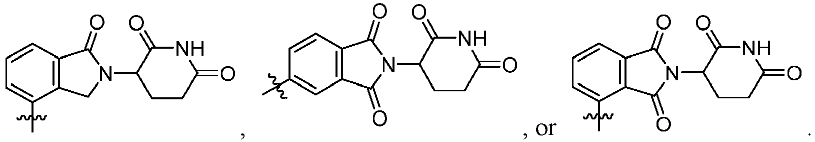

In a certain embodiment, in R¹, the C₁-C₄ alkoxy may be methoxy, ethoxy, n-propoxy, or isopropoxy.

In a certain embodiment, in X¹, in the 5- to 6-membered heterocycloalkylene and the 5- to 6-membered heterocycloalkylene substituted by R^{x1-1}, the heteroatom is N, and the number of heteroatoms is 1 or 2.

In a certain embodiment, in X¹, the 5- to 6-membered heterocycloalkylene may be piperidinylene or piperazinylene, and may be

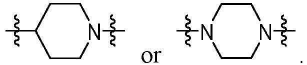

In a certain embodiment, in X¹, in the 5- to 6-membered heterocycloalkylene substituted by R^{x1-1}, the 5- to 6-membered heterocycloalkylene may be piperidinylene or piperazinylene, and may be

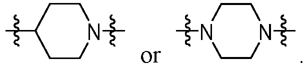

In a certain embodiment, in X¹, the number of R^{x1-1} may be 1, 2, or 3.

In a certain embodiment, in R^{x1-1}, the C₁-C₄ alkyl may be methyl or ethyl.

In a certain embodiment, in X¹, the 5- to 6-membered heterocycloalkylene substituted by R^{x1-1} may be

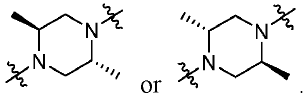

In a certain embodiment, X¹ may be -O- or

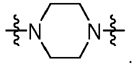

In a certain embodiment, in X², the C₁-C₆ alkylene may be methylene, 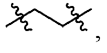 or

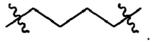

In a certain embodiment, in X², the C₂-C₁₅ heteroalkylene may be C₄-C₁₂ heteroalkylene.

In a certain embodiment, in X², in the C₂-C₁₅ heteroalkylene, the heteroatom is O and/or N, and the number of heteroatoms is 1, 2, 3, 4, or 5.

In a certain embodiment, in X², the C₂-C₁₅ heteroalkylene may be linked to the other group through the carbon atom.

In a certain embodiment, in R^{x2-1}, the C₁-C₄ alkyl is methyl or ethyl.

In a certain embodiment, in X², the C₂-C₁₅ heteroalkylene may be any one of the following structures: 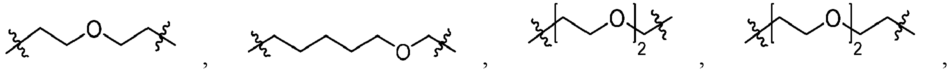 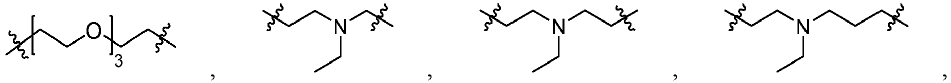 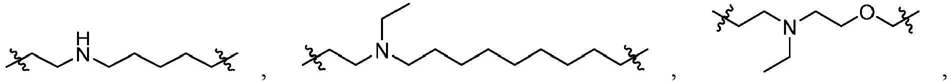 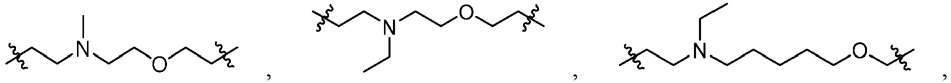 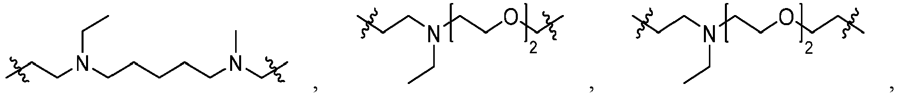 or

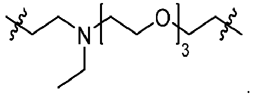

In a certain embodiment, X² may be absent, -C(=O)-, methylene, 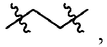 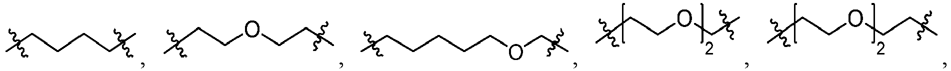 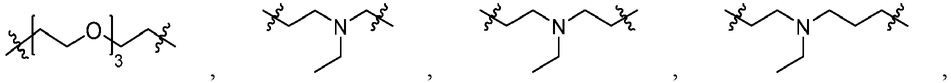 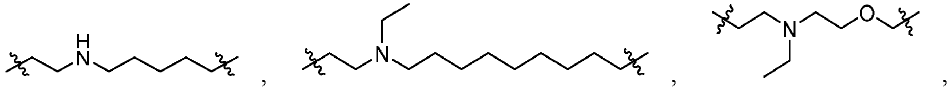 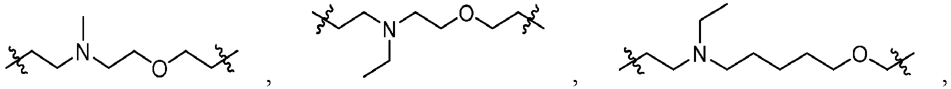 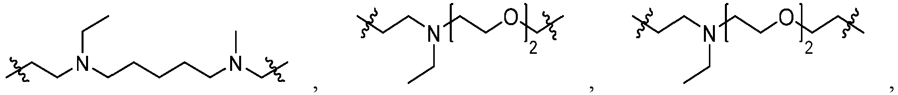 or

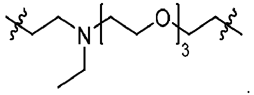

In a certain embodiment, in X³, in the 5- to 6-membered heterocycloalkylene and the 5- to 6-membered heterocycloalkylene substituted by R^{x3-1}, the heteroatom is N, and the number of heteroatoms is 1 or 2.

In a certain embodiment, in X³, the 5- to 6-membered heterocycloalkylene may be piperidinylene or piperazinylene, and may be

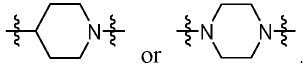

In a certain embodiment, in X³, in the 5- to 6-membered heterocycloalkylene substituted by R^{x3-1}, the 5- to 6-membered heterocycloalkylene may be piperidinylene or piperazinylene, and may be

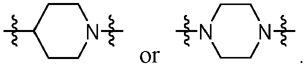

In a certain embodiment, in X³, the number of R^{x3-1} may be 1, 2, or 3.

In a certain embodiment, in R^{x3-1}, the C₁-C₄ alkyl may be methyl or ethyl.

In a certain embodiment, in X³, the 5- to 6-membered heterocycloalkylene substituted by R^{x3-1} may be

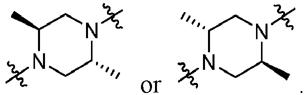

In a certain embodiment, in X³, the 5- to 6-membered heteroarylene may be triazolylene, and may be

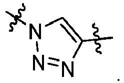

In a certain embodiment, in X³, in the 5- to 6-membered heteroarylene substituted by R^{x3-2}, the 5- to 6-membered heteroarylene may be triazolylene, and may be

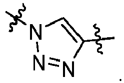

In a certain embodiment, in R^{x3-2}, the C₁-C₄ alkyl may be methyl or ethyl.

In a certain embodiment, X³ may be absent, 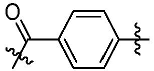 (e.g., 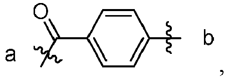 wherein a end is linked to X² and b end is linked to X⁴),

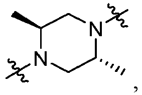

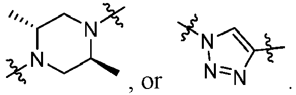

In a certain embodiment, in X⁴, the C₁-C₁₀ alkylene may be C₁-C₄ alkylene, and may be methylene or

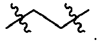

In a certain embodiment, in X⁴, the C₂-C₁₅ heteroalkylene may be C₂-C₄ heteroalkylene.

In a certain embodiment, in X⁴, in the C₂-C₁₅ heteroalkylene, the heteroatom is O and/or N, and the number of heteroatoms is 1 or 2.

In a certain embodiment, in X⁴, the C₂-C₁₅ heteroalkylene may be C₂-C₄ heteroalkylene in which the heteroatom is O and/or N and the number of heteroatoms is 1 or 2, and may be

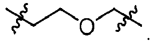

In a certain embodiment, X⁴ may be absent, methylene,

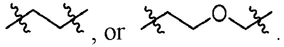

In a certain embodiment, in X⁵, in the 5- to 6-membered heterocycloalkylene and the 5- to 6-membered heterocycloalkylene substituted by R^{x5-1}, the heteroatom is N, and the number of heteroatoms is 1 or 2.

In a certain embodiment, in X⁵, the 5- to 6-membered heterocycloalkylene may be piperidinylene or piperazinylene, and may be

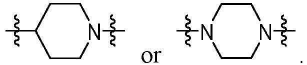

In a certain embodiment, in X⁵, in the 5- to 6-membered heterocycloalkylene substituted by R^{x5-1}, the 5- to 6-membered heterocycloalkylene may be piperidinylene or piperazinylene, and may be

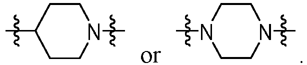

In a certain embodiment, in X⁵, the number of R^{x5-1} may be 1, 2, or 3.

In a certain embodiment, in R^{x5-1}, the C₁-C₄ alkyl may be methyl or ethyl.

In a certain embodiment, in X⁵, the 5- to 6-membered heterocycloalkylene substituted by R^{x5-1} may be

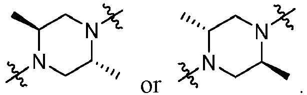

In a certain embodiment, X⁵ may be absent or

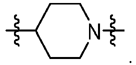

In a certain embodiment, in X⁶, in the 5- to 6-membered heterocycloalkylene and the 5- to 6-membered heterocycloalkylene substituted by R^{x6-1}, the heteroatom is N, and the number of heteroatoms is 1 or 2.

In a certain embodiment, in X⁶, the 5- to 6-membered heterocycloalkylene may be piperidinylene or piperazinylene, and may be

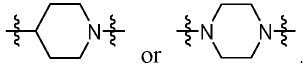

In a certain embodiment, in X⁶, in the 5- to 6-membered heterocycloalkylene substituted by R^{x6-1}, the 5- to 6-membered heterocycloalkylene may be piperidinylene or piperazinylene, and may be

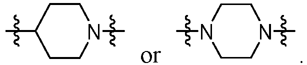

In a certain embodiment, in X⁶, the number of R^{x6-1} may be 1, 2, or 3.

In a certain embodiment, in R^{x6-1}, the C₁-C₄ alkyl may be methyl or ethyl.

In a certain embodiment, in X⁶, the 5- to 6-membered heterocycloalkylene substituted by R^{x6-1} may be

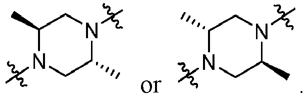

In a certain embodiment, in X⁶, in the -C(=O)-5- to 6-membered heterocycloalkylene, the heteroatom is N, and the number of heteroatoms is 1 or 2.

In a certain embodiment, in X⁶, in the -C(=O)-5- to 6-membered heterocycloalkylene, the 5- to 6-membered heterocycloalkylene may be piperidinylene or piperazinylene, and may be

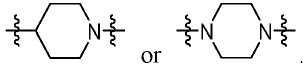

In a certain embodiment, in X⁶, the -C(=O)-5- to 6-membered heterocycloalkylene may be

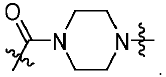

In a certain embodiment, X⁶ may be linked to R² through O or N.

In a certain embodiment, X⁶ may be absent, O, NH, -C(=O)-NH-,

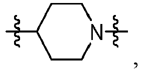

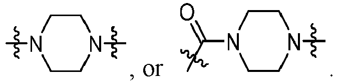

In a certain embodiment, R² may be

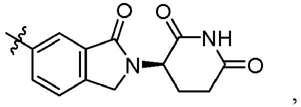

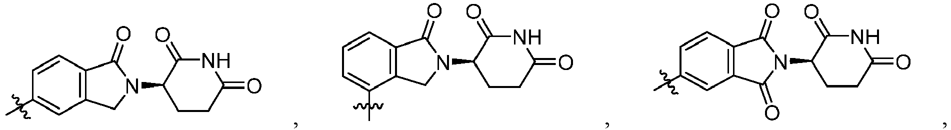

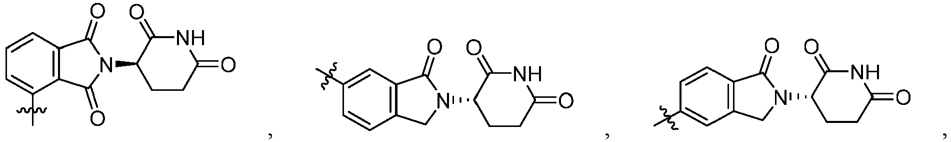

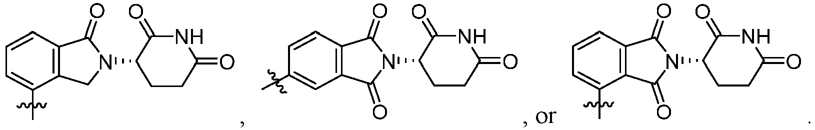

In a certain embodiment, the pharmaceutically acceptable salt may be hydrochloride.

In a certain embodiment, in R^{a}, the C₁-C₄ alkyl may be methyl or ethyl.

In a certain embodiment, in R^{b}, the C₁-C₄ alkyl may be methyl or ethyl.

In a certain embodiment, in R³, in the 3- to 6-membered heterocycloalkyl, the heteroatom is N, and the number of heteroatoms is 1.

In a certain embodiment, in R³, the 3- to 6-membered heterocycloalkyl may be

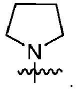

In a certain embodiment, R³ may be

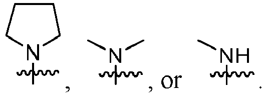

In a certain embodiment, the chiral carbon atom in R² may be in S configuration and R configuration, for example, the ratio of *S* configuration to *R* configuration is 1:1.

In a certain embodiment, the chiral carbon atom in R¹, X¹, X², X³, X⁴, X⁵, and X⁶ may be in S configuration and *R* configuration, for example, the ratio of S configuration to *R* configuration is 1:1.

The left end of a specific structural moiety with two bonding sites in the present disclosure (e.g., 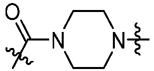 in X⁶) can be attached to the left end moiety of formula **I** (e.g., X⁵), or can be attached to the right end moiety of formula **I** (e.g., R²).

The left end of a specific structural moiety with two bonding sites in the present disclosure (e.g., 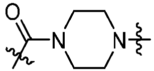 in X⁶) can be attached to the left end moiety of formula **III** (e.g., X⁵), or can be attached to the right end moiety of formula **III** (e.g., R²).

In a certain embodiment, 1, 2, 3, or 4 of X³, X⁴, X⁵, and X⁶ may be absent.

In a certain embodiment, 1, 2, 3, or 4 of X², X³, X⁴, X⁵, and X⁶ may be absent.

In a certain embodiment, X³, X⁴, and X⁵ may be absent.

In a certain embodiment, X², X³, X⁴, and X⁵ may be absent.

In a certain embodiment, X¹ may be -O-.

In a certain embodiment, X² may be C₁-C₆ alkylene, and may be methylene, 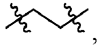 or

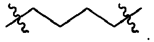

In a certain embodiment, X² may be -C(=O)- or C₁-C₆ alkylene, and may be -C(=O)-, methylene,

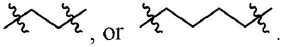

In a certain embodiment, X⁶ may be 5- to 6-membered heterocycloalkylene, -C(=O)-5- to 6-membered heterocycloalkylene, or 5- to 6-membered heterocycloalkylene substituted by R^{x6-1}.

In a certain embodiment, R¹ may be hydroxyl.

In a certain embodiment, R² may be

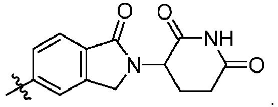

In a certain embodiment, X³, X⁴, and X⁵ may be absent, X¹ may be -O-, X² may be C₁-C₆ alkylene, and X⁶ may be 5- to 6-membered heterocycloalkylene, -C(=O)-5- to 6-membered heterocycloalkylene, or 5- to 6-membered heterocycloalkylene substituted by R^{x6-1}.

In a certain embodiment, R¹ may be hydroxyl, X³, X⁴, and X⁵ may be absent, X¹ may be -O-, X² may be C₁-C₆ alkylene, X⁶ may be 5- to 6-membered heterocycloalkylene, -C(=O)-5- to 6-membered heterocycloalkylene, or 5- to 6-membered heterocycloalkylene substituted by R^{x6-1}, and R² may be

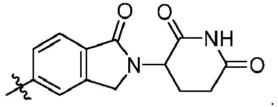

In a certain embodiment, R¹ may be hydroxyl;
X¹ may be -O-;
X² may be C₁-C₆ alkylene or C₂-C₁₅ heteroalkylene;
X³ may be absent, 5- to 6-membered heterocycloalkylene, or 5- to 6-membered heterocycloalkylene substituted by R^{x3-1}, in the 5- to 6-membered heterocycloalkylene and the 5- to 6-membered heterocycloalkylene substituted by R^{x3-1}, the heteroatom is one or more than one of O, S, and N, and the number of heteroatoms is 1, 2, or 3;
X⁴ may be absent or C₂-C₁₅ heteroalkylene; in the C₂-C₁₅ heteroalkylene, the heteroatom is one or more than one of O, S, and N, and the number of heteroatoms is 1, 2, 3, 4, 5, 6, 7, 8, or 9;
X⁵ may be absent;
X⁶ may be 5- to 6-membered heterocycloalkylene, -C(=O)-5- to 6-membered heterocycloalkylene, or 5- to 6-membered heterocycloalkylene substituted by R^{x6-1}, in the 5- to 6-membered heterocycloalkylene, the -C(=O)-5- to 6-membered heterocycloalkylene, and the 5- to 6-membered heterocycloalkylene substituted by R^{x1-1}, the heteroatom is one or more than one of O, S, and N, and the number of heteroatoms is 1, 2, or 3;
R² may be

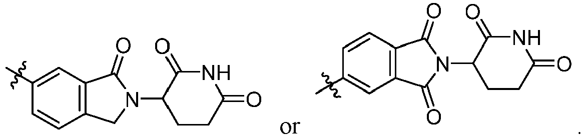

In a certain embodiment, R¹ may be hydroxyl;
X¹ may be -O-;
X² may be C₁-C₆ alkylene, C₂-C₁₅ heteroalkylene, or C₂-C₁₅ heteroalkylene substituted by R^{x2-1};
R^{x2-1} is independently C₁-C₄ alkyl;
X³ may be absent, 5- to 6-membered heterocycloalkylene, or 5- to 6-membered heterocycloalkylene substituted by R^{x3-1}, in the 5- to 6-membered heterocycloalkylene and the 5- to 6-membered heterocycloalkylene substituted by R^{x3-1}, the heteroatom is one or more than one of O, S, and N, and the number of heteroatoms is 1, 2, or 3;
X⁴ may be absent or C₂-C₁₅ heteroalkylene; in the C₂-C₁₅ heteroalkylene, the heteroatom is one or more than one of O, S, and N, and the number of heteroatoms is 1, 2, 3, 4, 5, 6, 7, 8, or 9;
X⁵ may be absent;
X⁶ may be 5- to 6-membered heterocycloalkylene, -C(=O)-5- to 6-membered heterocycloalkylene, or 5- to 6-membered heterocycloalkylene substituted by R^{x6-1}, in the 5- to 6-membered heterocycloalkylene, the -C(=O)-5- to 6-membered heterocycloalkylene, and the 5- to 6-membered heterocycloalkylene substituted by R^{x1-1}, the heteroatom is one or more than one of O, S, and N, and the number of heteroatoms is 1, 2, or 3;
R² may be

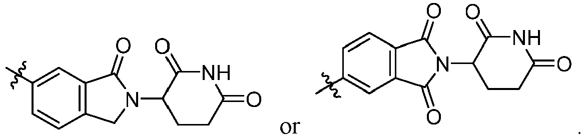

In a certain embodiment, R¹ may be hydroxyl;
X¹ may be -O-;
X² may be C₁-C₆ alkylene, C₂-C₁₅ heteroalkylene, or C₂-C₁₅ heteroalkylene substituted by R^{x2-1}; in the C₂-C₁₅ heteroalkylene, the heteroatom is one or more than one of O, S, and N, and the number of heteroatoms is 1, 2, 3, 4, 5, 6, 7, 8, or 9;
R^{x2-1} is independently C₁-C₄ alkyl;
X³ may be absent, 5- to 6-membered heterocycloalkylene, or 5- to 6-membered heterocycloalkylene substituted by R^{x3-1}, in the 5- to 6-membered heterocycloalkylene and the 5- to 6-membered heterocycloalkylene substituted by R^{x3-1}, the heteroatom is one or more than one of O, S, and N, and the number of heteroatoms is 1, 2, or 3;
X⁴ may be absent, -C(=O)-Ph-, or C₂-C₁₅ heteroalkylene; in the C₂-C₁₅ heteroalkylene, the heteroatom is one or more than one of O, S, and N, and the number of heteroatoms is 1, 2, 3, 4, 5, 6, 7, 8, or 9;
X⁵ may be absent, 5- to 6-membered heterocycloalkylene, or 5- to 6-membered heterocycloalkylene substituted by R^{x5-1}; in the 5- to 6-membered heterocycloalkylene and the 5- to 6-membered heterocycloalkylene substituted by R^{x5-1}, the heteroatom is one or more than one of O, S, and N, and the number of heteroatoms is 1, 2, or 3;
X⁶ may be absent, 5- to 6-membered heterocycloalkylene, -C(=O)-5- to 6-membered heterocycloalkylene, or 5- to 6-membered heterocycloalkylene substituted by R^{x6-1}, in the 5- to 6-membered heterocycloalkylene, the -C(=O)-5- to 6-membered heterocycloalkylene, and the 5- to 6-membered heterocycloalkylene substituted by R^{x1-1}, the heteroatom is one or more than one of O, S, and N, and the number of heteroatoms is 1, 2, or 3;
R² may be

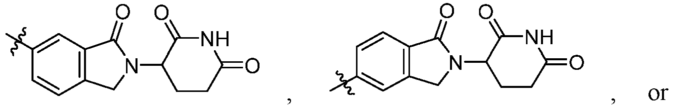

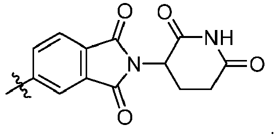

In a certain embodiment, R¹ may be hydroxyl;
X¹ may be -O-;
X² may be C₁-C₆ alkylene, C₂-C₁₅ heteroalkylene, or C₂-C₁₅ heteroalkylene substituted by R^{x2-1}; in the C₂-C₁₅ heteroalkylene, the heteroatom is one or more than one of O, S, and N, and the number of heteroatoms is 1, 2, 3, 4, 5, 6, 7, 8, or 9;
R^{x2-1} is independently C₁-C₄ alkyl;
X³ may be absent, 5- to 6-membered heterocycloalkylene, or 5- to 6-membered heterocycloalkylene substituted by R^{x3-1}, in the 5- to 6-membered heterocycloalkylene and the 5- to 6-membered heterocycloalkylene substituted by R^{x3-1}, the heteroatom is one or more than one of O, S, and N, and the number of heteroatoms is 1, 2, or 3;
X⁴ may be absent, -C(=O)-Ph-, or C₂-C₁₅ heteroalkylene; in the C₂-C₁₅ heteroalkylene, the heteroatom is one or more than one of O, S, and N, and the number of heteroatoms is 1, 2, 3, 4, 5, 6, 7, 8, or 9;
X⁵ may be absent, 5- to 6-membered heterocycloalkylene, or 5- to 6-membered heterocycloalkylene substituted by R^{x5-1}; in the 5- to 6-membered heterocycloalkylene and the 5- to 6-membered heterocycloalkylene substituted by R^{x5-1}, the heteroatom is one or more than one of O, S, and N, and the number of heteroatoms is 1, 2, or 3;
X⁶ may be absent, 5- to 6-membered heterocycloalkylene, -C(=O)-5- to 6-membered heterocycloalkylene, or 5- to 6-membered heterocycloalkylene substituted by R^{x6-1}, in the 5- to 6-membered heterocycloalkylene, the -C(=O)-5- to 6-membered heterocycloalkylene, and the 5- to 6-membered heterocycloalkylene substituted by R^{x1-1}, the heteroatom is one or more than one of O, S, and N, and the number of heteroatoms is 1, 2, or 3;
R² may be 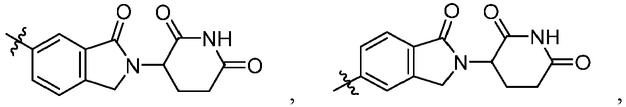 or

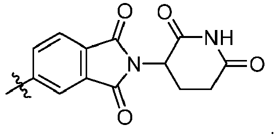

In a certain embodiment, R¹ may be hydroxyl;
X¹ may be -O-;
X² may be C₁-C₆ alkylene;
X³ may be absent; X⁴ may be absent; and X⁵ may be absent;
X⁶ may be 5- to 6-membered heterocycloalkylene, -C(=O)-5- to 6-membered heterocycloalkylene, or 5- to 6-membered heterocycloalkylene substituted by R^{x6-1}, in the 5- to 6-membered heterocycloalkylene, the -C(=O)-5- to 6-membered heterocycloalkylene, and the 5- to 6-membered heterocycloalkylene substituted by R^{x1-1}, the heteroatom is one or more than one of O, S, and N, and the number of heteroatoms is 1, 2, or 3;
R² may be

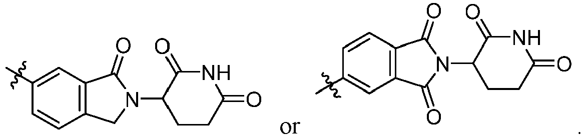

In a certain embodiment, R¹ may be hydroxyl;
X¹ may be -O-;
X² may be C₁-C₆ alkylene, C₂-C₁₅ heteroalkylene, or C₂-C₁₅ heteroalkylene substituted by R^{x2-1}; in the C₂-C₁₅ heteroalkylene, the heteroatom is one or more than one of O, S, and N, and the number of heteroatoms is 1, 2, 3, 4, 5, 6, 7, 8, or 9;
R^{x2-1} is independently C₁-C₄ alkyl;
X³ may be absent or -C(=O)-Ph-;
X⁴ may be absent or C₁-C₁₀ alkylene;
X⁵ may be absent, 5- to 6-membered heterocycloalkylene, or 5- to 6-membered heterocycloalkylene substituted by R^{x5-1}; in the 5- to 6-membered heterocycloalkylene and the 5- to 6-membered heterocycloalkylene substituted by R^{x5-1}, the heteroatom is one or more than one of O, S, and N, and the number of heteroatoms is 1, 2, or 3;
X⁶ may be -O-, -NH-, 5- to 6-membered heterocycloalkylene, -C(=O)-5- to 6-membered heterocycloalkylene, or 5- to 6-membered heterocycloalkylene substituted by R^{x6-1}, in the 5- to 6-membered heterocycloalkylene, the -C(=O)-5- to 6-membered heterocycloalkylene, and the 5- to 6-membered heterocycloalkylene substituted by R^{x1-1}, the heteroatom is one or more than one of O, S, and N, and the number of heteroatoms is 1, 2, or 3;
R² may be

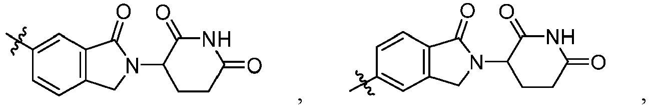

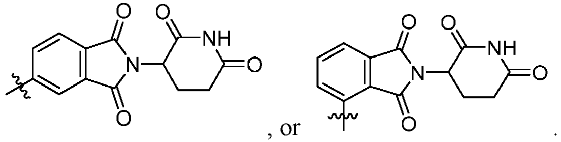

In a certain embodiment, R¹ may be hydroxyl or C₁-C₄ alkoxy;
X¹ may be -O-;
X² may be C₁-C₆ alkylene;
X³ may be absent; X⁴ may be absent; and X⁵ may be absent;
X⁶ may be 5- to 6-membered heterocycloalkylene, -C(=O)-5- to 6-membered heterocycloalkylene, or 5- to 6-membered heterocycloalkylene substituted by R^{x6-1}, in the 5- to 6-membered heterocycloalkylene, the -C(=O)-5- to 6-membered heterocycloalkylene, and the 5- to 6-membered heterocycloalkylene substituted by R^{x1-1}, the heteroatom is one or more than one of O, S, and N, and the number of heteroatoms is 1, 2, or 3;
R² may be

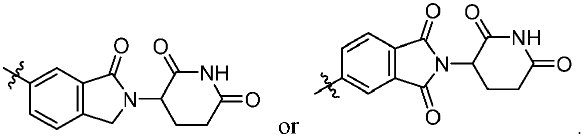

In a certain embodiment, R¹ is cyano, hydroxyl, or C₁-C₄ alkoxy;
X¹ is -O-, 5- to 6-membered heterocycloalkylene, or 5- to 6-membered heterocycloalkylene substituted by R^{x1-1}, in the 5- to 6-membered heterocycloalkylene and the 5- to 6-membered heterocycloalkylene substituted by R^{x1-1}, the heteroatom is one or more than one of O, S, and N, and the number of heteroatoms is 1, 2, or 3;
R^{x1-1} is independently C₁-C₄ alkyl;
X² is C₁-C₆ alkylene or C₂-C₁₅ heteroalkylene; in the C₂-C₁₅ heteroalkylene, the heteroatom is one or more than one of O, S, and N, and the number of heteroatoms is 1, 2, 3, 4, 5, 6, 7, 8, or 9;
X³ is absent, -C(=O)-Ph-, 5- to 6-membered heterocycloalkylene, 5- to 6-membered heteroarylene, 5- to 6-membered heterocycloalkylene substituted by R^{x3-1}, or 5- to 6-membered heteroarylene substituted by R^{x3-2}; in the 5- to 6-membered heterocycloalkylene and the 5- to 6-membered heterocycloalkylene substituted by R^{x3-1}, the heteroatom is one or more than one of O, S, and N, and the number of heteroatoms is 1, 2, or 3; in the 5- to 6-membered heteroarylene and the 5- to 6-membered heteroarylene substituted by R^{x3-2}, the heteroatom is one or more than one of O, S, and N, and the number of heteroatoms is 1, 2, or 3;
R^{x3-1} and R^{x3-2} are independently C₁-C₄ alkyl;
X⁴ is absent, C₁-C₁₀ alkylene, or C₂-C₁₅ heteroalkylene; in the C₂-C₁₅ heteroalkylene, the heteroatom is one or more than one of O, S, and N, and the number of heteroatoms is 1, 2, 3, 4, 5, 6, 7, 8, or 9;
X⁵ is absent, 5- to 6-membered heterocycloalkylene, or 5- to 6-membered heterocycloalkylene substituted by R^{x5-1}; in the 5- to 6-membered heterocycloalkylene and the 5- to 6-membered heterocycloalkylene substituted by R^{x5-1}, the heteroatom is one or more than one of O, S, and N, and the number of heteroatoms is 1, 2, or 3;
R^{x5-1} is independently C₁-C₄ alkyl;
X⁶ is absent, -O-, -NH-, -C(=O)-NH-, 5- to 6-membered heterocycloalkylene, -C(=O)-5- to 6-membered heterocycloalkylene, or 5- to 6-membered heterocycloalkylene substituted by R^{x6-1}, in the 5- to 6-membered heterocycloalkylene, the -C(=O)-5- to 6-membered heterocycloalkylene, and the 5- to 6-membered heterocycloalkylene substituted by R^{x1-1}, the heteroatom is one or more than one of O, S, and N, and the number of heteroatoms is 1, 2, or 3;
R^{x6-1} is independently C₁-C₄ alkyl;
R² is

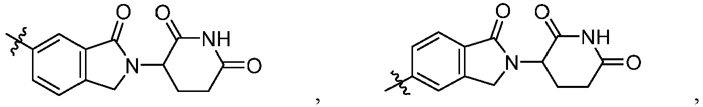

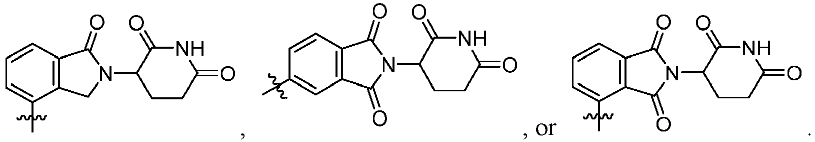

In a certain embodiment, in the naphthalene ring-containing compound of formula **II**,
R¹ is hydrogen or hydroxyl;
X¹ is -O-;
X² is -C(=O)- or C₁-C₆ alkylene;
X³ is absent;
X⁴ is absent;
X⁵ is absent;
X⁶ is 5- to 6-membered heterocycloalkylene; in the 5- to 6-membered heterocycloalkylene, the heteroatom is N, and the number of heteroatoms is 1, 2, or 3;
R² is 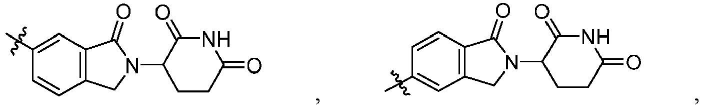 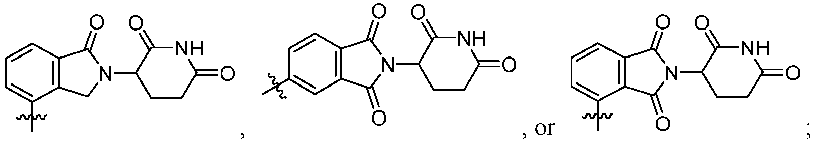
R³ is 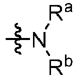 or 3- to 6-membered heterocycloalkyl, R^{a} and R^{b} are independently hydrogen or C₁-C₄ alkyl; the 3- to 6-membered heterocycloalkyl is linked to carbonyl through N; in the 3- to 6-membered heterocycloalkyl, the heteroatom is N, and the number of heteroatoms is 1 or 2.

In a certain embodiment, -X¹-X²-X³-X⁴-X⁵-X⁶- may be 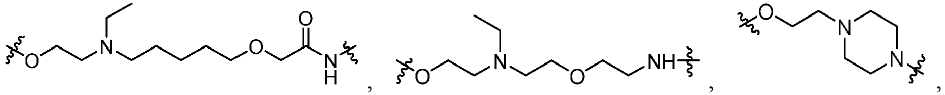 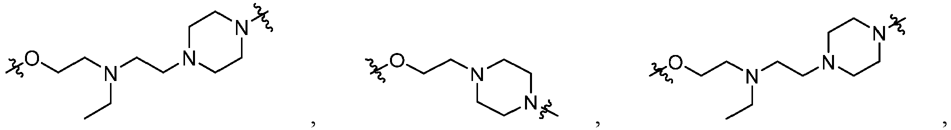 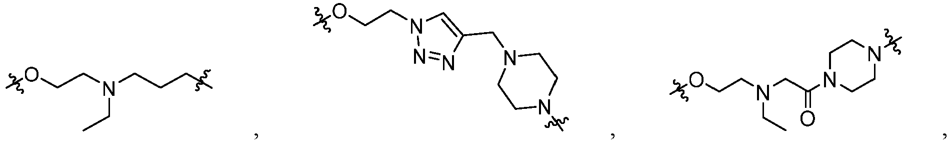 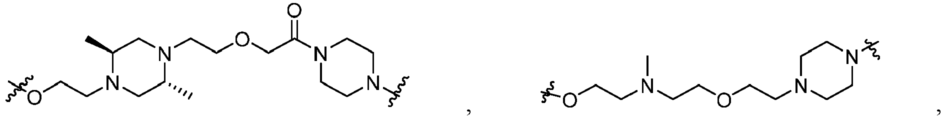 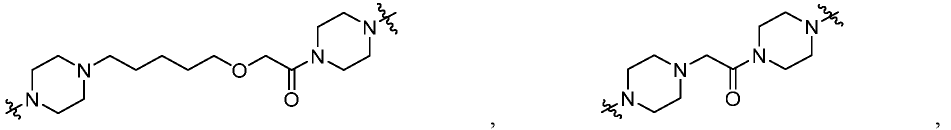 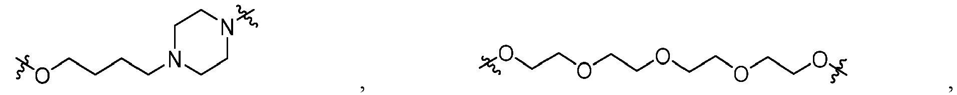 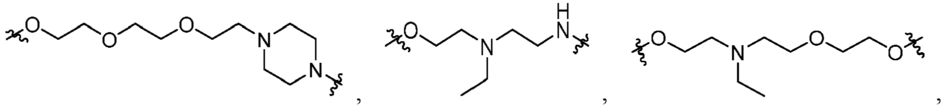 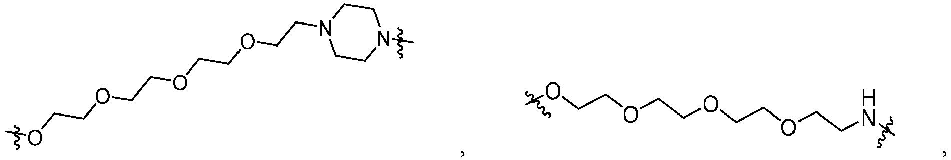 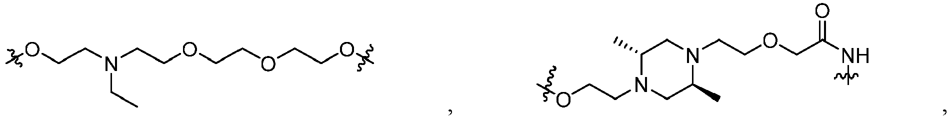 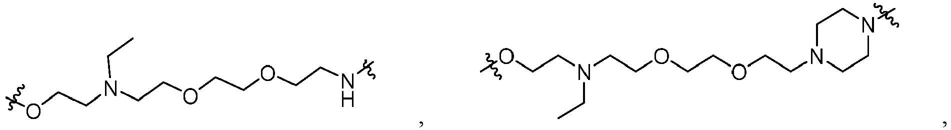 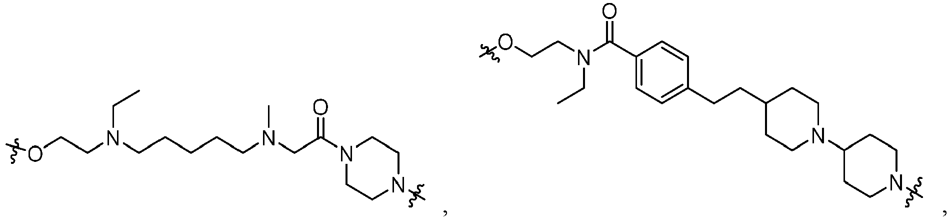 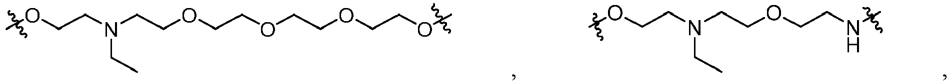 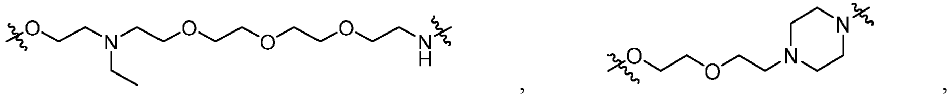 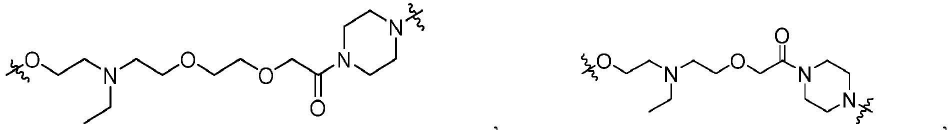 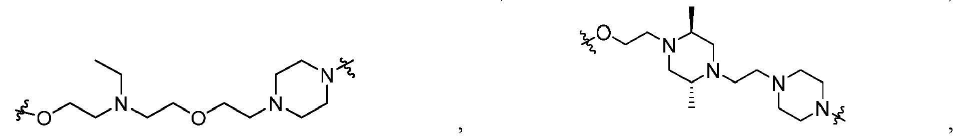 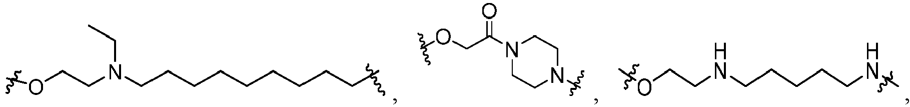 or

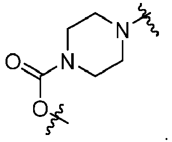

In a certain embodiment, in the naphthalene ring-containing compound of formula **III**, the pharmaceutically acceptable salt thereof, the solvate thereof, or the solvate of the pharmaceutically acceptable salt thereof, the naphthalene ring-containing compound of formula **III** may be as represented by any one of the following structures:

| Compound No. | Structural formula |
|---|---|
| **D1** | 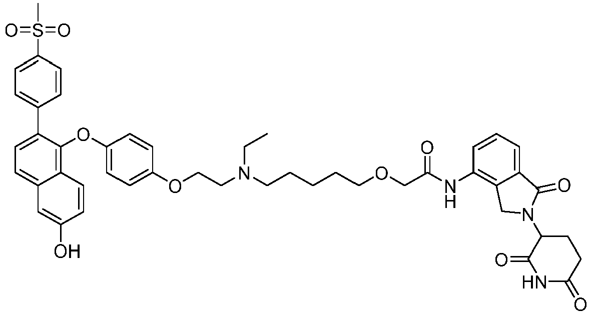 |
| **D2** | 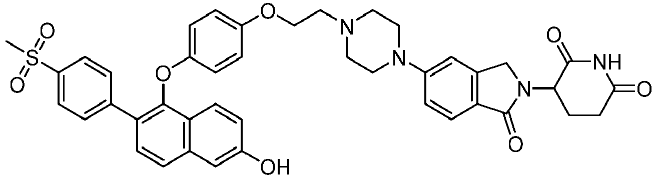 |
| **D57** | 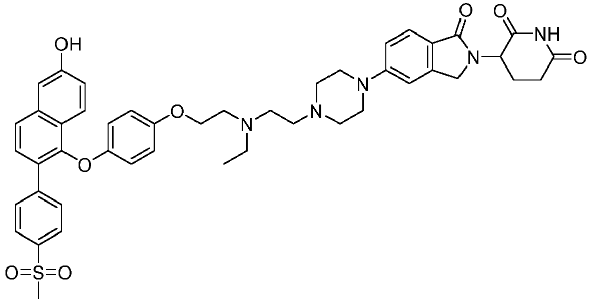 |
| **D3** | 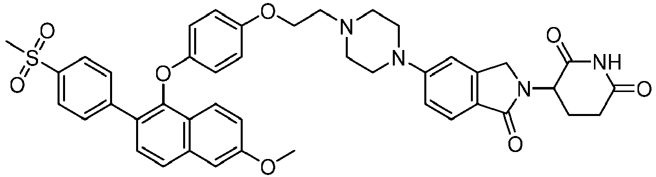 |
| **D4** | 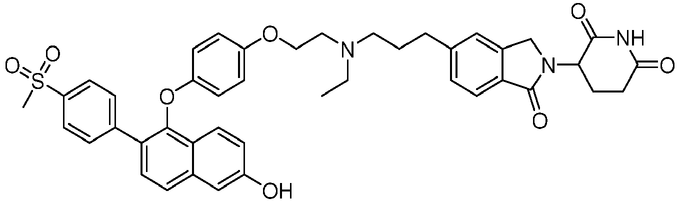 |
| **D5** | 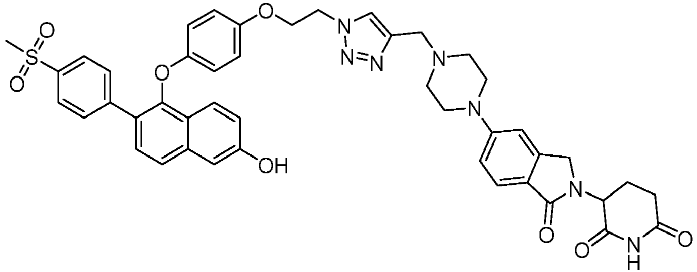 |
| **D58** | 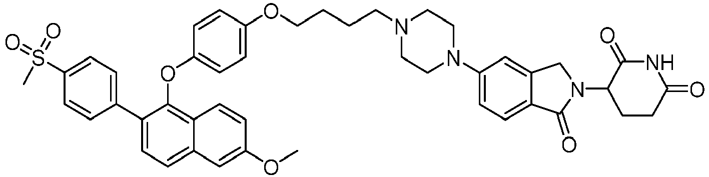 |
| **D6** | 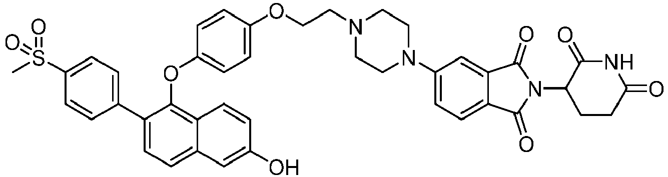 |
| **D7** | 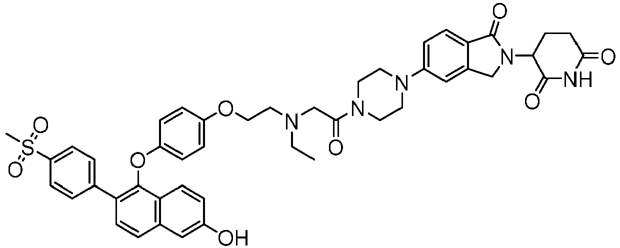 |
| **D8** | 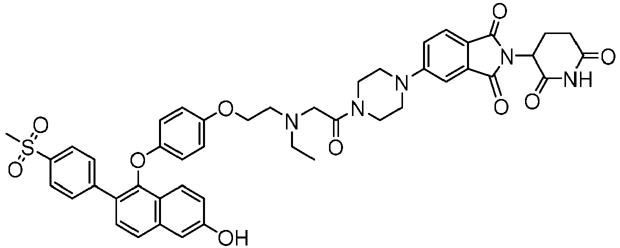 |
| **D9** | 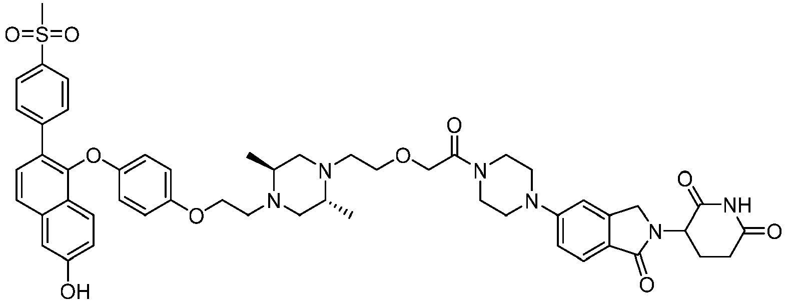 |
| **D10** | 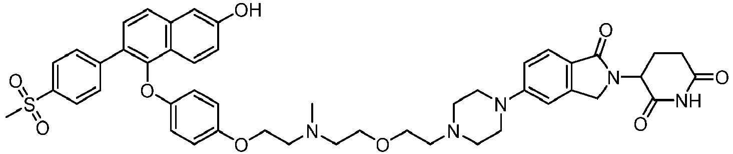 |
| **D11** | 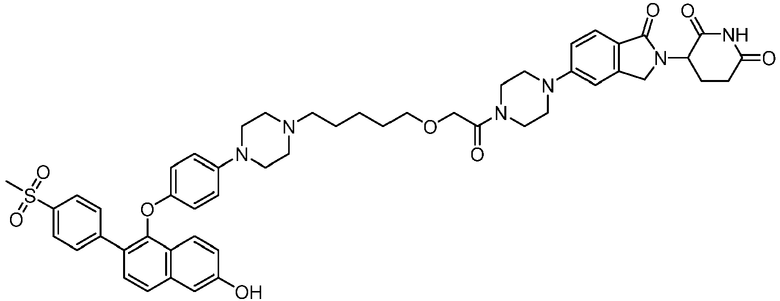 |
| **D12** | 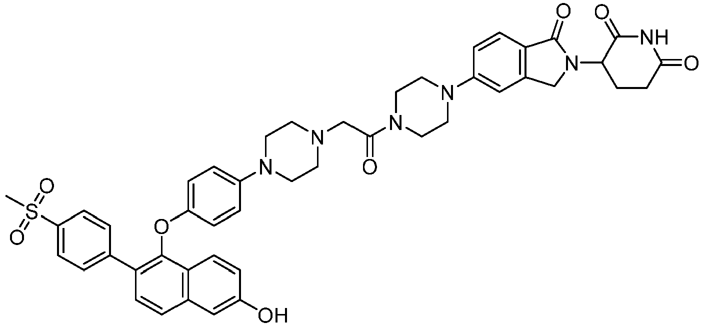 |
| **D13** | 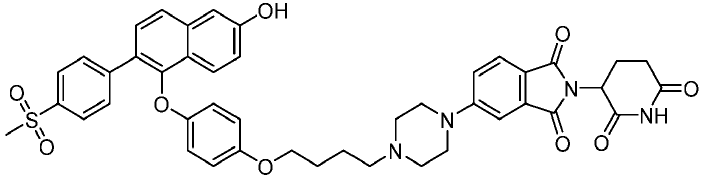 |
| **D59** | 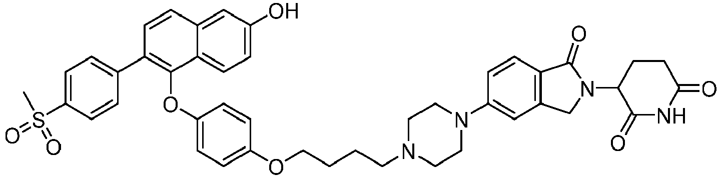 |
| **D14** | 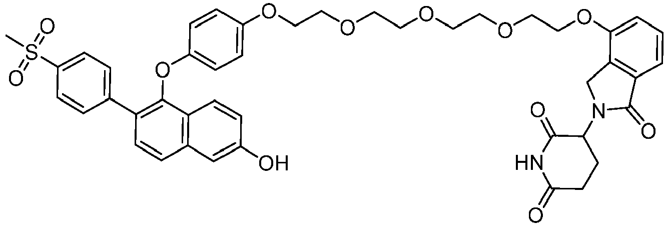 |
| **D15** | 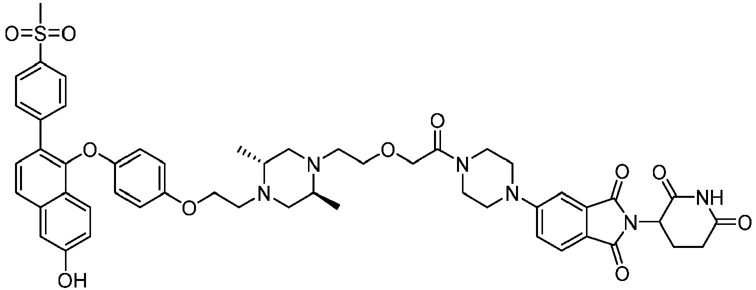 |
| **D16** | 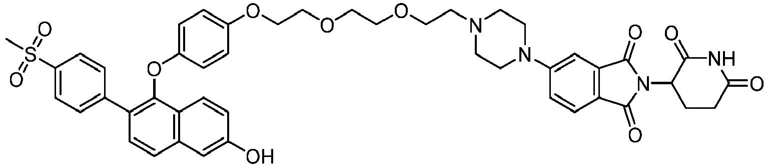 |
| **D17** | 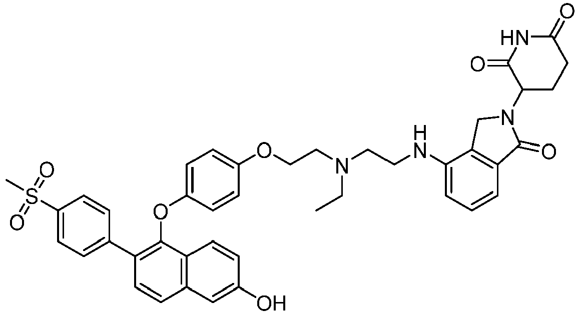 |
| **D18** | 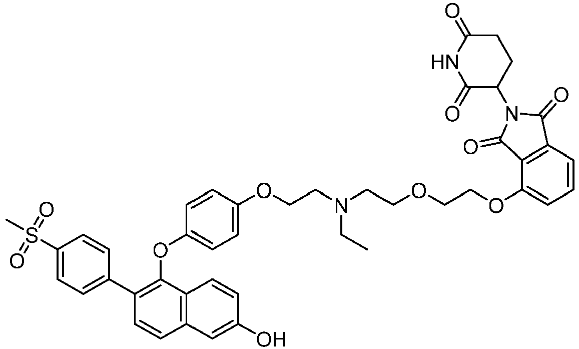 |
| **D19** | 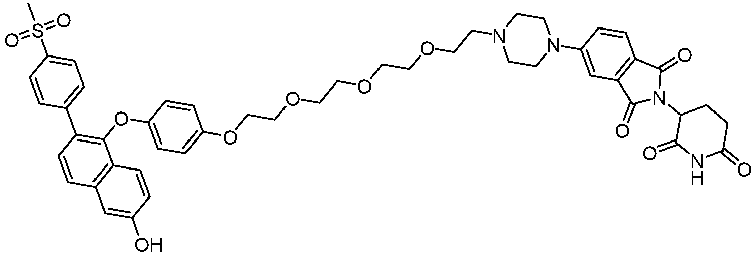 |
| **D20** | 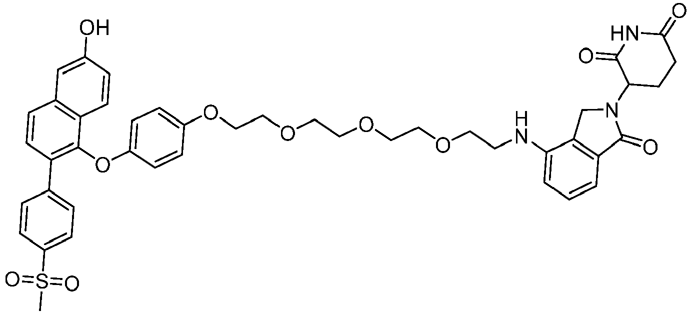 |
| **D21** | 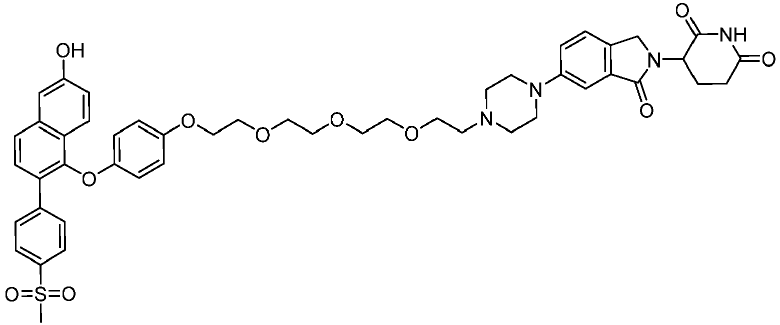 |
| **D22** | 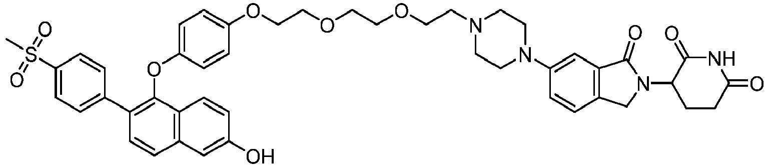 |
| **D13** | 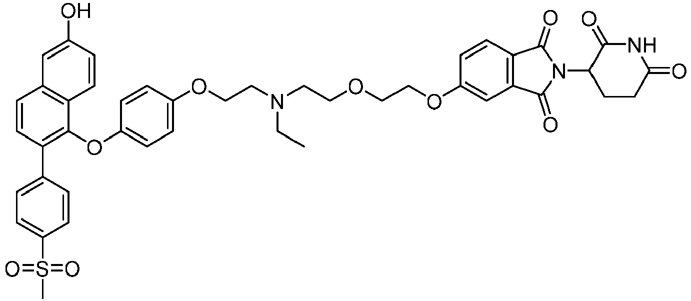 |
| **D24** | 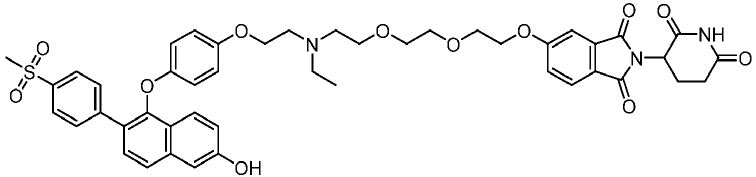 |
| **D25** | 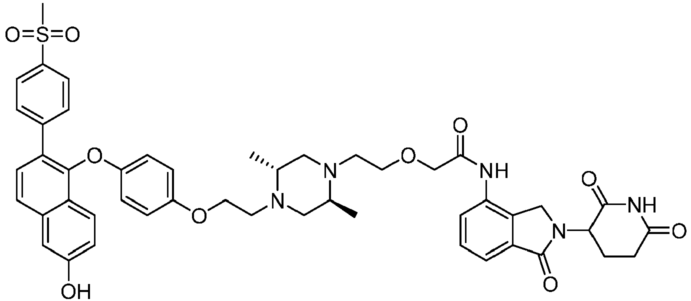 |
| **D26** | 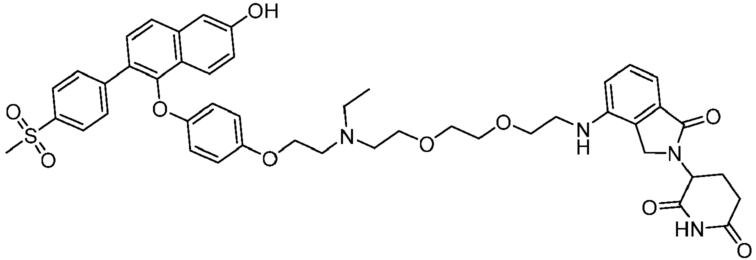 |
| **D27** | 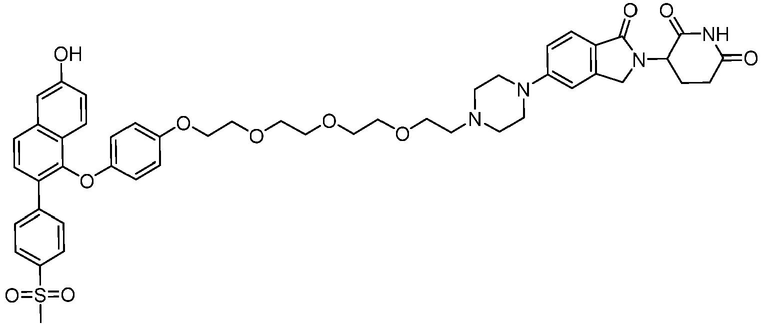 |
| **D28** | 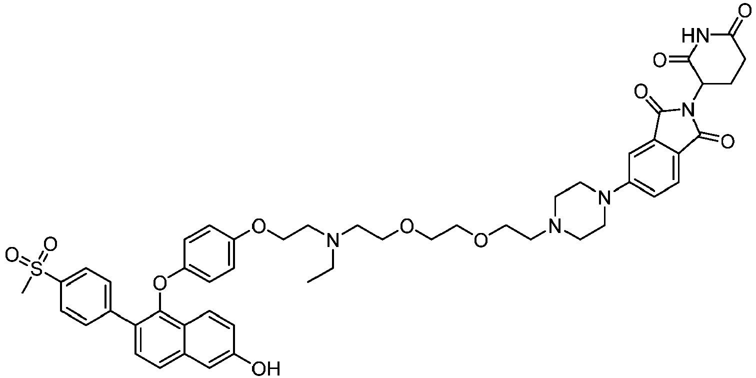 |
| **D29** | 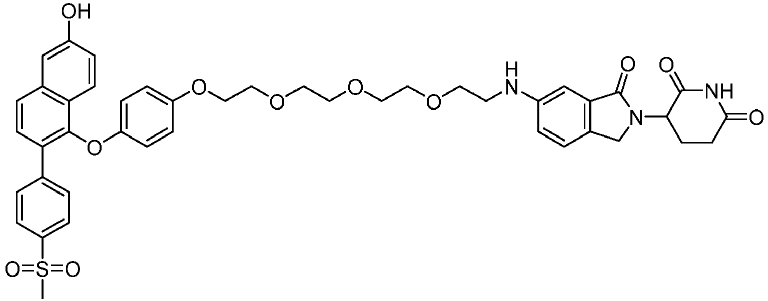 |
| **D30** | 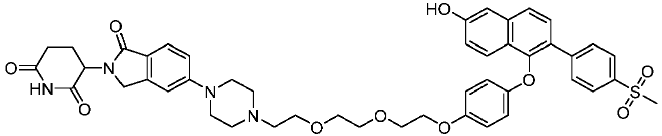 |
| **D60** | 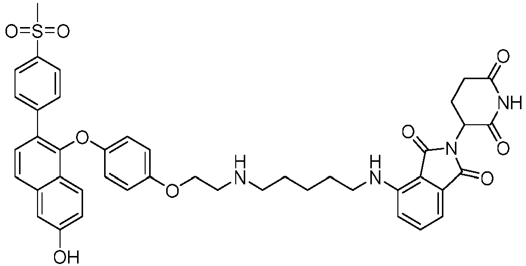 |
| **D31** | 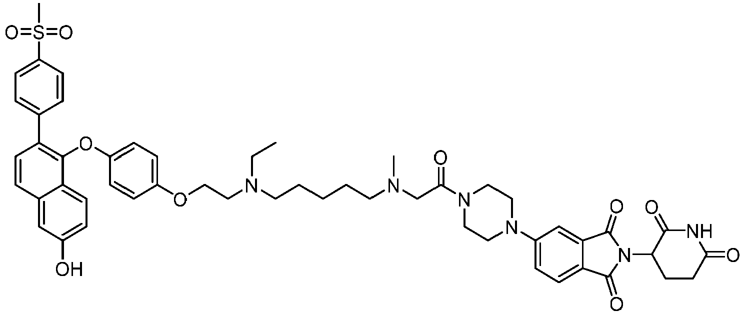 |
| **D32** | 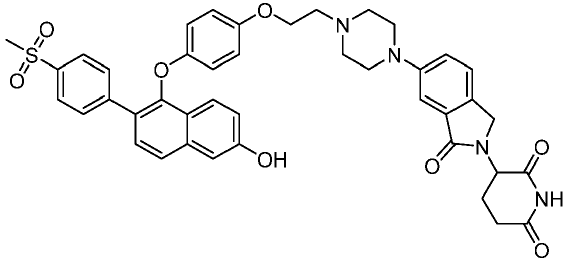 |
| **D33** | 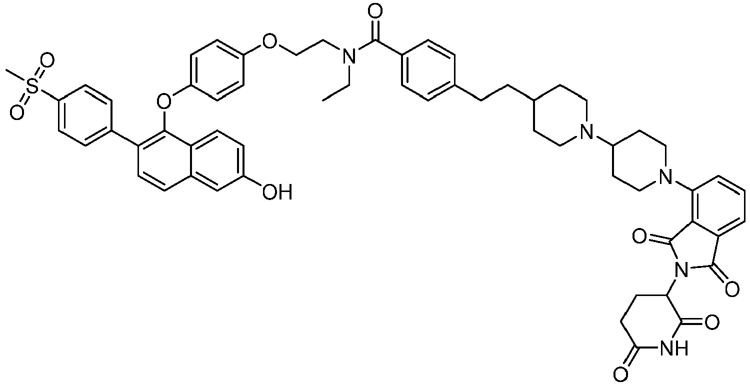 |
| **D34** | 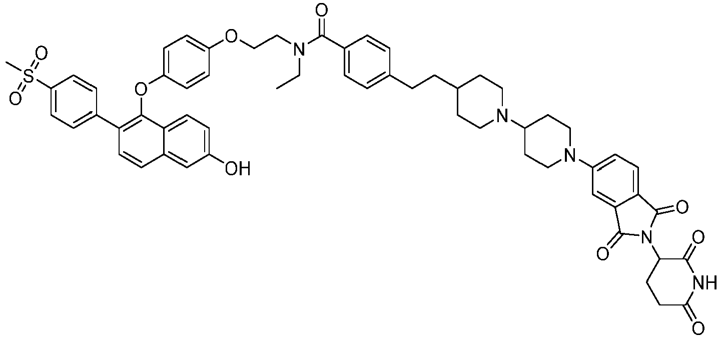 |
| **D35** | 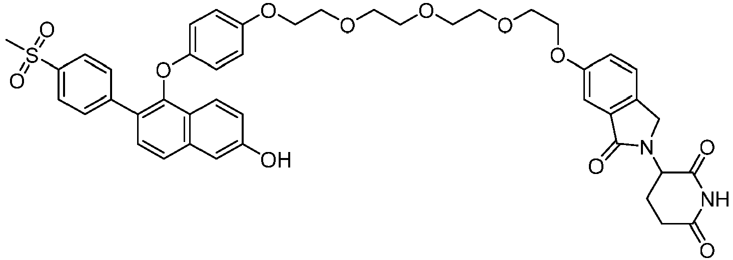 |
| **D36** | 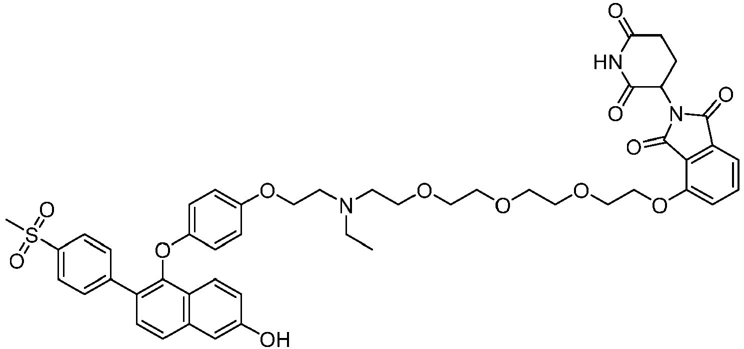 |
| **D37** | 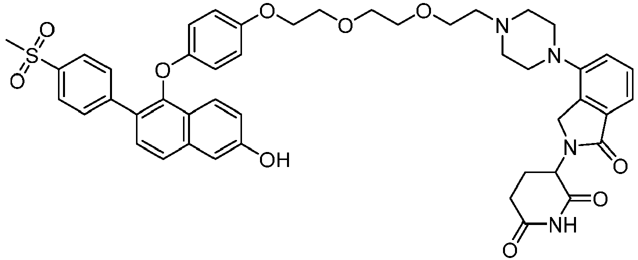 |
| **D38** | 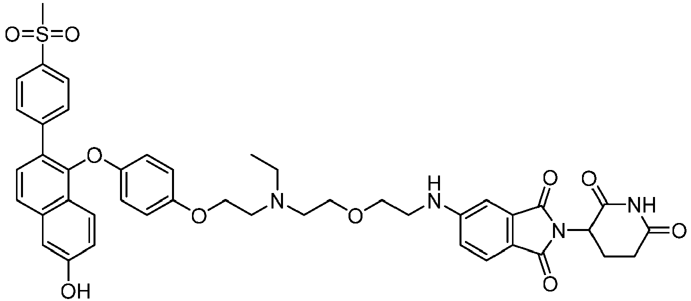 |
| **D39** | 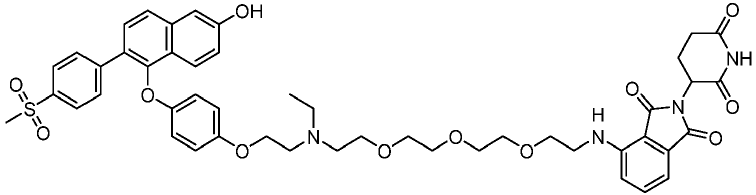 |
| **D40** | 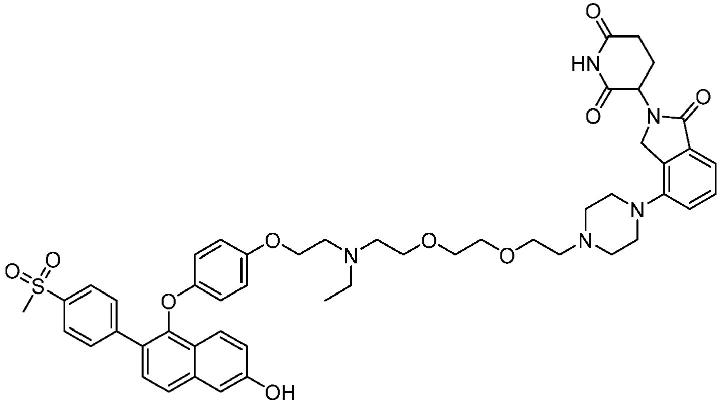 |
| **D41** | 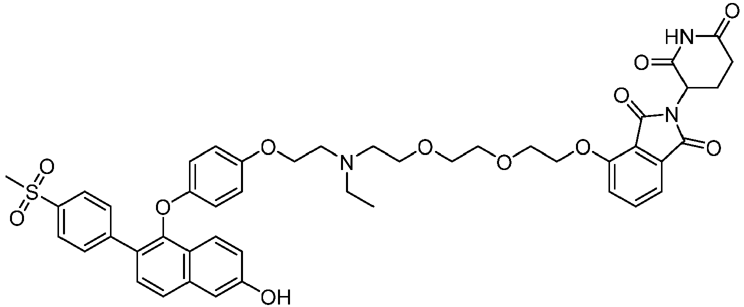 |
| **D42** | 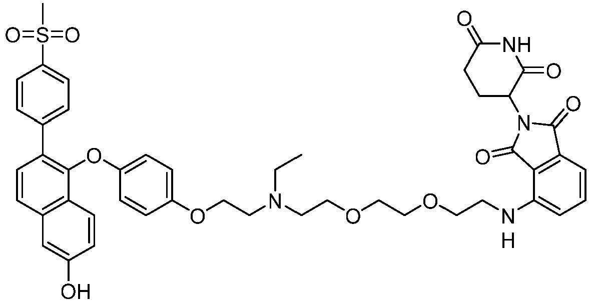 |
| **D43** | 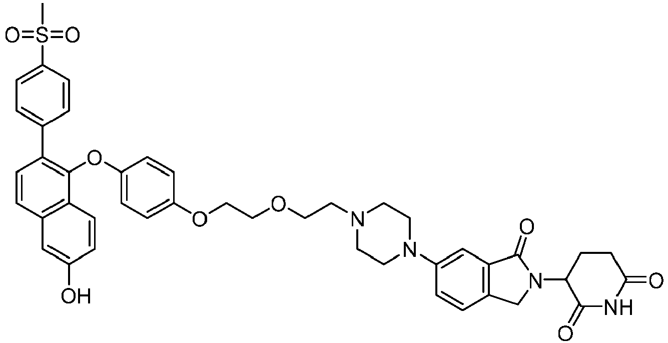 |
| **D44** | 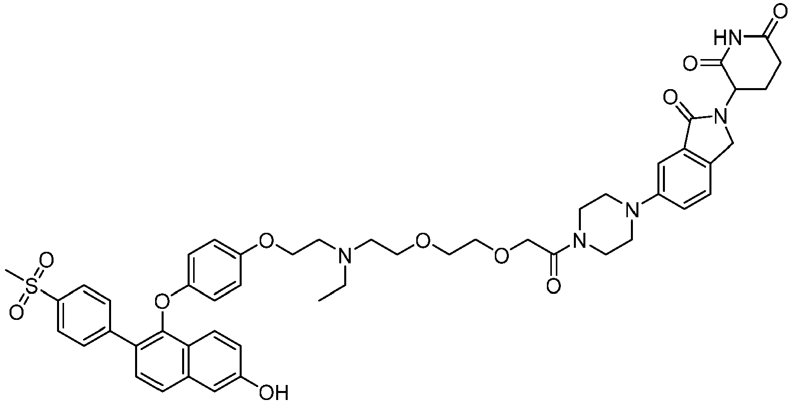 |
| **D45** | 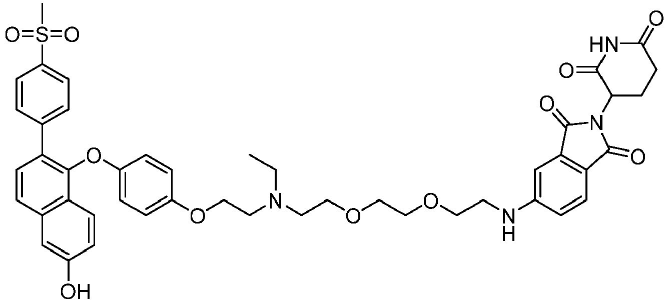 |
| **D46** | 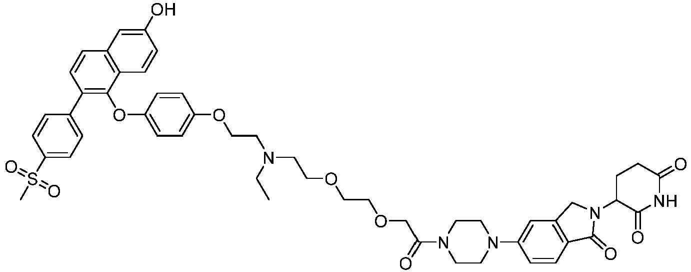 |
| **D47** | 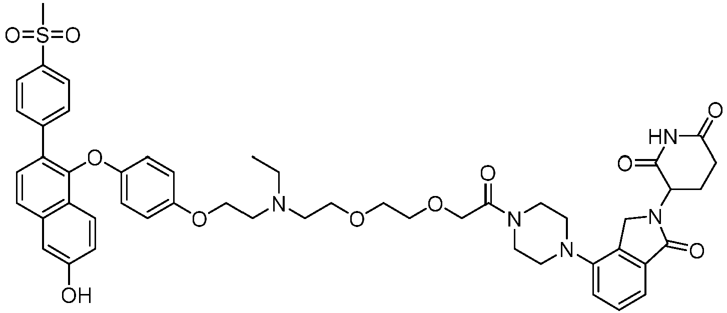 |
| **D48** | 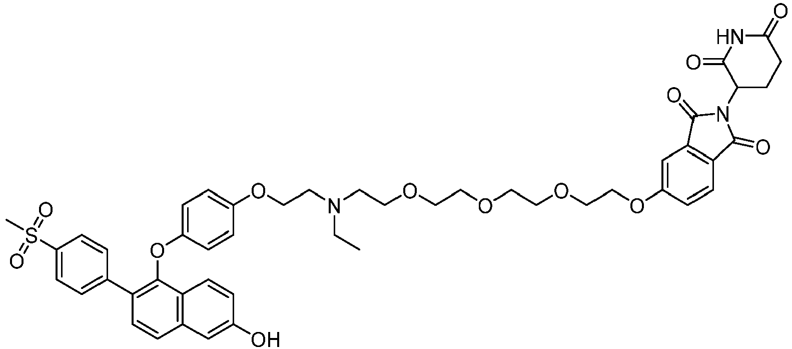 |
| **D49** | 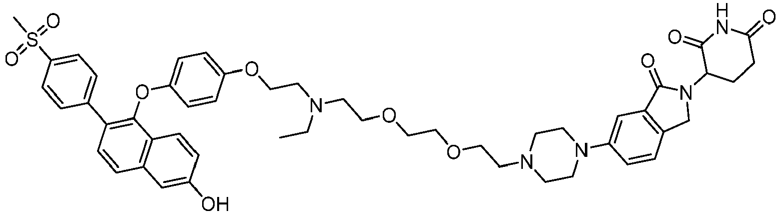 |
| **D50** | 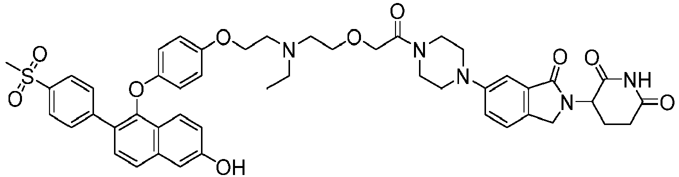 |
| **D51** | 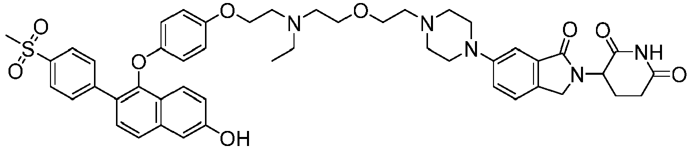 |
| **D52** | 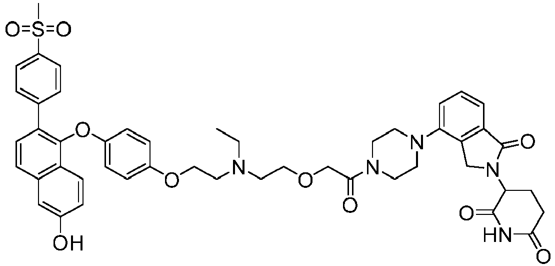 |
| **D61** | 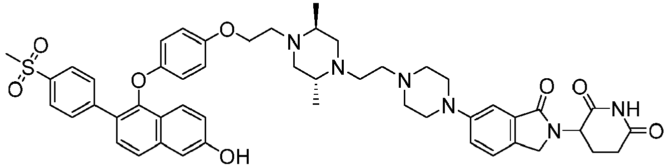 |
| **D53** | 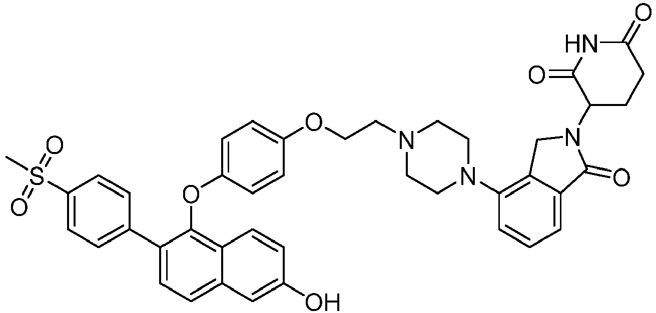 |
| **D62** | 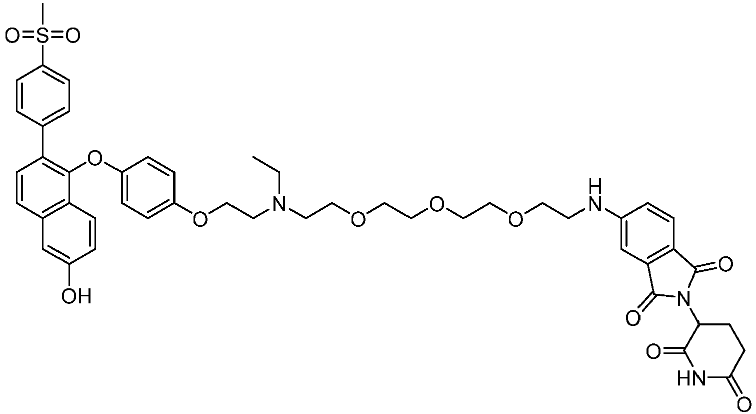 |
| **D63** | 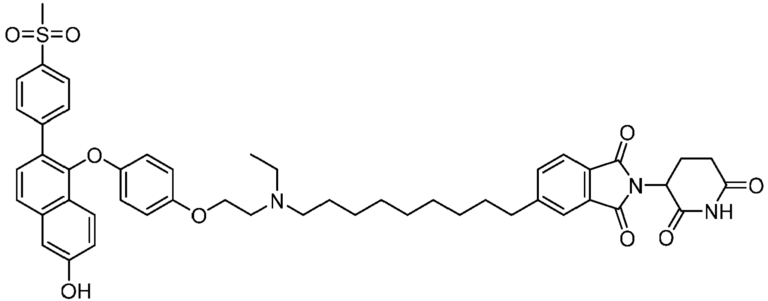 |
| **D54** | 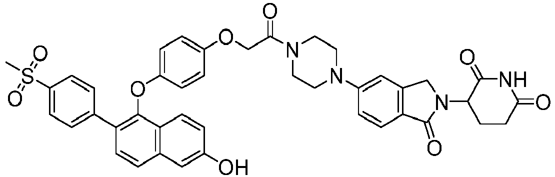 |
| **D55** | 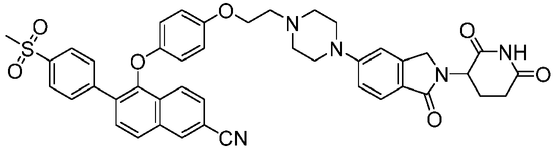 |
| **D56** | 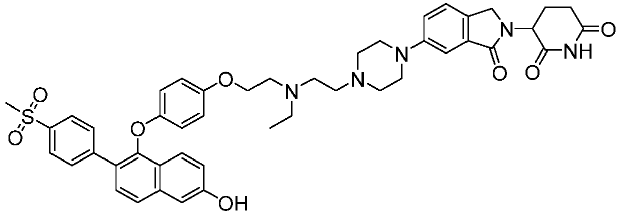 |
| **D64** | 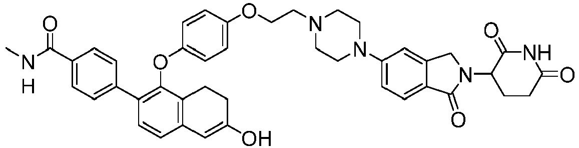 |
| **D65** | 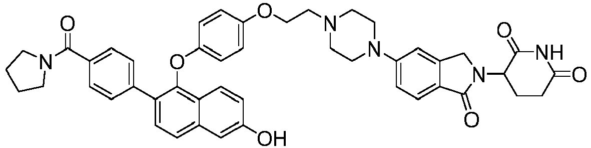 |
| **D66** | 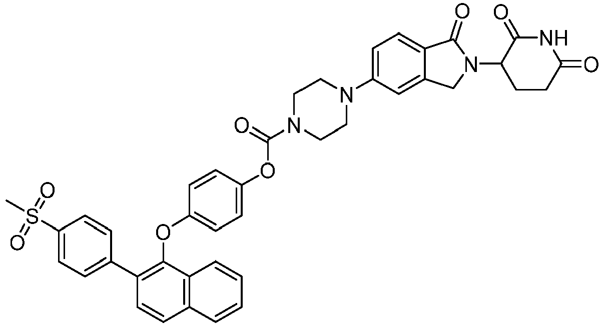 |
| **D67** | 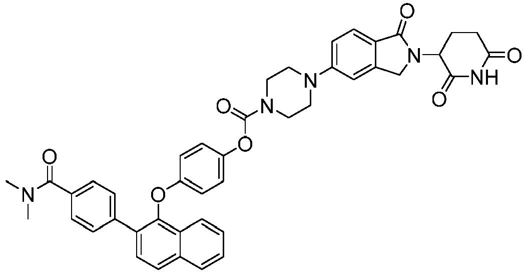 |
| **D68** | 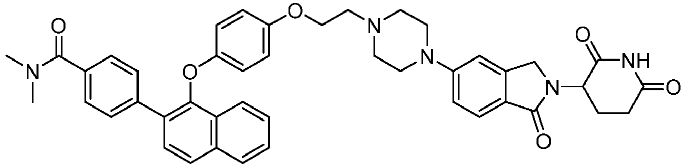 |
| **D69** | 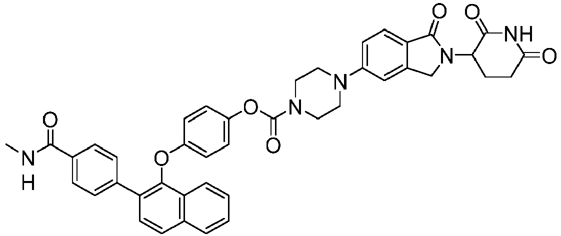 |

.

In a certain embodiment, the pharmaceutically acceptable salt of the naphthalene ring-containing compound of formula **III** may be

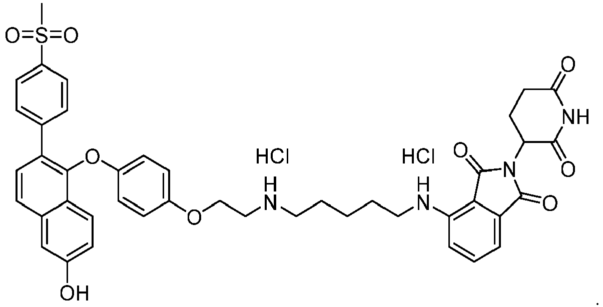

The present disclosure provides a naphthalene ring-containing compound of formula **IV** or a salt thereof; 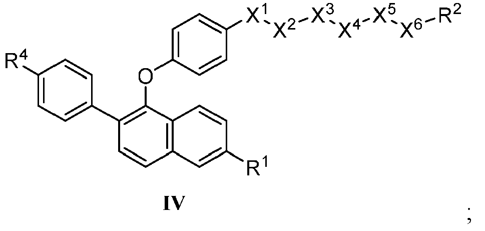
wherein R⁴ is -SO₂-C₁-C₄ alkyl or 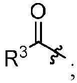 R³ is 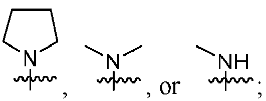
X¹ is -O-;
X², X³, X⁴, X⁵, and X⁶ are as previously described; X², X³, X⁴, X⁵, and X⁶ are not simultaneously absent;
R² is hydrogen, 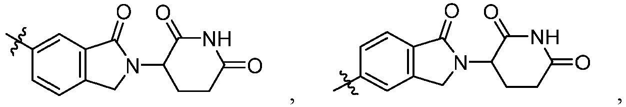 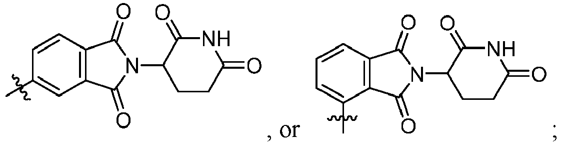
R¹ is hydrogen, benzyl, hydroxyl, or C₁-C₄ alkoxy.

The present disclosure also provides a compound or a salt thereof, which has any one of the following structures: 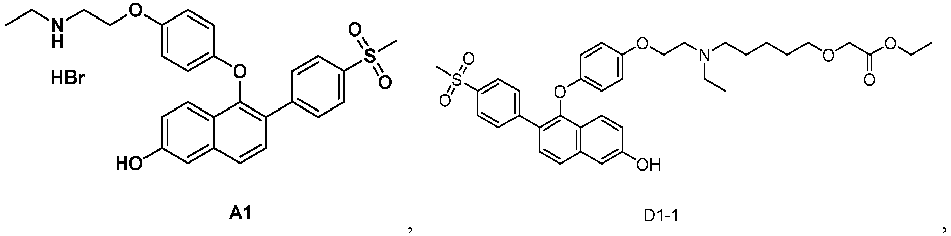 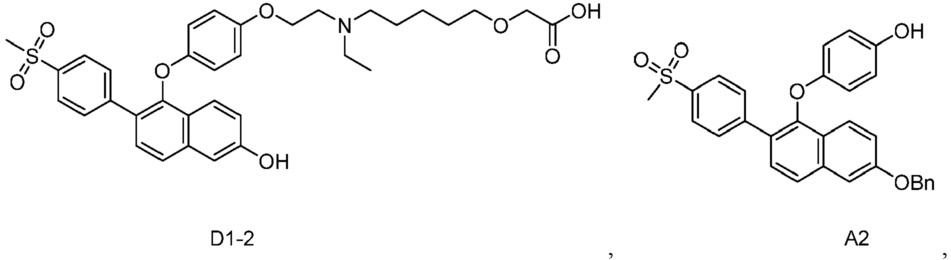 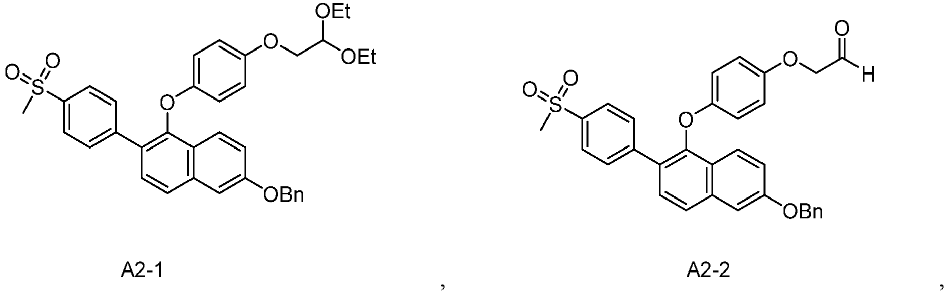 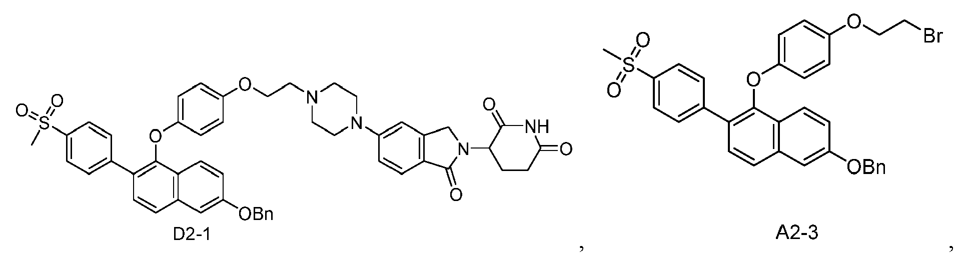 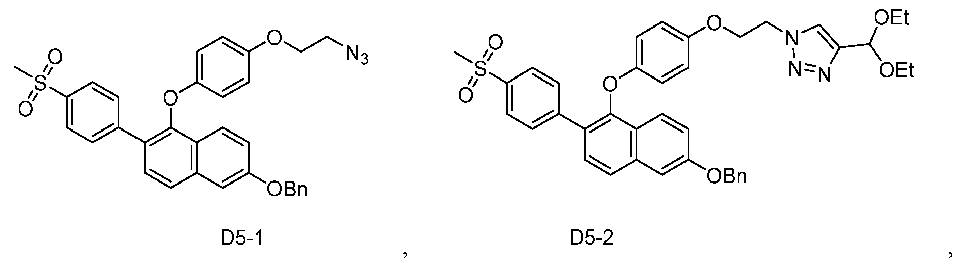 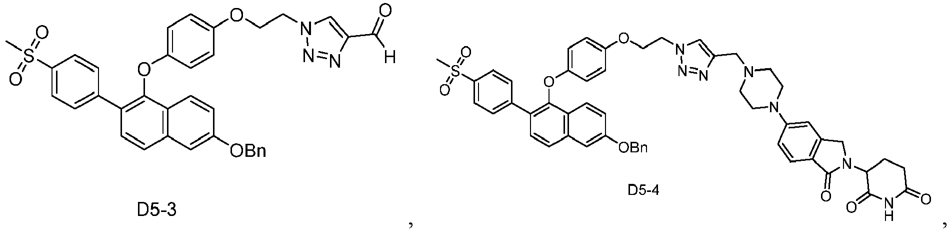 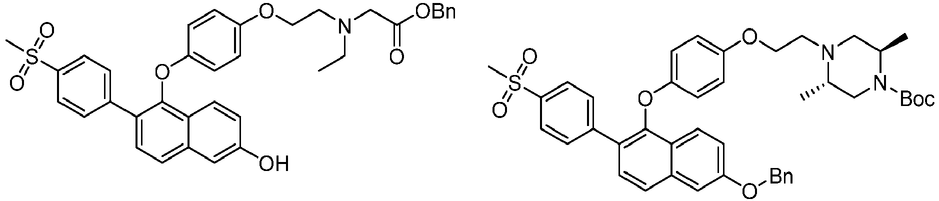 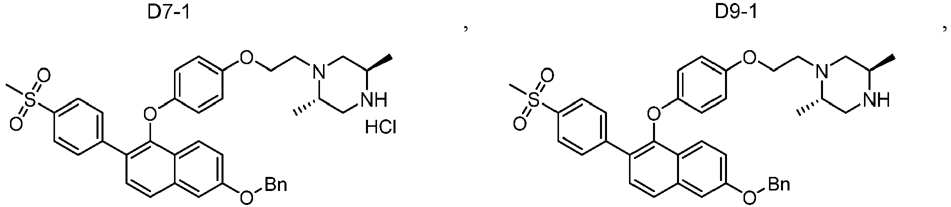 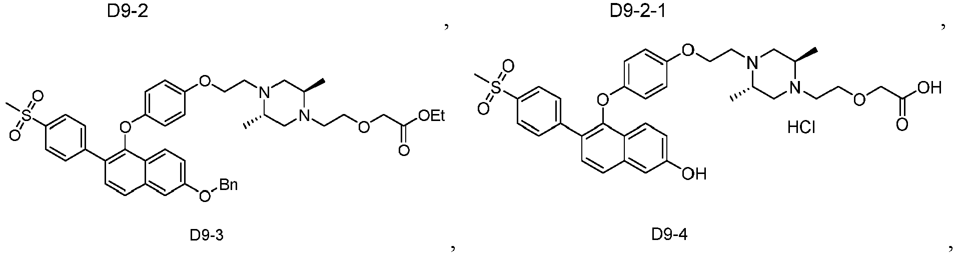 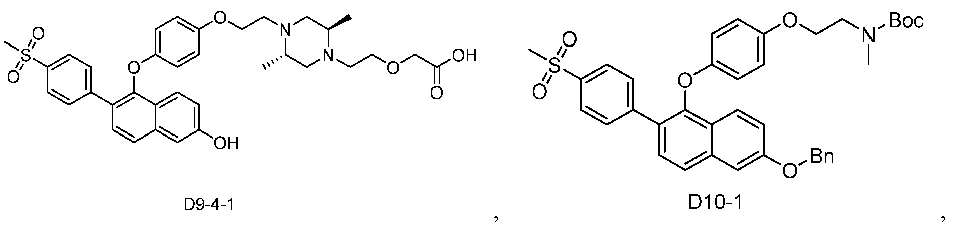 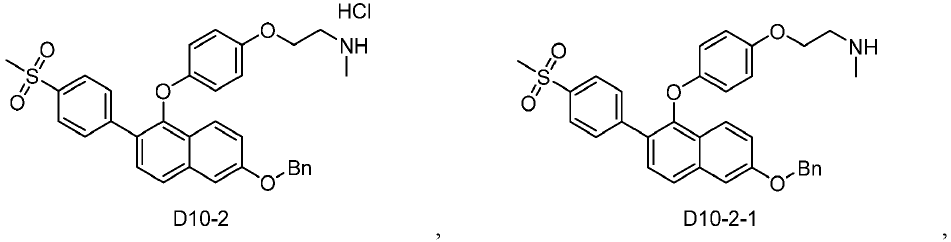 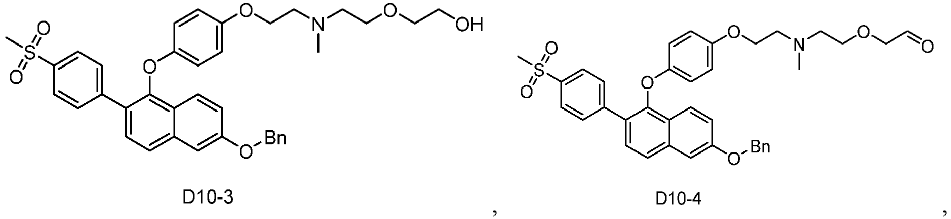 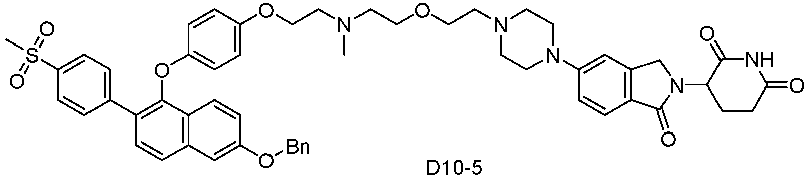 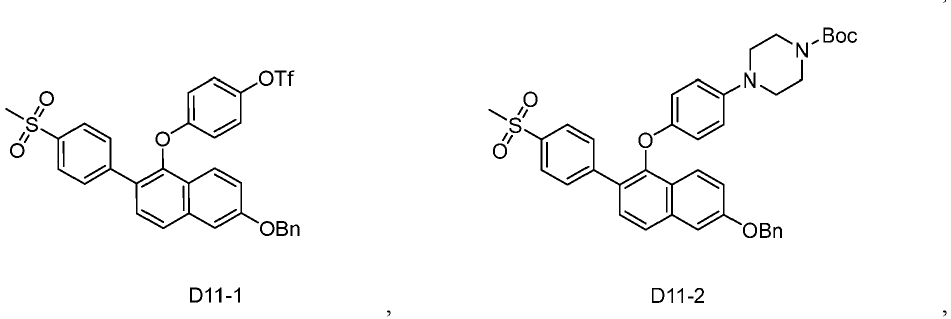 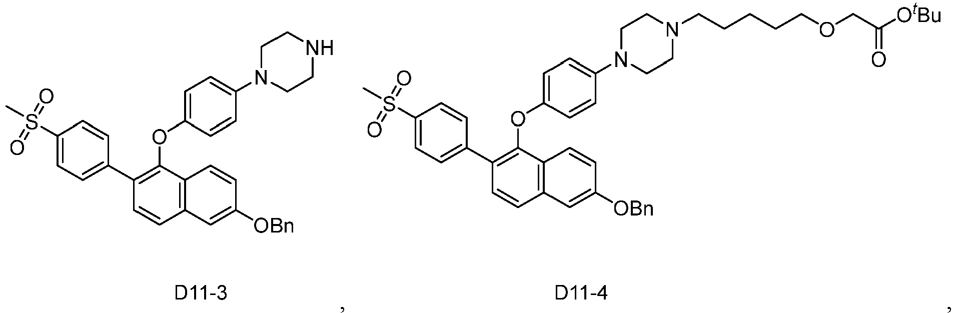 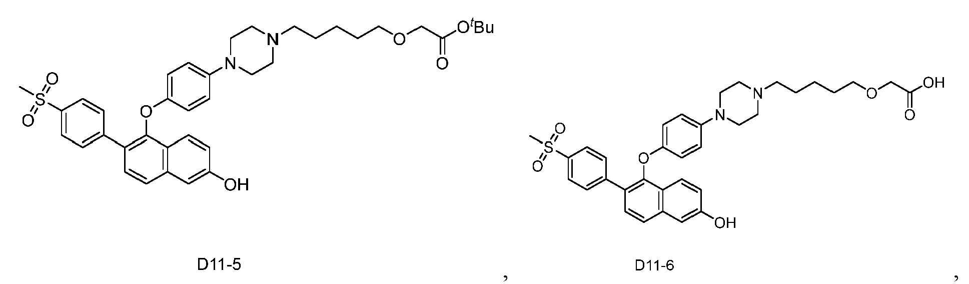 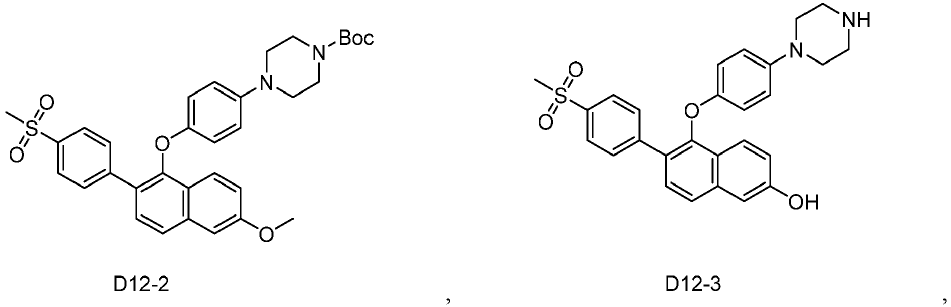 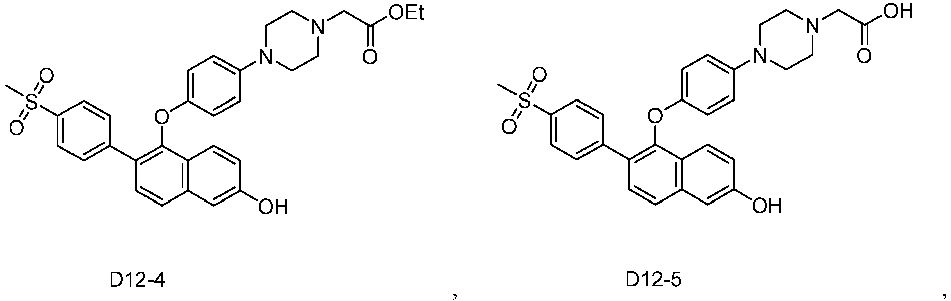 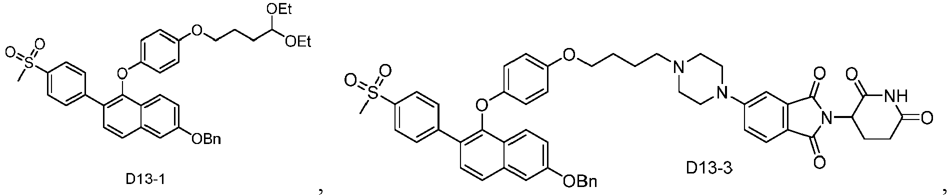 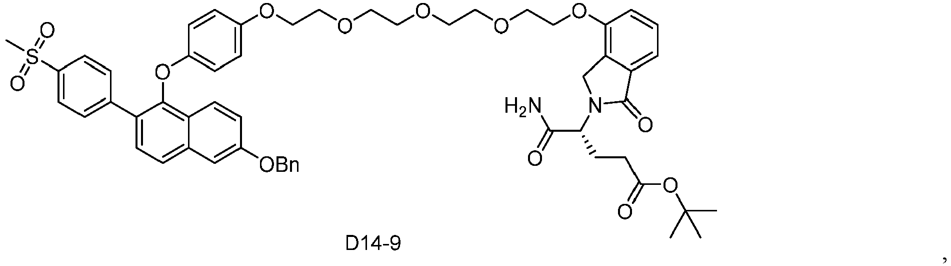 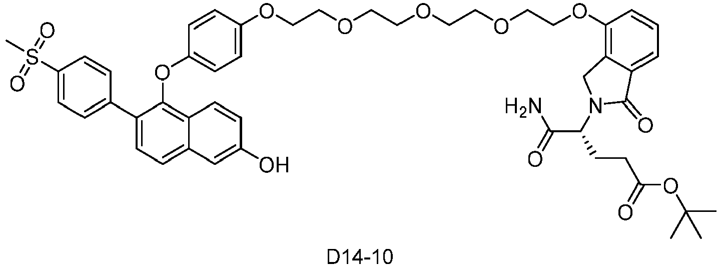 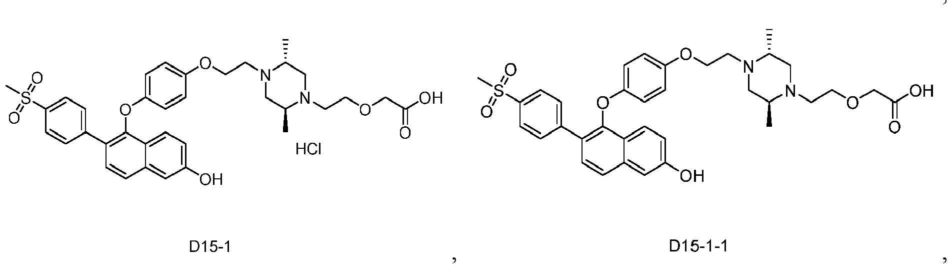 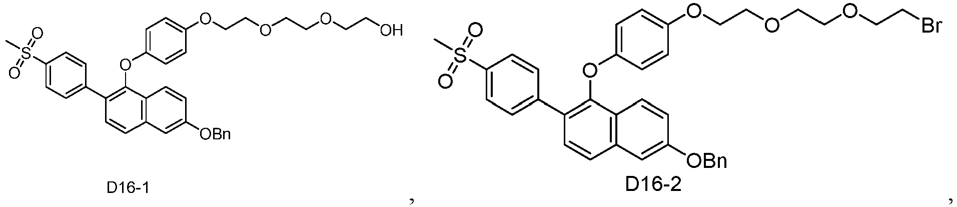 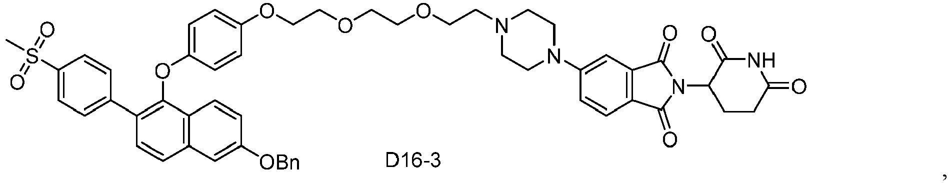 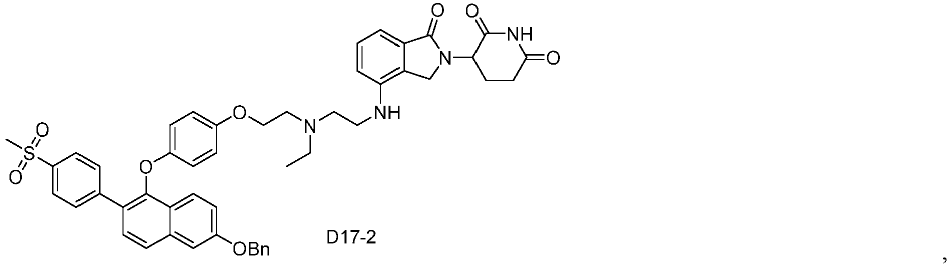 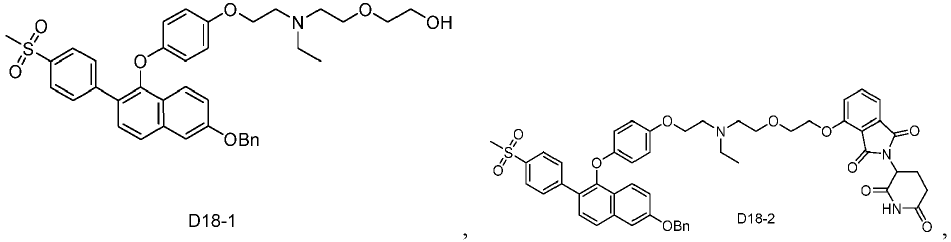 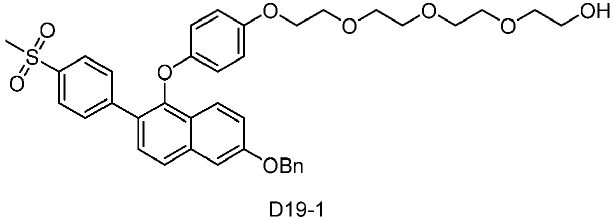 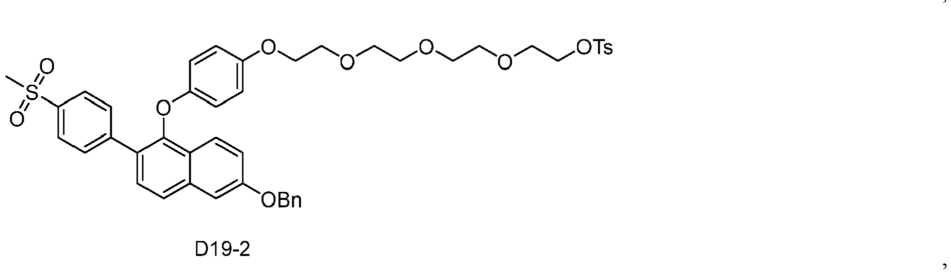 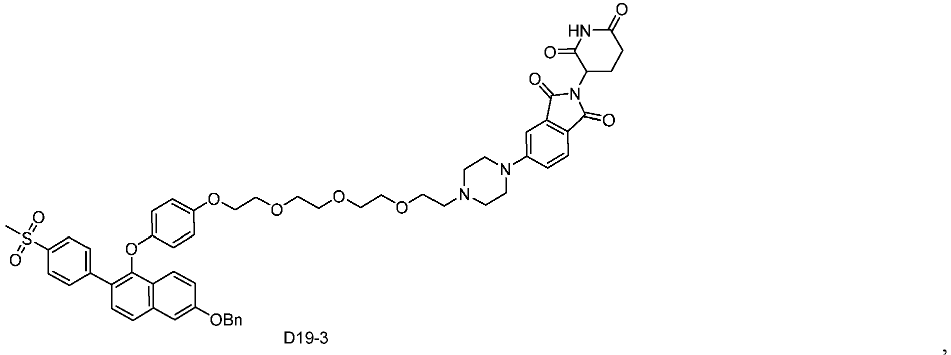 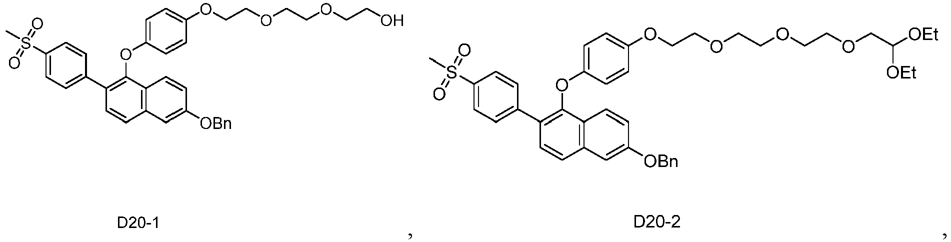 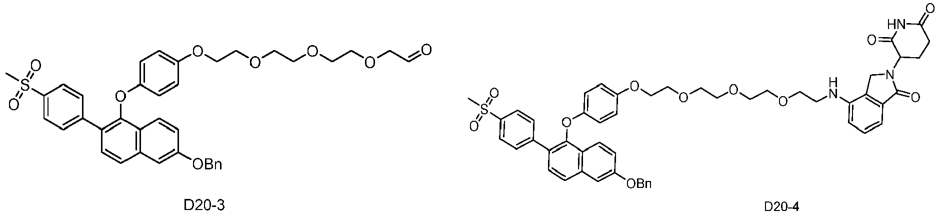 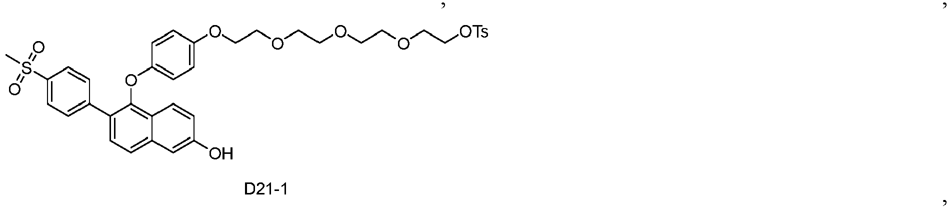 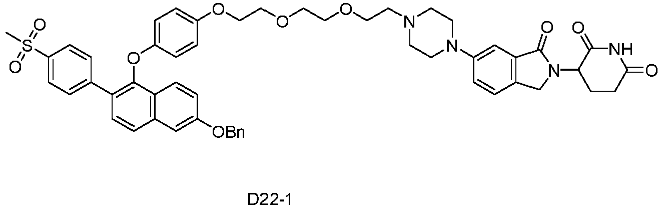 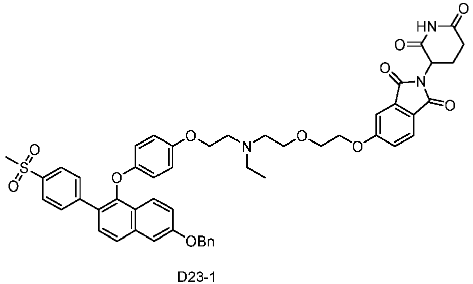 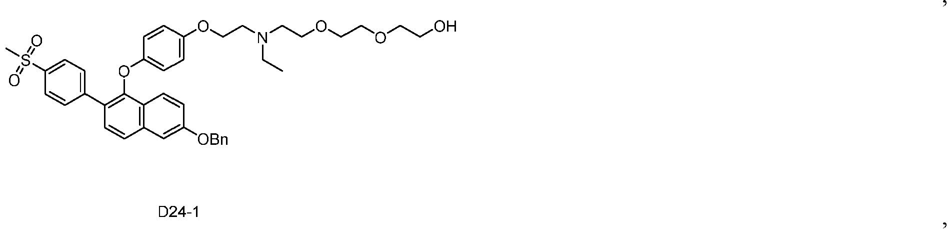 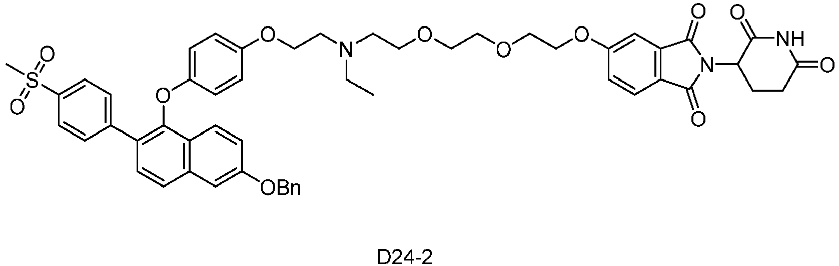 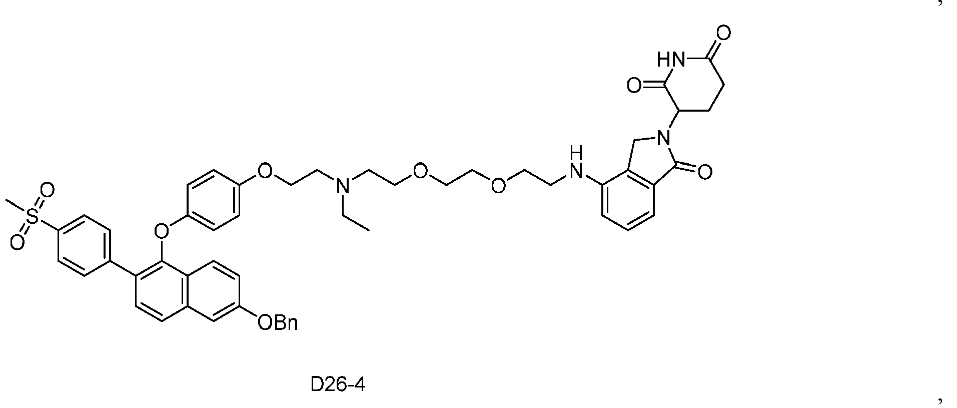 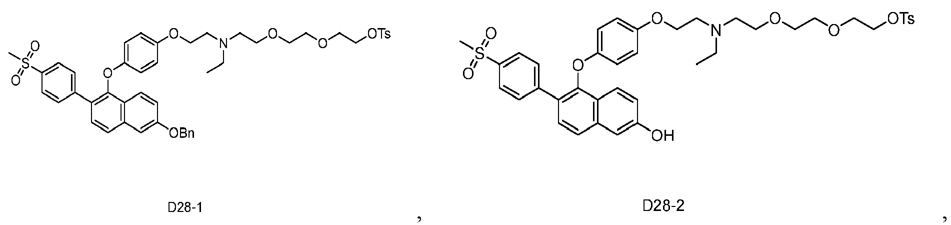 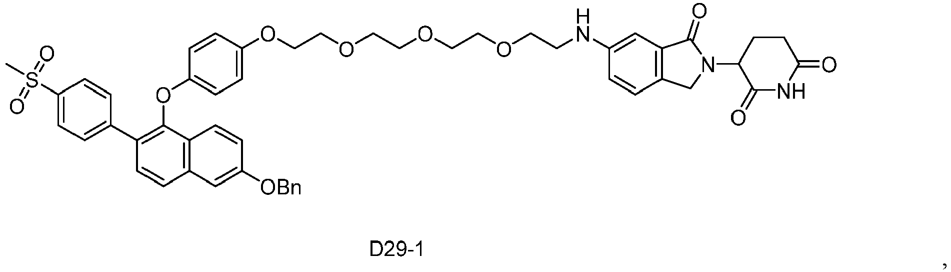 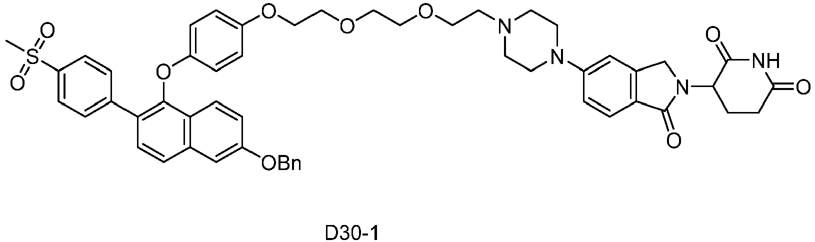 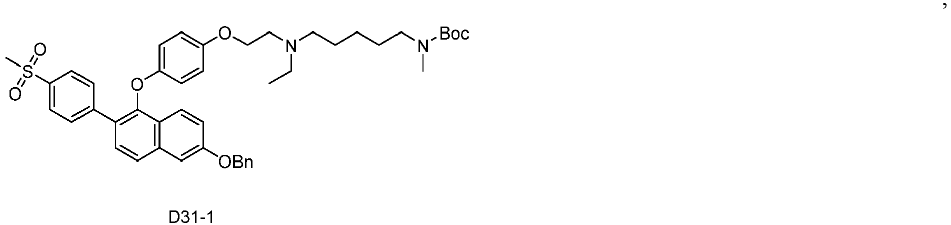 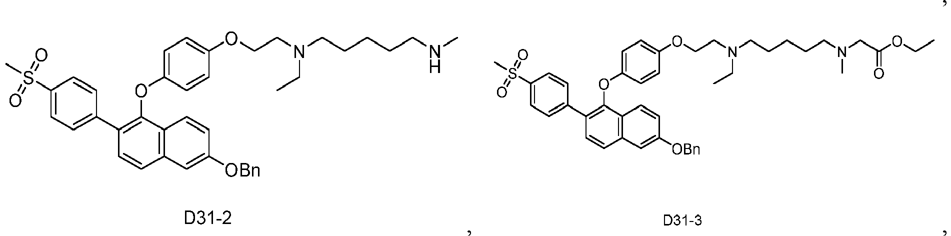 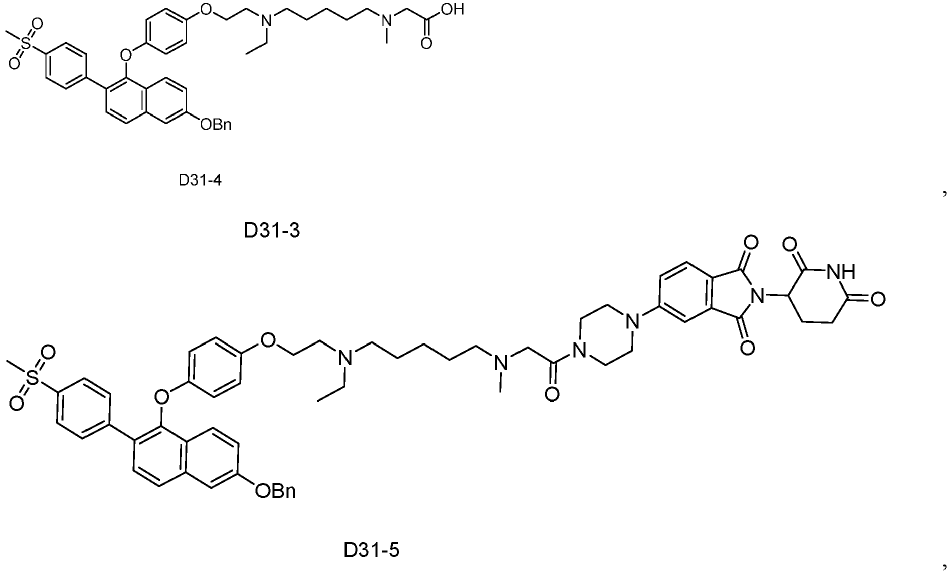 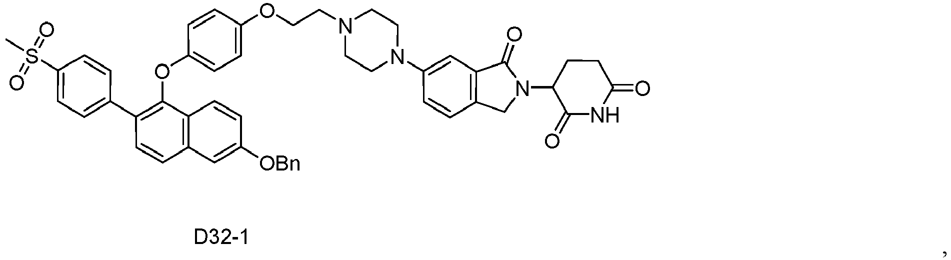 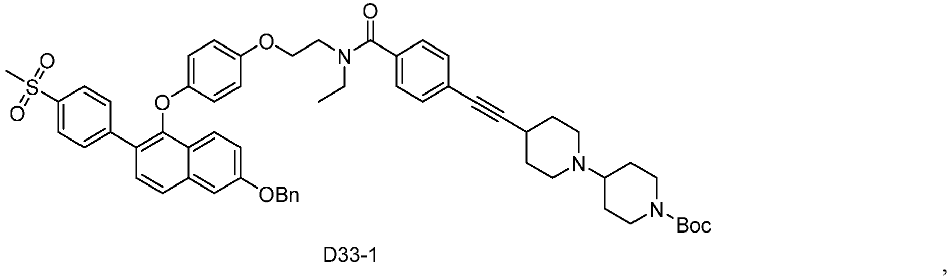 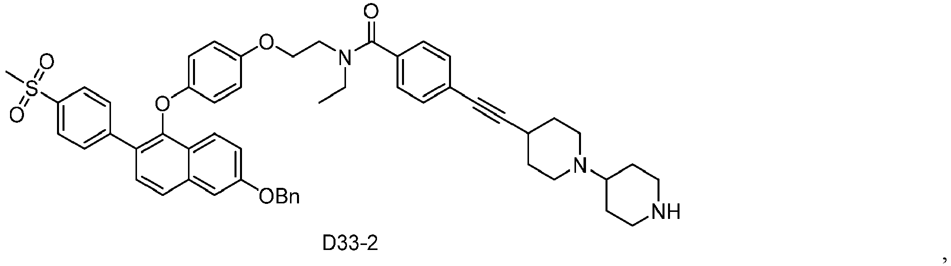 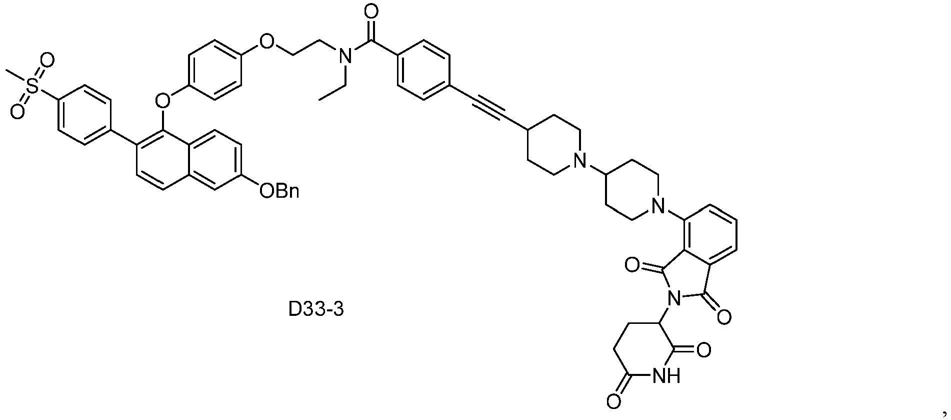 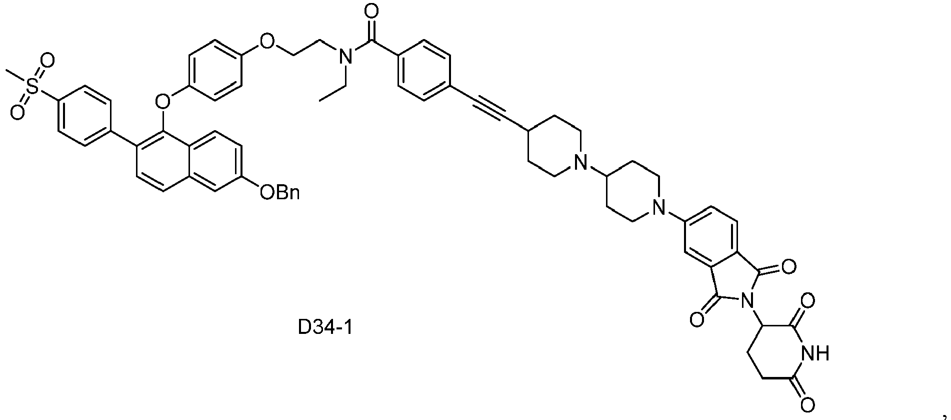 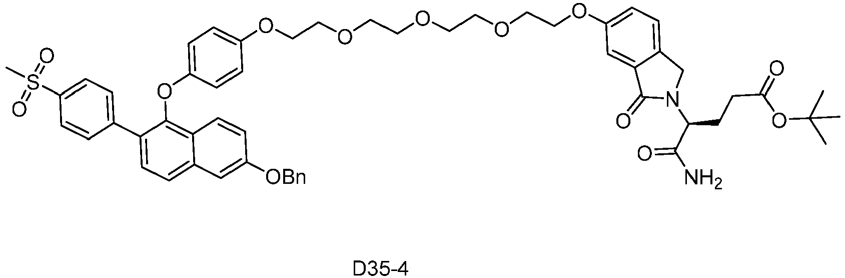 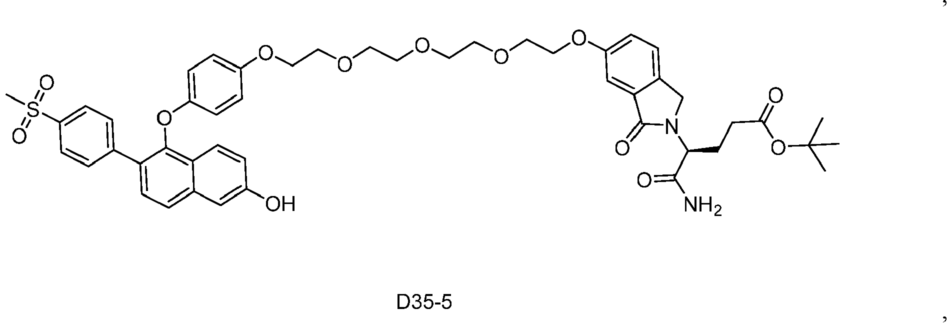 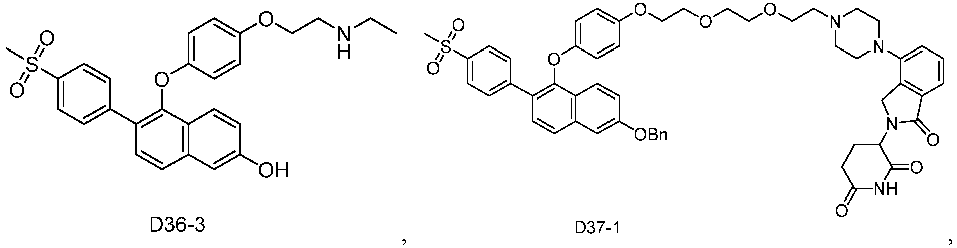 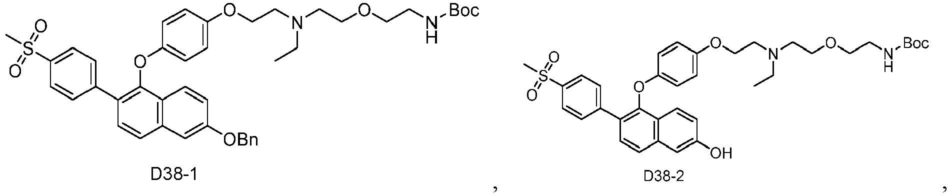 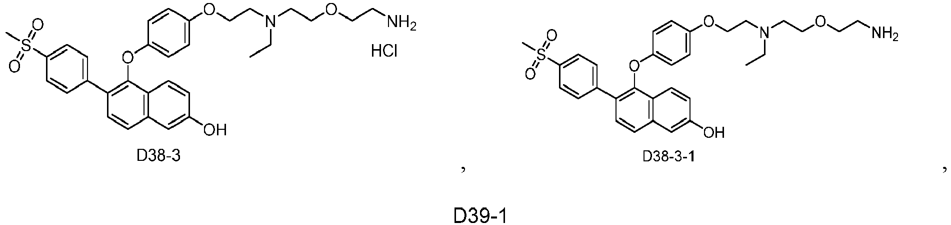 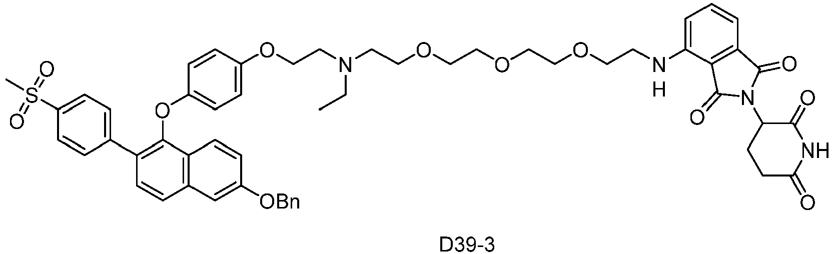 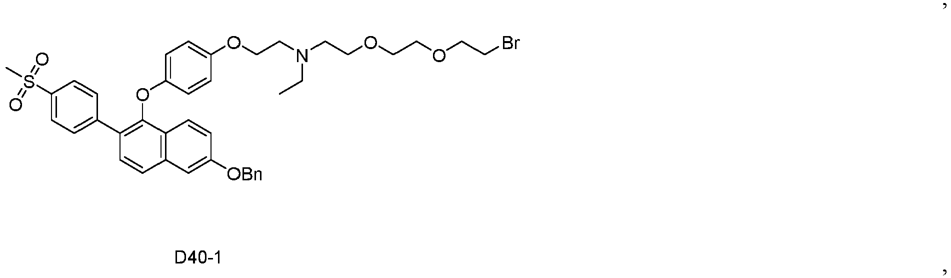 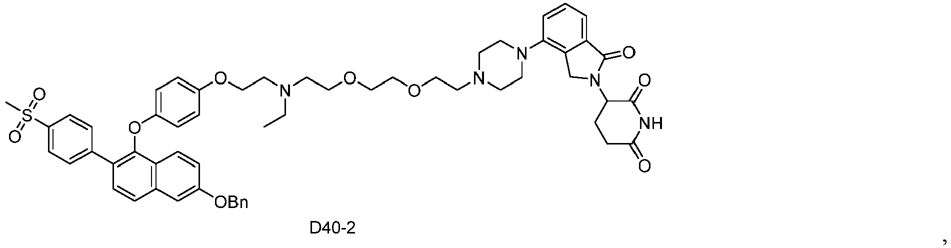 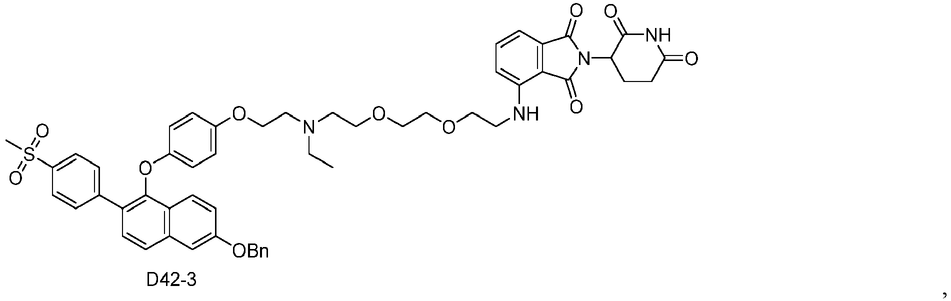 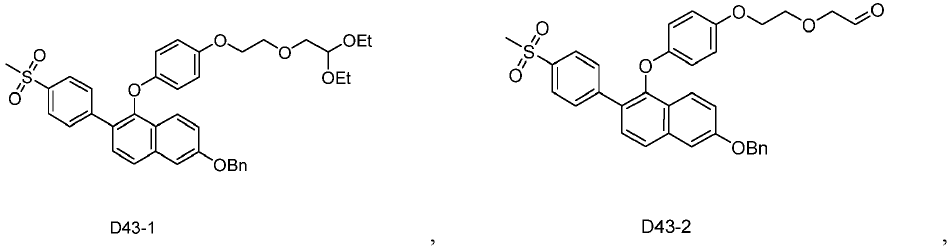 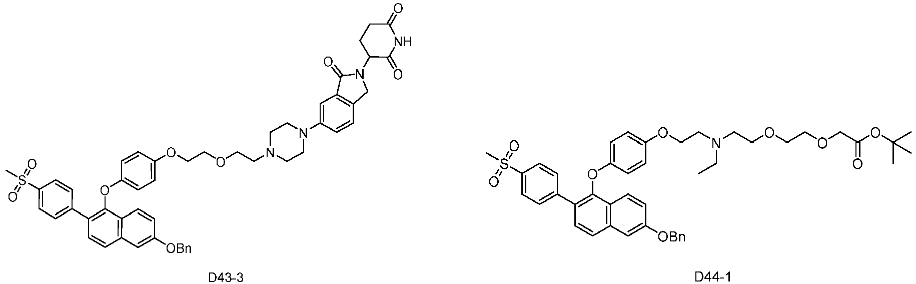 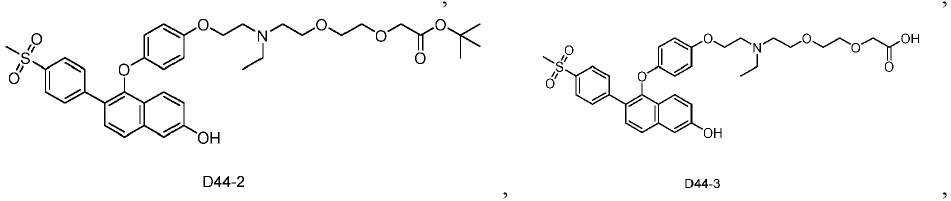 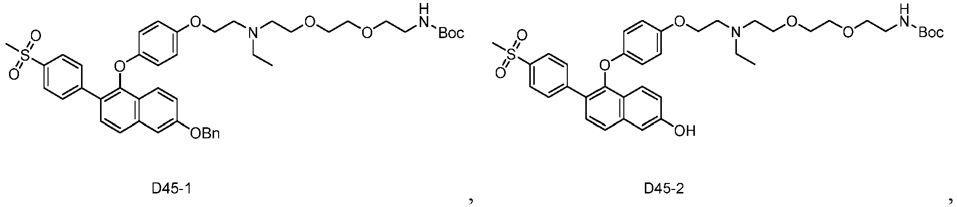 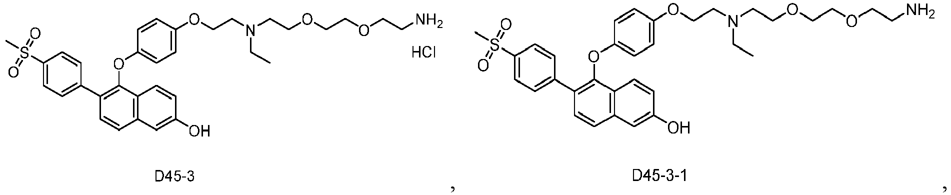 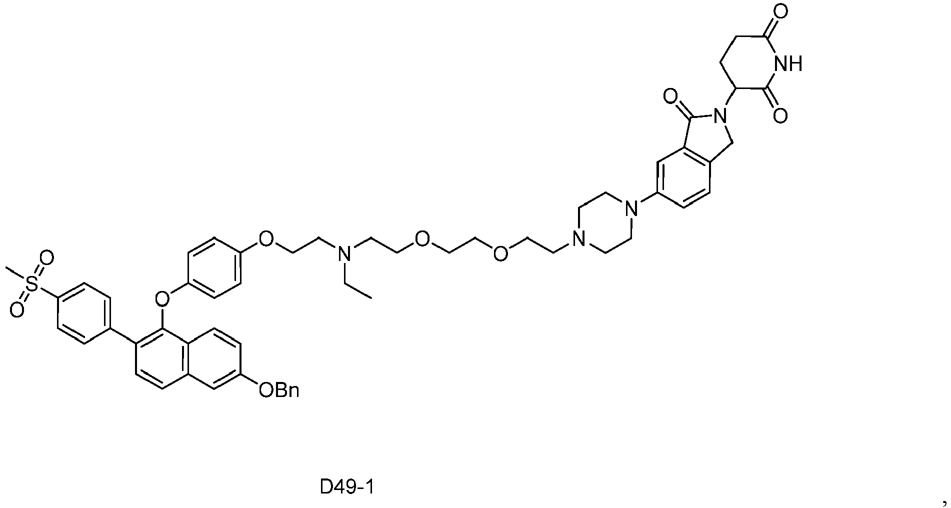 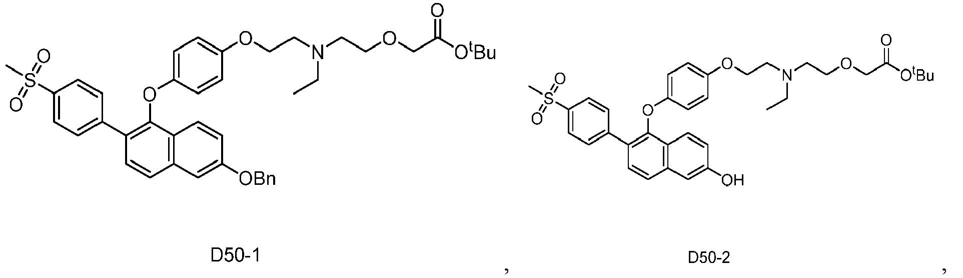 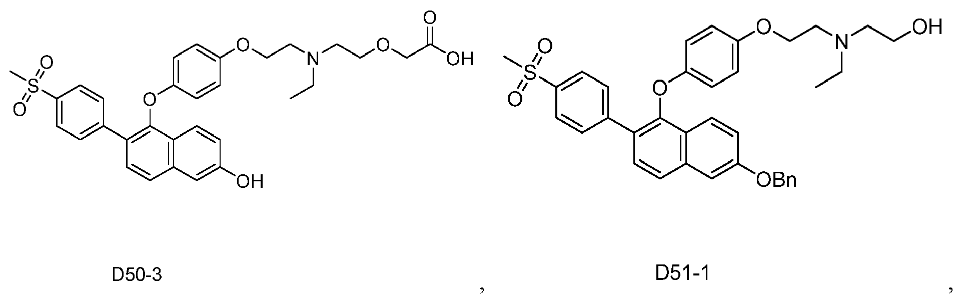 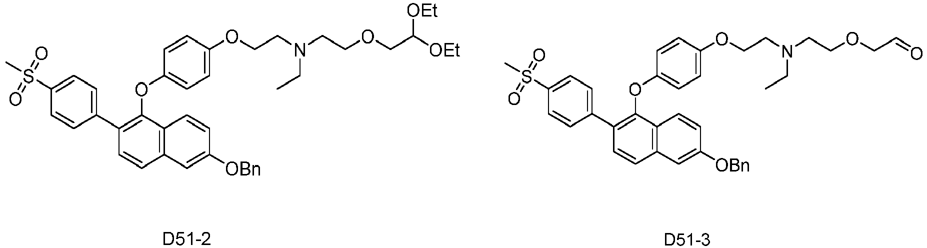 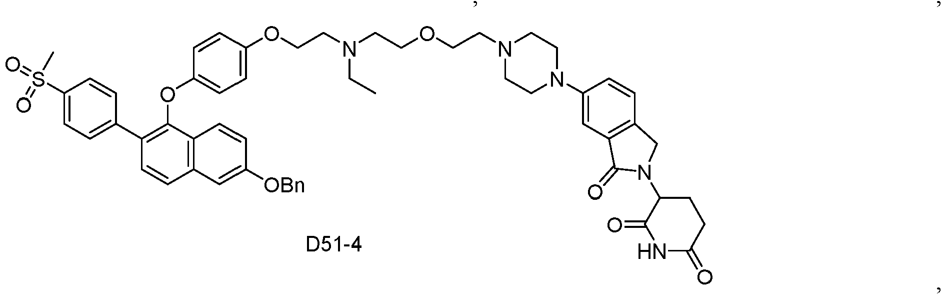 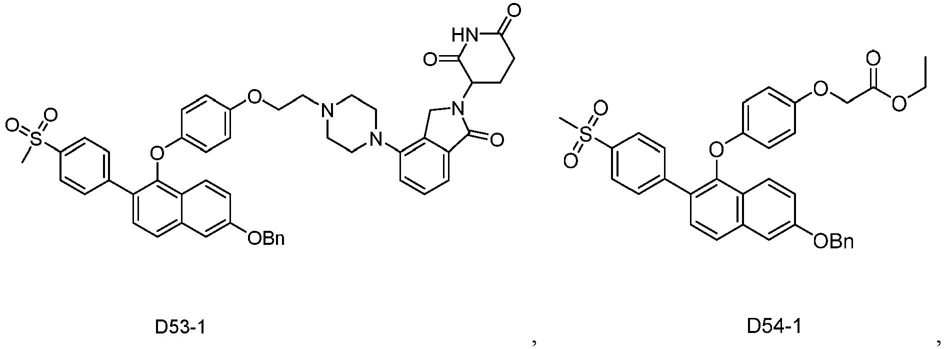 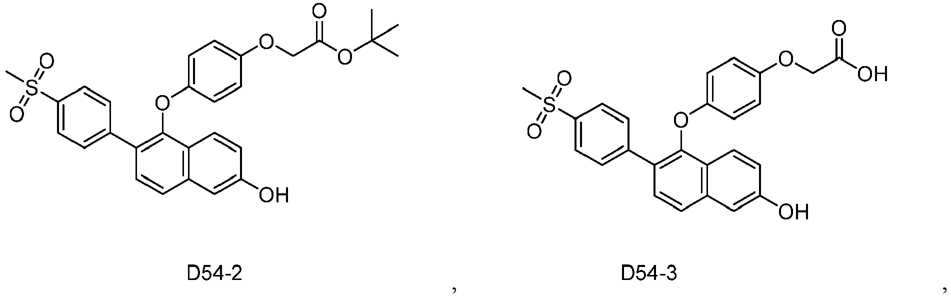 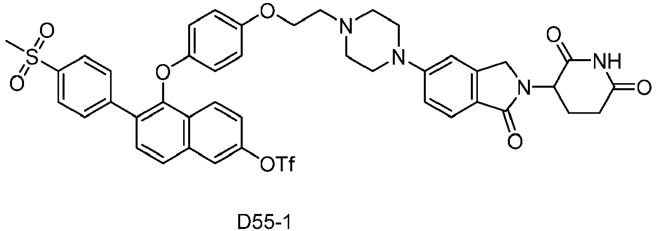 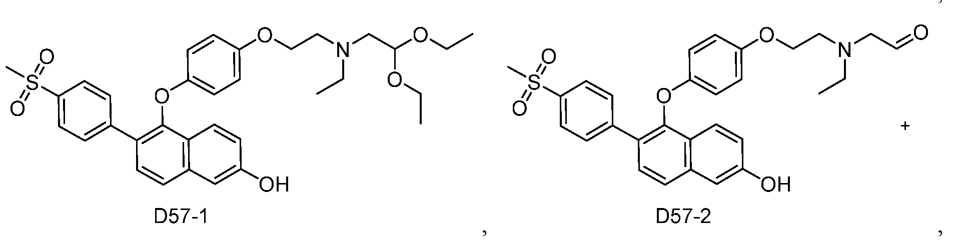 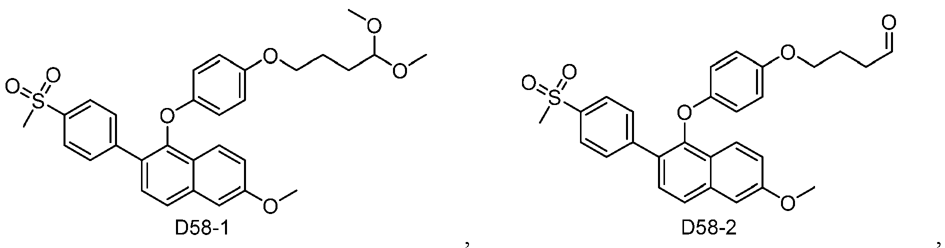 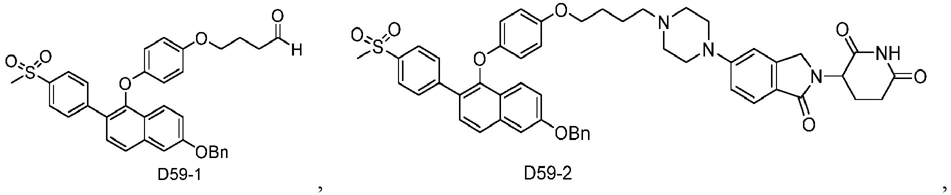 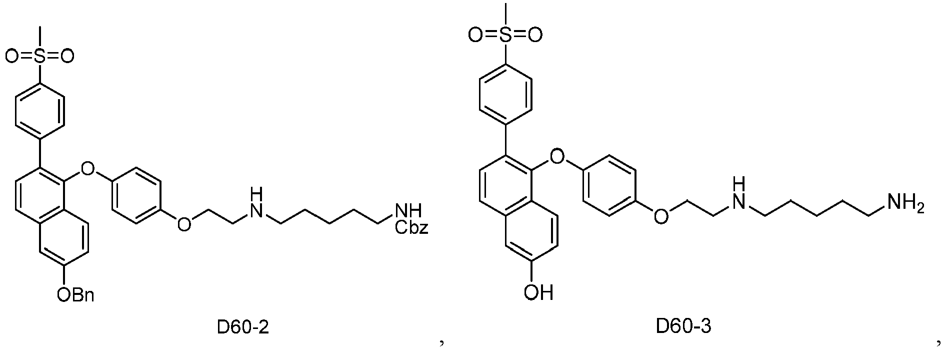 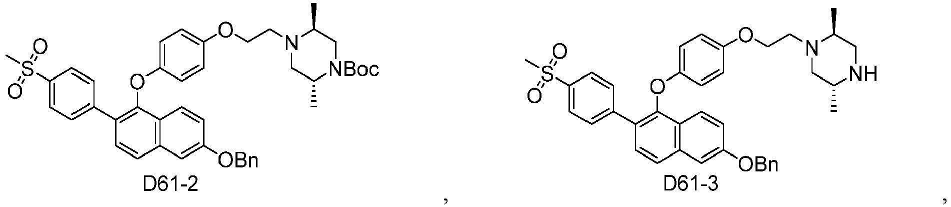 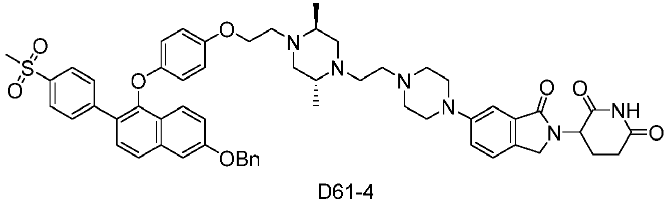 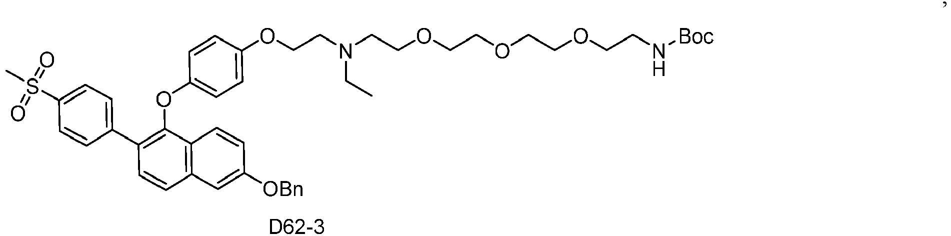 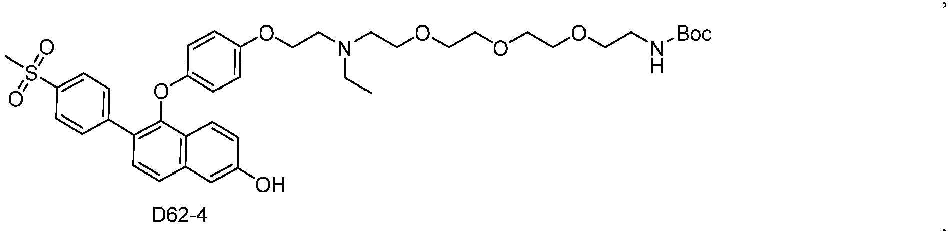 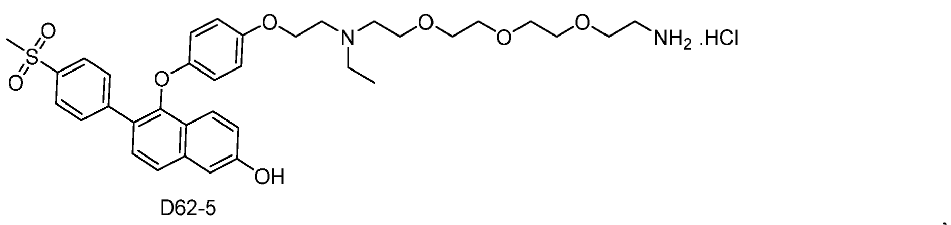 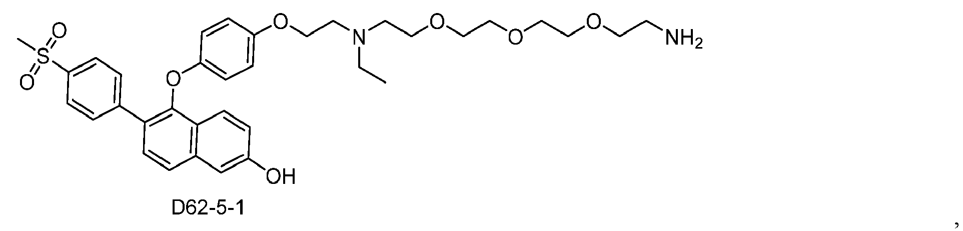 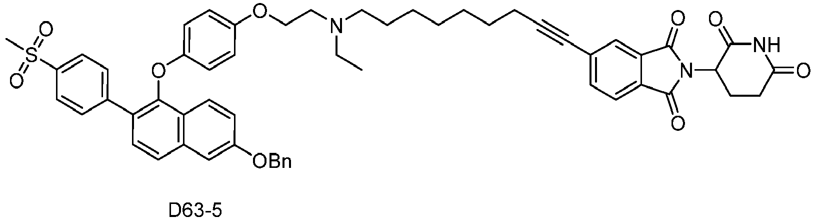 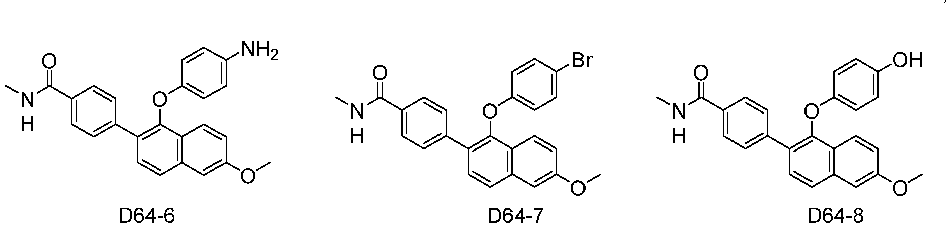 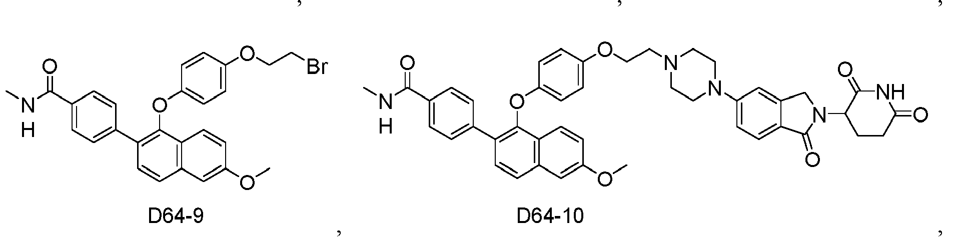 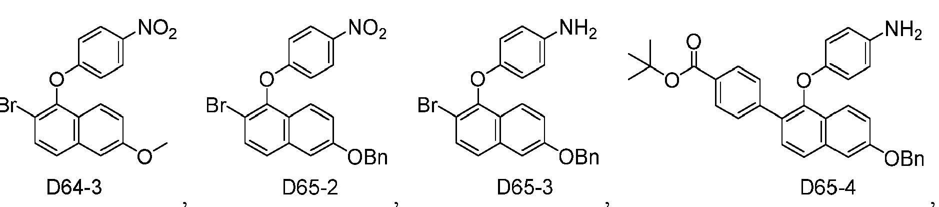 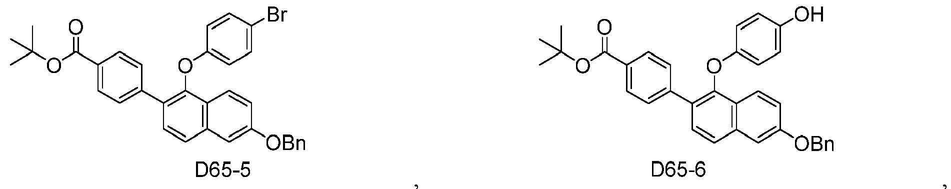 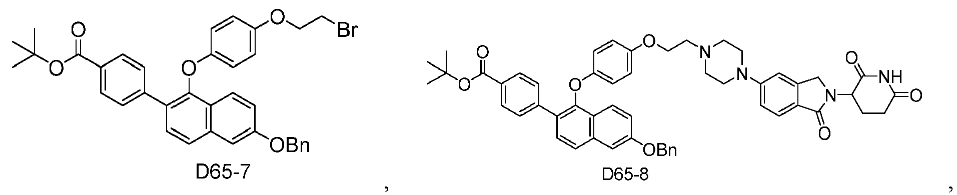 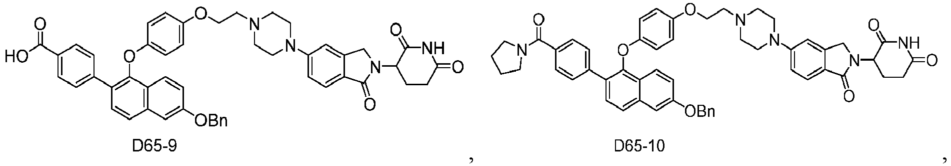 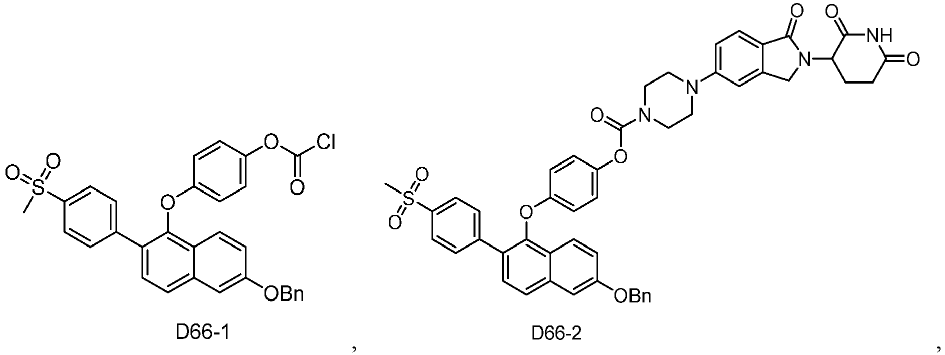 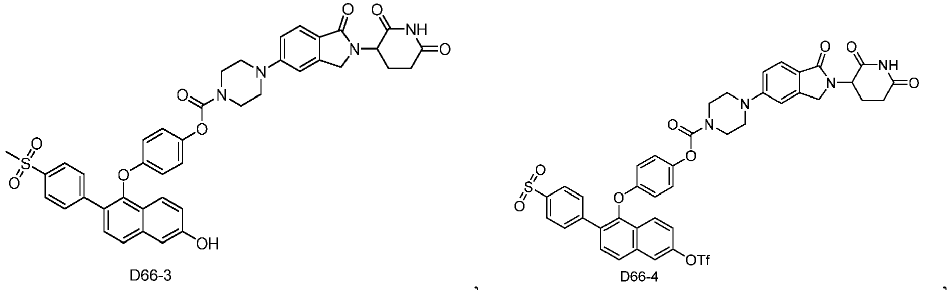 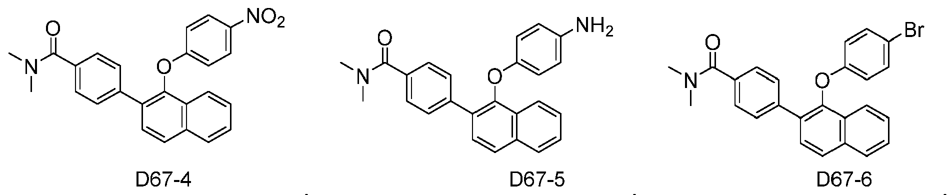 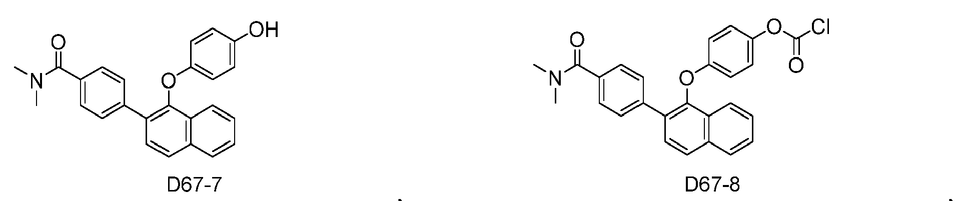 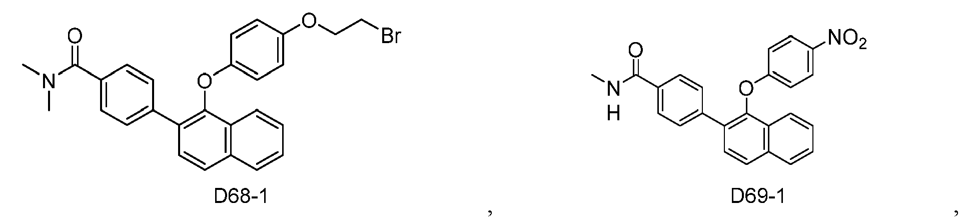 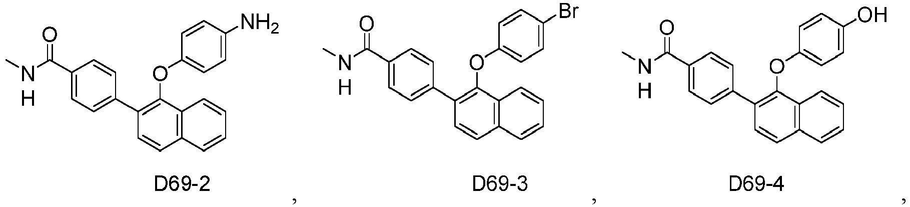 or

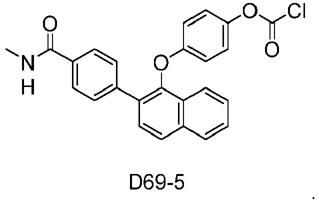

The present disclosure also provides a pharmaceutical composition comprising substance **A** and a pharmaceutical excipient;
the substance **A** is the naphthalene ring-containing compound of formula **I**, the pharmaceutically acceptable salt thereof, the solvate thereof, or the solvate of the pharmaceutically acceptable salt thereof.

In the pharmaceutical composition, the substance **A** may be a therapeutically effective amount of substance **A.**

The present disclosure also provides a pharmaceutical composition comprising substance **B** and a pharmaceutical excipient;
the substance **B** is the naphthalene ring-containing compound of formula **III**, the pharmaceutically acceptable salt thereof, the solvate thereof, or the solvate of the pharmaceutically acceptable salt thereof.

In the pharmaceutical composition, the substance **B** may be a therapeutically effective amount of substance **B.**

The present disclosure also provides a use of substance **A** or the pharmaceutical composition in the manufacture of a medicament;
the substance **A** is the naphthalene ring-containing compound of formula **I**, the pharmaceutically acceptable salt thereof, the solvate thereof, or the solvate of the pharmaceutically acceptable salt thereof; and the medicament is a medicament for the treatment or prevention of cancer.

The present disclosure also provides a use of substance **B** or the pharmaceutical composition in the manufacture of a medicament;

the substance **A** is the naphthalene ring-containing compound of formula **III**, the pharmaceutically acceptable salt thereof, the solvate thereof, or the solvate of the pharmaceutically acceptable salt thereof; and the medicament is a medicament for the treatment or prevention of cancer.

In the use, the cancer may be breast cancer. In the use, the breast cancer may be Luminal A breast cancer, Luminal B breast cancer, HER2-positive breast cancer, or triple-negative breast cancer, and may be triple-negative breast cancer.

In the use, the Luminal A breast cancer is an ER-positive and/or PR-positive, and HER2-negative breast cancer. The Luminal A breast cancer may be non-drug-resistant and non-mutated Luminal Abreast cancer, drug-resistant and non-mutated Luminal Abreast cancer, or drug-resistant and mutated Luminal A breast cancer.

In the use, the drug-resistant and non-mutated Luminal A breast cancer may be tamoxifen-resistant and non-mutated Luminal A breast cancer.

In the use, the drug-resistant and mutated Luminal A breast cancer may be ER^{D538G}-mutated Luminal A breast cancer, and may also be tamoxifen- and/or fulvestrant-resistant and ER^{D538G}-mutated Luminal Abreast cancer, and may also be tamoxifen- and fulvestrant-resistant and ER^{D538G}-mutated Luminal A breast cancer.

In the use, the drug-resistant and mutated Luminal A breast cancer may be ER^{Y537S}-mutated Luminal A breast cancer, and may also be tamoxifen- and/or fulvestrant-resistant and ER^{Y537S}-mutated Luminal Abreast cancer, and may also be tamoxifen- and fulvestrant-resistant and ER^{Y537S}-mutated Luminal A breast cancer.

Unless otherwise specified, the terms used in the present disclosure have the following meanings:

The term "alkyl" refers to a straight or branched chain alkyl group with a specified number of carbon atoms (e.g., C₁-C₄). Alkyl includes, but is not limited to, methyl, ethyl, n-propyl, isopropyl, n-butyl, tert-butyl, isobutyl, sec-butyl, etc.

The term "absent" means that the two moieties to which the moiety is attached are directly linked by a single bond. For example, "X² is absent" means that X¹ and X³ are directly linked by a single bond; "X² and X³ are absent" means that X¹ and X⁴ are directly linked by a single bond.

The term "heterocycloalkylene" refers to a cyclic group with a specified number of ring atoms (e.g., 5- to 6-membered), a specified number of heteroatoms (e.g., 1, 2, or 3), and a specified type of heteroatom (e.g., one or more than one of N, O, and S), which is a saturated monocyclic ring; it is a divalent functional group, which is linked to other moieties by two single bonds. Heterocycloalkylene includes, but is not limited to, tetrahydropyrrolylene, tetrahydrofurylene, morpholinylene, piperidinylene, etc.

The term "heteroarylene" refers to a cyclic group with a specified number of ring atoms (e.g., 5- to 6-membered), a specified number of heteroatoms (e.g., 1, 2, or 3), and a specified type of heteroatom (e.g., one or more than one of N, O, and S), which is a monocyclic ring with aromaticity (complying with Huckel's rule); it is a divalent functional group, which is linked to other moieties by two single bonds. Heteroarylene includes, but is not limited to, furylene, pyrrolylene, etc.

The term "alkylene" refers to a straight or branched chain alkylene group with a specified number of carbon atoms (e.g., C₁-C₄); it is a divalent functional group, which is linked to other moieties by two single bonds. Alkylene includes, but is not limited to, methylene, ethylene, etc.

The term "heteroalkylene" refers to a group formed by the substitution of carbon atoms in the alkylene group by heteroatoms, having a specified number of carbon atoms (e.g., C₁-C₄), a specified number of heteroatoms (e.g., 1, 2, or 3), and a specified type of heteroatom (e.g., one or more than one of N, O, and S), which may be straight or branched; it is a divalent functional group, which is linked to other moieties by two single bonds. Heteroalkylene includes, but is not limited to, 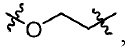 etc.

The term "alkoxy" refers to the group R^{X}-O-, wherein R^{X} is the alkyl as defined above.

The "-" at the end of a moiety means that the moiety is attached to other moieties in the molecule through this site. For example, CH₃-C(=O)- refers to acetyl.

The 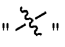 in a moiety means that the moiety is attached to other moieties in the molecule through this site. For example, 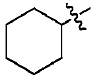 refers to cyclohexyl.

When an arbitrary variable (e.g., R^{x1-1}) appears many times in the definition of a compound, their definitions are independent of each other and have no influence on each other. For example, 5- to 6-membered heterocycloalkylene substituted by 3 R^{x1-1} means that 5- to 6-membered heterocycloalkylene will be substituted by 3 R^{x1-1}, and the definitions of 3 R^{x1-1} are independent of each other and have no influence on each other.

The term "pharmaceutically acceptable salt" refers to a salt obtained by reacting a compound with a pharmaceutically acceptable (relatively non-toxic, safe, and suitable for a patient) acid or base. When the compound contains a relatively acidic functional group, a base addition salt can be obtained by bringing the free form of the compound into contact with a sufficient amount of the pharmaceutically acceptable base in a suitable inert solvent. Pharmaceutically acceptable base addition salts include, but are not limited to, sodium salts, potassium salts, calcium salts, aluminum salts, magnesium salts, bismuth salts, ammonium salts, etc. When the compound contains a relatively basic functional group, an acid addition salt can be obtained by bringing the free form of the compound into contact with a sufficient amount of the pharmaceutically acceptable acid in a suitable inert solvent. Pharmaceutically acceptable acid addition salts include, but are not limited to, hydrochloride, sulfate, methanesulfonate, etc. For details, refer to Handbook of Pharmaceutical Salts: Properties, Selection, and Use (P. Heinrich Stahl, 2002).

The term "patient" refers to any animal, preferably a mammal, most preferably a human, that has been or will be treated. Mammals include, but are not limited to, cows, horses, sheep, pigs, cats, dogs, mice, rats, rabbits, guinea pigs, monkeys, humans, etc.

The term "solvate" refers to a substance formed by crystallization of a compound with a solvent (including, but not limited to, water, methanol, ethanol, etc.). Solvates include a stoichiometric solvate and a non-stoichiometric solvate.

The term "solvate of pharmaceutically acceptable salt" refers to a substance formed by combination of a compound with a pharmaceutically acceptable (relatively non-toxic, safe, and suitable for a patient) acid or base, and solvent (including, but not limited to, water, methanol, ethanol, etc.), wherein the pharmaceutically acceptable salt has the same meaning as the term "pharmaceutically acceptable salt" above, and the solvent is stoichiometric or non-stoichiometric. The solvate of pharmaceutically acceptable salt includes, but is not limited to, hydrochloride monohydrate.

The term "therapeutically effective amount" refers to the amount of a compound administered to a patient that is sufficient to effectively treat a disease. The therapeutically effective amount will vary depending on the compound, the type of the disease, the severity of the disease, the age of the patient, etc., but it can be adjusted by those skilled in the art as appropriate.

The term "pharmaceutical excipients" refers to the excipients and additives used in the manufacture of drugs and the formulation of prescriptions, which are all substances contained in pharmaceutical preparations except active ingredients. For details, refer to Pharmacopoeia of the People's Republic of China (2020 Edition) or Handbook of Pharmaceutical Excipients (Raymond C Rowe, 2009).

The term "treatment" refers to any one of the following: (1) alleviating one or more biological manifestations of a disease; (2) interfering with one or more points in the biological cascade that causes the disease; (3) slowing the progression of one or more biological manifestations of the disease.

The term "prevention" refers to a reduced risk of developing a disease.

On the basis of not violating the common knowledge in the art, the preferred conditions above can be arbitrarily combined to obtain the preferred examples of the present disclosure.

The reagents and raw materials used in the present disclosure are all commercially available.

The positive progressive effect of the present disclosure is that the compound has a novel structure and shows better activity on breast cancer.

### DETAILED DESCRIPTION OF THE PREFERRED EMBODIMENT

The present disclosure is further described below by the way of examples, but the present disclosure is not thereby limited to the scope of the described examples. The experimental methods for which the specific conditions are not specified in the following examples are selected according to the conventional methods and conditions, or according to the commodity instructions.

The meaning of each abbreviation in the present disclosure is shown in the following table:

| Abbreviation | Full name |
|---|---|
| DPEPhos | (Oxydi-2,1-phenylene)bis(diphenylphosphine) |
| DDQ | 2,3-Dichloro-5,6-dicyano-p-benzoquinone |
| DIPEA | Diisopropylethyl amine |
| DMF | N,N-Dimethylformamide |
| HATU | 2-(7-Azabenzotriazol-1-yl)-N,N,N',N'-tetramethyluronium hexafluorophosphate |
| DMAP | 4-Dimethylaminopyridine |
| DIAD | Diisopropyl azodicarboxylate |
| Pd(dppf)Cl₂.DCM | [1,1'-Bis(diphenylphosphino)ferrocene]dichloropalladium(II) dichloromethane complex |
| Pd₂(dba)₃ | Tris(dibenzylideneacetone)dipalladium |
| tBuXPhos | 2-Di-tert-butylphosphino-2',4',6'-triisopropylbiphenyl |
| Flash purification | Flash chromatography |
| TFA | Trifluoroacetic acid |

### Example 1: Synthesis of N-(2-(2,6-dioxopiperidin-3-yl)-1-oxoisoindolin-4-yl)-2-((5-(ethyl(2-(4-((6-hydroxy-2-(4-(methylsulfonyl)phenyl)naphthalen-1-yl)oxy)phenoxy)ethyl)amino)pentyl)oxy)acetamide (compound D1)

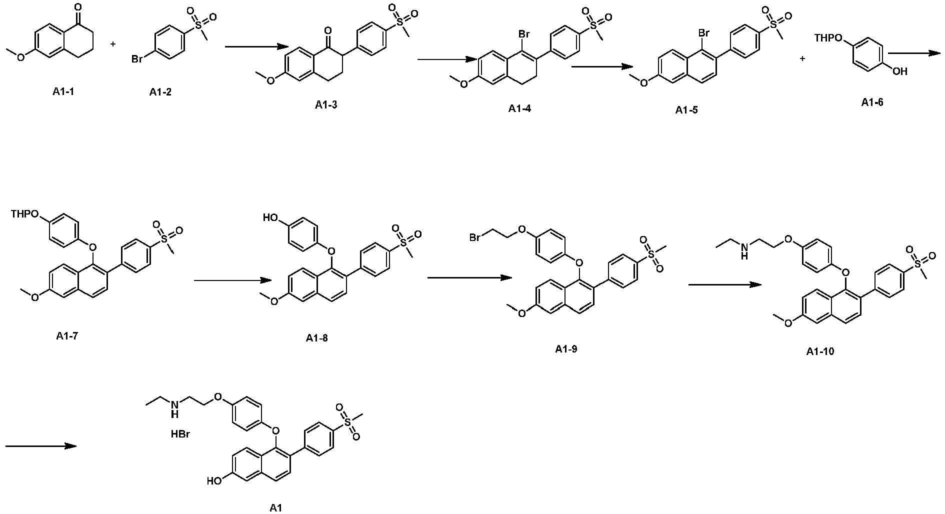

6-Methoxy-3,4-dihydronaphthalen-1(2H)-one (compound A1-1) (17.6 g, 100 mmol) and 1-bromo-4-(methylsulfonyl)benzene (compound A1-2) (23.5 g, 100 mmol) were placed in a 500 mL round-bottom flask. Toluene (200 mL), sodium tert-butoxide (24 g, 250 mmol), DPEPhos (1.34 mg, 2.5 mmol), and palladium acetate (560 mg, 2.5 mmol) were added thereto. The reaction mixture was replaced with nitrogen, reacted at 85°C for 3 hours, concentrated, extracted twice with water (200 mL) and dichloromethane (100 mL), and the phases were separated. The organic phase was dried and concentrated to obtain 6-methoxy-2-(4-(methylsulfonyl)phenyl)-3,4-dihydronaphthalene-1(2H)-one (compound A1-3) (32 g, purity: 90%, yield: 96.9%) as a pale yellow solid.

Compound A1-3 (32 g, 96.9 mmol) was placed in a 100 mL round-bottom flask, and phosphorus tribromide (78.7 g, 290.8 mmol) and anhydrous toluene (200 mL) were added thereto. The reaction mixture was refluxed for 20 hours, cooled, poured into ice water (500 mL) to quench, stirred for 1 hour, and extracted twice with dichloromethane (150 mL). The organic phases were combined, washed with saturated sodium bicarbonate aqueous solution (100 mL), and the phases were separated. The organic phase was concentrated to obtain a crude product (32 g), which was slurried with a mixed solvent of petroleum ether (50 mL) and ethyl acetate (50 mL), and filtered to obtain 7-methoxy-4-bromo-3-(4-(methylsulfonyl)phenyl)-1,2-dihydronaphthalene (compound A1-4) (22 g, purity: 80%, yield: 57.9%) as a pale yellow solid.

Compound A1-4 (22 g, 55.9 mmol) and DDQ (30 g, 132.1 mmol) were placed in a 500 mL round-bottom flask, and anhydrous toluene (200 mL) was added thereto. The reaction mixture was reacted at 70°C for 10 hours, concentrated, added with dichloromethane (150 mL) to dissolve the crude product, and the insoluble material was filtered off. The organic phases were washed three times with saturated potassium carbonate aqueous solution (100 mL), combined, and concentrated. The crude product was recrystallized with dichloromethane and petroleum ether to obtain 1-bromo-6-methoxy-2-(4-(methylsulfonyl)phenyl)naphthalene (compound A1-5) (14 g, purity: 98%, yield: 63.6%) as a solid.

Compound A1-5 (14 g, 35.78 mmol), 4-((tetrahydro-2H-pyran-2-yl)oxy)phenol (compound A1-6) (13.9 g, 71.56 mmol), and cesium carbonate (35.3 g, 107.34 mmol) were placed in a 250 mL round-bottom flask, and cuprous chloride (1.79 g, 17.89 mmol) and anhydrous DMSO (150 mL) were added thereto. The reaction mixture was replaced with nitrogen, reacted at 160°C for 8 hours, diluted with water (500 mL), and extracted twice with dichloromethane (150 mL). The organic phases were combined, dried, and concentrated to obtain 2-(4-((6-methoxy-2-(4-(methylsulfonyl)phenyl)naphthalen-1-yl)oxy)phenoxy)tetrahydro-2H-pyran (compound A1-7) (crude product, 21 g, yield: 100%) as a gray solid.

Compound A1-7 (21 g, 41.7 mmol) was placed in methanol (100 mL), and p-toluenesulfonic acid (7.1 g, 41.7 mmol) was added thereto. The reaction mixture was stirred and reacted at room temperature for 10 hours, and concentrated. The crude product was dissolved in dichloromethane (150 mL), and then washed twice with saturated sodium bicarbonate solution (100 mL). The organic phases were combined, dried, and concentrated to obtain 4-((6-methoxy-2-(4-(methylsulfonyl)phenyl)naphthalen-1-yl)oxy)phenol (compound A1-8) (14 g, purity: 90%, yield: 93%) as a pale yellow solid.

¹H-NMR (400 MHz, DMSO-*d*₆): δ 9.07 (s, 1H), 7.84 - .99 (m, 5H), 7.80 (d, *J* = 5.2Hz, 1H), 7.67 (d, *J* = 8.4Hz, 1H), 7.50 (d, *J* = 6.4Hz, 1H), 7.20 (dd, *J* = 9.2Hz, 2.4Hz, 1H), 6.50 - 6.58 (m, 4H), 3.93 (s, 3H), 3.25 (s, 3H).

Compound A1-8 (1.0 g, 2.38 mmol), 1,2-dibromoethane (4.46 g, 23.74 mmol), and cesium carbonate (2.32 g, 7.13 mmol) were placed in acetonitrile (10 mL), and the reaction mixture was stirred at 70°C for 16 hours. After the reaction was completed, the reaction mixture was filtered, and concentrated to dryness to obtain an oily liquid, which was purified by silica gel column chromatography (dichloromethane/ethyl acetate = 10/1) to obtain 1-(4-(2-bromoethoxy)phenoxy)-6-methoxy-2-(4-(methylsulfonyl)phenyl)naphthalene (compound A1-9) (1 g, purity: 90%, yield: 80%) as a white solid.

Compound A1-9 (0.8 g, 1.52 mmol) and ethylamine solution (8 mL, 1 M tetrahydrofuran solution) were placed in DMF (2 mL), and the reaction mixture was heated to 80°C and stirred for 3 hours. The reaction mixture was concentrated under vacuum, and the crude product was purified by silica gel column chromatography (dichloromethane/methanol = 10/1) to obtain N-ethyl-2-(4-((6-methoxy-2-(4-(methylsulfonyl)phenyl)naphthalen-1-yl)oxy)phenoxy)ethan-1-amine (compound A1-10) (0.4 g, purity: 96%, yield: 53.6%) as a white solid.

Compound A1-10 (0.4 g, 0.81 mmol) was dissolved in anhydrous dichloromethane (12 mL), and the mixture was added dropwise to a mixture of boron tribromide (4 mL) (1 M, dichloromethane solution) and anhydrous dichloromethane (4 mL) under an ice bath. After the dropwise addition was completed, the reaction mixture was warmed to room temperature and stirred for 30 minutes. The reaction mixture was added with methanol to quench, and concentrated. The crude product was liberated and then purified by silica gel column chromatography (dichloromethane/methanol = 10/1) to obtain 5-(4-(2-(ethylamino)ethoxy)phenoxy)-6-(4-(methylsulfonyl)phenyl)naphthalen-2-ol hydrobromide (compound A1) (0.3 g, purity: 92%, yield: 77.2%) as a yellow oily liquid.

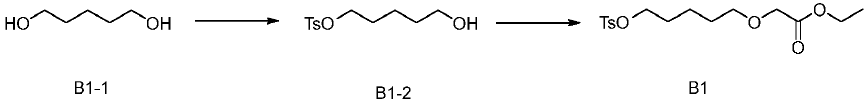

1,5-Pentanediol (5 g, 48.0 mmol) (compound B1-1), 4-dimethylaminopyridine (0.587 g, 4.80 mmol), and triethylamine (14.57 g, 144.0 mmol) were added to dichloromethane (500.0 mL) at room temperature, and p-toluenesulfonyl chloride (8.24 g, 43.2 mmol) was added thereto. The resulting reaction mixture was stirred at room temperature for 16 hours, then sequentially washed three times with water (100.0 mL), and washed with saturated brine (100.0 mL). The organic phase was dried over anhydrous sodium sulfate, filtered, and concentrated under reduced pressure. The crude product was purified by silica gel column chromatography (dichloromethane/methanol = 10/1) to obtain 5-hydroxypentyl 4-methylbenzenesulfonate (compound B1-2) (5.0 g, yield: 40%).

Boron trifluoride diethyl etherate (0.02 mL, 0.19 mmol) was added dropwise to a solution of compound B 1-2 (5.0 g, 19.4 mmol) in dichloromethane (40.0 mL) at 0°C, and then a solution of ethyl diazoacetate in dichloromethane (6.0 M, 10.0 mL) was added dropwise thereto. The resulting reaction mixture was warmed to room temperature, stirred for 2 hours, and diluted with water (100.0 mL). The organic phase was separated, and the aqueous phase was extracted three times with dichloromethane (100.0 mL). The organic phases were combined, dried over anhydrous sodium sulfate, filtered, and concentrated under reduced pressure. The crude product was purified by silica gel column chromatography (dichloromethane/methanol = 10/1) to obtain ethyl 2-((5-(tosyloxy)pentyl)oxy)acetate (compound B1) (4.0 g, yield: 70%).

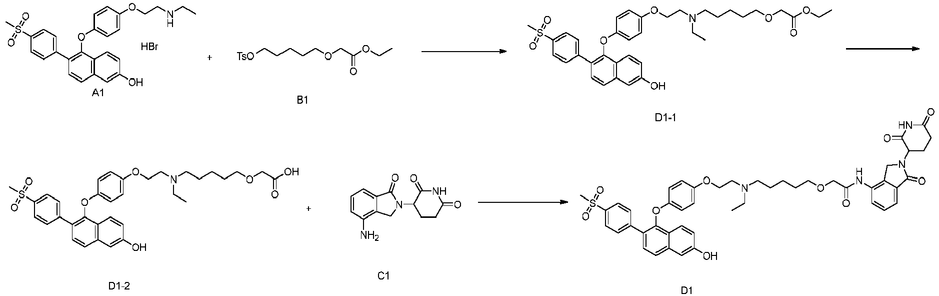

Compound B1 (288.0 mg, 0.84 mmol) was added to a solution of compound A1 crude product (200.0 mg, 0.42 mmol) and DIPEA (163.0 mg, 1.26 mmol) in DMF (5.0 mL) at room temperature. The resulting mixture was stirred at 80°C for 3 hours, cooled to room temperature, and concentrated under reduced pressure to remove the organic solvent. The crude product was purified by silica gel column chromatography (dichloromethane/methanol = 10/1) to obtain ethyl 2-((5-(ethyl(2-(4-((6-hydroxy-2-(4-(methylsulfonyl)phenyl)naphthalen-1-yl)oxy)phenoxy)ethyl)amino)pentyl)oxy)acetate (compound D1-1) (160.0 mg, yield: 65%).

Compound D1-1 (160.0 mg, 0.25 mmol) and lithium hydroxide monohydrate (31.0 mg, 0.74 mmol) were added to a mixed solvent of ethanol (3.0 mL) and water (2.0 mL) at room temperature. The resulting mixture was stirred at 50°C for 30 minutes, cooled to room temperature, added with hydrochloric acid aqueous solution (1.0 M) to adjust the pH of the aqueous phase to about 3 to 4, and concentrated to dryness under reduced pressure. The crude product was purified by prep-HPLC to obtain 2-((5-(ethyl(2-(4-((6-hydroxy-2-(4-(methylsulfonyl)phenyl)naphthalen-1-yl)oxy)phenoxy)ethyl)amino)pentyl)oxy)acetic acid (compound D1-2) (100.0 mg, yield: 64%).

Compound D1-2 (50.0 mg, 0.080 mmol) and 2-ethoxy-1-ethoxycarbonyl-1,2-dihydroquinoline (40.0 mg, 0.16 mmol) were added to a mixed solvent of dichloromethane (2.0 mL) and N-methylpyrrolidone (0.4 mL) at room temperature. The resulting mixture was stirred at room temperature for 10 minutes, and then added with compound C1 (31.0 mg, 0.12 mmol). The reaction mixture was stirred at 50°C for 4 hours, cooled to room temperature, and concentrated to dryness under reduced pressure. The crude product was purified by prep-HPLC to obtain compound D1 (27.0 mg, yield: 39%).

¹H NMR (400 MHz, DMSO-*d*₆) δ 11.03 (s, 1H), 9.74 (s, 1H), 8.36 (s, 1H), 7.92 (d, *J* = 8.4 Hz, 2H), 7.85 (d, *J* = 8.4 Hz, 2H), 7.76 (dd, *J* = 16.4, 8.9 Hz, 3H), 7.63 - 7.55 (m, 2H), 7.55 - 7.47 (m, 1H), 7.27 (d, *J* = 1.8 Hz, 1H), 7.12 (dd, *J* = 9.1, 2.0 Hz, 1H), 6.74 (d, *J* = 9.0 Hz, 2H), 6.61 (d, *J* = 9.0 Hz, 2H), 5.16 (dd, *J* = 13.3, 5.0 Hz, 1H), 4.39 (q, *J* = 17.4 Hz, 2H), 4.08 (s, 2H), 3.86 (t, *J* = 6.0 Hz, 2H), 3.38 - 3.35 (m, 2H), 3.23 (s, 3H), 2.98 - 2.87 (m, 1H), 2.69 (t, *J* = 6.0 Hz, 2H), 2.61 (d, *J* = 16.5 Hz, 1H), 2.49 (d, *J* = 7.0 Hz, 2H), 2.46 - 2.40 (m, 2H), 2.40 - 2.30 (m, 1H), 2.05 - 1.99 (m, 1H), 1.58 (p, *J* = 6.7 Hz, 2H), 1.45 - 1.36 (m, 2H), 1.35 - 1.29 (m, 2H), 0.93 (t, *J* = 7.0 Hz, 3H).

MS calculated for 862.32; found 863.5 [M+H]⁺.

### Example 2: Synthesis of 3-(5-(4-(2-(4-((6-hydroxy-2-(4-(methylsulfonyl)phenyl)naphthalen-1-yl)oxy)phenoxy)ethyl)piperazin-1-yl)-1-oxoisoindolin-2-yl)piperidine-2,6-dione (compound D2)

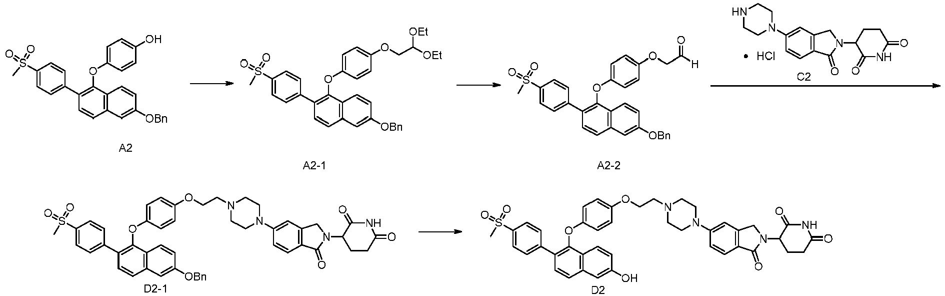

4-((6-(Benzyloxy)-2-(4-(methylsulfonyl)phenyl)naphthalen-1-yl)oxy)phenol (compound A2) (4.96 g, 10.0 mmol) and cesium carbonate (6.51 g, 20.0 mmol) were added to DMF (100.0 mL) at room temperature. The resulting mixture was stirred at 70°C for 30 minutes, and then bromoacetaldehyde diethyl acetal (10.0 g, 50.0 mmol) was added thereto. The reaction mixture was then heated to 100°C, stirred for 10 hours, cooled to room temperature, and diluted with water (100.0 mL). The resulting mixture was extracted twice with dichloromethane (80.0 mL). The organic phases were combined, dried over anhydrous sodium sulfate, filtered, and concentrated under reduced pressure. The crude product was purified by silica gel column chromatography (dichloromethane/methanol = 10/1) to obtain 6-(benzyloxy)-1-(4-(2,2-diethoxyethoxy)phenoxy)-2-(4-(methylsulfonyl)phenyl)naphthalene (compound A2-1) (6.0 g, yield: 98%).

Trifluoroacetic acid (0.5 mL, 21.0 mmol) was added dropwise to a solution of compound A2-1 (1.3 g, 2.12 mmol) in dichloromethane (20.0 mL) at room temperature. The resulting mixture was stirred at room temperature for 5 hours, diluted with dichloromethane (30.0 mL), and then washed twice with saturated sodium bicarbonate aqueous solution (20.0 mL). The organic phase was separated, dried over anhydrous sodium sulfate, filtered, and concentrated to dryness under reduced pressure to obtain a crude product of 2-(4-((6-(benzyloxy)-2-(4-(methylsulfonyl)phenyl)naphthalen-1-yl)oxy)phenoxy)acetaldehyde (compound A2-2) (0.90 g, yield: 80%).

The crude product of compound A2-2 (538.0 mg, 1.0 mmol), 3-(1-oxo-5-(piperazin-1-yl)isoindolin-2-yl)piperidine-2,6-dione hydrochloride (compound C2) (364.0 mg, 1.0 mmol), and potassium acetate (200.0 mg, 2.0 mmol) were sequentially added to a mixed solvent of dichloromethane (20.0 mL) and methanol (5.0 mL) at room temperature. The resulting mixture was stirred at room temperature for 30 minutes, then added with sodium triacetoxyborohydride (422.0 mg, 2.0 mmol), stirred for another 18 hours, and diluted with water (30.0 mL). The organic phase was separated, and the aqueous phase was extracted twice with dichloromethane (10.0 mL). The organic phases were combined, dried over anhydrous sodium sulfate, filtered, and concentrated under reduced pressure. The crude product was purified by silica gel column chromatography (dichloromethane/methanol = 10/1) to obtain 3-(5-(4-(2-(4-((6-(benzyloxy)-2-(4-(methylsulfonyl)phenyl)naphthalen-1-yl)oxy)phenoxy)ethyl)piperazin-1-yl)-1-oxoisoindolin-2-yl)piperidine-2,6-dione (compound D2-1) (700.0 mg, yield: 82%).

Wet palladium on carbon (200 mg, 10 wt%) was added to a solution of compound D2-1 (700.0 mg, 0.81 mmol) in tetrahydrofuran (20.0 mL) at room temperature. The reaction mixture was stirred for 5 hours under hydrogen atmosphere, filtered through diatomite, and the filter cake was washed twice with a mixed solvent of dichloromethane (10 mL) and methanol (1 mL). The filtrate was concentrated under reduced pressure, and the crude product was purified by prep-HPLC to obtain compound D2 (290.0 mg, yield: 47%).

¹H-NMR (400MHz, DMSO-*d*₆): δ 10.97 (s, 1H), 10.01 (s, 1H), 7.93 (d, *J* = 12Hz, 2H), 7.86 (d, *J* = 12 Hz, 2H), 7.76 (d, *J* = 8 Hz,2H), 7.62 (d, *J* = 8 Hz, 1H), 7.52 (d, *J* = 8 Hz, 2H), 7.28(d, *J* = 1.6 Hz, 2H), 7.06 - 7.14 (m, 3H), 6.78 - 6.83 (d, *J=* 4 Hz, 2H), 6.65 (d, *J=* 4 Hz, 2H), 5.08 (dd, *J* = 8 Hz, 1H), 4.25 (d, *J* = 16 Hz, 1H), 4.33 (d, *J* = 16 Hz, 1H), 3.99 (m, 2H), 3.27 (m, 2H), 3.24 (m, 3H), 2.88 - 2.97 (m, 1H), 2.68 - 2.71 (m, 2H), 2.62 - 2.65 (m, 6H), 2.37 - 2.41 (m, 1H), 1.95 - 2.01(m, 1H).

MS calculated for 760.26; found 761.2 [M+H]⁺.

### Example 3: Synthesis of 3-(5-(4-(2-(4-((6-methoxy-2-(4-(methylsulfonyl)phenyl)naphthalen-1-yl)oxy)phenoxy)ethyl)piperazin-1-yl)-1-oxoisoindolin-2-yl)piperidine-2,6-dione (compound D3)

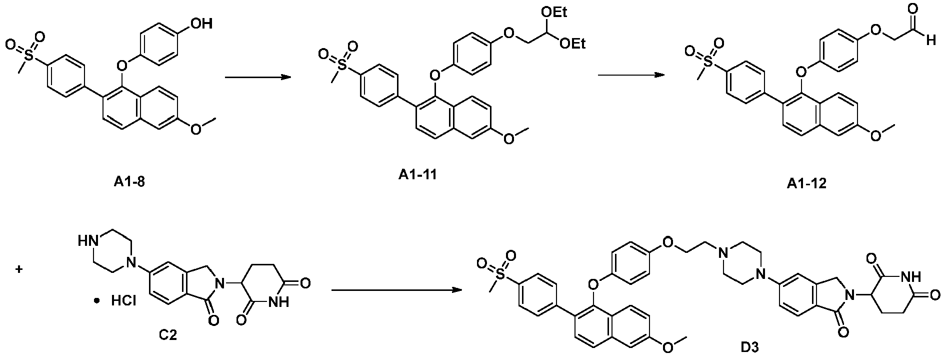

4-((6-Methoxy-2-(4-(methylsulfonyl)phenyl)naphthalen-1-yl)oxy)phenol (compound A1-8) (3.0 g, 7.14 mmol), bromoacetaldehyde diethyl acetal (14.1 g, 71.4 mmol), and potassium carbonate (1.97 g, 14.3 mmol) were sequentially added to acetonitrile (50.0 mL) at room temperature. The resulting mixture was stirred at 65°C for 10 hours, cooled to room temperature, diluted with water (100.0 mL), and extracted twice with dichloromethane (100.0 mL). The organic phases were combined, dried over anhydrous sodium sulfate, filtered, and concentrated under reduced pressure. The crude product was purified by silica gel column chromatography (dichloromethane/petroleum ether = 3/1) to obtain 1-(4-(2,2-diethoxyethoxy)phenoxy)-6-methoxy-2-(4-(methylsulfonyl)phenyl)naphthalene (compound A1-11) (2.3 g, yield: 61%).

p-Toluenesulfonic acid (200.0 mg, 1.2 mmol) was added to a solution of compound A1-11 (370.0 mg, 0.69 mmol) in tetrahydrofuran (10.0 mL) at room temperature. The resulting mixture was stirred at 70°C for 2 hours, cooled to room temperature, diluted with dichloromethane (30.0 mL), then washed with water (30.0 mL), dried over anhydrous sodium sulfate, filtered, and concentrated to dryness under reduced pressure to obtain a crude product of 2-(4-((6-methoxy-2-(4-(methylsulfonyl)phenyl)naphthalen-1-yl)oxy)phenoxy)acetaldehyde (compound A1-12) (200.0 mg, yield: 62%).

The crude product of compound A1-12 (200.0 mg, 0.43 mmol), compound C2 (80.0 mg, 0.22 mmol), and potassium acetate (172.0 mg, 1.72 mmol) were sequentially added to a mixed solvent of dichloromethane (5.0 mL) and methanol (5.0 mL) at room temperature. The resulting mixture was stirred at room temperature for 40 minutes, then added with sodium triacetoxyborohydride (47.5 mg, 0.22 mmol), stirred for another 18 hours, and diluted with water (20.0 mL). The organic phase was separated, and the aqueous phase was extracted twice with dichloromethane (15.0 mL). The organic phases were combined, dried over anhydrous sodium sulfate, filtered, and the filtrate was concentrated under reduced pressure. The crude product was purified by prep-HPLC to obtain compound D3 (100.0 mg, yield: 59%).

¹H NMR (400 MHz, DMSO) δ 10.98 (s, 1H), 7.97 - 7.87 (m, 6H), 7.79 (d, *J* = 9.2 Hz, 1H), 7.70 (d, *J* = 8.6 Hz, 1H), 7.60 - 7.50 (m, 2H), 7.26 - 7.18 (m, 1H), 7.12 (s, 2H), 6.82 (d, *J* = 7.9 Hz, 2H), 6.65 (d, *J* = 8.5 Hz, 2H), 5.08 (dd, *J* = 13.4, 5.0 Hz, 1H), 4.36 (d, *J* = 16.4 Hz, 1H), 4.23 (d, *J* = 17.0 Hz, 1H), 3.93 (s, 3H), 3.26 (s, 5H), 2.93 (t, *J* = 12.9 Hz, 2H), 2.61 (d, *J* = 16.4 Hz, 2H), 2.44 - 2.32 (m, 1H), 1.99 (d, *J* = 6.0 Hz, 1H), 1.46 - 1.14 (m, 3H).

MS calculated for 774.27; found 388.3 [M/2+H]⁺.

### Example 4: Synthesis of 3-(5-(3-(ethyl(2-(4-((6-hydroxy-2-(4-(methylsulfonyl)phenyl)naphthalen-1-yl)oxy)phenoxy)ethyl)amino)propyl)-1-oxoisoindolin-2-yl)piperidine-2,6-dione (compound D4)

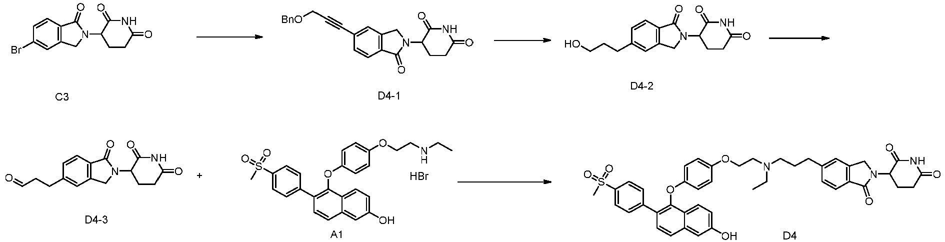

3-(5-Bromo-1-oxoisoindolin-2-yl)piperidine-2,6-dione (compound C3) (400.0 mg, 1.24 mmol), benzyl propargyl ether (362.0 mg, 2.48 mmol), cuprous iodide (23.6 mg, 0.124 mmol), bis(triphenylphosphine)palladium(II) chloride (87.0 mg, 0.124 mmol), and triethylamine (2.5 g, 2.48 mmol) were sequentially added to DMF (15.0 mL) at room temperature. The resulting mixture was stirred at 80°C for 2 hours, cooled to room temperature, and concentrated under reduced pressure to remove the organic solvent. The crude product was purified by silica gel column chromatography (dichloromethane/methanol = 10/1) to obtain 3-(5-(3-(benzyloxy)prop-1-yn-1-yl)-1-oxoisoindolin-2-yl)piperidine-2,6-dione (compound D4-1) (650.0 mg, yield: 100%).

Wet palladium on carbon (200 mg, 10 wt%) was added to a solution of compound D4-1 (600.0 mg, 1.54 mmol) in tetrahydrofuran (15.0 mL) at room temperature. The reaction mixture was stirred for 10 hours under hydrogen atmosphere, filtered through diatomite, and the filter cake was washed twice with a mixed solvent of dichloromethane (10 mL) and methanol (0.5 mL). The filtrate was concentrated to dryness under reduced pressure to obtain a crude product of 3-(5-(3-hydroxypropyl)-1-oxoisoindolin-2-yl)piperidine-2,6-dione (compound D4-2) (350.0 mg, yield: 75%).

Dess-Martin periodinane (982.8 mg, 2.32 mmol) was added to a solution of the crude product of compound D4-2 (350.0 mg, 1.16 mmol) in dichloromethane (15.0 mL) at room temperature. The reaction mixture was stirred at room temperature for 18 hours, diluted with dichloromethane (20.0 mL), then washed with saturated sodium bicarbonate aqueous solution (30.0 mL), dried over anhydrous sodium sulfate, filtered, and the filtrate was concentrated to dryness under reduced pressure to obtain a crude product of 3-(2-(2,6-dioxopiperidin-3-yl)-1-oxoisoindolin-5-yl)propanal (compound D4-3) (180.0 mg, crude yield: 52%).

The crude product of compound D4-3 (180.0 mg, 0.6 mmol), compound A1 (335.1 mg, 0.6 mmol), and potassium acetate (240.0 mg, 2.4 mmol) were sequentially added to a mixed solvent of dichloromethane (15.0 mL) and methanol (5.0 mL) at room temperature. The resulting mixture was stirred at room temperature for 30 minutes, then added with sodium triacetoxyborohydride (250.0 mg, 1.2 mmol), stirred for another 18 hours, and diluted with water (50.0 mL) and dichloromethane (50.0 mL). The organic phase was separated, and the aqueous phase was extracted twice with dichloromethane (20.0 mL). The organic phases were combined, dried over anhydrous sodium sulfate, filtered, and the filtrate was concentrated under reduced pressure. The crude product was purified by prep-HPLC to obtain compound D4 (47.0 mg, yield: 10%).

¹H NMR (400 MHz, DMSO) δ 11.02 (s, 1H), 10.11 (s, 1H), 7.89 (dd, *J* = 27.2, 8.3 Hz, 4H), 7.76 (dd, *J* = 17.5, 8.9 Hz, 2H), 7.61 (t, *J* = 7.3 Hz, 2H), 7.43 (s, 1H), 7.34 (d, *J* = 7.8 Hz, 1H), 7.28 (d, *J* = 1.6 Hz, 1H), 7.15 - 7.08 (m, 1H), 6.74 (d, *J* = 9.0 Hz, 2H), 6.62 (d, *J* = 9.0 Hz, 2H), 5.12 (dd, *J* = 13.2, 4.9 Hz, 1H), 4.35 (dd, *J* = 50.8, 17.3 Hz, 2H), 3.87 (t, *J* = 5.4 Hz, 2H), 3.24 (s, 3H), 3.00 - 2.87 (m, 1H), 2.76 - 2.57 (m, 5H), 2.49 - 2.35 (m, 3H), 2.04 - 1.96 (m, 1H), 1.78 - 1.68 (m, 2H), 1.25 (s, 1H), 0.94 (t, *J* = 7.0 Hz, 3H).

MS calculated for 761.28; found 381.6 [M/2+H]⁺.

### Example 5: Synthesis of 3-(5-(4-((1-(2-(4-((6-hydroxy-2-(4-(methylsulfonyl)phenyl)naphthalen-1-yl)oxy)phenoxy)ethyl)-1H-1,2,3-triazol-4-yl)methyl)piperazin-1-yl)-1-oxoisoindolin-2-yl)piperidine-2,6-dione (compound D5)

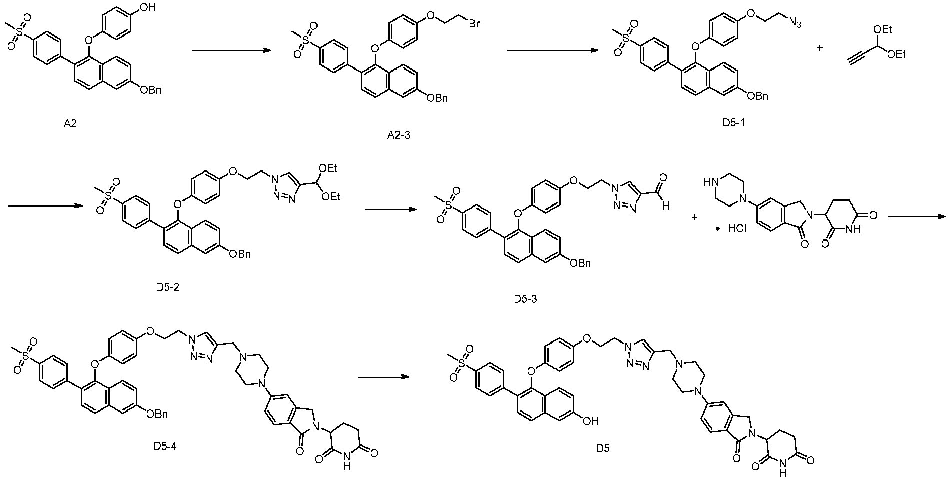

Cesium carbonate (1.97 g, 2.0 mmol) and 1,2-dibromoethane (1.88 g, 10.0 mmol) were sequentially added to a solution of compound A2 (1.0 g, 2.0 mmol) in acetonitrile (10.0 mL) at room temperature. The resulting mixture was stirred at 80°C for 18 hours, cooled to room temperature, diluted with water (50.0 mL), and then extracted three times with ethyl acetate (50.0 mL). The organic phases were combined, washed with saturated brine (40.0 mL), dried over anhydrous sodium sulfate, filtered, and concentrated under reduced pressure. The crude product was purified by silica gel column chromatography (dichloromethane/petroleum ether = 1/1) to obtain 6-(benzyloxy)-1-(4-(2-bromoethoxy)phenoxy)-2-(4-(methylsulfonyl)phenyl)naphthalene (compound A2-3) (450.0 mg, yield: 38%).

Azidotrimethylsilane (128.9 mg, 1.13 mmol) and a solution of tetra-n-butylammonium fluoride in tetrahydrofuran (1.0 M, 1.12 mL, 1.12 mmol) were sequentially added to a solution of compound A2-3 (450.0 mg, 0.75 mmol) in DMF (5.0 mL) at room temperature. The resulting mixture was stirred at room temperature for 18 hours, diluted with water (20.0 mL), and then extracted three times with ethyl acetate (20.0 mL). The organic phases were combined, washed with saturated brine (20.0 mL), dried over anhydrous sodium sulfate, filtered, and concentrated under reduced pressure. The crude product was purified by silica gel column chromatography (dichloromethane/petroleum ether = 2/1) to obtain 1-(4-(2-azidoethoxy)phenoxy)-6-(benzyloxy)-2-(4-(methylsulfonyl)phenyl)naphthalene (compound D5-1) (400.0 mg, yield: 95%).

3,3-Diethoxy-1-propyne (170.0 mg, 1.42 mmol), cuprous iodide (253.0 mg, 1.42 mmol), and triethylamine (200.0 mg, 2.13 mmol) were sequentially added to a solution of compound D5-1 (400.0 mg, 0.71 mmol) in tetrahydrofuran (5.0 mL) at room temperature. The resulting mixture was stirred at room temperature for 3 hours, diluted with water (20.0 mL), and then extracted three times with ethyl acetate (20.0 mL). The organic phases were combined, washed with saturated brine (20.0 mL), dried over anhydrous sodium sulfate, filtered, and concentrated under reduced pressure. The crude product was purified by silica gel column chromatography (dichloromethane/ethyl acetate = 20/1) to obtain 1-(2-(4-((6-(benzyloxy)-2-(4-(methylsulfonyl)phenyl)naphthalen-1-yl)oxy)phenoxy)ethyl)-4-(diethoxymethyl)-1*H*-1,2,3-triazole (compound D5-2) (340.0 mg, yield: 70%).

Hydrochloric acid aqueous solution (1.0 M, 3.0 mL, 3 mmol) was added dropwise to a solution of compound D5-2 (340.0 mg, 0.49 mmol) in tetrahydrofuran (3.0 mL) at room temperature. The reaction mixture was stirred at 50°C for 1 hour, cooled to room temperature, diluted with water (20.0 mL), and then extracted three times with ethyl acetate (20.0 mL). The organic phases were combined, washed with saturated brine (20.0 mL), dried over anhydrous sodium sulfate, filtered, and concentrated to dryness under reduced pressure to obtain a crude product of 1-(2-(4-((6-(benzyloxy)-2-(4-(methylsulfonyl)phenyl)naphthalen-1-yl)oxy)phenoxy)ethyl)-1*H*-1,2,3-triazole-4-carbaldehyde (compound D5-3) (340.0 mg, yield > 100%).

The crude product of compound D5-3 (170.0 mg, 0.27 mmol), 3-(1-oxo-5-(piperazin-1-yl)isoindolin-2-yl)piperidine-2,6-dione hydrochloride (100.0 mg, 0.27 mmol), and potassium acetate (80.9 mg, 0.81 mmol) were sequentially added to a mixed solvent of dichloromethane (1.0 mL) and methanol (1.0 mL) at room temperature. The resulting mixture was stirred at room temperature for 30 minutes, then added with sodium triacetoxyborohydride (114.0 mg, 0.54 mmol), stirred for another 3 hours, and diluted with water (10.0 mL) and dichloromethane (10.0 mL). The organic phase was separated, and the aqueous phase was extracted three times with dichloromethane (10.0 mL). The organic phases were combined, dried over anhydrous sodium sulfate, filtered, and the filtrate was concentrated under reduced pressure. The crude product was purified by silica gel column chromatography (dichloromethane/methanol = 20/1) to obtain 3-(5-(4-((1-(2-(4-((6-(benzyloxy)-2-(4-(methylsulfonyl)phenyl)naphthalen-1-yl)oxy)phenoxy)ethyl)-1H-1,2,3-triazol-4-yl)methyl)piperazin-1-yl)-1-oxoisoindolin-2-yl)piperidine-2,6-dione (compound D5-4) (160.0 mg, yield: 62%).

Wet palladium on carbon (20 mg, 10 wt%) was added to a solution of compound D5-4 (160.0 mg, 0.17 mmol) in tetrahydrofuran (5.0 mL) at room temperature. The reaction mixture was stirred for 3 hours under hydrogen atmosphere, filtered through diatomite, and the filter cake was washed twice with a mixed solvent of dichloromethane (10 mL) and methanol (1 mL). The filtrate was concentrated to dryness under reduced pressure, and the crude product was purified by prep-HPLC to obtain compound D5 (11.7 mg, yield: 8%).

¹H NMR (400 MHz, DMSO) δ 10.97 (s, 1H), 8.05 (s, 1H), 7.91 (t, *J* = 10.1 Hz, 3H), 7.85 (t, *J* = 8.3 Hz, 2H), 7.77 (d, *J* = 9.3 Hz, 1H), 7.68 (d, *J* = 8.6 Hz, 1H), 7.61 (d, *J* = 2.0 Hz, 1H), 7.53 (t, *J* = 6.2 Hz, 3H), 7.43 (t, *J* = 7.7 Hz, 2H), 7.38 (t, *J* = 7.0 Hz, 1H), 7.31 - 7.22 (m, 1H), 7.04 (d, *J* = 8.7 Hz, 2H), 6.75 (d, *J* = 9.1 Hz, 2H), 6.62 (d, *J* = 9.0 Hz, 2H), 5.27 (s, 2H), 5.07 (dd, *J* = 13.0, 4.9 Hz, 1H), 4.71 - 4.65 (m, 2H), 4.39 - 4.17 (m, 4H), 3.61 (s, 2H), 3.29 - 3.24 (m, 4H), 3.24 (s, 3H).

MS calculated for 841.29; found 842.3 [M+H]⁺.

### Example 6: Synthesis of 2-(2,6-dioxopiperidin-3-yl)-5-(4-(2-(4-((6-hydroxy-2-(4-(methylsulfonyl)phenyl)naphthalen-1-yl)oxy)phenoxy)ethyl)piperazin-1-yl)isoindoline-1,3-dione (compound D6)

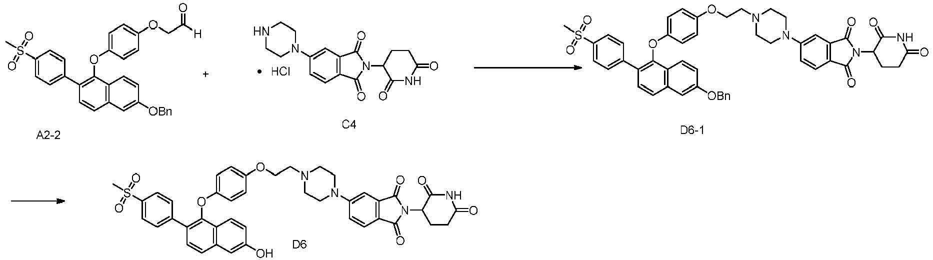

The crude product of compound A2-2 (200.0 mg, 0.37 mmol), 2-(2,6-dioxopiperidin-3-yl)-5-(piperazin-1-yl)isoindoline-1,3-dione hydrochloride (compound C4) (140.0 mg, 0.37 mmol), and potassium acetate (74.0 mg, 0.74 mmol) were sequentially added to a mixed solvent of dichloromethane (10.0 mL) and methanol (2.0 mL) at room temperature. The resulting mixture was stirred at room temperature for 30 minutes, then added with sodium triacetoxyborohydride (156.0 mg, 0.74 mmol), stirred for another 18 hours, and diluted with water (20.0 mL) and dichloromethane (20.0 mL). The organic phase was separated, and the aqueous phase was extracted twice with dichloromethane (10.0 mL). The organic phases were combined, dried over anhydrous sodium sulfate, filtered, and concentrated under reduced pressure. The crude product was purified by silica gel column chromatography (dichloromethane/methanol = 10/1) to obtain 3-(5-(4-(2-(4-((6-(benzyloxy)-2-(4-(methylsulfonyl)phenyl)naphthalen-1-yl)oxy)phenoxy)ethyl)piperazin-1-yl)-1,3-dioxoisoindolin-2-yl)piperidine-2,4,6-trione (compound D6-1) (100.0 mg, yield: 30%).

Wet palladium on carbon (100 mg, 10 wt%) was added to a solution of compound D6-1 (100.0 mg, 0.12 mmol) in tetrahydrofuran (10.0 mL) at room temperature. The reaction mixture was stirred for 5 hours under hydrogen atmosphere, filtered through diatomite, and the filter cake was washed twice with a mixed solvent of dichloromethane (10 mL) and methanol (0.5 mL). The filtrate was concentrated under reduced pressure, and the crude product was purified by prep-HPLC to obtain compound D6 (34.0 mg, yield: 36%).

¹H NMR (400 MHz, DMSO) δ 11.15 (s, 1H), 10.14 (s, 1H), 7.92 (dd, *J* = 19.3, 8.5 Hz, 4H), 7.84 - 7.72 (m, 3H), 7.64 (d, *J* = 8.6 Hz, 1H), 7.51 (s, 1H), 7.37 (d, *J* = 8.8 Hz, 1H), 7.30 (s, 1H), 7.14 (d, *J* = 7.5 Hz, 1H), 6.86 (d, *J* = 8.8 Hz, 2H), 6.69 (d, *J* = 8.8 Hz, 2H), 5.13 (dd, *J* = 13.0, 5.4 Hz, 1H), 4.24 (s, 4H), 3.61 (d, *J* = 33.0 Hz, 4H), 3.27 (s, 6H), 2.92 (s, 1H), 2.76 (s, 1H), 2.62 (d, *J* = 17.8 Hz, 1H), 2.07 (s, 1H).

MS calculated for 774.24; found 775.2 [M+H]⁺.

### Example 7: Synthesis of 3-(5-(4-(N-ethyl-N-(2-(4-((6-hydroxy-2-(4-(methylsulfonyl)phenyl)naphthalen-1-yl)oxy)phenoxy)ethyl)glycyl)piperazin-1-yl)-1-oxoisoindolin-2-yl)piperidine-2,6-dione (compound D7)

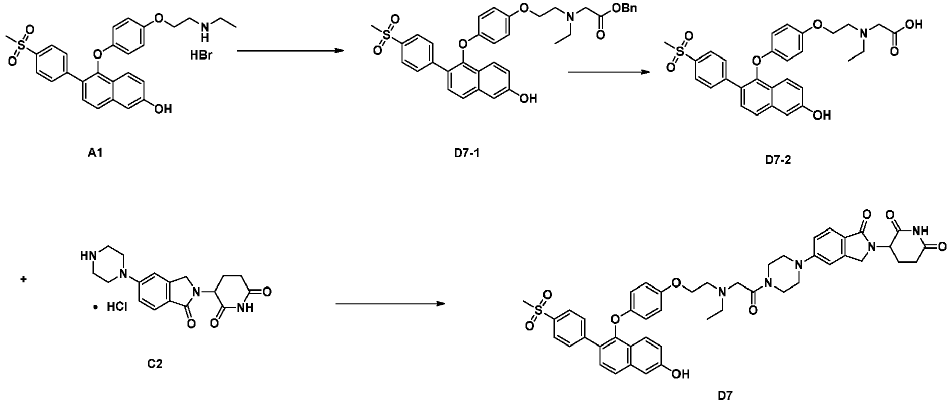

Compound A1 (500.0 mg, 1.05 mmol), benzyl 2-bromoacetate (626.0 mg, 2.10 mmol), and triethylamine (319.0 mg, 3.15 mmol) were sequentially added to DMF (15.0 mL) at room temperature. The resulting mixture was stirred at 80°C for 3 hours, cooled to room temperature, and concentrated under reduced pressure to remove the organic solvent. The crude product was purified by silica gel column chromatography (dichloromethane/methanol = 10/1) to obtain benzyl N-ethyl-N-(2-(4-((6-hydroxy-2-(4-(methylsulfonyl)phenyl)naphthalen-1-yl)oxy)phenoxy)ethyl)glycinate (compound D7-1) (300.0 mg, yield: 46%).

Wet palladium on carbon (160 mg, 10 wt%) was added to a solution of compound D7-1 (200.0 mg, 0.32 mmol) in tetrahydrofuran (10.0 mL) at room temperature. The reaction mixture was stirred for 6 hours under hydrogen atmosphere, filtered through diatomite, and the filter cake was washed twice with a mixed solvent of dichloromethane (10 mL) and methanol (0.5 mL). The filtrate was concentrated to dryness under reduced pressure to obtain a crude product of N-ethyl-N-(2-(4-((6-hydroxy-2-(4-(methylsulfonyl)phenyl)naphthalen-1-yl)oxy)phenoxy)ethyl)glycine (compound D7-2) (160.0 mg, crude yield: 93%).

The crude product of compound D7-2 (50.0 mg, 0.093 mmol) and HATU (56.0 mg, 0.14 mmol) were sequentially added to DMF (3.0 mL) at room temperature. The resulting mixture was stirred at room temperature for 30 minutes, and then sequentially added with compound C2 (51.0 mg, 0.14 mmol) and DIPEA (60.0 mg, 0.47 mmol). The reaction mixture was stirred at room temperature for 1 hour, and concentrated under reduced pressure to remove the organic solvent. The crude product was purified by prep-HPLC to obtain compound D7 (15.0 mg, yield: 19%).

¹H NMR (400 MHz, DMSO) δ 10.99 (s, 1H), 10.09 (s, 1H), 7.92 (d, *J* = 7.7 Hz, 2H), 7.85 (d, *J* = 7.8 Hz, 2H), 7.79 (d, *J* = 8.5 Hz, 1H), 7.73 (d, *J* = 9.2 Hz, 1H), 7.60 (d, *J* = 8.5 Hz, 1H), 7.55 (d, *J* = 8.3 Hz, 1H), 7.27 (s, 1H), 7.11 (d, *J* = 9.2 Hz, 1H), 7.05 (d, *J* = 7.6 Hz, 2H), 6.74 (d, *J* = 8.5 Hz, 2H), 6.60 (d, *J* = 8.2 Hz, 2H), 5.08 (dd, *J* = 13.0, 4.2 Hz, 1H), 4.28 (dd, *J* = 46.4, 16.9 Hz, 2H), 3.93 (s, 2H), 3.68 (s, 2H), 3.55 (s, 2H), 3.29 (s, 3H), 3.23 (s, 6H), 2.94 (t, *J* = 13.0 Hz, 1H), 2.79 (d, *J* = 5.6 Hz, 2H), 2.65 - 2.58 (m, 3H), 2.46 - 2.33 (m, 1H), 2.00 (s, 1H), 0.98 (t, *J* = 6.8 Hz, 3H).

MS calculated for 845.31; found 423.9 [M/2+H]⁺.

### Example 8: Synthesis of 2-(2,6-dioxopiperidin-3-yl)-5-(4-(N-ethyl-N-(2-(4-((6-hydroxy-2-(4-(methylsulfonyl)phenyl)naphthalen-1-yl)oxy)phenoxy)ethyl)glycyl)piperazin-1-yl)isoindoline-1,3-dione (compound D8)

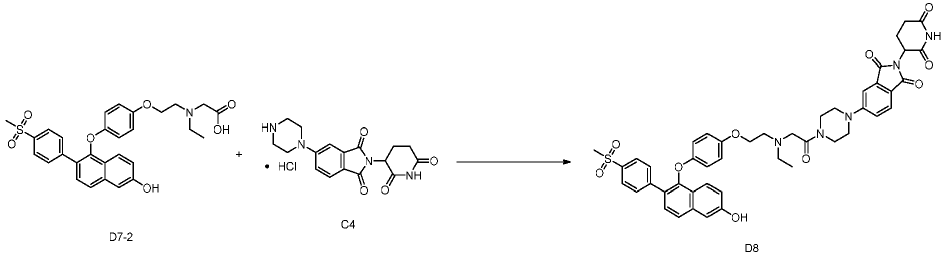

The crude product of compound D7-2 (50.0 mg, 0.093 mmol) and HATU (56.0 mg, 0.14 mmol) were sequentially added to DMF (3.0 mL) at room temperature. The resulting mixture was stirred at room temperature for 30 minutes, and then sequentially added with compound C4 (53.0 mg, 0.14 mmol) and DIPEA (60.0 mg, 0.47 mmol). The reaction mixture was stirred at room temperature for 1 hour, and concentrated under reduced pressure to remove the organic solvent. The crude product was purified by prep-HPLC to obtain compound D8 (5.0 mg, yield: 6.3%).

¹H NMR (400 MHz, DMSO) δ 11.19 - 11.08 (m, 1H), 10.24 - 10.13 (m, 1H), 7.92 (d, *J* = 8.1 Hz, 2H), 7.85 (d, *J* = 7.9 Hz, 2H), 7.80 - 7.67 (m, 2H), 7.59 (d, *J* = 8.9 Hz, 1H), 7.34 (s, 1H), 7.29 - 7.18 (m, 2H), 7.10 (d, *J* = 9.2 Hz, 1H), 6.74 (d, *J* = 8.3 Hz, 2H), 6.60 (d, *J* = 8.7 Hz, 1H), 5.35 (s, 1H), 3.92 (s, 2H), 3.69 (s, 2H), 3.55 (s, 3H), 3.45 (s, 4H), 3.32 - 3.27 (m, 2H), 3.23 (s, 3H), 2.79 (s, 2H), 2.64 - 2.60 (m, 2H), 2.09 - 1.95 (m, 4H), 0.98 (t, *J* = 6.5 Hz, 3H).

MS calculated for 859.29; found 430.3 [M/2+H]⁺.

### Example 9: Synthesis of 3-(5-(4-(2-(2-((2R,5S)-4-(2-(4-((6-hydroxy-2-(4-(methylsulfonyl)phenyl)naphthalen-1-yl)oxy)phenoxy)ethyl)-2,5-dimethylpiperazin-1-yl)ethoxy)acetyl)piperazin-1-yl)-1-oxoisoindolin-2-yl)piperidine-2,6-dione (compound D9)

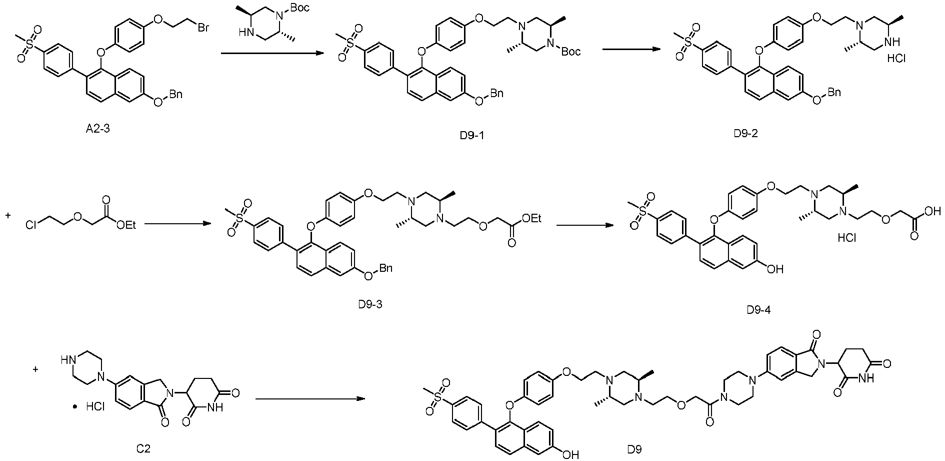

tert-Butyl (2R,SS)-2,5-dimethylpiperazine-1-carboxylate (182.6 mg, 0.85 mmol), potassium carbonate (686.0 mg, 2.13 mmol), and potassium iodide (82.5 mg, 0.21 mmol) were sequentially added to a solution of compound A2-3 (426.0 mg, 0.71 mmol) in DMF (10.0 mL) at room temperature. The reaction mixture was stirred at 80°C for 18 hours, cooled to room temperature, and diluted with water (15.0 mL). The resulting mixture was extracted three times with ethyl acetate (15.0 mL). The organic phases were combined, washed with saturated brine (20.0 mL), dried over anhydrous sodium sulfate, filtered, and the filtrate was concentrated under reduced pressure. The resulting residue was purified by silica gel column chromatography (eluent: petroleum ether/ethyl acetate = 1/1) to obtain tert-butyl (2R,5S)-4-(2-(4-((6-(benzyloxy)-2-(4-(methylsulfonyl)phenyl)naphthalen-1-yl)oxy)phenoxy)ethyl)-2, 5-dimethylpiperazine-1-carboxylate (compound D9-1) (900.0 mg, yield: 75%).

Compound D9-1 (900.0 mg, 1.2 mmol) was added to a mixed solvent of ethyl acetate (3.0 mL) and dichloromethane (3.0 mL) at room temperature, and then hydrochloric acid aqueous solution (4.0 M, 3.0 mL, 12 mmol) was added dropwise thereto. The reaction mixture was stirred at room temperature for 2 hours, and concentrated to dryness under reduced pressure to obtain a crude product of (2S,SR)-1-(2-(4-((6-(benzyloxy)-2-(4-(methylsulfonyl)phenyl)naphthalen-1-yl)oxy)phenoxy)ethyl)-2, 5-dimethylpiperazine hydrochloride (compound D9-2) (900.0 mg, yield > 100%).

Ethyl 2-(2-chloroethoxy)acetate (219.0 mg, 1.31 mmol), potassium carbonate (455.0 mg, 3.29 mmol), and potassium iodide (54.8 mg, 0.33 mmol) were sequentially added to a solution of the crude product of compound D9-2 (700.0 mg, 0.99 mmol) in DMF (10.0 mL) at room temperature. The reaction mixture was stirred at 110°C for 18 hours, cooled to room temperature, and diluted with water (30.0 mL). The resulting mixture was extracted three times with ethyl acetate (20.0 mL). The organic phases were combined, washed with saturated brine (20.0 mL), dried over anhydrous sodium sulfate, filtered, and the filtrate was concentrated under reduced pressure. The crude product was purified by silica gel column chromatography (dichloromethane/methanol = 10/1) to obtain ethyl 2-(2-((2R,5S)-4-(2-(4-((6-(benzyloxy)-2-(4-(methylsulfonyl)phenyl)naphthalen-1-yl)oxy)phenoxy)ethyl)-2,5-dimethylpiperazin-1-yl)ethoxy)acetate (compound D9-3) (500.0 mg, yield: 59%).

Wet palladium on carbon (50 mg, 10 wt%) was added to a solution of compound D9-3 (500.0 mg, 0.66 mmol) in tetrahydrofuran (5.0 mL) at room temperature. The reaction mixture was stirred for 3 hours under hydrogen atmosphere, filtered through diatomite, and the filter cake was washed twice with a mixed solvent of dichloromethane (10 mL) and methanol (0.5 mL). The filtrate was concentrated to dryness under reduced pressure, and the resulting residue was added with a mixed solvent of methanol (2.0 mL) and water (2.0 mL), and then added with lithium hydroxide monohydrate (30.0 mg, 0.71 mmol). The resulting mixture was stirred at room temperature for 3 hours, and added with hydrochloric acid aqueous solution (1.0 M) to adjust the pH of the aqueous phase to about 3 to 4. The resulting mixture was concentrated to dryness under reduced pressure to obtain a crude product of 2-(2-((2R,5S)-4-(2-(4-((6-hydroxy-2-(4-(methylsulfonyl)phenyl)naphthalen-1-yl)oxy)phenoxy)ethyl)-2,5-dimethylpiperazin-1-yl)ethoxy)acetic acid hydrochloride (compound D9-4) (400.0 mg, crude yield: 95%).

Compound C2 (158.7 mg, 0.34 mmol), HATU (125.4 mg, 0.34 mmol), and DIPEA (116.1 mg, 0.93 mmol) were sequentially added to a solution of the crude product of compound D9-4 (200.0 mg, 0.31 mmol) in DMF (2.0 mL) at room temperature. The resulting mixture was stirred at room temperature for 3 hours, diluted with water (10.0 mL), and then extracted three times with ethyl acetate (10.0 mL). The organic phases were combined, washed with saturated brine (20.0 mL), dried over anhydrous sodium sulfate, filtered, and concentrated under reduced pressure. The resulting residue was purified by silica gel column chromatography (eluent: dichloromethane/methanol = 10/1) to obtain compound D9 (20.0 mg, yield: 7%).

¹H NMR (400 MHz, DMSO-*d*₆) δ 10.99 (s, 1H), 10.11 (s, 1H), 7.93 (d, *J* = 8.4 Hz, 2H), 7.86 (d, *J* = 8.4 Hz, 2H), 7.79 (d, *J* = 8.7 Hz, 1H), 7.74 (d, *J* = 9.1 Hz, 1H), 7.61 (d, *J* = 8.6 Hz, 1H), 7.57 (d, *J* = 8.2 Hz, 1H), 7.28 (d, *J* = 2.2 Hz, 1H), 7.15 - 7.08 (m, 3H), 6.76 (d, *J* = 9.1 Hz, 2H), 6.62 (d, *J* = 9.1 Hz, 2H), 5.08 (dd, *J* = 13.3, 5.1 Hz, 1H), 4.36 (d, *J* = 17.2 Hz, 1H), 4.24 (d, *J* = 16.9 Hz, 2H), 3.91 (s, 2H), 3.62 (s, 6H), 3.33 - 3.29 (m, 2H), 3.25 (s, 3H), 3.03 - 2.86 (m, 3H), 2.74 (d, *J* = 34.8 Hz, 1H), 2.60 (d, *J* = 16.6 Hz, 2H), 2.46 - 2.34 (m, 2H), 2.02 - 1.93 (m, 2H), 1.26 (s, 1H), 0.97 (s, 5H).

MS calculated for 958.39; found 480.4 [M/2+H]⁺.

### Example 10: Synthesis of 3-(5-(4-(2-(2-((2-(4-((6-hydroxy-2-(4-(methylsulfonyl)phenyl)naphthalen-1-yl)oxy)phenoxy)ethyl)methylamino)ethoxy)ethyl)piperazin-1-yl)-1-oxoisoindolin-2-yl)piperidine-2,6-dione (compound D10)

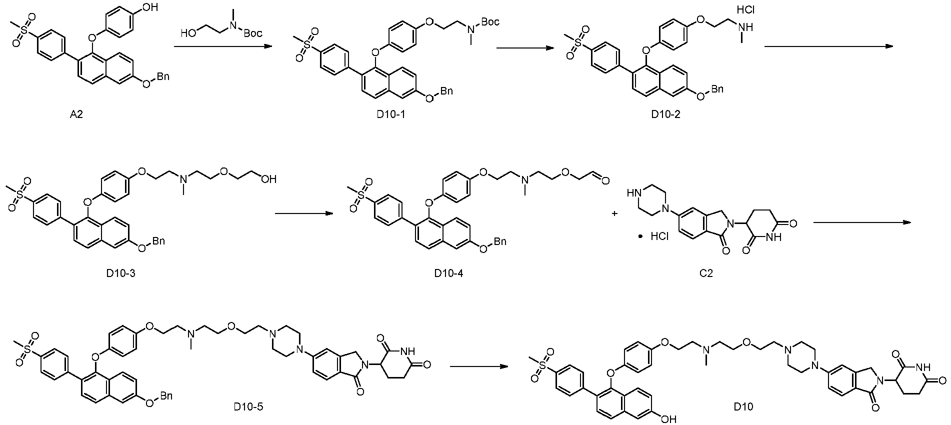

tert-Butyl (2-hydroxyethyl)(methyl)carbamate (423.4 mg, 2.4 mmol), diethyl azodicarboxylate (489.0 mg, 2.4 mmol), and triphenylphosphine (634.0 mg, 2.4 mmol) were sequentially added to a solution of compound A2 (1.0 g, 2.0 mmol) in tetrahydrofuran (10.0 mL) at room temperature. The reaction mixture was stirred at room temperature for 18 hours, and diluted with water (15.0 mL). The resulting mixture was extracted three times with ethyl acetate (15.0 mL). The organic phases were combined, washed with saturated brine (20.0 mL), dried over anhydrous sodium sulfate, filtered, and concentrated under reduced pressure. The crude product was purified by silica gel column chromatography (petroleum ether/ethyl acetate = 1/1) to obtain tert-butyl (2-(4-((6-(benzyloxy)-2-(4-(methylsulfonyl)phenyl)naphthalen-1-yl)oxy)phenoxy)ethyl)(methyl)carbamate (compound D10-1) (800.0 mg, yield: 61%).

A solution of hydrochloric acid in ethyl acetate (4.0 M, 10.0 mL, 40.0 mmol) was added dropwise to a solution of compound D10-1 (800.0 mg, 1.2 mmol) in 1,4-dioxane (10.0 mL) at room temperature. The resulting mixture was stirred at room temperature for 18 hours, and concentrated to dryness under reduced pressure to obtain a crude product of (2-(4-((6-(benzyloxy)-2-(4-(methylsulfonyl)phenyl)naphthalen-1-yl)oxy)phenoxy)-N-methylethylamine hydrochloride (compound D10-2) (800.0 mg, yield > 100%).

2-(2-Hydroxyethoxy)ethyl 4-methylbenzenesulfonate (382.0 mg, 1.48 mmol) and potassium carbonate (513.4 mg, 3.71 mmol) were sequentially added to a solution of the crude product of compound D10-2 (800.0 mg, 1.2 mmol) in DMF (10.0 mL) at room temperature. The resulting mixture was stirred at room temperature for 18 hours, and diluted with water (20.0 mL). The resulting mixture was extracted three times with ethyl acetate (20.0 mL). The organic phases were combined, washed with saturated brine (20.0 mL), dried over anhydrous sodium sulfate, filtered, and concentrated under reduced pressure. The crude product was purified by silica gel column chromatography (petroleum ether/ethyl acetate = 1/1) to obtain 2-(2-((2-(4-((6-(benzyloxy)-2-(4-(methylsulfonyl)phenyl)naphthalen-1-yl)oxy)phenoxy)ethyl)methylamino)ethoxy)ethanol (compound D10-3) (250.0 mg, yield: 25%).

A solution of dimethyl sulfoxide (121.9 mg, 1.56 mmol) in dichloromethane (2 mL) was added dropwise to a solution of oxalyl chloride (148.6 mg, 1.17 mmol) in dichloromethane (3.0 mL) at -70°C. The resulting mixture was stirred at -70°C for 30 minutes, and a solution of compound D10-3 (250.0 mg, 0.39 mmol) in dichloromethane (2.0 mL) was added thereto. The reaction mixture was stirred for 1 hour, then added with triethylamine (315.7 mg, 3.12 mmol), and stirred for another 1 hour. The resulting mixture was warmed to room temperature, stirred for 1 hour, diluted with water (10.0 mL), and extracted three times with ethyl acetate (10.0 mL). The organic phases were combined, washed with saturated brine (20.0 mL), dried over anhydrous sodium sulfate, filtered, and concentrated to dryness under reduced pressure to obtain a crude product of 2-(2-((2-(4-((6-(benzyloxy)-2-(4-(methylsulfonyl)phenyl)naphthalen-1-yl)oxy)phenoxy)ethyl)methylamino)ethoxy)acetaldehyde (compound D10-4) (200.0 mg, crude yield: 80%).

The crude product of compound D10-4 (200.0 mg, 0.31 mmol), compound C2 (112.0 mg, 0.31 mmol), and potassium acetate (60.9 mg, 0.62 mmol) were sequentially added to a mixed solvent of dichloromethane (2.0 mL) and methanol (2.0 mL) at room temperature. The resulting mixture was stirred at room temperature for 30 minutes, then added with sodium cyanoborohydride (39.0 mg, 0.62 mmol), stirred for another 2 hours, and diluted with water (10.0 mL) and dichloromethane (10.0 mL). The organic phase was separated, and the aqueous phase was extracted twice with dichloromethane (10.0 mL). The organic phases were combined, dried over anhydrous sodium sulfate, filtered, and concentrated under reduced pressure. The crude product was purified by silica gel column chromatography (dichloromethane/methanol = 10/1) to obtain 3-(5-(4-(2-(2-((2-(4-((6-(benzyloxy)-2-(4-(methylsulfonyl)phenyl)naphthalen-1-yl)oxy)phenoxy)ethyl)methylamino)ethoxy)ethyl)piperazin-1-yl)-1-oxoisoindolin-2-yl)piperidine-2,6-dione (compound D10-5) (80.0 mg, yield: 22%).

Wet palladium on carbon (20 mg, 10 wt%) was added to a solution of compound D10-5 (80.0 mg, 0.084 mmol) in methanol (3.0 mL) at room temperature. The reaction mixture was stirred for 3 hours under hydrogen atmosphere, filtered through diatomite, and the filter cake was washed twice with a mixed solvent of dichloromethane (10 mL) and methanol (0.5 mL). The filtrate was concentrated to dryness under reduced pressure, and the crude product was purified by silica gel column chromatography (dichloromethane/methanol = 10/1) to obtain compound D10 (14.4 mg, yield: 20%).

¹H NMR (400 MHz, DMSO-*d*₆) δ 10.97 (s, 1H), 7.95 - 7.82 (m, 4H), 7.78 (d, *J* = 8.7 Hz, 1H), 7.74 (d, *J* = 9.0 Hz, 1H), 7.60 (d, *J* = 8.5 Hz, 1H), 7.54 (d, *J* = 8.3 Hz, 1H), 7.29 (d, *J* = 2.1 Hz, 1H), 7.12 (dd, *J* = 9.2, 2.3 Hz, 1H), 7.05 (d, *J* = 8.9 Hz, 2H), 6.78 (d, *J* = 9.0 Hz, 2H), 6.62 (d, *J* = 9.1 Hz, 2H), 5.07 (dd, *J* = 13.1, 4.9 Hz, 1H), 4.39 - 4.19 (m, 1H), 3.98 (s, 1H), 3.55 (s, 5H), 3.26 (d, *J* = 18.0 Hz, 9H), 2.97 - 2.87 (m, 2H), 2.76 (s, 1H), 2.59 (s, 6H), 2.45 - 2.33 (m, 3H), 1.99 (s, 1H), 1.94 (s, 2H), 1.40 - 1.18 (m, 4H).

MS calculated for 861.34; found 862.3 [M+H]⁺.

### Example 11: Synthesis of 3-(5-(4-(2-((5-(4-(4-((6-hydroxy-2-(4-(methylsulfonyl)phenyl)naphthalen-1-yl)oxy)phenyl)piperazin-1-yl)pentyl)oxy)acetyl)piperazin-1-yl)-1-oxoisoindolin-2-yl)piperidine-2,6-dione (compound D11)

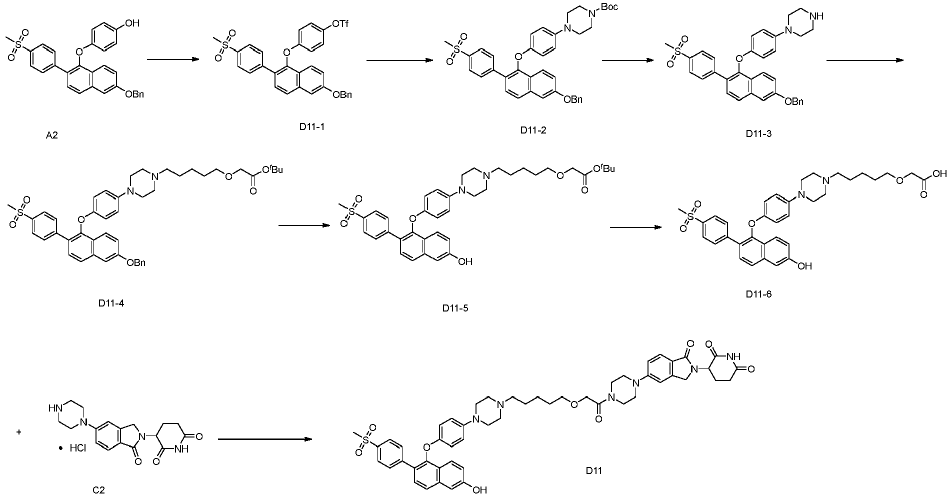

Pyridine (0.32 mL, 4.0 mmol) and trifluoromethanesulfonic anhydride (0.4 mL, 2.4 mmol) were added to a solution of compound A2 (0.993 g, 2.0 mmol) in dichloromethane (20.0 mL) at 0°C. The reaction mixture was warmed to room temperature, stirred for 2 hours, diluted with dichloromethane (30.0 mL), sequentially washed with hydrochloric acid aqueous solution (0.1 M, 40.0 mL), saturated sodium bicarbonate aqueous solution (20.0 mL), saturated brine (20.0 mL), and water (20.0 mL), then dried over anhydrous sodium sulfate, filtered, and concentrated to dryness under reduced pressure to obtain a crude product of 4-((6-(benzyloxy)-2-(4-(methylsulfonyl)phenyl)naphthalen-1-yl)oxy)phenyl trifluoromethanesulfonate (compound D11-1) (1.26 g, yield: 100%).

The crude product of compound D11-1 (629.0 mg, 1.0 mmol), tert-butyl piperazine-1-carboxylate (279.0 mg, 1.5 mmol), palladium(II) acetate (34.0 mg, 0.15 mmol), 2,2'-bis(diphenylphosphino)-1,1'-binaphthyl (187.0 mg, 0.3 mmol), and cesium carbonate (652.0 mg, 2.0 mmol) were sequentially added to toluene (10.0 mL) at room temperature. The resulting mixture was stirred at 100°C for 18 hours, cooled to room temperature, and concentrated under reduced pressure to remove the organic solvent. The crude product was purified by silica gel column chromatography (petroleum ether/ethyl acetate = 2/1) to obtain tert-butyl 4-(4-((6-(benzyloxy)-2-(4-(methylsulfonyl)phenyl)naphthalen-1-yl)oxy)phenyl)piperazine-1-carboxylate (compound D11-2) (275.0 mg, yield: 41%).

Trifluoroacetic acid (2.0 mL) was added to a solution of compound D11-2 (275.0 mg, 0.41 mmol) in dichloromethane (5.0 mL) at room temperature. The reaction mixture was stirred at room temperature for 4 hours, and concentrated to dryness under reduced pressure. The resulting residue was added with dichloromethane (5.0 mL), and added with saturated potassium carbonate aqueous solution to adjust the pH of the aqueous phase to about 7 to 8. The resulting mixture was filtered under reduced pressure, and the filter cake was washed twice with dichloromethane (10.0 mL), and the filtrate was concentrated to dryness under reduced pressure to obtain 1-(4-((6-(benzyloxy)-2-(4-(methylsulfonyl)phenyl)naphthalen-1-yl)phenyl)piperazine (compound D11-3) (280.0 mg, yield: 100%).

Compound D11-3 (280.0 mg, 0.41 mmol), tert-butyl 2-((5-tosyloxy)pentyl)oxy)acetate (153 mg, 0.41 mmol) (compound B1), and potassium carbonate (113 mg, 0.82 mmol) were sequentially added to DMF (2.0 mL) at room temperature. The resulting mixture was stirred at 80°C for 30 hours, cooled to room temperature, and concentrated to dryness under reduced pressure. The crude product was purified by silica gel column chromatography (dichloromethane/methanol = 20/1) to obtain tert-butyl 2-((5-(4-(4-((6-(benzyloxy)-2-(4-(methylsulfonyl)phenyl)naphthalen-1-yl)oxy)phenyl)piperazin-1-yl)pentyl)oxy)acetate (compound D11-4) (210.0 mg, yield: 67%).

Wet palladium on carbon (100 mg, 10 wt%) was added to a solution of compound D11-4 (210.0 mg, 0.27 mmol) in methanol (5.0 mL) and tetrahydrofuran (2.0 mL) at room temperature. The reaction mixture was stirred for 18 hours under hydrogen atmosphere, filtered through diatomite, and the filter cake was washed twice with a mixed solvent of dichloromethane (10 mL) and methanol (0.5 mL). The filtrate was concentrated to dryness under reduced pressure to obtain a crude product of tert-butyl 2-((5-(4-(4-((6-hydroxy-2-(4-(methylsulfonyl)phenyl)naphthalen-1-yl)oxy)phenyl)piperazin-1-yl)pentyl)oxy)acetate (compound D11-5) (163.0 mg, yield: 89%).

Trifluoroacetic acid (1.0 mL) was added to a solution of the crude product of compound D11-5 (163.0 mg, 0.24 mmol) in dichloromethane (5.0 mL) at room temperature. The resulting mixture was stirred at room temperature for 4 hours, and concentrated to dryness under reduced pressure to obtain a crude product of 2-((5-(4-(4-((6-hydroxy-2-(4-(methylsulfonyl)phenyl)naphthalen-1-yl)oxy)phenyl)piperazin-1-yl)pentyl)oxy)acetic acid (compound D11-6) (230.0 mg, yield > 100%).

The crude product of compound D11-6 (120.0 mg, 0.09 mmol) and compound C2 (36.0 mg, 0.10 mmol) were added to DMF (1.0 mL) at room temperature, and then DIPEA (46.0 mg, 0.36 mmol) and HATU (34.0 mg, 0.09 mmol) were sequentially added thereto. The resulting mixture was stirred at room temperature for 1 hour, and concentrated under reduced pressure to remove the organic solvent. The resulting residue was added with dichloromethane (20.0 mL), washed twice with water (10.0 mL), dried over anhydrous sodium sulfate, filtered, and the filtrate was concentrated under reduced pressure. The crude product was purified by prep-HPLC to obtain compound D11 (25.0 mg, yield: 30%).

¹H NMR (400 MHz, DMSO-*d*₆) δ 10.95 (s, 1H), 10.05 (s, 1H), 7.91 (d, *J* = 8.6 Hz, 2H), 7.85 (d, *J* = 8.6 Hz, 2H), 7.77 (d, *J* = 8.7 Hz, 1H), 7.72 (d, *J* = 9.1 Hz, 1H), 7.59 (d, *J* = 8.6 Hz, 1H), 7.55 (d, *J* = 8.3 Hz, 1H), 7.25 (d, *J* = 2.3 Hz, 1H), 7.11 - 7.05 (m, 3H), 6.88 - 6.70 (m, 2H), 6.59 (d, *J* = 8.5 Hz, 2H), 5.05 (dd, *J* = 13.2, 5.1 Hz, 1H), 4.36 - 4.31 (m, 1H), 4.24 - 4.18 (m, 1H), 4.18 - 4.14 (m, 2H), 3.65 - 3.53 (m, 8H), 3.48 - 3.40 (m, 4H), 3.22 (s, 3H), 3.17 - 3.04 (m, 3H), 2.95 - 2.78 (m, 4H), 2.62 - 2.57 (m, 1H), 2.41 - 2.33 (m, 1H), 2.01 - 1.92 (m, 1H), 1.57 - 1.51 (m, 2H), 1.37 - 1.32 (m, 2H), 1.29 - 1.27 (m, 2H).

MS calculated for 928.38; found 465.4 [M/2+H]⁺.

### Example 12: Synthesis of 3-(5-(4-(2-(4-(4-((6-hydroxy-2-(4-(methylsulfonyl)phenyl)naphthalen-1-yl)oxy)phenyl)piperazin-1-yl)acetyl)piperazin-1-yl)-1-oxoisoindolin-2-yl)piperidine-2,6-dione (compound D12)

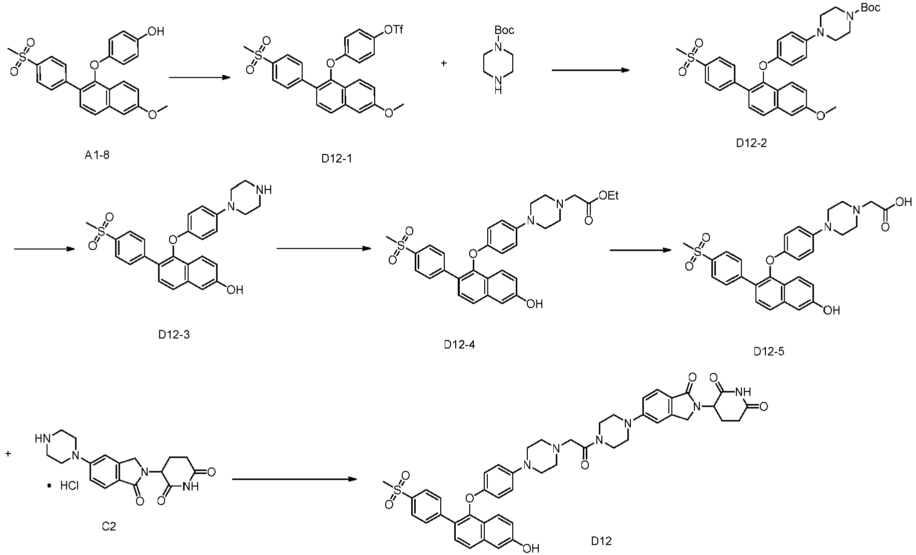

Pyridine (0.16 mL, 2.0 mmol) and trifluoromethanesulfonic anhydride (0.2 mL, 1.2 mmol) were added to a solution of compound A1-8 (420.0 mg, 1.0 mmol) in dichloromethane (10.0 mL) at 0°C. The reaction mixture was warmed to room temperature, stirred for 2 hours, diluted with dichloromethane (30.0 mL), sequentially washed with hydrochloric acid aqueous solution (0.1 M, 40.0 mL), saturated sodium bicarbonate aqueous solution (20.0 mL), saturated brine (20.0 mL), and water (20.0 mL), then dried over anhydrous sodium sulfate, filtered, and the filtrate was concentrated to dryness under reduced pressure to obtain a crude product of 4-((6-(methoxy)-2-(4-(methylsulfonyl)phenyl)naphthalen-1-yl)oxy)phenyl trifluoromethanesulfonate (compound D12-1) (540.0 mg, crude yield: 100%).

Compound D12-1 (274.0 mg, 1.47 mmol), palladium(II) acetate (33.0 mg, 0.15 mmol), 2,2'-bis(diphenylphosphino)-1,1'-binaphthyl (187.0 mg, 0.3 mmol), and cesium carbonate (639.0 mg, 1.96 mmol) were sequentially added to toluene (10.0 mL) at room temperature. The resulting mixture was stirred at 100°C for 18 hours, cooled to room temperature, and concentrated under reduced pressure to remove the organic solvent. The crude product was purified by silica gel column chromatography (petroleum ether/ethyl acetate = 2/1) to obtain (compound D12-2) (415.0 mg, yield: 72%).

A solution of boron tribromide in dichloromethane (1.0 M, 2.1 mL, 2.1 mmol) was added dropwise to a solution of compound D12-2 (415.0 mg, 0.70 mmol) in dichloromethane (10.0 mL) at 0°C. The resulting mixture was warmed to room temperature, stirred for 2 hours, then cooled to 0°C, sequentially diluted with methanol (1.0 mL) and dichloromethane (20.0 mL), and added with saturated sodium bicarbonate aqueous solution to adjust the pH of the aqueous phase to about 7 to 8. The organic phase was separated, and the aqueous phase was extracted twice with dichloromethane (10.0 mL). The organic phases were combined, dried over anhydrous sodium sulfate, filtered, and the filtrate was concentrated under reduced pressure. The crude product was purified by silica gel column chromatography (dichloromethane/methanol = 10/1) to obtain 6-(4-(methylsulfonyl)phenyl)-5-(4-(piperazin-1-yl)phenoxy)naphthalen-2-ol (compound D12-3) (200.0 mg, yield: 60%).

DIPEA (0.2 mL, 1.26 mmol) and ethyl bromoacetate (70 mg, 0.42 mmol) were sequentially added to a solution of compound D12-3 (200.0 mg, 0.42 mmol) in DMF (6.0 mL) at 0°C. The resulting mixture was warmed to room temperature, stirred for 2 hours, diluted with water (15.0 mL), and then extracted twice with ethyl acetate (20.0 mL). The organic phases were combined, dried over anhydrous sodium sulfate, filtered, and the filtrate was concentrated under reduced pressure. The crude product was purified by silica gel column chromatography (dichloromethane/methanol = 10/1) to obtain ethyl 2-(4-(4-((6-hydroxy-2-(4-(methylsulfonyl)phenyl)naphthalen-1-yl)oxy)phenyl)piperazin-1-yl)acetate (compound D12-4) (200.0 mg, yield: 85%).

Compound D12-4 (200.0 mg, 0.36 mmol) was added to a mixed solvent of tetrahydrofuran (6.0 mL) and water (1.0 mL) at room temperature, and then lithium hydroxide monohydrate (30 mg, 0.71 mmol) was added thereto. The reaction mixture was stirred at room temperature for 1 hour, and then added with hydrochloric acid aqueous solution (1.0 M) to adjust the pH of the aqueous phase to about 5 to 6. The resulting mixture was concentrated to dryness under reduced pressure to obtain a crude product of 2-(4-(4-((6-hydroxy-2-(4-(methylsulfonyl)phenyl)naphthalen-1-yl)oxy)phenyl)piperazin-1-yl)acetic acid (compound D12-5) (210.0 mg, yield > 100%).

The crude product of compound D12-5 (95.0 mg, 0.18 mmol) and compound C2 (72.0 mg, 0.20 mmol) were added to DMF (1.5 mL) at room temperature, and then DIPEA (93.0 mg, 0.72 mmol) and HATU (68.0 mg, 0.18 mmol) were sequentially added thereto. The resulting mixture was stirred at room temperature for 1 hour, and concentrated under reduced pressure to remove the organic solvent. The resulting residue was added with dichloromethane (20.0 mL), washed twice with water (10.0 mL), dried over anhydrous sodium sulfate, filtered, and the filtrate was concentrated under reduced pressure. The crude product was purified by prep-HPLC to obtain compound D12 (18.0 mg, yield: 12%).

¹H NMR (400 MHz, DMSO-*d*₆) δ 10.94 (s, 1H), 10.05 (s, 1H), 7.90 (d, *J* = 8.6 Hz, 2H), 7.87 - 7.78 (m, 2H), 7.73 (dd, *J* = 15.9, 8.9 Hz, 2H), 7.55 (dd, *J* = 19.7, 8.5 Hz, 2H), 7.24 (d, *J* = 2.4 Hz, 1H), 7.07 (qd, *J* = 4.5, 4.0, 2.3 Hz, 3H), 6.74 (d, *J* = 9.1 Hz, 2H), 6.55 (d, *J* = 9.1 Hz, 2H), 5.04 (dd, *J* = 13.2, 5.1 Hz, 1H), 4.32 (d, *J* = 16.9 Hz, 1H), 4.20 (d, *J* = 16.9 Hz, 1H), 3.75 - 3.56 (m, 4H), 3.30 - 3.19 (m, 13H), 3.00 - 2.92 (m, 4H), 2.92 - 2.83 (m, 1H), 2.63 - 2.56 (m, 1H), 2.45 - 2.28 (m, 1H), 2.03 - 1.89 (m, 1H).

MS calculated for 842.31; found 422.3 [M/2+H]⁺.

### Example 13: Synthesis of 2-(2,6-dioxopiperidin-3-yl)-5-(4-(4-(4-((6-hydroxy-2-(4-(methylsulfonyl)phenyl)naphthalen-1-yl)oxy)phenoxy)butyl)piperazin-1-yl)isoindoline-1,3-dione (compound D13)

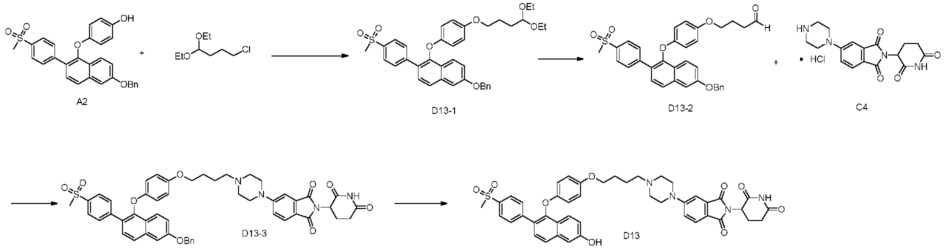

Compound A2 (1.45 g, 8.0 mmol) and potassium carbonate (552.0 mg, 4.0 mmol) were added to DMF (20.0 mL) at room temperature. The resulting mixture was stirred at 80°C for 10 hours, cooled to room temperature, diluted with water (50.0 mL), and then extracted twice with ethyl acetate (20.0 mL). The organic phases were combined, dried over anhydrous sodium sulfate, filtered, and the filtrate was concentrated under reduced pressure. The crude product was purified by silica gel column chromatography (petroleum ether/dichloromethane = 1/1) to obtain 6-(benzyloxy)-1-(4-(4,4-diethoxybutoxy)phenoxy)-2-(4-(methylsulfonyl)phenyl)naphthalene (compound D13-1) (750.0 mg, yield: 58%).

Hydrochloric acid aqueous solution (6.0 M, 1.95 mL, 11.7 mmol) was added dropwise to a solution of compound D13-1 (750.0 mg, 1.17 mmol) in tetrahydrofuran (15.0 mL) at room temperature. The resulting mixture was stirred at 50°C for 3 hours, cooled to room temperature, and concentrated to dryness under reduced pressure to obtain a crude product of 4-(4-((6-(benzyloxy)-2-(4-(methylsulfonyl)phenyl)naphthalen-1-yl)oxy)phenoxy)butanal (compound D13-2) (740.0 mg, yield > 100%).

The crude product of compound D13-2 (370.0 mg, 0.44 mmol), compound C4 (168.0 mg, 0.44 mmol), and potassium acetate (44.2 mg, 0.44 mmol) were sequentially added to dichloromethane (10.0 mL) at room temperature. The resulting mixture was stirred at room temperature for 30 minutes, then added with sodium triacetoxyborohydride (195.0 mg, 0.88 mmol), stirred for another 18 hours, and diluted with water (30.0 mL) and dichloromethane (15.0 mL). The organic phase was separated, and the aqueous phase was extracted twice with dichloromethane (10.0 mL). The organic phases were combined, dried over anhydrous sodium sulfate, filtered, and the filtrate was concentrated under reduced pressure. The crude product was purified by silica gel column chromatography (dichloromethane) to obtain 5-(4-(4-(4-((6-(benzyloxy)-2-(4-(methylsulfonyl)phenyl)naphthalen-1-yl)oxy)phenoxy)butyl)piperazin-1-yl)-2-(2,6-dioxopiperidin-3-yl)isoindoline-1,3-dione (compound D13-3) (200.0 mg, yield: 51%).

Wet palladium on carbon (100 mg, 10 wt%) was added to a solution of compound D13-3 (200.0 mg, 0.22 mmol) in tetrahydrofuran (15.0 mL) at room temperature. The reaction mixture was stirred for 10 hours under hydrogen atmosphere, and filtered through diatomite. The filter cake was washed twice with a mixed solvent of dichloromethane (10 mL) and methanol (0.5 mL), and the filtrate was concentrated to dryness under reduced pressure. The crude product was purified by prep-HPLC to obtain compound D13 (13.8 mg, yield: 8%).

¹H-NMR(400 MHz, DMSO-*d*₆) δ 11.1 (s, 1H), 8.23 (s, 1H), 7.94 (d, *J* = 8.0 Hz, 2H), 7.85 (d, *J* = 8.0 Hz, 2H), 7.74 - 7.79 (m, 2H), 7.69 (s, *J* = 12 Hz, 1H), 7.59 (s, *J* = 8 Hz, 1H), 7.35 (s, 1H), 7.25 (m, 2H), 7.10 (s, *J* = 4 Hz, 1H), 6.62 (d, *J* = 8 Hz, 2H), 6.75 (d, *J* = 8 Hz, 2H), 5.09 (dd, *J* = 12 Hz, 4 Hz, 1H), 3.86 (t, *J* = 4 Hz, 2H), 3.3 (m, 4H), 3.25 (m, 3H), 2.9 (m, 1H), 2.6 (m, 5H), 2.40 (m, 1H), 2.35 (m, 2H), 2.0 (m, 1H), 1.57 (m, 2H), 1.68 (m, 2H).

MS calculated for 802.27; found 803.1 [M+H]⁺.

### Example 14: Synthesis of 3-(4-(2-(2-(2-(2-(4-((6-hydroxy-2-(4-(methylsulfonyl)phenyl)naphthalen-1-yl)oxy)phenoxy)ethoxy)ethoxy)ethoxy)ethoxy)-1-oxoisoindolin-2-yl)piperidine-2,6-dione (compound D14)

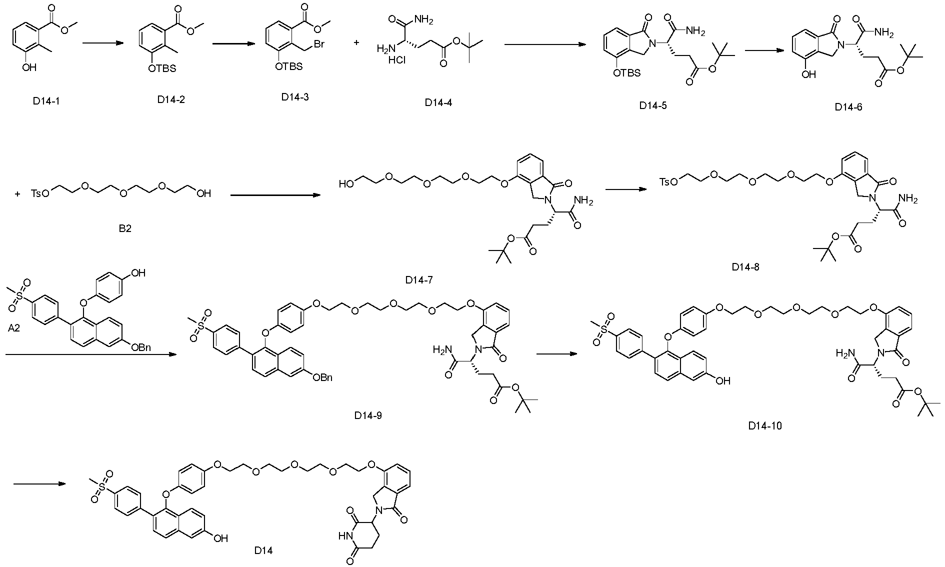

Methyl 3-hydroxy-2-methylbenzoate (compound D14-1) (3.0 g, 18.1 mmol), tertbutyldimethylsilyl chloride (3.54 g, 23.5 mmol), and imidazole (2.5 g, 36.2 mmol) were sequentially added to dichloromethane (30.0 mL) at room temperature. The resulting mixture was stirred at room temperature for 18 hours, and diluted with water (50.0 mL). The organic phase was separated, and the aqueous phase was extracted twice with dichloromethane (50.0 mL). The organic phases were combined, dried over anhydrous sodium sulfate, filtered, and concentrated under reduced pressure. The crude product was purified by silica gel column chromatography (petroleum ether/ethyl acetate = 100/1) to obtain methyl 3-((tertbutyldimethylsilyl)oxy)-2-methylbenzoate (compound D14-2) (3.0 g, yield: 71%).

Compound D14-2 (1.5 g, 5.36 mmol), N-bromosuccinimide (1.05 g, 5.89 mmol), and azobisisobutyronitrile (89.0 mg, 0.54 mmol) were sequentially added to carbon tetrachloride (10.0 mL) at room temperature. The resulting mixture was stirred at 90°C for 18 hours, cooled to room temperature, and filtered under reduced pressure. The filtrate was concentrated to dryness under reduced pressure to obtain a crude product of methyl 2-(bromomethyl)-3-((tert-butyldimethylsilyl)oxy)benzoate (compound D14-3) (1.8 g, yield > 100%).

The crude product of compound D14-3 (1.8 g, 5.03 mmol), tert-butyl (*S*)-4,5-diamino-5-oxopentanoate hydrochloride (compound D14-4) (1.44 g, 6.03 mmol), and DIPEA (2.7 mL, 15.1 mmol) were sequentially added to acetonitrile (20.0 mL) at room temperature. The resulting mixture was stirred at room temperature for 1 hour, heated to 90°C and stirred for 3 hours, cooled to room temperature, and concentrated to dryness under reduced pressure. The crude product was purified by silica gel column chromatography (dichloromethane/methanol = 10/1) to obtain tert-butyl (*S*)-5-amino-4-(4-((tert-butyldimethylsilyl)oxy)-1-oxoisoindolin-2-yl)-5-oxopentanoate (compound D14-5) (1.1 g, yield: 49%).

Tetra-n-butylammonium fluoride (1.279 g, 4.9 mmol) was added to a solution of compound D14-5 (1.1 g, 2.45 mmol) in tetrahydrofuran (25.0 mL) at room temperature. The resulting mixture was stirred at room temperature for 1 hour, and concentrated to dryness under reduced pressure. The resulting residue was added with ethyl acetate (50.0 mL), washed twice with saturated ammonium chloride aqueous solution (25 mL), then dried over anhydrous sodium sulfate, filtered, and the filtrate was concentrated under reduced pressure. The crude product was purified by silica gel column chromatography (dichloromethane/methanol = 10/1) to obtain tert-butyl (*S*)-5-amino-4-(4-hydroxy-1-oxoisoindolin-2-yl)-5-oxopentanoate (compound D14-6) (600 mg, yield: 36%).

Compound D14-6 (300.0 mg, 0.90 mmol), compound B2 (409.0 mg, 1.18 mmol), and potassium carbonate (621.0 mg, 4.5 mmol) were sequentially added to acetonitrile (25.0 mL) at room temperature. The resulting mixture was stirred at 90°C for 10 hours, cooled to room temperature, and concentrated to dryness under reduced pressure. The crude product was purified by silica gel column chromatography (dichloromethane/methanol = 10/1) to obtain tert-butyl (*S*)-5-amino-4-(4-(2-(2-(2-(2-hydroxyethoxy)ethoxy)ethoxy)ethoxy)-1-oxoisoindolin-2-yl)-5-oxopentanoate (compound D14-7) (400.0 mg, yield: 71%).

Compound D14-7 (400 mg, 0.78 mmol), p-toluenesulfonyl chloride (193.0 mg, 1.01 mmol), triethylamine (0.33 mL, 2.34 mmol), and 4-dimethylaminopyridine (9.6 mg, 0.078 mmol) were sequentially added to dichloromethane (25.0 mL) at room temperature. The resulting mixture was stirred at room temperature for 18 hours, and concentrated to dryness under reduced pressure. The crude product was purified by silica gel column chromatography (dichloromethane/methanol = 10/1) to obtain tert-butyl (*S*)-5-amino-5-oxo-4-(1-oxo-4-(2-(2-(2-(2-(tosyloxy)ethoxy)ethoxy)ethoxy)isoindolin-2-yl)-5-oxopentanoate (compound D14-8) (470 mg, yield: 90%).

Compound D14-8 (470 mg, 0.71 mmol), compound A2 (421.0 mg, 0.85 mmol), and potassium carbonate (294.0 mg, 2.13 mmol) were sequentially added to DMF (15.0 mL) at room temperature. The resulting mixture was stirred at 85°C for 10 hours, cooled to room temperature, and concentrated to dryness under reduced pressure. The crude product was purified by silica gel column chromatography (dichloromethane/methanol = 10/1) to obtain tert-butyl (*S*)-5-amino-4-(4-(2-(2-(2-(2-(4-((6-(benzyloxy)-2-(4-(methylsulfonyl)phenyl)naphthalen-1-yl)oxy)phenoxy)ethoxy)ethoxy)ethoxy)ethoxy)-1-oxoisoindolin-2-yl)-5-oxopentanoate (compound D14-9) (730.0 mg, yield: 99%).

Compound D14-9 (732 mg, 0.74 mmol) was added to a mixed solvent of methanol (15.0 mL) and tetrahydrofuran (10.0 mL) at room temperature, and then wet palladium on carbon (300 mg, 10 wt%) was added thereto. The resulting mixture was stirred at room temperature for 18 hours, and filtered through diatomite. The filter cake was washed twice with a mixed solvent of dichloromethane (10 mL) and methanol (0.5 mL), and the filtrate was concentrated to dryness under reduced pressure. The crude product was purified by silica gel column chromatography (dichloromethane/methanol = 10/1) to obtain tert-butyl (*S*)-5-amino-4-(4-(2-(2-(2-(2-(4-((6-hydroxy)-2-(4-(methylsulfonyl)phenyl)naphthalen-1-yl)oxy)phenoxy)ethoxy)ethoxy)ethoxy)ethoxy)-1-oxoisoindolin-2-yl)-5-oxopentanoate (compound D14-10) (516 mg, yield: 78%).

Compound D14-10 (258.0 mg, 0.29 mmol) and benzenesulfonic acid (136 mg, 0.86 mmol) were sequentially added to acetonitrile (5.0 mL) at room temperature. The resulting mixture was stirred at 100°C for 16 hours, cooled to room temperature, and concentrated to dryness under reduced pressure. The crude product was purified by silica gel column chromatography (dichloromethane/methanol = 10/1) to obtain compound D14 (58.0 mg, yield: 23%).

¹H NMR (400 MHz, DMSO-*d*₆) δ:10.98 (s, 1H), 10.06 (s, 1H), 7.93 (d, *J* = 8.2 Hz, 2H), 7.86 (d, *J* = 8.2 Hz, 2H), 7.77 (dd, *J* = 16.0, 8.9 Hz, 2H), 7.61 (d, *J* = 8.6 Hz, 1H), 7.48 (t, *J* = 7.8 Hz, 1H), 7.33 (d, *J* = 7.5 Hz, 1H), 7.30 - 7.23 (m, 2H), 7.12 (dd, *J* = 9.3, 2.4 Hz, 1H), 6.76 (d, *J* = 9.0 Hz, 2H), 6.62 (d, *J* = 8.7 Hz, 2H), 4.32 - 4.20 (m, 3H), 3.93 (t, *J* = 4.7 Hz, 2H), 3.77 (t, *J* = 4.6 Hz, 2H), 3.68 - 3.50 (m, 12H), 3.24 (s, 3H), 2.99 - 2.86 (m, 1H), 2.59 (d, *J* = 18.1 Hz, 1H), 2.06 - 1.93 (m, 1H).

MS calculated for 824.90; found 825.0 [M+H]⁺.

### Example 15: Synthesis of 2-(2,6-dioxopiperidin-3-yl)-5-(4-(2-(2-((2S,SR)-4-(2-(4-((6-hydroxy-2-(4-(methylsulfonyl)phenyl)naphthalen-1-yl)oxy)phenoxy)ethyl)-2,5-dimethylpiperazin-1-yl)ethoxy)acetyl)piperazin-1-yl)isoindoline-1,3-dione (compound D15)

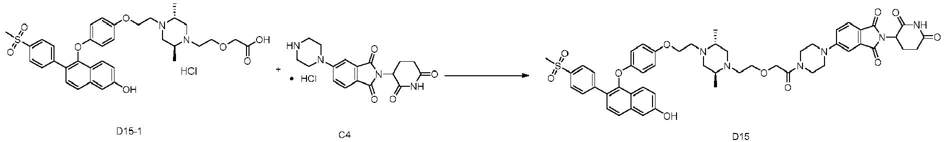

Compound D15-1 was prepared with reference to the preparation method of D9-4, and the difference was that tert-butyl (2S,SR)-2,5-dimethylpiperazine-1-carboxylate was used instead of tert-butyl (2R,5S)-2,5-dimethylpiperazine-1-carboxylate.

2-(2-((2S,SR)-4-(2-(4-((6-Hydroxy-2-(4-(methylsulfonyl)phenyl)naphthalen-1-yl)oxy)phenoxy)ethyl)-2,5-dimethylpiperazin-1-yl)ethoxy)acetic acid hydrochloride (compound D15-1) (100 mg, 0.13 mmol), compound C4 (74.0 mg. 0.196 mmol), DIPEA (84.0 mg, 0.65 mmol), and HATU (74.5 mg, 0.196 mmol) were sequentially added to DMF (3.0 mL) at room temperature. The resulting mixture was stirred at room temperature for 16 hours, and concentrated to dryness under reduced pressure. The crude product was purified by prep-HPLC to obtain compound D15 (20.0 mg, yield: 18%).

¹H NMR (400 MHz, DMSO) δ 11.08 (s, 1H), 10.28 - 10.07 (m, 1H), 8.19 (s, 1H), 7.90 (d, *J* = 8.3 Hz, 2H), 7.83 (d, *J* = 8.3 Hz, 2H), 7.78 - 7.66 (m, 3H), 7.57 (d, *J* = 8.6 Hz, 1H), 7.35 (s, 1H), 7.29 - 7.21 (m, 2H), 7.11 (d, *J* = 9.0 Hz, 1H), 6.72 (d, *J* = 8.9 Hz, 2H), 6.58 (d, *J* = 8.9 Hz, 2H), 5.07 (dd, *J* = 13.2, 5.4 Hz, 1H), 4.20 (s, 2H), 3.87 (s, 2H), 3.55 (d, *J* = 13.1 Hz, 10H), 3.21 (s, 3H), 3.01 - 2.76 (m, 6H), 2.59 (s, 1H), 2.54 (s, 1H), 2.46 - 2.31 (m, 1H), 2.13 - 1.92 (m, 1H), 1.30 - 1.21 (m, 4H), 0.95 (dd, *J* = 9.7, 5.9 Hz, 6H).

MS calculated for 972.37; found 487.4 [M/2+H]⁺.

### Example 16: Synthesis of 2-(2,6-dioxopiperidin-3-yl)-5-(4-(2-(2-(2-(4-((6-hydroxy-2-(4-(methylsulfonyl)phenyl)naphthalen-1-yl)oxy)phenoxy)ethoxy)ethoxy)ethyl)piperazin-1-yl)isoindoline-1,3-dione (compound D16)

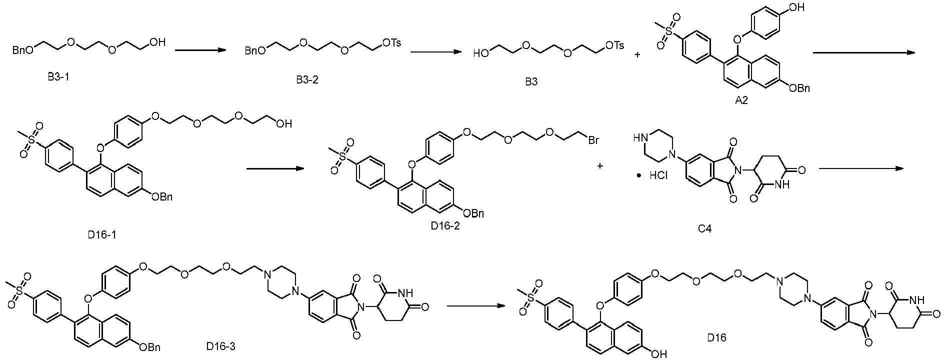

p-Toluenesulfonyl chloride (9.52 g, 50 mmol) was added to a solution of triethylene glycol monobenzyl ether (compound B3-1) (10.0 g, 41.6 mmol), triethylamine (11.5 mL, 83.2 mmol), and 4-dimethylaminopyridine (508 mg, 4.2 mmol) in dichloromethane (100.0 mL) in batches at 0°C. The reaction mixture was stirred at room temperature for 16 hours, cooled to 0°C, and added with hydrochloric acid aqueous solution (1.0 M) to adjust the pH of the aqueous phase to about 6 to 7. The resulting mixture was extracted twice with dichloromethane (200.0 mL). The organic phases were combined, washed with saturated brine (100.0 mL), dried over anhydrous sodium sulfate, filtered, and the filtrate was concentrated under reduced pressure. The crude product was purified by silica gel column chromatography (petroleum ether/ethyl acetate = 2/1) to obtain 2-(2-(2-(benzyloxy)ethoxy)ethoxy)ethyl 4-methylbenzenesulfonate (compound B3-2) (13.28 g, yield: 81%).

Wet palladium on carbon (1.5 g, 10 wt%) was added to a solution of compound B3-2 (13.28 g, 33.7 mmol) in methanol (200.0 mL) at room temperature. The reaction mixture was stirred for 16 hours under hydrogen atmosphere, filtered through diatomite, and the filtrate was concentrated to dryness under reduced pressure to obtain 2-(2-(2-hydroxyethoxy)ethoxy)ethyl 4-methylbenzenesulfonate (compound B3) (9.9 g, yield: 97%).

Compound B3 (2.0 g, 6.57 mmol), compound A2 (3.91 g, 7.88 mmol), and potassium carbonate (2.72 g, 19.7 mmol) were sequentially added to DMF (30.0 mL) at room temperature. The reaction mixture was stirred at 85°C for 16 hours, cooled to room temperature, diluted with water (150.0 mL), and extracted twice with dichloromethane (100.0 mL). The organic phases were combined, washed with saturated brine (100.0 mL), dried over anhydrous sodium sulfate, filtered, and the filtrate was concentrated under reduced pressure. The crude product was purified by silica gel column chromatography (petroleum ether/ethyl acetate = 2/1) to obtain 2-(2-(2-(4-((6-(benzyloxy)-2-(4-(methylsulfonyl)phenyl)naphthalen-1-yl)oxy)phenoxy)ethoxy)ethoxy)ethanol (compound D16-1) (3.5 g, yield: 85%).

Carbon tetrabromide (473 mg, 1.43 mmol) was slowly added to a solution of compound D16-1 (600.0 mg, 0.95 mmol) and triphenylphosphine (374 mg, 1.43 mmol) in dichloromethane (8.0 mL) at 0°C. The reaction mixture was stirred at room temperature for 2 hours, and concentrated to dryness under reduced pressure. The crude product was purified by silica gel column chromatography (petroleum ether/ethyl acetate = 2/1) to obtain 6-(benzyloxy)-1-(4-(2-(2-(2-bromoethoxy)ethoxy)ethoxy)phenoxy)-2-(4-(methylsulfonyl)phenyl)naphthalene (compound D16-2) (630.0 mg, yield: 96%).

Compound D16-2 (260.0 mg, 0.376 mmol), compound C4 (143 mg, 0.376 mmol), DIPEA (0.27 mL, 1.5 mmol), and potassium iodide (6.0 mg, 0.04 mmol) were sequentially added to acetonitrile (5.0 mL) at room temperature. The reaction mixture was stirred at 85°C for 16 hours, cooled to room temperature, and concentrated to dryness under reduced pressure. The resulting residue was added with water (10.0 mL), and extracted twice with dichloromethane (10.0 mL). The organic phases were combined, washed with saturated brine (10.0 mL), dried over anhydrous sodium sulfate, filtered, and the filtrate was concentrated under reduced pressure. The crude product was purified by silica gel column chromatography (dichloromethane/methanol = 10/1) to obtain 5-(4-(2-(2-(2-(4-((6-(benzyloxy)-2-(4-(methylsulfonyl)phenyl)naphthalen-1-yl)oxy)phenoxy)ethoxy)ethoxy)ethyl)piperazin-1-yl)-2-(2,6-dioxopiperidin-3-yl)isoindoline-1,3-dione (compound D16-3) (165.0 mg, yield: 46%).

Compound D16-3 (70.0 mg, 0.07 mmol) was added to a mixed solvent of methanol (7.0 mL) and tetrahydrofuran (3.0 mL) at room temperature, and then wet palladium on carbon (70 mg, 10 wt%) was added thereto. The reaction mixture was stirred for 16 hours under hydrogen atmosphere, filtered through diatomite, and the filtrate was concentrated under reduced pressure. The crude product was purified by silica gel column chromatography (dichloromethane/methanol = 10/1) to obtain compound D16 (17.0 mg, yield: 27%).

¹H NMR (400 MHz, DMSO-*d*₆) δ: 11.10 (s, 1H), 10.05 (s, 1H), 7.92 (d, *J* = 8.4 Hz, 2H), 7.84 (d, *J* = 8.3 Hz, 2H), 7.80 - 7.65 (m, 3H), 7.59 (d, *J* = 8.6 Hz, 1H), 7.33 (s, 1H), 7.28 - 7.21 (m, 2H), 7.10 (d, *J* = 9.1 Hz, 1H), 6.78 (d, *J* = 9.0 Hz, 2H), 6.62 (d, *J* = 9.0 Hz, 2H), 5.09 (dd, *J* = 12.7, 5.3 Hz, 1H), 3.95 (d, *J* = 5.1 Hz, 2H), 3.69 (d, *J* = 4.1 Hz, 2H), 3.61 - 3.49 (m, 7H), 3.38 (d, *J* = 16.8 Hz, 5H), 3.34 - 3.29 (m, 2H), 3.23 (s, 3H), 2.97 - 2.84 (m, 1H), 2.72 - 2.56 (m, 4H), 2.07 - 1.99 (m, 1H).

MS calculated for 862.29; found 863.2 [M+H]⁺.

### Example 17: Synthesis of 3-(4-((2-(ethyl(2-(4-((6-hydroxy-2-(4-(methylsulfonyl)phenyl)naphthalen-1-yl)oxy)phenoxy)ethyl)amino)ethyl)amino)-1-oxoisoindolin-2-yl)piperidine-2,6-dione (compound D17)

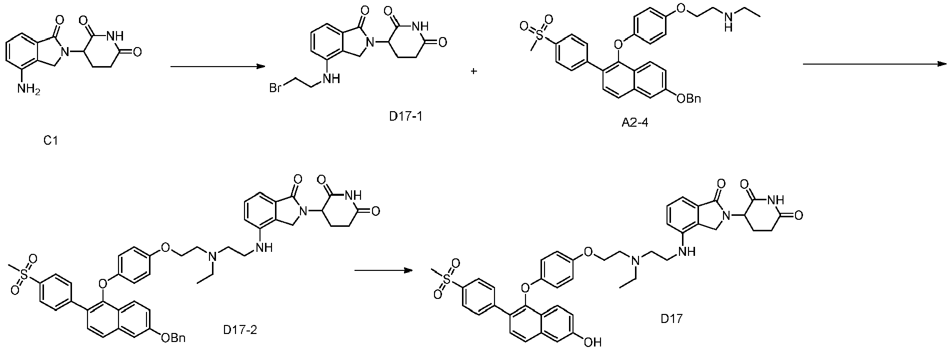

1,2-Dibromoethane (448 mg, 2.4 mmol) and DIPEA (780.0 mg, 6.0 mmol) were sequentially added to a solution of compound C1 (518.0 mg, 2.0 mmol) in N-methylpyrrolidone (10.0 mL) at room temperature. The resulting mixture was stirred at 110°C for 2 hours, cooled to room temperature, and diluted with water (100.0 mL). The resulting mixture was filtered under reduced pressure, and the filter cake was dried under vacuum to obtain a crude product of 3-(4-((2-bromoethyl)amino)-1-oxoisoindolin-2-yl)piperidine-2,6-dione (compound D17-1) (680 mg, crude yield: 93%).

2-(4-((6-(Benzyloxy)-2-(4-(methylsulfonyl)phenyl)naphthalen-1-yl)oxy)phenoxy)-N-ethylethan-1-amine (compound A2-4) (356.5 mg, 0.628 mmol) and DIPEA (162.0 mg, 1.26 mmol) were sequentially added to a solution of compound D17-1 (230 mg, 0.628 mmol) in N-methylpyrrolidone (10.0 mL) at room temperature. The resulting mixture was stirred at 100°C for 4 hours, cooled to room temperature, diluted with water (100.0 mL), and then extracted twice with ethyl acetate (25.0 mL). The organic phases were combined, dried over anhydrous sodium sulfate, filtered, and the filtrate was concentrated under reduced pressure. The crude product was purified by silica gel column chromatography (dichloromethane/methanol = 10/1) to obtain 3-(4-((2-((2-(4-((6-(benzyloxy)-2-(4-(methylsulfonyl)phenyl)naphthalen-1-yl)oxy)phenoxy)ethyl)(ethyl)amino)ethyl)amino)-1-oxoisoindolin-2-yl)piperidine-2,6-dione (compound D17-2) (150.0 mg, yield: 28%).

Wet palladium on carbon (50 mg, 10 wt%) was added to a solution of compound D17-2 (150.0 mg, 0.176 mmol) in tetrahydrofuran (10.0 mL) at room temperature. The resulting mixture was stirred for 10 hours under hydrogen atmosphere, filtered through diatomite, and the filtrate was concentrated to dryness under reduced pressure. The crude product was purified by prep-HPLC to obtain compound D17 (22.0 mg, 16%) as a product.

¹H-NMR(400MHz, DMSO-*d*₆), δ 11.1 (s, 1H), 8.23 (s, 1H), 7.94 (d, *J* = 8.0 Hz, 2H), 7.85 (d, *J* = 8.0 Hz, 2H), 7.74 - 7.79 (m, 2H), 7.60 (s, *J* = 12 Hz, 1H),7.28 (m, 2H), 7.10 (s, 1H), 6.94 (s, 1H), 6.76 (s, 1H), 6.70 (d, *J* = 8 Hz, 2H), 6.60 (d, *J* = 8 Hz, 2H), 5.39 (m, 1H), 5.10 (m, 1H), 4.10 - 4.22 (m, 2H), 3.90 (t, *J* = 4 Hz, 2H), 3.25 (m, 5H), 2.92 - 2.95 (m, 1H), 2.79 - 2.82 (m, 2H), 2.69 - 2.73 (m, 2H), 2.60 - 2.64 (m, 3H), 2.27 - 2.32 (m, 1H), 1.99 - 2.01 (m, 1H), 0.96 (t, *J* = 8 Hz, 3H).

MS calculated for 762.27; found 763.3 [M+H]⁺.

### Example 18: Synthesis of 2-(2,6-dioxopiperidin-3-yl)-4-(2-(2-(ethyl(2-(4-((6-hydroxy-2-(4-(methylsulfonyl)phenyl)naphthalen-1-yl)oxy)phenoxy)ethyl)amino)ethoxy)ethoxy)isoindoline-1,3-dione (compound D18)

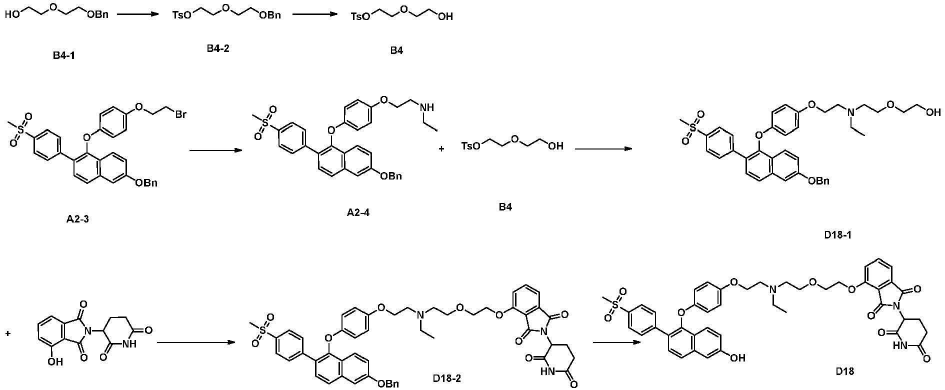

Triethylamine (15.0 mL, 108.0 mmol) and p-toluenesulfonyl chloride (12.4 g, 64.8 mmol) were sequentially added to a solution of 2-(2-(benzyloxy)ethoxy)ethanol (compound B4-1) (10.6 g, 54.0 mmol) and 4-dimethylaminopyridine (660.0 mg, 5.4 mmol) in dichloromethane (250.0 mL) at 0°C. The reaction mixture was warmed to room temperature, stirred for 16 hours, added with water (200.0 mL), stirred for 3 minutes, and the organic phase was separated. The aqueous phase was extracted twice with dichloromethane (100.0 mL). The organic phases were combined, washed with saturated ammonium chloride aqueous solution (50.0 mL) and saturated brine (50.0 mL), dried over anhydrous sodium sulfate, filtered, and the filtrate was concentrated under reduced pressure. The crude product was purified by silica gel column chromatography (dichloromethane/petroleum ether = 2/1) to obtain 2-(2-(benzyloxy)ethoxy)ethyl 4-methylbenzenesulfonate (compound B4-2) (16.8 g, yield: 89%).

Compound B4-2 (16.8 g, 47.9 mmol) was added to a mixed solvent of methanol (70.0 mL) and tetrahydrofuran (70.0 mL) at room temperature, and then wet palladium on carbon (1.7 g, 10 wt%) was added thereto. The resulting mixture was stirred for 16 hours under hydrogen atmosphere, and filtered through diatomite. The filter cake was washed three times with methanol (10.0 mL), and the filtrate was concentrated to dryness under reduced pressure to obtain a crude product of 2-(2-hydroxyethoxy)ethyl 4-methylbenzenesulfonate (compound B4) (12.4 g, yield: 99%).

Compound A2-3 (10.0 g, 16.6 mmol) was added to a solution of ethylamine in tetrahydrofuran (2.0 M, 83.0 mL, 166.0 mmol) at room temperature, and then water (10 mL) was added thereto. The resulting mixture was stirred at 70°C for 18 hours, cooled to room temperature, and concentrated to dryness under reduced pressure. The resulting mixture was added with water (20 mL), and then extracted three times with dichloromethane (20.0 mL). The organic phases were combined, dried over anhydrous sodium sulfate, filtered, and the filtrate was concentrated under reduced pressure. The crude product was purified by silica gel column chromatography (dichloromethane/methanol = 10/1) to obtain 2-(4-((6-(benzyloxy)-2-(4-(methylsulfonyl)phenyl)naphthalen-yl)oxy)phenoxy)-N-ethylethylamine (compound A2-4) (8.7 g, yield: 93%).

Compound A2-4 (500.0 mg, 0.88 mmol), the crude product of compound B4 (229 mg, 0.88 mmol), potassium carbonate (365.0 mg, 2.64 mmol), and potassium iodide (44.0 mg, 0.26 mmol) were sequentially added to a mixed solvent of acetonitrile (9.0 mL) and DMF (4.0 mL) at room temperature. The resulting mixture was stirred at 80°C for 24 hours, cooled to room temperature, and concentrated to dryness under reduced pressure. The crude product was purified by silica gel column chromatography (dichloromethane/methanol = 10/1) to obtain 2-(2-((2-(4-((6-(benzyloxy)-2-(4-(methylsulfonyl)phenyl)naphthalen-1-yl)oxy)phenoxy)ethyl)(ethyl)amino)ethoxy)ethanol (compound D18-1) (415.0 mg, yield: 72%).

Triphenylphosphine (114 mg, 0.43 mmol) and diethyl azodicarboxylate (120 mg, 0.59 mmol) were sequentially added to a solution of compound D18-1 (160 mg, 0.24 mmol) and a crude product of 2-(2,6-dioxopiperidin-3-yl)-4-hydroxyisoindoline-1,3-dione (78 mg, 0.28 mmol) in tetrahydrofuran (4.0 mL) at 0°C. The resulting mixture was warmed to room temperature, stirred for 12 hours, and concentrated to dryness under reduced pressure. The crude product was purified by silica gel column chromatography (dichloromethane/methanol = 10/1) to obtain 4-(2-(2-((2-(4-((6-(benzyloxy)-2-(4-(methylsulfonyl)phenyl)naphthalen-1-yl)oxy)phenoxy)ethyl)ethylamino)ethoxy)ethoxy)-2-(2,6-dioxopiperidin-3-yl)isoindoline-1,3-dione (compound D18-2) (120.0 mg, yield: 55%).

Compound D18-2 (120 mg, 0.13 mmol) was added to a mixed solvent of methanol (5.0 mL) and tetrahydrofuran (5.0 mL) at room temperature, and then wet palladium on carbon (100 mg, 10 wt%) was added thereto. The resulting mixture was stirred for 16 hours under hydrogen atmosphere, filtered through diatomite, and the filtrate was concentrated under reduced pressure. The crude product was purified by silica gel column chromatography (dichloromethane/methanol = 10/1) to obtain compound D18 (15 mg, yield: 14%).

¹H NMR (400 MHz, DMSO-*d*₆) δ 11.08 (s, 1H), 10.03 (s, 1H), 7.89 (d, *J* = 8.1 Hz, 2H), 7.83 (d, *J* = 8.1 Hz, 2H), 7.79 - 7.69 (m, 3H), 7.57 (d, *J* = 8.5 Hz, 1H), 7.48 (d, *J* = 8.5 Hz, 1H), 7.41 (d, *J* =7.0 Hz, 1H), 7.25 (s, 1H), 7.09 (d, *J* = 9.1 Hz, 1H), 6.69 (d, *J* = 8.4 Hz, 2H), 6.57 (d, *J* = 8.4 Hz,2H), 5.06 (dd, *J* = 12.8, 5.4 Hz, 1H), 4.31 (s, 2H), 3.97 - 3.71 (m, 4H), 3.67 - 3.49 (m, 2H), 3.20 (s, 3H), 2.92 - 2.80 (m, 2H), 2.78 - 2.53 (m, 6H), 2.47 - 2.39 (m, 1H), 2.03 - 1.92 (m, 1H), 1.04 - 0.79 (m, 3H).

MS calculated for 821.26; found 822.2 [M+H]⁺.

### Example 19: Synthesis of 2-(2,6-dioxopiperidin-3-yl)-5-(4-(2-(2-(2-(2-(4-((6-hydroxy-2-(4-(methylsulfonyl)phenyl)naphthalen-1-yl)oxy)phenoxy)ethoxy)ethoxy)ethoxy)piperazin-1-yl)isoindoline-1,3 -dione (compound D19)

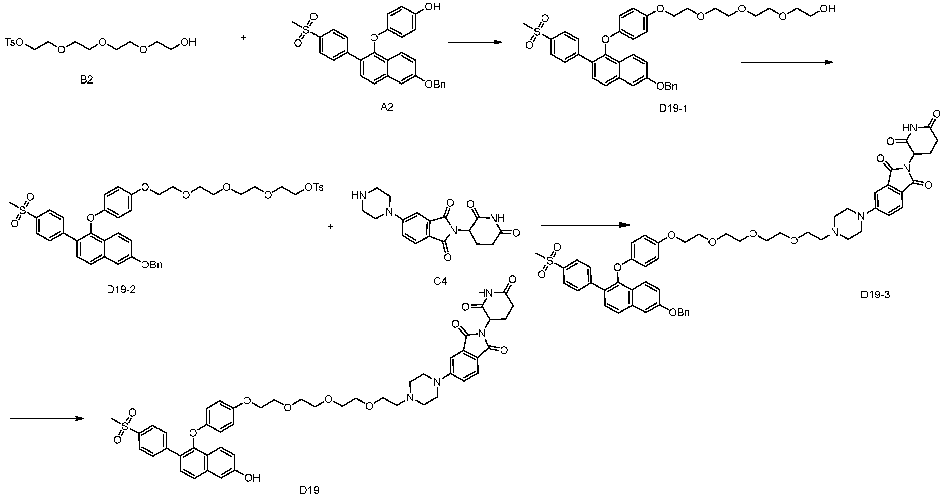

2-(2-(2-(2-(2-Hydroxyethoxy)ethoxy 4-methylbenzenesulfonate (compound B2) (1.4 g, 4.03 mmol), compound A2 (2 g, 4.03 mmol), and potassium carbonate (1.7 g, 12.1 mmol) were sequentially added to DMF (40 mL) at room temperature, and the resulting mixture was replaced with nitrogen and then stirred at 90°C for 15 hours. The resulting mixture was cooled to room temperature, and concentrated under reduced pressure. The crude product was purified by silica gel column chromatography (petroleum ether/dichloromethane/ethyl acetate = 1/1/17) to obtain 2-(2-(2-(2-(4-((6-(benzyloxy)-2-(4-(methylsulfonyl)phenyl)naphthalen-1-yl)oxy)phenoxy)ethoxy)ethoxy)ethoxy)ethan-1-ol (compound D19-1) (1.6 g, yield: 59%).

Compound D19-1 (780 mg, 1.16 mmol), TsCl (288 mg, 1.51 mmol), triethylamine (0.3 mL, 2.32 mmol), and DMAP (15 mg, 0.12 mmol) were sequentially added to dichloromethane (15 mL) at room temperature. The reaction mixture was stirred at room temperature for 16 hours, and concentrated under reduced pressure. The crude product was purified by silica gel column chromatography (petroleum ether/ethyl acetate = 8/1) to obtain 2-(2-(2-(2-(4-((6-(benzyloxy)-2-(4-(methylsulfonyl)phenyl)naphthalen-1-yl)oxy)phenoxy)ethoxy)ethoxy)ethoxy)ethyl 4-methylbenzenesulfonate (compound D19-2) (850 mg, yield: 86%).

Compound D19-2 (150 mg, 0.18 mmol), compound C4 (82.5 g, 0.22 mmol), DIPEA (0.125 mL), and potassium iodide (3 mg, 0.018 mmol) were added to acetonitrile (5 mL) at room temperature. The reaction mixture was stirred at 100°C for 12 hours, and concentrated to dryness. The crude product was purified by silica gel column chromatography (dichloromethane/methanol = 10/1) to obtain 5-(4-(2-(2-(2-(2-(4-((6-(benzyloxy)-2-(4-(methylsulfonyl)phenyl)naphthalen-1-yl)oxy)phenoxy)ethoxy)ethoxy)ethoxy)ethyl)piperazin-1-yl)-2-(2,6-dioxopiperidin-3-yl)isoindoline-1,3-dione (compound D19-3) (220 mg, yield: 52%).

Compound D19-3 (110 mg, 0.11 mmol) and wet palladium on carbon (100 mg, 10 wt%) were sequentially added to methanol/tetrahydrofuran (13 mL/13 mL) at room temperature, and the mixture was stirred at room temperature for 18 hours. The reaction mixture was filtered, and the resulting filtrate was concentrated to dryness. The crude product was purified by silica gel column chromatography (dichloromethane/methanol = 10/1) to obtain compound D19 (15 mg, yield: 15%).

¹H NMR (400 MHz, DMSO-*d*₆) δ: 11.08 (s, 1H), 10.04 (s, 1H), 7.92 - 7.88 (m, 2H), 7.85 - 7.81 (m, 2H), 7.77 - 7.74 (m, 1H), 7.71 (d, *J* = 9.1 Hz, 1H), 7.66 (d, *J* = 8.5 Hz, 1H), 7.57 (d, *J* = 8.6 Hz, 1H), 7.32 (d, *J* = 2.4 Hz, 1H), 7.26 - 7.20 (m, 2H), 7.08 (dd, *J* = 9.1, 2.4 Hz, 1H), 6.77 - 6.72 (m, 2H), 6.62 - 6.57 (m, 2H), 3.95 - 3.90 (m, 2H), 3.65 (t, *J* = 4.8 Hz, 2H), 3.56 - 3.46 (m, 11H), 3.42 - 3.36 (m, 4H), 3.30 (s, 2H), 3.21 (s, 4H), 2.88 (ddd, *J* = 16.5, 13.6, 5.4 Hz, 1H), 2.53 (d, *J* = 5.7 Hz, 5H), 2.05 - 1.95 (m, 2H).

MS calculated for 906.31; found 907.0 [M+H]⁺.

### Example 20: Synthesis of 3-(4-((2-(2-(2-(2-(4-((6-hydroxy-2-(4-(methylsulfonyl)phenyl)naphthalen-1-yl)oxy)phenoxy)ethoxy)ethoxy)ethoxy)ethyl)amino)-1-oxoisoindolin-2-yl)piperidine-2,6-dione (compound D20)

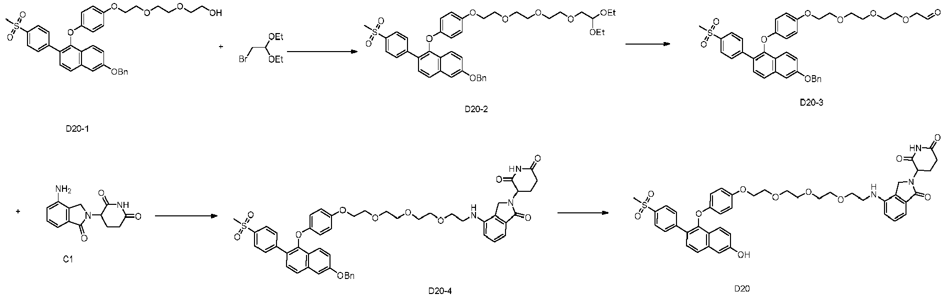

2-(2-(2-(4-((6-(Benzyloxy)-2-(4-(methylsulfonyl)phenyl)naphthalen-1-yl)oxy)phenoxy)ethoxy)ethoxy)ethan-1-ol (compound D20-1) (800.0 mg, 1.27 mmol) was slowly added to a suspension of sodium hydride (66.0 mg, 1.65 mmol) in tetrahydrofuran (6.0 mL) at 0°C, and the reaction mixture was stirred at room temperature for 45 minutes. The reaction mixture was cooled to 0°C, slowly added with bromoacetaldehyde diethyl acetal (1.25 g, 6.35 mmol), heated to 55°C, and stirred for 16 hours. The reaction mixture was concentrated under reduced pressure, added with saturated ammonium chloride aqueous solution (15.0 mL), and extracted twice with dichloromethane (10.0 mL). The organic phases were combined, washed with saturated brine (10.0 mL), dried over anhydrous sodium sulfate, filtered, and concentrated under reduced pressure. The crude product was purified by silica gel column chromatography (petroleum ether/ethyl acetate = 4/1) to obtain 1-(4-((6-(benzyloxy)-2-(4-(methylsulfonyl)phenyl)naphthalen-1-yl)oxy)phenoxy)-11-ethoxy-3,6,9,12-tetraoxatetradecane (compound D20-2) (1.0 g, yield: 100%).

Compound D20-2 (500.0 mg, 0.67 mmol) was added to a mixed solvent of concentrated hydrochloric acid (0.6 mL), water (0.6 mL), and tetrahydrofuran (6.0 mL) at room temperature, and the reaction mixture was heated to 50°C and stirred for 2 hours. The reaction mixture was concentrated under reduced pressure, added with water (10.0 mL), and extracted twice with dichloromethane (10.0 mL). The organic phases were combined, washed with saturated brine (10.0 mL), dried over anhydrous sodium sulfate, filtered, and concentrated under reduced pressure to obtain 2-(2-(2-(2-(4-((6-(benzyloxy)-2-(4-(methylsulfonyl)phenyl)naphthalen-1-yl)oxy)phenoxy)ethoxy)ethoxy)ethoxy)acetaldehyde (compound D20-3) (450 mg, yield: 100%).

Compound D20-3 (450.0 mg, 0.67 mmol), compound C1 (158.0 mg, 0.61 mmol), and two drops of acetic acid were sequentially added to methanol (5.0 mL) at room temperature. The reaction mixture was stirred at room temperature for 15 minutes, added with sodium triacetoxyborohydride (323.0 mg, 1.53 mmol), and stirred at 20°C for 16 hours. The reaction mixture was added with sodium cyanoborohydride (115.0 mg, 1.83 mmol), and stirred at 30°C for 4 hours. The reaction mixture was concentrated under reduced pressure, and the crude product was purified by silica gel column chromatography (dichloromethane/methanol = 10/1) to obtain 3-(4-((2-(2-(2-(2-(4-((6-(benzyloxy)-2-(4-(methylsulfonyl)phenyl)naphthalen-1-yl)oxy)phenoxy)ethoxy)ethoxy)ethoxy)ethyl)amino)-1-oxoisoindolin-2-yl)piperidine-2,6-dione (compound D20-4) (88 mg, yield: 15.8%).

Compound D20-4 (88.0 mg, 0.1 mmol) and wet palladium on carbon (70.0 mg, 10 wt%) were sequentially added to a mixed solvent of methanol (7.0 mL) and tetrahydrofuran (3.0 mL) at room temperature. The reaction mixture was stirred at 20°C for 16 hours under hydrogen atmosphere, filtered through diatomite, and the filtrate was concentrated under reduced pressure. The crude product was purified by a silica gel plate (dichloromethane/methanol = 10/1) to obtain compound D20 (47 mg, yield: 59.3%).

¹H NMR (400 MHz, DMSO-*d*₆) δ: 11.06 (s, 1H), 10.11 (s, 1H), 7.96 (d, *J* = 8.5 Hz, 2H), 7.89 (d, *J* = 8.5 Hz, 2H), 7.80 (dd, *J* = 21.6, 8.9 Hz, 2H), 7.64 (d, *J* = 8.6 Hz, 1H), 7.32 (dd, *J* = 8.0, 5.1 Hz, 2H), 7.15 (dd, *J* = 9.1, 2.3 Hz, 1H), 6.98 (t, *J* = 8.5 Hz, 1H), 6.85 (t, *J* = 8.6 Hz, 1H), 6.79 (d, *J* = 9.1 Hz, 2H), 6.65 (t, *J* = 6.4 Hz, 2H), 5.62 (t, *J* = 5.7 Hz, 1H), 5.16 (dd, *J* = 13.3, 5.1 Hz, 1H), 4.27 (t, *J* = 12.9 Hz, 1H), 4.17 (d, *J* = 17.1 Hz, 1H), 4.01 - 3.93 (m, 2H), 3.71 - 3.61 (m, 4H), 3.60 - 3.53 (m, 8H), 3.35 (dd, *J* = 11.5, 5.7 Hz, 2H), 3.27 (s, 3H), 3.04 - 2.91 (m, 1H), 2.72 - 2.62 (m, 1H), 2.44 - 2.29 (m, 1H), 2.13 - 2.02 (m, 1H).

MS calculated for 823.28; found 824.2 [M+H]⁺.

### Example 21: Synthesis of 3-(6-(4-(2-(2-(2-(2-(2-(4-((6-hydroxy-2-(4-(methylsulfonyl)phenyl)naphthalen-1-yl)oxy)phenoxy)ethoxy)ethoxy)ethoxy)ethyl)piperazin-1-yl)-1-oxoisoindolin-2-yl)piperidine-2,6-dione (compound D21)

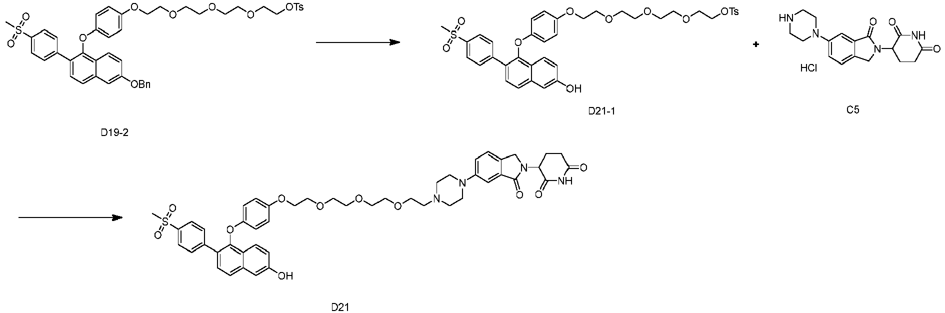

Compound D19-2 (300 mg, 0.36 mmol) and wet palladium on carbon (384 mg, 10 wt%) were sequentially added to tetrahydrofuran/methanol (15 mL/10 mL) at room temperature, and the resulting mixture was stirred at 22°C for 16 hours under hydrogen atmosphere. The mixture was filtered, and the resulting filtrate was concentrated under reduced pressure. The crude product was purified by silica gel column chromatography (dichloromethane/methanol = 10/1) to obtain 2-(2-(2-(2-(4-((6-hydroxy-2-(4-(methylsulfonyl)phenyl)naphthalen-1-yl)oxy)phenoxy)ethoxy)ethoxy)ethoxy)ethyl 4-methylbenzenesulfonate (compound D21-1) (278 mg, yield: 100%).

Compound D21-1 (50 mg, 0.068 mmol), 3-(1-oxo-6-(piperazin-1-yl)isoindolin-2-yl)piperidine-2,6-dione (compound C5) (27 mg, 0.075 mmol), DIPEA (0.06 mL, 0.34 mmol), and potassium iodide (1.1 mg, 0.0068 mmol) were sequentially added to acetonitrile (3 mL) at room temperature, and the resulting mixture was replaced with nitrogen and then stirred at 90°C for 16 hours. The mixture was cooled to room temperature, and concentrated under reduced pressure. The crude product was purified by silica gel column chromatography (dichloromethane/methanol = 10/1) to obtain compound D21 (13 mg, yield: 21%).

¹H NMR (400 MHz, DMSO-d₆) δ: 10.97 (s, 1H), 8.36 (s, 1H), 7.90 (d, *J* = 8.4 Hz, 2H), 7.85 - 7.80 (m, 2H), 7.73 (dd, *J* = 22.3, 8.9 Hz, 2H), 7.57 (d, *J* = 8.6 Hz, 1H), 7.40 (d, *J* = 8.4 Hz, 1H), 7.26 - 7.21 (m, 2H), 7.14 (d, *J* = 2.3 Hz, 1H), 7.09 (dd, *J* = 9.1, 2.3 Hz, 1H), 6.77 - 6.71 (m, 2H), 6.62 - 6.56 (m, 2H), 5.09 (dd, *J* = 13.3, 5.1 Hz, 1H), 4.32 (d, *J* = 16.7 Hz, 1H), 4.20 (d, *J* = 16.7 Hz, 1H), 3.93 (dd, *J* = 5.7, 3.7 Hz, 2H), 3.65 (dd, *J* = 5.7, 3.7 Hz, 3H), 3.58 - 3.44 (m, 13H), 3.15 (t, *J* = 5.0 Hz, 4H), 2.95 - 2.86 (m, 1H), 2.62 - 2.53 (m, 5H), 2.40 - 2.34 (m, 1H), 2.02 - 1.95 (m, 1H).

MS calculated for 893.02; found 894.2 [M+H]⁺.

### Example 22: Synthesis of 3-(6-(4-(2-(2-(2-(4-(((6-hydroxy-2-(4-(methylsulfonyl))phenyl)naphthalen-1-yl)oxy)phenoxy)ethoxy)ethoxy) 3-(6-(4-(2-(2-(2-(4-((6-hydroxy-2-(4-(methylsulfonyl)phenyl)naphthalen-1-yl)oxy)phenoxy)ethoxy)ethoxy)ethyl)piperazin-1-yl)-1-oxoisoindolin-2-yl)piperidine-2,6-dione (compound D22)

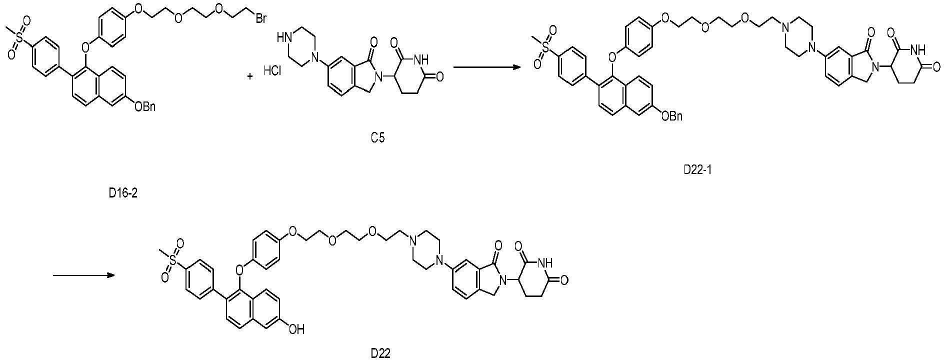

Compound D16-2 (260.0 mg, 0.376 mmol), hydrochloride of compound C5 (137 mg, 0.376 mmol), DIPEA (0.27 mL, 1.5 mmol), and potassium iodide (6 mg, 0.04 mmol) were sequentially added to acetonitrile (5.0 mL) at room temperature. The reaction mixture was stirred at 100°C for 16 hours. The reaction mixture was cooled to room temperature, concentrated under reduced pressure, added with water (10.0 mL), and extracted twice with dichloromethane (10.0 mL). The organic phases were combined, washed with saturated brine (10.0 mL), dried over anhydrous sodium sulfate, filtered, and concentrated under reduced pressure. The crude product was purified by silica gel column chromatography (dichloromethane/methanol = 10/1) to obtain 3-(6-(4-(2-(2-(2-(4-((6-(benzyloxy)-2-(4-(methylsulfonyl)phenyl)naphthalen-1-yl)oxy)phenoxy)ethoxy) 3-(6-(4-(2-(2-(2-(4-((6-(benzyloxy)-2-(4-(methylsulfonyl)phenyl)naphthalen-1-yl)oxy)phenoxy)ethoxy))3-ethoxy)ethyl)piperazin-1-yl)-1-oxoisoindolin-2-yl)piperidine-2,6-dione (compound D22-1) (106 mg, yield: 30%).

Compound D22-1 (106.0 mg, 0.11 mmol) and wet palladium on carbon (80.0 mg, 10 wt%) were sequentially added to a mixed solvent of methanol (7.0 mL) and tetrahydrofuran (3.0 mL) at room temperature. The reaction mixture was stirred at 20°C for 16 hours under hydrogen atmosphere, filtered through diatomite, and the filtrate was concentrated under reduced pressure. The crude product was purified by silica gel column chromatography (dichloromethane/methanol = 10/1) to obtain compound D22 (24.1 mg, yield: 25.1%).

¹H NMR (400 MHz, DMSO-d₆) δ: 10.97 (s, 1H), 10.05 (s, 1H), 7.90 (d, *J* = 8.4 Hz, 2H), 7.83 (d, *J* = 8.5 Hz, 2H), 7.78 - 7.70 (m, 2H), 7.58 (d, *J* = 8.6 Hz, 1H), 7.40 (d, *J* = 8.5 Hz, 1H), 7.26 - 7.20 (m, 2H), 7.15 (s, 1H), 7.09 (dd, *J* = 9.1, 2.3 Hz, 1H), 6.75 (d, *J* = 9.1 Hz, 2H), 6.60 (d, *J* = 9.1 Hz, 2H), 5.10 (dd, *J* = 13.3, 5.1 Hz, 1H), 4.33 (d, *J* = 16.7 Hz, 1H), 4.21 (d, *J* = 16.7 Hz, 1H), 3.98 - 3.90 (m, 2H), 3.71 - 3.63 (m, 2H), 3.60 - 3.48 (m, 6H), 3.21 (s, 3H), 3.15 (s, 4H), 2.98 - 2.84 (m, 1H), 2.66 - 2.51 (m, 7H), 2.38 (tt, *J* = 13.3, 6.6 Hz, 1H), 2.04 - 1.94 (m, 1H).

MS calculated for 848.31; found 849.2 [M+H]⁺.

### Example 23: Synthesis of 2-(2,6-dioxopiperidin-3-yl)-5-(2-(2-(ethyl(2-(4-((6-hydroxy-2-(4-(methylsulfonyl)phenyl)naphthalen-1-yl)oxy)phenoxy)ethyl)amino)ethoxy)ethoxy)isoindoline-1,3-dione (compound D23)

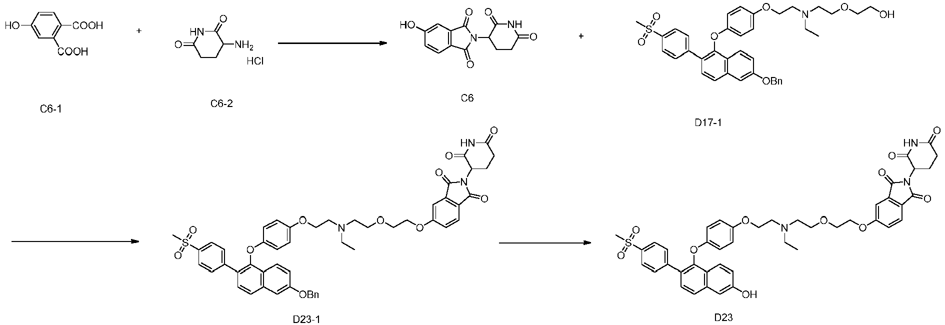

Sodium acetate (8.1 g, 98.8 mmol) and 4-hydroxyphthalic acid (compound C6-1) (6 g, 32.9 mmol) were added to a solution of 3-aminopiperidine-2,6-dione hydrochloride (compound C6-2) (8.13 g, 49.4 mmol) in acetic acid (90 mL). The mixture was heated to 120°C and stirred for 18 hours. The mixture was concentrated under vacuum, dispersed in water, and filtered. The filter cake was washed with water and then dried under vacuum. The crude product was purified by silica gel column chromatography (dichloromethane/methanol = 10/1) to obtain 2-(2,6-dioxopiperidin-3-yl)-5-hydroxyisoindoline-1,3-dione (compound C6) (8.7 g, yield: 58%) as a yellow solid.

Triphenylphosphine (191 mg, 0.73 mmol) and DIAD (148 mg, 0.73 mmol) were added to a solution of compound D17-1 (160 mg, 0.24 mmol) and compound C6 (66 mg, 0.24 mmol) in tetrahydrofuran (6 mL) at 0°C, and the reaction mixture was warmed to room temperature and stirred for 18 hours. The reaction mixture was concentrated under vacuum and the crude product was purified by silica gel column chromatography (dichloromethane/methanol = 10/1) to obtain 5-(2-(2-((2-(4-((6-(benzyloxy)-2-(4-(methylsulfonyl)phenyl)naphthalen-1-yl)oxy)phenoxy)ethyl)(ethyl)amino)ethoxy)ethoxy)-2-(2,6-dioxopiperidin-3-yl)isoindoline-1,3-dione (compound D23-1) (90 mg, yield: 41%) as a yellow gel.

Wet palladium on carbon (100.0 mg, 10 wt%) was added to a solution of compound D23-1 (110 mg, 0.12 mmol) in methanol (5 mL)/tetrahydrofuran (5 mL). The mixture was stirred at 25°C for 16 hours under hydrogen balloon atmosphere. The mixture was filtered, concentrated under vacuum, and purified by silica gel column chromatography (dichloromethane/methanol = 10/1) to obtain compound D23 (8 mg, yield: 8%) as a white solid.

¹H NMR (500 MHz, DMSO-*d*₆) δ 11.06 (s, 1H), 10.04 (s, 1H), 7.90 (d, *J* = 8.5 Hz, 2H), 7.83 (d, *J* = 8.5 Hz, 2H), 7.76 (d, *J* = 8.6 Hz, 1H), 7.72 (d, *J* = 3.5 Hz, 1H), 7.71 (d, *J* = 4.4 Hz, 1H), 7.57 (d, = 8.6 Hz, 1H), 7.24 (d, *J* = 2.4 Hz, 1H), 7.20 - 7.12 (m, 2H), 7.09 (dd, *J* = 9.1, 2.4 Hz, 1H), 6.72 (d, *J* = 8.5 Hz, 2H), 6.58 (d, *J* = 8.5 Hz, 2H), 5.14 (dd, *J* = 13.0, 5.4 Hz, 1H), 3.95 - 3.66 (m, 4H), 3.52 - 3.35 (m, 4H), 3.21 (s, 3H), 2.99 - 2.88 (m, 1H), 2.81 - 2.63 (m, 3H), 2.62 - 2.51 (m, 5H), 2.04 - 1.97 (m, 1H), 0.99 - 0.82 (m, 3H).

MS calculated for 821.26; found 822.2 [M+H]⁺.

### Example 24: Synthesis of 2-(2,6-dioxopiperidin-3-yl)-5-(2-(2-(2-(ethyl(2-(4-((6-hydroxy-2-(4-(methylsulfonyl)phenyl)naphthalen-1-yl)oxy)phenoxy)ethyl)amino)ethoxy)ethoxy)ethoxy)isoindoline-1,3-dione (compound D24)

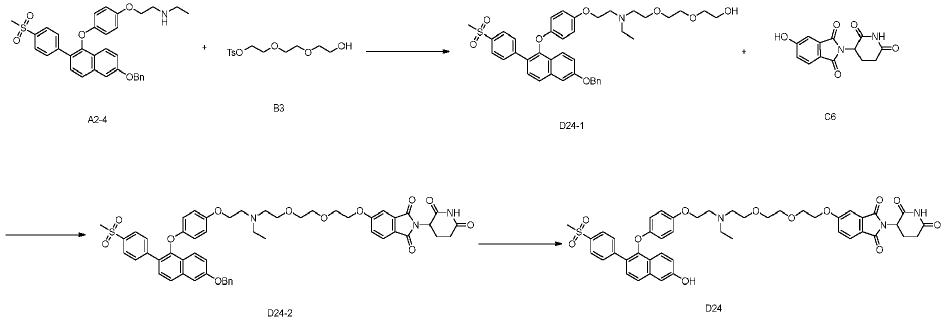

A reaction mixture of compound A2-4 (300 mg, 0.53 mmol), compound B3 (161 mg, 0.53 mmol), potassium carbonate (220 mg, 1.59 mmol), and potassium iodide (26 mg, 0.16 mmol) in DMF (4 mL) was stirred at 80°C for 18 hours. The mixture was cooled, concentrated under vacuum, and purified by silica gel column chromatography (dichloromethane/methanol = 10/1) to obtain 2-(2-(2-((2-(4-((6-(benzyloxy)-2-(4-(methylsulfonyl)phenyl)naphthalen-1-yl)oxy)phenoxy)ethyl)(ethyl)amino)ethoxy)ethoxy)ethan-1-ol (compound D24-1) (320 mg, yield: 86%) as a white solid.

Triphenylphosphine (362 mg, 1.38 mmol) and DIAD (279 mg, 1.38 mmol) were added to a solution of compound D24-1 (320 mg, 0.46 mmol) and compound C6 (138 mg, 0.5 mmol) in tetrahydrofuran (5 mL) at 0°C, and the reaction mixture was warmed to room temperature and stirred for 18 hours. The reaction mixture was concentrated under vacuum and purified by silica gel column chromatography (dichloromethane/methanol = 10/1) to obtain 5-(2-(2-(2-((2-(4-((6-(benzyloxy)-2-(4-(methylsulfonyl)phenyl)naphthalen-1-yl)oxy)phenoxy)ethyl)(ethyl)amino)ethoxy)ethoxy)ethoxy)-2-(2,6-dioxopiperidin-3-yl)isoindoline-1,3-dione (compound D24-2) (190 mg, yield: 43%) as a yellow gel.

Wet palladium on carbon (150.0 mg, 10 wt%) was added to a solution of compound D24-2 (190 mg, 0.2 mmol) in methanol (2 mL)/tetrahydrofuran (10 mL). The mixture was stirred at 30°C for 18 hours under hydrogen balloon atmosphere. The mixture was filtered, washed with dichloromethane/tetrahydrofuran/methanol, concentrated under vacuum, and the crude product was purified by pre-HPLC to obtain compound D24 (25 mg, yield: 14%) as a yellow solid.

¹H NMR (400 MHz, DMSO-*d*₆) δ 8.19 (s, 1H), 7.90 (d, *J* = 8.3 Hz, 2H), 7.82 (d, *J* = 8.3 Hz, 2H), 7.78 - 7.67 (m, 3H), 7.57 (d, *J* = 8.6 Hz, 1H), 7.24 (d, *J* = 2.3 Hz, 1H), 7.19 - 7.12 (m, 2H), 7.09 (dd, *J* = 9.1, 2.4 Hz, 1H), 6.71 (d, *J* = 9.1 Hz, 2H), 6.58 (d, *J* = 9.1 Hz, 2H), 5.14 (dd, *J* = 13.0, 5.4 Hz, 1H), 3.88 - 3.80 (m, 3H), 3.79 - 3.71 (m, 1H), 3.45 - 3.43 (m, 4H), 3.42 - 3.39 (m, 4H), 3.21 (s, 3H), 2.98 - 2.90 (m, 1H), 2.78 - 2.68 (m, 3H), 2.60 (t, *J* = 6.2 Hz, 2H), 2.53 (d, *J* = 7.6 Hz, 3H), 2.08 - 1.96 (m, 1H), 0.91 (t, *J* = 7.1 Hz, 3H).

MS calculated for 865.29; found 866.2 [M+H]⁺.

### Example 25: Synthesis of N-(2-(2,6-dioxopiperidin-3-yl)-1-oxoisoindolin-4-yl)-2-(2-((2 S, SR)-4-(2-(4-((6-hydroxy-2-(4-(methylsulfonyl)phenyl)naphthalen-1-yl)oxy)phenoxy)ethyl)-2,5-dimethylpiperazin-1-yl)ethoxy)acetamide (compound D25)

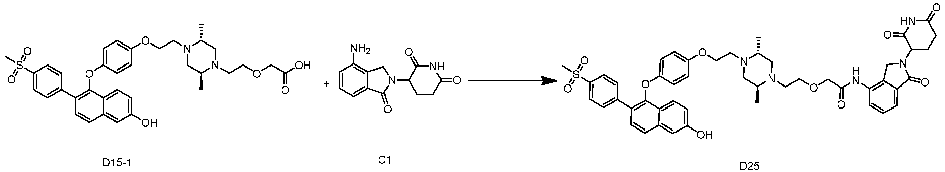

Compound D15-1 (150 mg, 0.23 mmol) was dissolved in DMF (1 mL), and compound C1 (59.6 mg, 0.23 mmol), TCFH (77.7 mg, 0.277 mmol), and NMI (56.6 mg, 0.69 mmol) were sequentially added thereto at room temperature. The reaction mixture was stirred at room temperature for 18 hours. The reaction mixture was diluted with water, and extracted three times with ethyl acetate. The organic phases were combined, washed with saturated brine, dried over anhydrous sodium sulfate, and evaporated to dryness by rotary evaporation. The crude product was purified by pre-HPLC to obtain compound D25 (11.8 mg, yield: 5.78%) as a white solid.

¹H NMR (400 MHz, DMSO-*d*₆) δ 11.06 (s, 1H), 9.89 (s, 1H), 7.93 (d, *J* = 8.6 Hz, 2H), 7.88 (d, *J* = 8.5 Hz, 2H), 7.80 (d, *J* = 8.8 Hz, 2H), 7.75 (d, *J* = 9.2 Hz, 1H), 7.64 - 7.58 (m, 2H), 7.55 (t, *J* = 7.6 Hz, 1H), 7.29 (s, 1H), 7.13 (d, *J* = 9.2 Hz, 1H), 6.79 (d, *J* = 8.6 Hz, 2H), 6.64 (d, *J* = 9.0 Hz, 2H), 5.20 (dd, *J* = 13.1, 5.2 Hz, 1H), 4.46 - 4.33 (m, 2H), 4.24 (d, *J* = 6.0 Hz, 2H), 4.04 (s, 3H), 3.70 (s, 1H), 3.25 (s, 6H), 3.03 - 2.91 (m, 2H), 2.82 (s, 1H), 2.72 - 2.58 (m, 1H), 2.33 (d, *J* = 17.5 Hz, 1H), 2.06 (s, 1H), 1.36 - 1.07 (m, 8H).

MS calculated for 889.34; found 890.2 [M+H]⁺.

### Example 26: Synthesis of 3-(4-((2-(2-(2-(ethyl(2-(4-((6-hydroxy-2-(4-(methylsulfonyl)phenyl)naphthalen-1-yl)oxy)phenoxy)ethyl)amino)ethoxy)ethoxy)ethyl)amino)-1-oxoisoindolin-2-yl)piperidine-2,6-dione (compound D26)

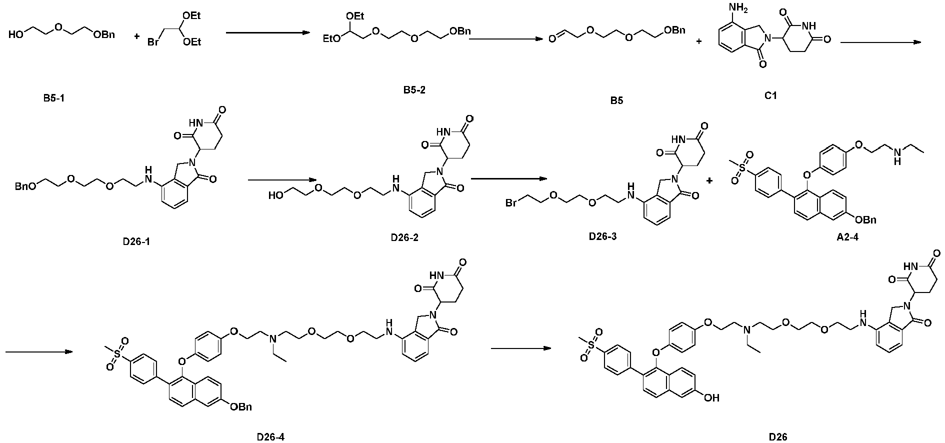

2-(2-(Benzyloxy)ethoxy)ethan-1-ol (compound B5-1) (500.0 mg, 2.55 mmol) was slowly added to a suspension of sodium hydride (132.0 mg, 3.3 mmol) in tetrahydrofuran (5.0 mL) at 0°C, and the reaction mixture was stirred at room temperature for 45 minutes. The reaction mixture was cooled to 0°C, slowly added with bromoacetaldehyde diethyl acetal (2.6 g, 13.2 mmol), heated to 55°C, and stirred for 16 hours. The reaction mixture was concentrated under reduced pressure, added with saturated ammonium chloride aqueous solution (15.0 mL), extracted twice with dichloromethane (10.0 mL), washed with saturated brine (10.0 mL), dried over anhydrous sodium sulfate, filtered, and concentrated under reduced pressure. The crude product was purified by silica gel column chromatography (petroleum ether/ethyl acetate = 4/1) to obtain 10-ethoxy-1-phenyl-2,5,8,11-tetraoxatridecane (compound B5-2) (383 mg, yield: 48.1%).

Compound B5-2 (173.0 mg, 0.55 mmol) was added to a mixed solvent of concentrated hydrochloric acid (0.3 mL), water (0.3 mL), and tetrahydrofuran (3.0 mL) at room temperature, and the reaction mixture was heated to 50°C and stirred for 1.5 hours. The reaction mixture was concentrated under reduced pressure, added with water (10.0 mL), and extracted twice with ethyl acetate (10.0 mL). The organic phases were combined, washed with saturated brine, dried over anhydrous sodium sulfate, filtered, and concentrated under reduced pressure to obtain 2-(2-(2-(benzyloxy)ethoxy)ethoxy)acetaldehyde (compound B5) (149 mg, yield: 100%).

Compound B5 (149.0 mg, 0.55 mmol), compound C1 (162.0 mg, 0.625 mmol), and two drops of acetic acid were sequentially added to methanol (3.0 mL) at room temperature. The reaction mixture was stirred at room temperature for 30 minutes, added with sodium triacetoxyborohydride (331.0 mg, 1.56 mmol), and stirred at 20°C for 16 hours. The reaction mixture was added with sodium cyanoborohydride (118.0 mg, 1.88 mmol), and stirred at 30°C for 5 hours. The reaction mixture was concentrated under reduced pressure and purified by silica gel column chromatography (dichloromethane/methanol = 10/1) to obtain 3-(4-((2-(2-(2-(2-(2-(benzyloxy)ethoxy)ethoxy)ethyl)amino)-1-oxoisoindolin-2-yl)piperidine-2,6-dione (compound D26-1) (140 mg, yield: 53%).

Compound D26-1 (140.0 mg, 0.29 mmol) and wet palladium on carbon (100.0 mg, 10 wt%) were sequentially added to methanol (10.0 mL) at room temperature. The reaction mixture was stirred at 20°C for 16 hours under hydrogen atmosphere, filtered through diatomite, and the filtrate was concentrated under reduced pressure to obtain 3-(4-((2-(2-(2-(2-hydroxyethoxy)ethoxy)ethyl)amino)-1-oxoisoindolin-2-yl)piperidine-2,6-dione (compound D26-2) (100 mg, yield: 88%).

Carbon tetrabromide (475 mg, 1.43 mmol) was slowly added to a solution of compound D26-2 (374.0 mg, 0.955 mmol) and triphenylphosphine (375 mg, 1.43 mmol) in dichloromethane (8.0 mL) at 0°C, and the reaction mixture was stirred at room temperature for 16 hours. The reaction mixture was concentrated under reduced pressure and purified by silica gel column chromatography (dichloromethane/methanol = 10/1) to obtain 3-(4-((2-(2-(2-bromoethoxy)ethoxy)ethyl)amino)-1-oxoisoindolin-2-yl)piperidine-2,6-dione (compound D26-3) (410 mg, yield: 94.5%).

Compound D26-3 (410.0 mg, 0.9 mmol), compound A2-4 (406 mg, 0.72 mmol), DIPEA (0.39 mL, 2.2 mmol), and potassium iodide (15 mg, 0.09 mmol) were sequentially added to DMF (5.0 mL) at room temperature. The reaction mixture was stirred at 85°C for 16 hours. The reaction mixture was cooled to room temperature, concentrated under reduced pressure, added with water (10.0 mL), and extracted twice with dichloromethane (10.0 mL). The organic phases were combined, washed with saturated brine (10.0 mL), dried over anhydrous sodium sulfate, filtered, and concentrated under reduced pressure. The crude product was purified by silica gel column chromatography (dichloromethane/methanol = 10/1) to obtain 3-(4-((2-(2-(2-((2-(4-((6-(benzyloxy)-2-(4-(methylsulfonyl)phenyl)naphthalen-1-yl)oxy)phenoxy)ethyl)(ethyl)amino)ethoxy)ethoxy)ethyl)amino)-1-oxoisoindolin-2-yl)piperidine-2,6-dione (compound D26-4) (220 mg, yield: 32.7%).

Compound D26-4 (220.0 mg, 0.23 mmol) and wet palladium on carbon (100.0 mg, 10 wt%) were sequentially added to a mixed solvent of methanol (15.0 mL) and tetrahydrofuran (5.0 mL) at room temperature. The reaction mixture was stirred at 20°C for 16 hours under hydrogen atmosphere, filtered through diatomite, and the filtrate was concentrated under reduced pressure. The crude product was purified by silica gel column chromatography (dichloromethane/methanol = 10/1) to obtain compound D26 (62 mg, yield: 31.2%).

¹H NMR (400 MHz, DMSO-d₆) δ: 11.01 (s, 1H), 10.06 (s, 1H), 7.92 (d, *J* = 8.4 Hz, 2H), 7.85 (d, *J* = 8.4 Hz, 2H), 7.82 - 7.71 (m, 2H), 7.59 (t, *J* = 8.6 Hz, 1H), 7.32 - 7.24 (m, 2H), 7.11 (dt, *J* = 10.1, 5.0 Hz, 1H), 6.95 (t, *J* = 6.8 Hz, 1H), 6.77 (dd, *J* = 21.1, 8.5 Hz, 3H), 6.61 (d, *J* = 9.1 Hz, 2H), 5.61 - 5.53 (m, 1H), 5.12 (dt, *J* = 12.6, 6.3 Hz, 1H), 4.19 (dd, *J* = 41.3, 17.1 Hz, 2H), 3.87 (t, *J* = 5.9 Hz, 2H), 3.63 (t, *J* = 6.3 Hz, 1H), 3.58 (t, *J* = 5.9 Hz, 2H), 3.52 (d, *J* = 4.5 Hz, 4H), 3.45 (t, *J* = 5.9 Hz, 2H), 3.32 - 3.27 (m, 2H), 3.23 (s, 3H), 2.94 (ddd, *J* = 18.1, 13.3, 5.0 Hz, 1H), 2.81 - 2.58 (m, 5H), 2.39 - 2.24 (m, 1H), 2.09 - 2.00 (m, 1H), 1.84 - 1.74 (m, 1H), 0.94 (t, *J* = 7.0 Hz, 3H).

MS calculated for 850.32; found 851.2 [M+H]⁺.

### Example 27: Synthesis of 3-(5-(4-(2-(2-(2-(2-(2-(4-((6-hydroxy-2-(4-(methylsulfonyl)phenyl)naphthalen-1-yl)oxy)phenoxy)ethoxy)ethoxy)ethoxy)ethyl)piperazin-1-yl)-1-oxoisoindolin-2-yl)piperidine-2,6-dione (compound D27)

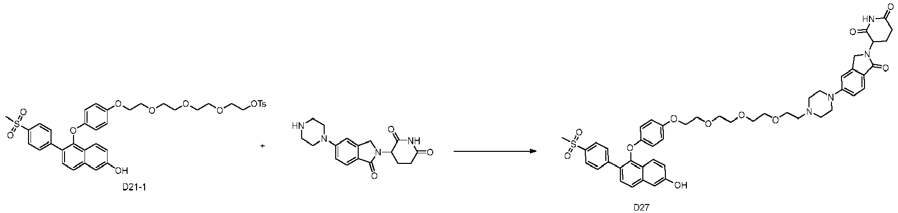

Compound D21-1, 3-(1-oxo-5-(piperazin-1-yl)isoindolin-2-yl)piperidine-2,6-dione (non-salt substance corresponding to compound C2) (27 mg, 0.075 mmol), DIPEA (0.06 mL, 0.34 mmol), and potassium iodide (1.1 mg, 0.0068 mmol) were sequentially added to acetonitrile (5 mL) at room temperature, and the resulting mixture was replaced with nitrogen and then stirred at 105°C for 16 hours. The mixture was cooled to room temperature, concentrated under reduced pressure, and purified by silica gel column chromatography (dichloromethane/methanol = 10/1) to obtain compound D27 (8.4 mg, yield: 14%).

¹H NMR (400 MHz, DMSO-*d*₆) δ 10.97 (s, 1H), 8.28 (s, 1H), 7.92 (d, *J* = 8.3 Hz, 2H), 7.85 (d, *J* = 8.3 Hz, 2H), 7.76 (dd, *J* = 17.6, 8.9 Hz, 2H), 7.60 (d, *J* = 8.6 Hz, 1H), 7.53 (d, *J* = 8.6 Hz, 1H), 7.27 (d, *J* = 2.3 Hz, 1H), 7.12 (dd, *J* = 9.1, 2.4 Hz, 1H), 7.05 (d, *J* = 8.1 Hz, 2H), 6.81 - 6.74 (m, 2H), 6.65 - 6.59 (m, 2H), 5.07 (dd, *J* = 13.3, 5.0 Hz, 1H), 4.34 (d, *J* = 16.9 Hz, 1H), 4.22 (d, *J* = 16.8 Hz, 1H), 4.00 - 3.92 (m, 2H), 3.68 (t, *J* = 4.6 Hz, 3H), 3.58 - 3.51 (m, 15H), 3.25 (d, *J* = 12.0 Hz, 11H), 2.92 (ddd, *J* = 18.0, 13.4, 5.3 Hz, 1H), 2.66 - 2.56 (m, 4H), 2.38 (dd, *J* = 13.4, 4.7 Hz, 1H), 1.98 (d, *J* = 14.4 Hz, 1H).

MS calculated for 892.3; found 893.0 [M+H]⁺.

### Example 28: Synthesis of 2-(2,6-dioxopiperidin-3-yl)-5-(4-(2-(2-(2-(ethyl(2-(4-((6-hydroxy-2-(4-(methylsulfonyl)phenyl)naphthalen-1-yl)oxy)phenoxy)ethyl)amino)ethoxy)ethoxy)ethyl)piperazin-1-yl)isoindoline-1,3-dione (compound D28)

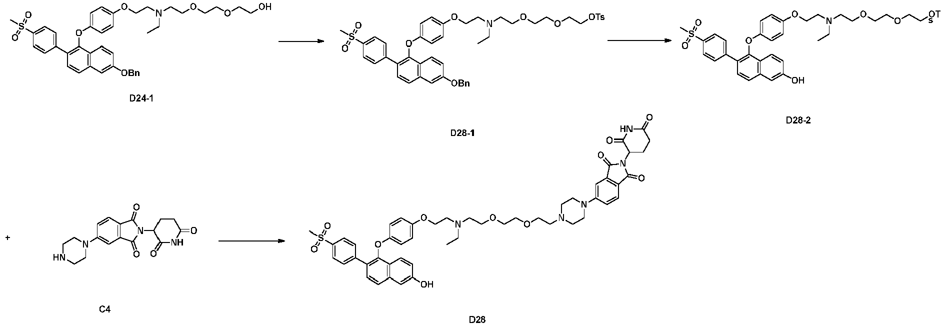

Compound D24-1 (280 mg, 0.4 mmol), TsCl (99 mg, 0.52 mmol), triethylamine (0.17 mL, 1.2 mmol), and DMAP (5 mg, 0.04 mmol) were sequentially added to dichloromethane (5 mL) at room temperature, and the resulting mixture was replaced with nitrogen and then stirred at room temperature for 16 hours. The mixture was cooled to room temperature, concentrated under reduced pressure, and purified by silica gel column chromatography (dichloromethane/methanol = 10/1) to obtain 2-(2-(2-((2-(4-((6-(benzyloxy)-2-(4-(methylsulfonyl)phenyl)naphthalen-1-yl)oxy)phenoxy)ethyl)(ethyl)amino)ethoxy)ethoxy)ethyl 4-methylbenzenesulfonate (compound D28-1) (360 mg, yield: 100%).

Compound D28-1 (120 mg, 0.14 mmol) and wet palladium on carbon (120.0 mg, 10 wt%) were sequentially added to tetrahydrofuran (10 mL)/methanol (7 mL) at room temperature, and the resulting mixture was replaced with nitrogen and then stirred at room temperature for 16 hours. The mixture was filtered, and the resulting filtrate was concentrated under reduced pressure and purified by silica gel column chromatography (dichloromethane/methanol = 10/1) to obtain 2-(2-(2-(ethyl(2-(4-((6-hydroxy-2-(4-(methylsulfonyl)phenyl)naphthalen-1-yl)oxy)phenoxy)ethyl)amino)ethoxy)ethoxy)ethyl 4-methylbenzenesulfonate (compound D28-2) (7 mg, yield: 73%).

Compound D28-2 (78 mg, 0.1 mmol), 2-(2,6-dioxopiperidin-3-yl)-5-(piperazin-1-yl)isoindoline-1,3-dione (non-salt substance corresponding to compound C4) (42 mg, 0.11 mmol), DIPEA (0.09 mL, 0.5 mmol), and potassium iodide (2 mg, 0.01 mmol) were sequentially added to acetonitrile (5 mL) at room temperature, and the resulting mixture was replaced with nitrogen and then stirred at 105°C for 16 hours. The mixture was cooled to room temperature, concentrated under reduced pressure, and purified by silica gel column chromatography (dichloromethane/methanol = 90/10) to obtain compound D28 (7.7 mg, yield: 8%).

¹H NMR (400 MHz, DMSO-*d*₆) δ 11.10 (s, 1H), 10.07 (s, 1H), 7.92 (d, *J* = 8.2 Hz, 2H), 7.86 (d, *J* = 8.3 Hz, 2H), 7.81 - 7.67 (m, 3H), 7.60 (d, *J* = 8.8 Hz, 1H), 7.35 (s, 1H), 7.30 - 7.23 (m, 2H), 7.14 - 7.09 (m, 1H), 6.78 (d, *J* = 8.5 Hz, 2H), 6.63 (d, *J* = 8.7 Hz, 2H), 5.10 (dd, *J* = 12.7, 5.4 Hz, 1H), 3.99 (s, 1H), 3.67 - 3.40 (m, 22H), 3.24 (s, 4H), 2.91 (s, 2H), 2.63 (s, 7H), 2.09 - 1.99 (m, 1H).

MS calculated for 933.36; found 934.0 [M+H]⁺.

### Example 29: Synthesis of 3-(6-((2-(2-(2-(2-(4-((6-hydroxy-2-(4-(methylsulfonyl)phenyl)naphthalen-1-yl)oxy)phenoxy)ethoxy)ethoxy)ethoxy)ethoxy)ethyl)amino)-1-oxoisoindolin-2-yl)piperidine-2,6-dione (compound D29)

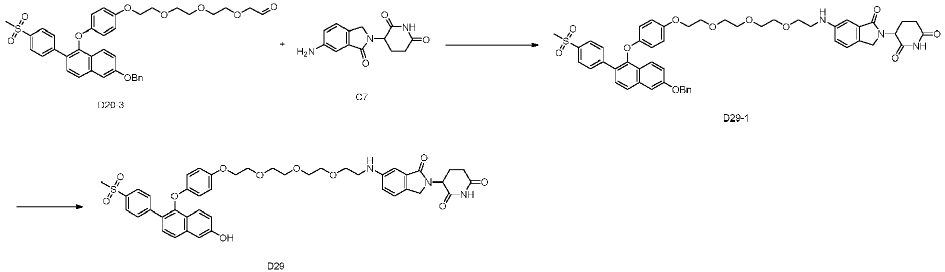

Compound D20-3 (450.0 mg, 0.67 mmol), 3-(6-amino-1-oxoisoindolin-2-yl)piperidine-2,6-dione (compound C7) (158.0 mg, 0.61 mmol), and three drops of acetic acid were sequentially added to a mixed solvent of methanol (5.0 mL) and dichloromethane (2.0 mL) at room temperature. The reaction mixture was stirred at room temperature for 20 minutes, added with sodium triacetoxyborohydride (323.0 mg, 1.53 mmol), and stirred at 20°C for 16 hours. The reaction mixture was added with sodium cyanoborohydride (115.0 mg, 1.83 mmol), and stirred at 30°C for 4 hours. The reaction mixture was concentrated under reduced pressure and purified by silica gel column chromatography (dichloromethane/methanol = 10/1) to obtain 3-(6-((2-(2-(2-(2-(4-((6-(benzyloxy)-2-(4-(methylsulfonyl)phenyl)naphthalen-1-yl)oxy)phenoxy)ethoxy)ethoxy)ethoxy)ethyl)amino)-1-oxoisoindolin-2-yl)piperidine-2,6-dione (compound D29-1) (125 mg, yield: 22.4%).

Compound D29-1 (125.0 mg, 0.136 mmol) and wet palladium on carbon (80.0 mg, 10 wt%) were sequentially added to a mixed solvent of methanol (8.0 mL) and tetrahydrofuran (3.0 mL) at room temperature. The reaction mixture was stirred at 20°C for 16 hours under hydrogen atmosphere, filtered through diatomite, and the filtrate was concentrated under reduced pressure. The crude product was purified by silica gel column chromatography (dichloromethane/methanol = 10/1) to obtain compound D29 (60 mg, yield: 53.3%).

¹H NMR (400 MHz, DMSO-d₆) δ: 10.94 (s, 1H), 10.02 (s, 1H), 7.93 (d, *J* = 8.4 Hz, 2H), 7.86 (d, *J* = 8.3 Hz, 2H), 7.77 (dd, *J* = 16.9, 8.9 Hz, 2H), 7.60 (t, *J* = 7.5 Hz, 1H), 7.27 (t, *J* = 5.7 Hz, 2H), 7.11 (dt, *J* = 8.9, 4.4 Hz, 1H), 6.94 - 6.83 (m, 2H), 6.76 (d, *J* = 9.1 Hz, 2H), 6.62 (d, *J* = 9.0 Hz, 2H), 5.88 (q, *J* = 6.0 Hz, 1H), 5.08 (dt, *J* = 15.5, 7.7 Hz, 1H), 4.20 (dt, *J* = 48.7, 15.0 Hz, 2H), 3.98 - 3.90 (m, 2H), 3.70 - 3.50 (m, 14H), 3.24 (s, 3H), 2.99 - 2.84 (m, 1H), 2.61 (d, *J* = 16.6 Hz, 1H), 2.45 - 2.32 (m, 1H), 1.83 - 1.75 (m, 1H).

MS calculated for 823.28; found 824.2 [M+H]⁺.

### Example 30: Synthesis of 3-(5-(4-(2-(2-(2-(4-((6-hydroxy-2-(4-(methylsulfonyl))phenyl)naphthalen-1-yl)oxy)phenoxy)ethoxy)ethoxy)ethyl)piperazin-1-yl)-1-oxoisoindolin-2-yl)piperidine-2,6-dione (compound D30)

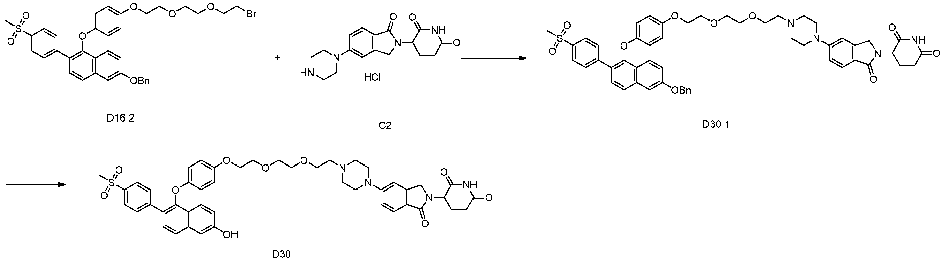

Compound D16-2 (78.0 mg, 0.11 mmol), compound C2 (41 mg, 0.11 mmol), DIPEA (78 µL, 0.44 mmol), and potassium iodide (2 mg, 0.01 mmol) were sequentially added to acetonitrile (3.0 mL) at room temperature. The reaction mixture was stirred at 90°C for 16 hours. The reaction mixture was cooled to room temperature, concentrated under reduced pressure, added with water (10.0 mL), and extracted twice with dichloromethane (10.0 mL). The organic phases were combined, washed with saturated brine (10.0 mL), dried over anhydrous sodium sulfate, filtered, and concentrated under reduced pressure. The crude product was purified by silica gel column chromatography (dichloromethane/methanol = 10/1) to obtain 3-(5-(4-(2-(2-(2-(4-((6-(benzyloxy)-2-(4-(methylsulfonyl)phenyl)naphthalen-1-yl)oxy)phenoxy)ethoxy))ethoxy)ethyl)piperazin-1-yl)-1-oxoisoindolin-2-yl)piperidine-2,6-dione (compound D30-1) (103 mg, yield: 97.2%).

Compound D30-1 (103.0 mg, 0.11 mmol) and wet palladium on carbon (80.0 mg, 10 wt%) were sequentially added to a mixed solvent of methanol (8.0 mL) and tetrahydrofuran (3.0 mL) at room temperature. The reaction mixture was stirred at 20°C for 16 hours under hydrogen atmosphere, filtered through diatomite, and the filtrate was concentrated under reduced pressure. The crude product was purified by silica gel column chromatography (dichloromethane/methanol = 10/1) to obtain compound D30 (12 mg, yield: 12.9%).

¹H NMR (400 MHz, DMSO-d₆) δ:10.96 (s, 1H), 10.06 (s, 1H), 7.92 (d, *J* = 8.4 Hz, 2H), 7.85 (d, *J* = 8.3 Hz, 2H), 7.76 (dd, *J* = 18.2, 8.9 Hz, 2H), 7.60 (d, *J* = 8.6 Hz, 1H), 7.53 (d, *J* = 8.2 Hz, 1H), 7.27 (t, *J* = 5.4 Hz, 1H), 7.13 - 7.01 (m, 3H), 6.76 (t, *J* = 10.5 Hz, 2H), 6.61 (t, *J* = 11.3 Hz, 2H), 5.06 (dt, *J* = 12.4, 6.2 Hz, 1H), 4.40 - 4.18 (m, 2H), 3.96 (s, 2H), 3.69 (d, *J* = 4.4 Hz, 3H), 3.55 (d, *J* = 7.7 Hz, 7H), 3.39 (s, 2H), 3.25 (d, *J* = 10.2 Hz, 7H), 2.92 (dd, *J* = 21.7, 8.9 Hz, 1H), 2.73 - 2.57 (m, 4H), 2.09 - 1.91 (m, 1H).

MS calculated for 848.31; found 849.2 [M+H]⁺.

### Example 31: Synthesis of 2-(2,6-dioxopiperidin-3-yl)-5-(4-(N-(5-(ethyl(2-(4-((6-hydroxy-2-(4-(methylsulfonyl))phenyl)naphthalen-1-yl)oxy)phenoxy)ethyl)aminopentyl)-N-methylglycyl)piperazin-1-yl)isoindoline-1,3-dione (compound D31)

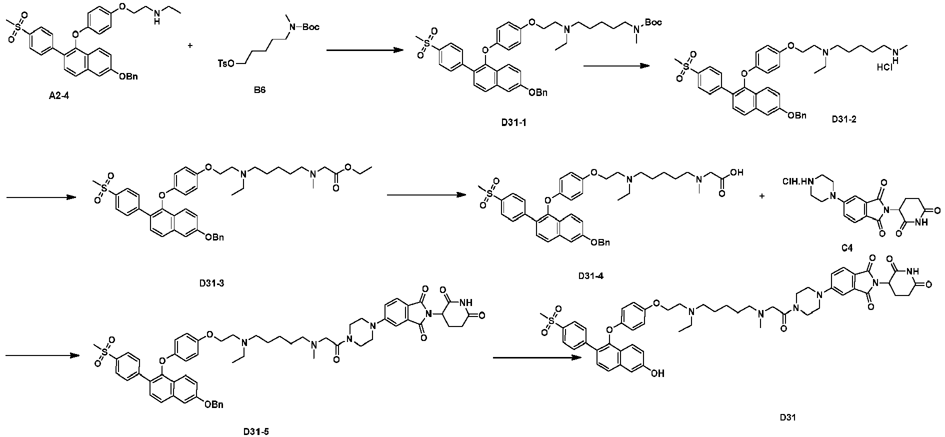

Compound B6 (466 mg, 1.26 mmol) was added to a solution of compound A2-4 (200 mg, 0.35 mmol) and triethylamine (178 mg, 1.76 mmol) in DMF (3 mL), and the reaction mixture was heated to 80°C and stirred for 4 hours. The reaction mixture was diluted with water (10 mL), and extracted three times with ethyl acetate (10 mL). The organic phases were combined, washed twice with saturated brine (10 mL), dried over anhydrous sodium sulfate, and concentrated to obtain tert-butyl (5-((2-(4-((6-(benzyloxy)-2-(4-(methylsulfonyl)phenyl)naphthalen-1-yl)oxy)phenoxy)ethyl)(ethyl)amino)pentyl)(methyl)carbamate (compound D31-1) (300 mg, yield: 100%) as a yellow oily liquid.

A solution of hydrochloric acid in dioxane (1 mL, 4 M) was added to a solution of compound D31-1 (300 mg, 0.35 mmol) in dichloromethane (7 mL) under an ice-water bath, and the reaction mixture was warmed to room temperature and stirred for 1 hour. The reaction mixture was concentrated, added with ethyl acetate and concentrated three times to obtain compound D31-2 (300 mg hydrochloride) as a yellow solid.

Ethyl bromoacetate (88 mg, 0.53 mmol) was added to a solution of compound D31-2 (300 mg, hydrochloride) and triethylamine (224 mg, 1.75 mmol) in DMF (5 mL), and the reaction mixture was heated to 90°C and stirred for 16 hours. The reaction mixture was concentrated with an oil pump and purified by silica gel column chromatography (dichloromethane/methanol = 10/1) to obtain ethyl N-(5-((2-(4-((6-(benzyloxy)-2-(4-(methylsulfonyl)phenyl)naphthalen-1-yl)oxy)phenoxy)ethyl)(ethyl)amino)pentyl)-N-methylglycinate (compound D31-3) (200 mg, yield: 75.9%) as a yellow solid.

Compound D31-3 (100 mg, 0.13 mmol) and lithium hydroxide monohydrate (17 mg, 0.398 mmol) were added to a mixed solution of tetrahydrofuran (3 mL) and water (1 mL), and the reaction mixture was stirred at room temperature for 1 hour. The pH was adjusted to 3 to 4, and the reaction mixture was concentrated to obtain N-(5-((2-(4-((6-(benzyloxy)-2-(4-(methylsulfonyl)phenyl)naphthalen-1-yl)oxy)phenoxy)ethyl)(ethyl)amino)pentyl)-N-methylglycine (compound D31-4) (100 mg, yield: 100%) as a white solid.

HATU (77 mg, 0.20 mmol) was added to a solution of compound D31-4 (100 mg, 0.13 mmol), compound C4 (76 mg, 0.20 mmol), and DIPEA (84 mg, 0.65 mmol) in DMF (3 mL), and the reaction mixture was stirred at room temperature for 1 hour. The reaction mixture was concentrated with an oil pump, and the crude product was purified by pre-HPLC4 to obtain 5-(4-(N-(5-((2-(4-((6-(benzyloxy)-2-(4-(methylsulfonyl)phenyl)naphthalen-1-yl)oxy)phenoxy)ethyl)(ethyl)amino)pentyl)-N-methylglycyl)piperazin-1-yl)-2-(2,6-dioxopiperidin-3-yl)isoindoline-1,3-dione (compound D31-5) (80 mg, yield: 54.8%) as a yellow solid.

Wet palladium on carbon (60 mg, 10 wt%) was added to a solution of compound D31-5 (60 mg, 0.052 mmol) in tetrahydrofuran (2 mL) and methanol (1 mL) under nitrogen atmosphere, and the reaction mixture was stirred at room temperature for 16 hours. After the reaction was completed, the reaction mixture was filtered and the filtrate was concentrated. The crude product was purified by prep-HPLC to obtain compound D31 (15 mg, yield: 30.2%) as a yellow solid.

¹H NMR (400 MHz, DMSO-*d*₆) δ 11.07 (s, 1H), 10.04 (s, 1H), 7.89 (d, *J* = 8.4 Hz, 2H), 7.83 (d, *J* = 8.4 Hz, 2H), 7.75 (d, *J* = 8.6 Hz, 1H), 7.69 (dd, *J=* 14.5, 8.8 Hz, 2H), 7.57 (d, *J* = 8.6 Hz, 1H), 7.33 (s, 1H), 7.25 (s, 1H), 7.22 (d, *J* = 8.8 Hz, 1H), 7.09 (d, *J* = 9.0 Hz, 1H), 6.72 (d, *J=* 9.2 Hz, 2H), 6.59 (d, *J* = 8.9 Hz, 2H), 5.07 (dd, *J=* 13.0, 5.0 Hz, 1H), 3.95 - 3.80 (m, 2H), 3.67 (s, 2H), 3.60 - 3.55 (m, 2H), 3.50 - 3.48 (m, 2H), 3.45 - 3.42 (m, 2H), 3.38 - 3.34 (m, 4H), 3.21 (s, 5H), 2.96 - 2.71 (m, 4H), 2.63 - 2.54 (m, 4H), 2.33 (s, 1H), 2.18 (s, 2H), 1.40 (s, 4H), 1.24 (s, 2H), 0.93 (s, 3H).

MS calculated for 958.39; found 480.4 [M/2+H]⁺.

### Example 32: Synthesis of 3-(6-(4-(2-(4-((6-hydroxy-2-(4-(methylsulfonyl)phenyl)naphthalen-1-yl)oxy)phenoxy)ethyl)piperazin-1-yl)-1-oxoisoindolin-2-yl)piperidine-2,6-dione (compound D32)

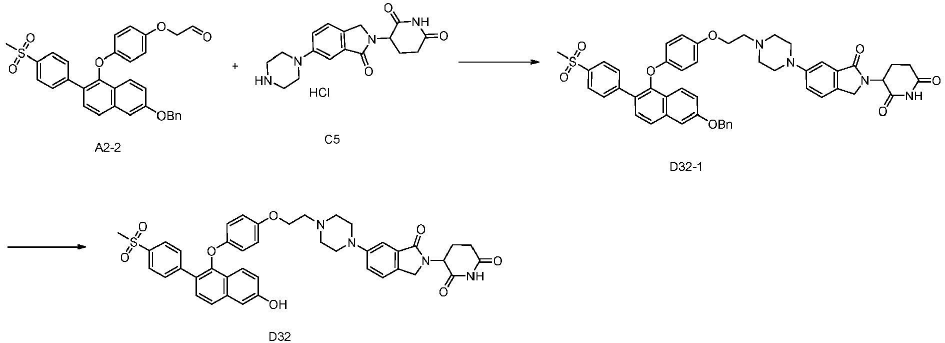

Compound A2-2 (450.0 mg, 0.836 mmol), hydrochloride of compound C5 (304 mg, 0.836 mmol), and potassium acetate (246.1 mg, 2.5 mmol) were sequentially added to a mixed solvent of methanol (5.0 mL) and dichloromethane (2.0 mL) at room temperature. The reaction mixture was stirred at room temperature for 10 minutes, added with sodium triacetoxyborohydride (538 mg, 2.5 mmol), and stirred at 20°C for 16 hours. The reaction mixture was concentrated under reduced pressure and purified by silica gel column chromatography (dichloromethane/methanol = 10/1) to obtain 3-(6-(4-(2-(4-((6-(benzyloxy)-2-(4-(methylsulfonyl)phenyl)naphthalen-1-yl)oxy)phenoxy)ethyl)piperazin-1-yl)-1-oxoisoindolin-2-yl)piperidine-2,6-dione (compound D32-1) (300 mg, yield: 42.2%).

Compound D32-1 (300 mg, 0.353 mmol) and wet palladium on carbon (100 mg, 10 wt%) were sequentially added to a mixed solvent of methanol (1.0 mL) and tetrahydrofuran (5.0 mL) at room temperature. The reaction mixture was stirred at 20°C for 16 hours under hydrogen atmosphere, filtered, and the filtrate was concentrated under reduced pressure and purified by silica gel column chromatography (dichloromethane/methanol = 10/1) to obtain compound D32 (95 mg, yield: 34%).

¹H NMR (400 MHz, DMSO-*d*₆) δ: 11.0 (s, 1H), 10.06 (s, 1H), 7.96 (d, *J* = 8.2 Hz, 2H), 7.86 (d, *J* = 8.3 Hz, 2H), 7.77 (dd, *J* = 16.2, 8.8 Hz, 2H), 7.62 (d, *J* = 7.5 Hz, 1H), 7.45 (d, *J* = 8.0 Hz, 1H), 7.27 (m, 2H), 7.11 (m, 2H), 6.86 - 6.75 (d, *J* = 12.0 Hz, 2H), 6.65 (d, *J* = 12.0 Hz, 2H), 5.14 (dd, *J* = 12.0 , 4.0 Hz, 1H), 4.35 (d, *J* = 16.0 Hz, 1H), 4.23 (d, *J* = 16.0 Hz, 1H), 4.05 - 3.90 (m, 2H), 3.24 (s, 3H), 3.20 - 3.10 (m, 4H), 3.00 - 2.85 (m, 1H), 2.75 - 2.57 (m, 7H), 2.45 - 2.32 (m, 1H), 2.08 - 1.92 (m, 1H).

MS calculated for 760.26; found 761.2 [M+H]⁺.

### Example 33: Synthesis of 4-(2-(1'-(2-(2,6-dioxopiperidin-3-yl)-1,3-dioxoisoindolin-4-yl)-[1,4'-bipiperidin]-4-yl)ethyl)-N-ethyl-N-(2-(4-((6-hydroxy-2-(4-(methylsulfonyl)phenyl)naphthalen-1-yl)oxy)phenoxy)ethyl)benzamide (compound D33)

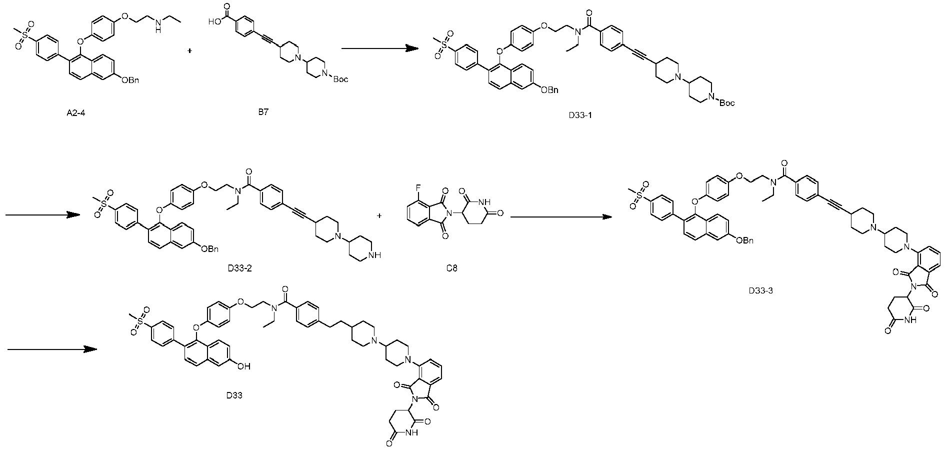

Compound A2-4 (500 mg, 0.88 mmol) was dissolved in anhydrous DMF (5 mL), and compound B7 was sequentially added thereto at room temperature. The reaction mixture was stirred at room temperature for 18 hours. The reaction mixture was diluted with water, and extracted three times with ethyl acetate. The organic phases were combined, washed with saturated brine, dried over anhydrous sodium sulfate, and evaporated to dryness by rotary evaporation. The crude product was purified by silica gel column chromatography (dichloromethane/methanol = 10/1) to obtain tert-butyl 4-((4-((2-(4-((6-(benzyloxy)-2-(4-(methylsulfonyl)phenyl)naphthalen-1-yl)oxy)phenoxy)ethyl)(ethyl)carbamoyl)phenyl)ethynyl)-[1,4'-bipiperidine]-1'-carboxylate (compound D33-1) (500 mg, yield: 59.17%).

Compound D33-1 (500 mg, 0.52 mmol) was dissolved in ethyl acetate (3 mL), and a solution of hydrochloric acid in ethyl acetate (4 M, 5 mL) was added thereto at room temperature. The reaction mixture was stirred at room temperature for 3 hours, and evaporated to dryness by rotary evaporation to obtain 4-([1,4'-bipiperidin]-4-ylethynyl)-N-(2-(4-((6-(benzyloxy)-2-(4-(methylsulfonyl)phenyl)naphthalen-1-yl)oxy)phenoxy)ethyl)-N-ethylbenzamide (compound D33-2) (467 mg, yield > 99%).

Compound D33-2 (200 mg, 0.22 mmol) was dissolved in DMSO (2 mL), and 2-(2,6-dioxopiperidin-3-yl)-4-fluoroisoindoline-1,3-dione (compound C8) (73.8 mg, 0.27 mmol) and DIPEA (85 mg, 0.66 mmol) were sequentially added thereto at room temperature. The reaction mixture was heated to 110°C and stirred for 18 hours. The reaction mixture was diluted with water, and extracted three times with ethyl acetate. The organic phases were combined, washed with saturated brine, dried over anhydrous sodium sulfate, evaporated to dryness by rotary evaporation, and purified by silica gel column chromatography (dichloromethane/methanol = 10/1) to obtain N-(2-(4-((6-(benzyloxy)-2-(4-(methylsulfonyl)phenyl)naphthalen-1-yl)oxy)phenoxy)ethyl)-4-((1'-(2-(2,6-dioxopiperidin-3-yl)-1,3-dioxoisoindolin-4-yl)-[1,4'-bipiperidin]-4-yl)ethynyl)-N-ethylbenzamide (compound D33-3) (200 mg, yield: 81.6%).

Compound D33-3 (200 mg, 0.179 mmol) was dissolved in acetonitrile (5 mL), and wet palladium on carbon (20 mg, 10 wt%) was added thereto at room temperature. The reaction mixture was stirred at room temperature for 18 hours. The reaction mixture was filtered and evaporated to dryness by rotary evaporation. The crude product was purified by prep-HPLC to obtain compound D33 (57.1 mg, yield: 31.0%).

¹H NMR (400 MHz, DMSO-*d*₆) δ 11.12 (s, 1H), 7.93 (d, *J* = 8.5 Hz, 2H), 7.86 (d, *J* = 8.4 Hz, 2H), 7.80 (d, *J=* 8.6 Hz, 1H), 7.77 - 7.71 (m, 2H), 7.61 (d, *J* = 8.6 Hz, 1H), 7.44 - 7.37 (m, 2H), 7.28 (s, 5H), 7.12 (dd, *J* = 9.2, 2.2 Hz, 1H), 6.80 (s, 1H), 6.63 (s, 2H), 5.12 (dd, *J* = 12.6, 5.5 Hz, 1H), 4.07 (s, 1H), 3.55 (d, *J* = 10.1 Hz, 4H), 3.39 (s, 2H), 3.24 (s, 5H), 3.08 - 2.84 (m, 5H), 2.65 (s, 3H), 2.17 (d, *J* = 11.9 Hz, 2H), 2.09 - 1.94 (m, 3H), 1.89 (d, *J* = 11.6 Hz, 2H), 1.65 (d, *J* = 66.2 Hz, 3H), 1.44 (s, 2H), 1.06 (s, 3H).

MS calculated for 1031.41; found 516.8 [M/2+H]⁺.

### Example 34: Synthesis of 4-(2-(1'-(2-(2,6-dioxopiperidin-3-yl)-1,3-dioxoisoindolin-5-yl)-[1,4'-bipiperidin]-4-yl)ethyl)-N-ethyl-N-(2-(4-((6-hydroxy-2-(4-(methylsulfonyl)phenyl)naphthalen-1-yl)oxy)phenoxy)ethyl)benzamide (compound D34)

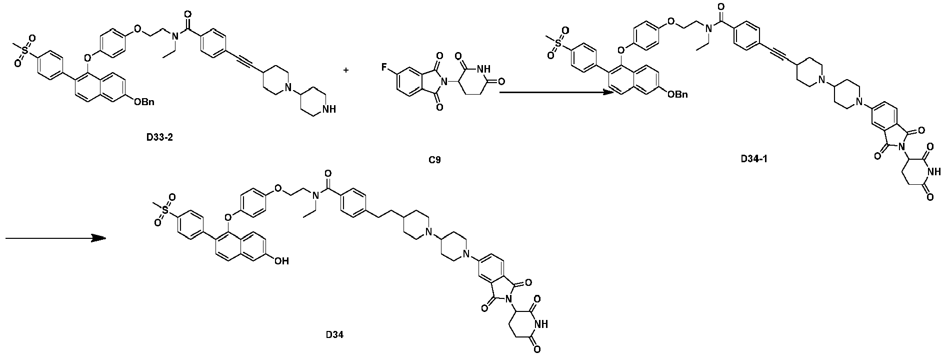

Compound D33-2 (200 mg, 0.22 mmol) was dissolved in DMSO (2 mL), and 2-(2,6-dioxopiperidin-3-yl)-5-fluoroisoindoline-1,3-dione (compound C9) (73.8 mg, 0.27 mmol) and DIPEA (85 mg, 0.66 mmol) were sequentially added thereto at room temperature. The reaction mixture was heated to 110°C and stirred for 18 hours. The reaction mixture was diluted with water, and extracted three times with ethyl acetate. The organic phases were combined, washed with saturated brine, dried over anhydrous sodium sulfate, evaporated to dryness by rotary evaporation, and purified by silica gel column chromatography (dichloromethane/methanol = 10/1) to obtain N-(2-(4-((6-(benzyloxy)-2-(4-(methylsulfonyl)phenyl)naphthalen-1-yl)oxy)phenoxy)ethyl)-4-((1'-(2-(2,6-dioxopiperidin-3-yl)-1,3-dioxoisoindolin-5-yl)-[1,4'-bipiperidin]-4-yl)ethynyl)-N-ethylbenzamide (compound D34-1) (200 mg, yield: 81.6%).

Compound D34-1 (200 mg, 0.179 mmol) was dissolved in acetonitrile (5 mL), and wet palladium on carbon (20 mg, 10 wt%) was added thereto at room temperature. The reaction mixture was stirred at room temperature for 18 hours. The reaction mixture was filtered and evaporated to dryness by rotary evaporation. The crude product was purified by prep-HPLC to obtain compound D34 (40.4 mg, yield: 22.0%).

¹H NMR (400 MHz, DMSO-*d*₆) δ 11.12 (s, 1H), 7.93 (d, *J* = 8.5 Hz, 2H), 7.86 (d, *J* = 8.4 Hz, 2H), 7.80 (d, *J* = 8.6 Hz, 1H), 7.77 - 7.71 (m, 2H), 7.61 (d, *J* = 8.6 Hz, 1H), 7.44 - 7.37 (m, 2H), 7.28 (s, 5H), 7.12 (dd, *J* = 9.2, 2.2 Hz, 1H), 6.80 (s, 1H), 6.63 (s, 2H), 5.12 (dd, *J* = 12.6, 5.5 Hz, 1H), 4.07 (s, 1H), 3.55 (d, *J* = 10.1 Hz, 4H), 3.39 (s, 2H), 3.24 (s, 5H), 3.08 - 2.84 (m, 5H), 2.65 (s, 3H), 2.17 (d, *J* = 11.9 Hz, 2H), 2.09 - 1.94 (m, 3H), 1.89 (d, *J* = 11.6 Hz, 2H), 1.65 (d, *J* = 66.2 Hz, 3H), 1.44 (s, 2H), 1.06 (s, 3H).

MS calculated for 1031.41; found 516.9 [M/2+H]⁺.

### Example 35: Synthesis of 3-(6-(2-(2-(2-(2-(4-((6-hydroxy-2-(4-(methylsulfonyl)phenyl)naphthalen-1-yl)oxy)phenoxy)ethoxy)ethoxy)ethoxy)ethoxy)-1-oxoisoindolin-2-yl)piperidine-2,6-dione (compound D35)

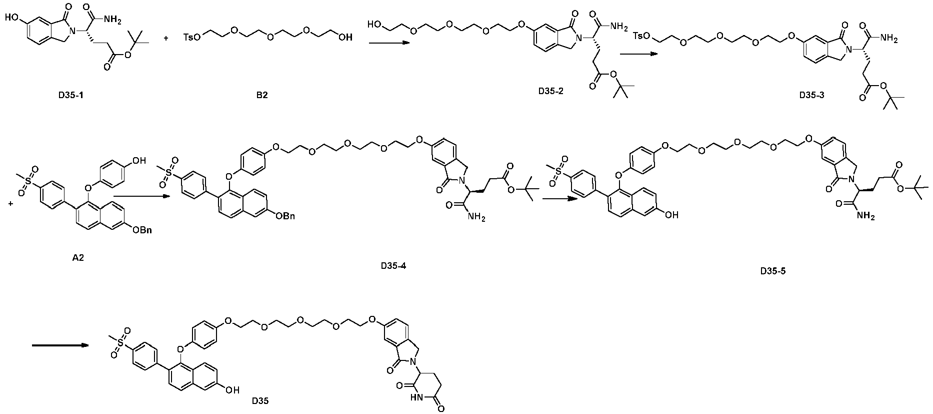

Compound B2 (689 mg, 1.98 mmol), tert-butyl (S)-5-amino-4-(6-hydroxy-1-oxoisoindolin-2-yl)-5-oxopentanoate (compound D35-1) (600 mg, 1.8 mmol), and potassium carbonate (1.2 g, 36.2 mmol) were sequentially added to acetonitrile (20 mL) at room temperature, and the resulting mixture was stirred at room temperature for 16 hours under nitrogen atmosphere at 107°C. The reaction mixture was cooled to room temperature, concentrated, and the crude product was purified by silica gel column chromatography (dichloromethane/methanol = 10/1) to obtain tert-butyl (S)-5-amino-4-(6-(2-(2-(2-(2-(2-(2-hydroxyethoxy)ethoxy)ethoxy)ethoxy)-1-oxoisoindolin-2-yl)-5-oxopentanoate (compound D35-2) (610 mg, yield: 67%).

Compound D35-2 (610 mg, 1.2 mmol), TsCl (296 mg, 1.55 mmol), triethylamine (0.5 mL, 3.6 mmol), and DMAP (15 mg, 0.12 mmol) were sequentially added to dichloromethane (10 mL) at room temperature, and the resulting mixture was stirred at room temperature for 18 hours. The reaction mixture was concentrated to obtain a residue, which was purified by silica gel column chromatography (dichloromethane/methanol = 10/1) to obtain tert-butyl (S)-5-amino-5-oxo-4-(1-oxo-6-(2-(2-(2-(2-(2-(tosyloxy)ethoxy)ethoxy)ethoxy)ethoxy)isoindolin-2-yl)pentanoate (compound D35-3) (724 mg, yield: 91%).

Compound D35-3 (230 mg, 0.35 mmol), compound A2 (188 mg, 0.38 mmol), and potassium carbonate (241 mg, 1.75 mmol) were sequentially added to DMF (5 mL) at room temperature. The mixture was stirred at 90°C for 8 hours, cooled to room temperature, concentrated under reduced pressure, and purified by silica gel column chromatography (dichloromethane/methanol = 10/1) to obtain tert-butyl (S)-5-amino-4-(6-(2-(2-(2-(2-(4-((6-(benzyloxy)-2-(4-(methylsulfonyl)phenyl)naphthalen-1-yl)oxy)phenoxy)ethoxy)ethoxy)ethoxy)-1-oxoisoindolin-2-yl)-5-oxopentanoate (compound D35-4) (180 mg, yield: 53%).

Compound D35-4 (180 mg, 0.18 mmol) and wet palladium on carbon (96.6 mg, 10 wt%) were added to tetrahydrofuran/methanol (10 mL/10 mL) at room temperature. The reaction mixture was stirred at room temperature for 16 hours under hydrogen atmosphere, filtered, and the resulting filtrate was concentrated to dryness. The crude product was purified by silica gel column chromatography (dichloromethane/methanol = 10/1) to obtain tert-butyl (S)-5-amino-4-(6-(2-(2-(2-(2-(4-((6-hydroxy-2-(4-(methylsulfonyl)phenyl)naphthalen-1-yl)oxy)phenoxy)ethoxy)ethoxy)ethoxy)ethoxy)-1-oxoisoindolin-2-yl)-5-oxopentanoate (compound D35-5) (160 mg, yield: 98%).

Compound D35-5 (160 mg, 0.18 mmol) and benzenesulfonic acid (90 mg, 0.57 mmol) were sequentially added to acetonitrile (5 mL) at room temperature, and the mixture was stirred at 100°C for 18 hours. The reaction mixture was cooled to room temperature, concentrated to dryness, and purified by silica gel column chromatography (dichloromethane/methanol = 10/1) to obtain compound D35 (10.6 mg, yield: 7%).

¹H NMR (400 MHz, DMSO-*d*₆) δ 10.97 (s, 1H), 8.28 (s, 1H), 7.92 (d, *J* = 8.3 Hz, 2H), 7.85 (d, *J* = 8.3 Hz, 2H), 7.76 (dd, *J* = 17.6, 8.9 Hz, 2H), 7.60 (d, *J* = 8.6 Hz, 1H), 7.53 (d, *J* = 8.6 Hz, 1H), 7.27 (d, *J* = 2.3 Hz, 1H), 7.12 (dd, *J* = 9.1, 2.4 Hz, 1H), 7.05 (d, *J* = 8.1 Hz, 2H), 6.81 - 6.74 (m, 2H), 6.65 - 6.59 (m, 2H), 5.07 (dd, *J* = 13.3, 5.0 Hz, 1H), 4.34 (d, *J* = 16.9 Hz, 1H), 4.22 (d, *J* = 16.8 Hz, 1H), 4.00 - 3.92 (m, 2H), 3.68 (m, 3H), 3.58 - 3.51 (m, 15H), 3.28 - 3.23 (m, 9H), 2.97 - 2.85 (m, 1H), 2.66 - 2.56 (m, 4H), 2.45 - 2.32 (m, 1H), 2.05 - 1.92 (m, 1H).

MS calculated for 824.3; found 825.0 [M+H]⁺.

### Example 36: Synthesis of 2-(2,6-dioxopiperidin-3-yl)-4-((3-ethyl-1-(4-((6-hydroxy-2-(4-(methylsulfonyl)phenyl)naphthalen-1-yl)oxy)phenoxy)-6,9,12-trioxa-3-aza-14-yl)oxy)isoindoline-1,3-dione (compound D36)

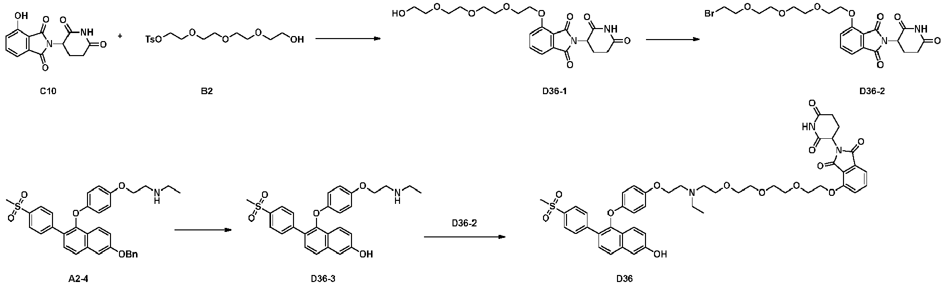

A mixture of 2-(2,6-dioxopiperidin-3-yl)-4-hydroxyisoindoline-1,3-dione (compound C10) (274 mg, 1.0 mmol), compound B2 (522 mg, 1.5 mmol), sodium bicarbonate (168 mg, 2.0 mmol), and sodium iodide (150 mg, 1.0 mmol) in DMF (2.5 mL) was stirred at 70°C for 20 hours. The mixture was cooled, concentrated and purified by silica gel column chromatography (dichloromethane/methanol = 10/1) to obtain 2-(2,6-dioxopiperidin-3-yl)-4-(2-(2-(2-(2-(2-hydroxyethoxy)ethoxy)ethoxy)ethoxy)isoindoline-1,3-dione (compound D36-1) (300 mg, yield: 66%) as a yellow gel.

Carbon tetrabromide (265 mg, 0.8 mmol) was added to a solution of compound D36-1 (300 mg, 0.66 mmol) and triphenylphosphine (210 mg, 0.8 mmol) in tetrahydrofuran (8 mL) at 0°C, and the reaction mixture was warmed to room temperature and stirred for 16 hours. The reaction mixture was concentrated and purified by silica gel column chromatography (dichloromethane/methanol = 10/1) to obtain 4-(2-(2-(2-(2-(2-bromoethoxy)ethoxy)ethoxy)ethoxy)-2-(2,6-dioxopiperidin-3-yl)isoindoline-1,3-dione (compound D36-2) (260 mg, yield: 77%) as a yellow gel.

Wet palladium on carbon (130 mg, 10 wt%) was added to a solution of compound A2-4 (130 mg, 0.23 mmol) in tetrahydrofuran (5 mL)/methanol (2 mL). The mixture was stirred at 25°C for 4 hours under hydrogen atmosphere. The reaction mixture was filtered, washed with dichloromethane/methanol, and concentrated under vacuum to obtain 5-(4-(2-(ethylamino)ethoxy)phenoxy)-6-(4-(methylsulfonyl)phenyl)naphthalen-2-ol (compound D36-3) (120 mg, yield: 100%).

A mixture of compound D36-3 (110 mg, 0.23 mmol), compound D36-2 (148 mg, 0.23 mmol), DIPEA (119 mg, 0.92 mmol), and potassium iodide (4 mg, 0.023 mmol) in DMF (4 mL) was stirred at 90°C for 18 hours. The reaction mixture was cooled, dissolved in dichloromethane, washed with water and saturated brine, concentrated and purified by silica gel column chromatography (dichloromethane/methanol = 10/1) to obtain compound D36 (30 mg, yield: 14%) as a white solid.

¹H NMR (400 MHz, DMSO-*d*₆) δ 11.09 (s, 1H), 10.04 (s, 1H), 7.90 (d, *J* = 8.5 Hz, 2H), 7.84 (d, *J* = 8.2 Hz, 2H), 7.81 - 7.74 (m, 2H), 7.72 (d, *J* = 9.1 Hz, 1H), 7.58 (d, *J* = 8.6 Hz, 1H), 7.49 (d, *J* = 8.6 Hz, 1H), 7.44 (d, *J* = 7.2 Hz, 1H), 7.25 (d, *J* = 2.3 Hz, 1H), 7.09 (dd, *J* = 9.1, 2.4 Hz, 1H), 6.81 - 6.68 (m, 2H), 6.66 - 6.54 (m, 2H), 5.07 (dd, *J* = 12.7, 5.4 Hz, 1H), 4.30 (t, *J* = 4.5 Hz, 2H), 3.84 - 3.68 (m, 3H), 3.67 - 3.57 (m, 3H), 3.57 - 3.39 (m, 8H), 3.21 (s, 3H), 2.93 - 2.82 (m, 1H), 2.79 - 2.65 (m, 1H), 2.64 - 2.51 (m, 7H), 2.07 - 1.94 (m, 1H), 1.29 - 1.10 (m, 3H).

MS calculated for 909.31; found 910.3 [M+H]⁺.

### Example 37: Synthesis of 3-(4-(4-(2-(2-(2-(4-((6-hydroxy-2-(4-(methylsulfonyl)phenyl)naphthalen-1-yl)oxy)phenoxy)ethoxy)ethoxy)ethyl)piperazin-1-yl)-1-oxoisoindolin-2-yl)piperidine-2,6-dione (compound D37)

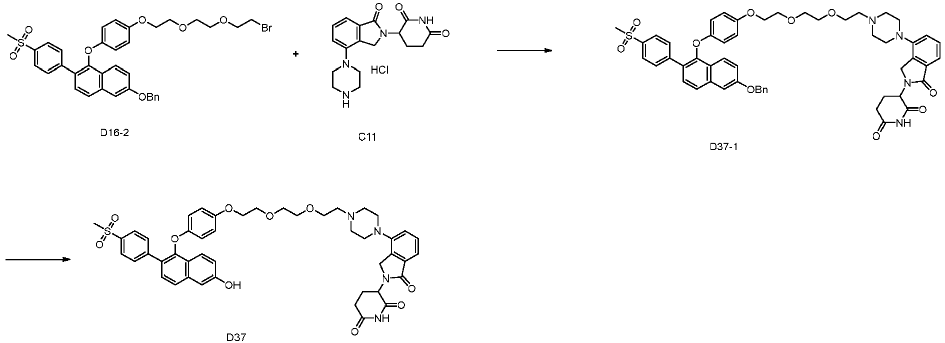

Compound D16-2 (220.0 mg, 0.318 mmol), compound C11 (116 mg, 0.318 mmol), DIPEA (0.23 mL, 1.27 mmol), and potassium iodide (5 mg, 0.03 mmol) were sequentially added to acetonitrile (5.0 mL) at room temperature. The reaction mixture was stirred at 90°C for 16 hours. The reaction mixture was cooled to room temperature, concentrated under reduced pressure, added with water (10.0 mL), and extracted twice with dichloromethane (10.0 mL). The organic phases were combined, washed with saturated brine, dried over anhydrous sodium sulfate, filtered, concentrated under reduced pressure, and purified by silica gel column chromatography (dichloromethane/methanol = 10/1) to obtain 3-(4-(4-(2-(2-(2-(4-((6-(benzyloxy)-2-(4-(methylsulfonyl)phenyl)naphthalen-1-yl)oxy)phenoxy)ethoxy))3-ethoxy)ethyl)piperazin-1-yl)-1-oxoisoindolin-2-yl)piperidine-2,6-dione (compound D37-1) (241 mg, yield: 80.7%).

Compound D37-1 (241.0 mg, 0.256 mmol) and wet palladium on carbon (100.0 mg, 10 wt%) were sequentially added to a mixed solvent of methanol (15.0 mL) and tetrahydrofuran (5.0 mL) at room temperature. The reaction mixture was stirred at 20°C for 16 hours under hydrogen atmosphere, filtered through diatomite, and the filtrate was concentrated under reduced pressure. The crude product was purified by silica gel column chromatography (dichloromethane/methanol = 10/1) to obtain compound D37 (130 mg, yield: 59.7%).

¹H NMR (400 MHz, DMSO-*d*₆) δ: 10.99 (s, 1H), 10.07 (s, 1H), 7.93 (d, *J* = 8.5 Hz, 2H), 7.86 (d, *J* = 8.5 Hz, 2H), 7.82 - 7.72 (m, 2H), 7.60 (t, *J* = 7.1 Hz, 1H), 7.48 - 7.41 (m, 1H), 7.34 (d, *J* = 7.4 Hz, 1H), 7.28 (t, *J* = 4.7 Hz, 1H), 7.13 (dt, *J* = 8.6, 7.5 Hz, 2H), 6.78 (d, *J* = 9.1 Hz, 2H), 6.63 (d, *J* = 9.1 Hz, 2H), 5.14 (dt, *J* = 12.8, 6.3 Hz, 1H), 4.51 - 4.42 (m, 1H), 4.37 - 4.28 (m, 1H), 4.00 - 3.94 (m, 2H), 3.73 - 3.67 (m, 2H), 3.60 - 3.52 (m, 6H), 3.24 (s, 3H), 3.07 (d, *J* = 4.4 Hz, 4H), 3.00 - 2.89 (m, 1H), 2.67 - 2.55 (m, 7H), 2.52 - 2.44 (m, 1H), 2.05 - 1.97 (m, 1H).

MS calculated for 848.31; found 849.2 [M+H]⁺.

### Example 38: Synthesis of 2-(2,6-dioxopiperidin-3-yl)-5-((2-(2-(ethyl(2-(4-((6-hydroxy-2-(4-(methylsulfonyl)phenyl)naphthalen-1-yl)oxy)phenoxy)ethyl)amino)ethoxy)ethyl)amino)indoline-1,3-dione (compound D38)

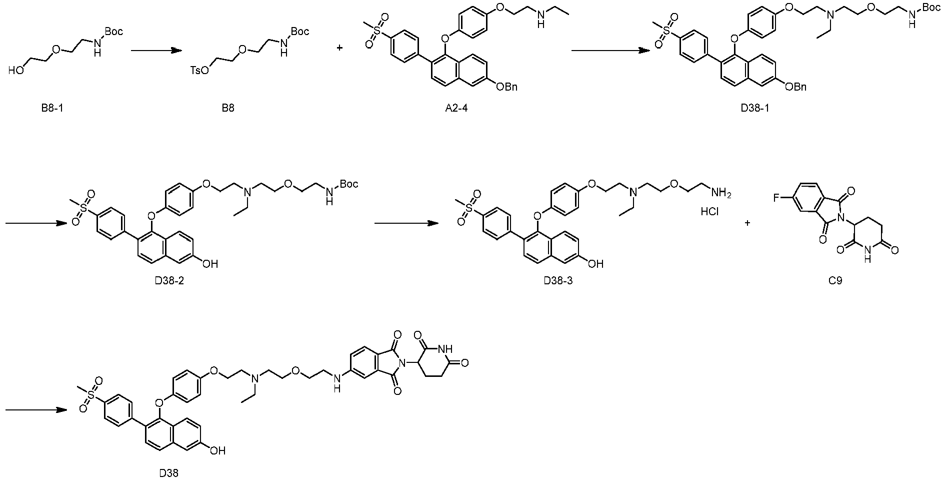

p-Toluenesulfonyl chloride (5.6 g, 29.23 mmol) was added to a solution of tert-butyl (2-(2-hydroxyethoxy)ethyl)carbamate (compound B8-1) (5 g, 24.36 mmol), DMAP (297.6 mg, 2.436 mmol), and triethylamine (7.39 g, 73.08 mmol) in dichloromethane (400 mL), and the reaction mixture was stirred at room temperature for 16 hours. The reaction mixture was concentrated, and the crude product was purified by silica gel column chromatography (dichloromethane/methanol = 10/1) to obtain 2-(2-((tert-butoxycarbonyl)amino)ethoxy)ethyl 4-methylbenzenesulfonate (compound B8) (7.0 g, yield: 79.9%) as a transparent oil.

Compound A2-4 (1.0 g, 1.76 mmol), compound B8 (949 mg, 2.64 mmol), and triethylamine (534 mg, 5.28 mmol) were dissolved in DMF (5 mL), and the reaction mixture was stirred at 80°C for 16 hours. The reaction mixture was concentrated to obtain a crude product, which was purified by silica gel column chromatography (dichloromethane/methanol = 10/1) to obtain tert-butyl (2-(2-((2-(4-((6-(benzyloxy)-2-(4-(methylsulfonyl)phenyl)naphthalen-1-yl)oxy)phenoxy)ethyl)(ethyl)amino)ethoxy)ethyl)carbamate (compound D38-1) (900 mg, yield: 67.7%) as a white solid.

Compound D38-1 (500 mg, 0.66 mmol) was dissolved in methanol (9 mL) and ethyl acetate (1 mL), and wet palladium on carbon (100 mg, 10 wt%) was added thereto. The reaction mixture was replaced with hydrogen three times, and stirred at room temperature for 16 hours. The reaction mixture was filtered, and the filtrate was concentrated to obtain tert-butyl (2-(2-(ethyl(2-(4-(((6-hydroxy-2-(4-(methylsulfonyl))phenyl)naphthalen-1-yl)oxy)phenoxy)ethyl)amino)ethoxy)ethyl)carbamate (compound D38-2) (300 mg, yield: 68.2%) as a white solid.

Compound D38-2 (300 mg, 0.45 mmol) was dissolved in dioxane (5 mL), a solution of hydrochloric acid in dioxane (1.2 mL, 4 M) was slowly added thereto at 0°C, and the reaction mixture was stirred at room temperature for 2 hours. The reaction mixture was concentrated to obtain 5-(4-(2-((2-(2-(2-aminoethoxy)ethyl)(ethyl)amino)ethoxy)phenoxy)-6-(4-(methylsulfonyl)phenyl)naphthalen-2-ol hydrochloride (compound D38-3) (150 mg, yield: 55.4%) as a white solid.

Compound D38-3 (150 mg, 0.25 mmol) and DIPEA (97 mg, 0.75 mmol) were dissolved in dimethyl sulfoxide (1 mL), then 2-(2,6-dioxopiperidin-3-yl)-5-fluoroisoindoline-1,3-dione (compound C9) (69 mg, 0.25 mmol) was added thereto, and the reaction mixture was stirred at 120°C for 6 hours under nitrogen atmosphere. The reaction mixture was evaporated to dryness by rotary evaporation, and the crude product was purified by prep-HPLC to obtain compound D38 (25 mg, yield: 12.2%) as a yellow solid.

¹H NMR (400 MHz, DMSO) δ 11.01 (s, 1H), 10.09 (s, 1H), 7.88 (s, 2H), 7.83 (s, 2H), 7.73 (dd, *J* = 16.0, 8.8 Hz, 2H), 7.57 (d, *J* = 8.8 Hz, 1H), 7.52 (d, *J* = 8.3 Hz, 1H), 7.26 (s, 1H), 7.18 - 7.06 (m, 2H), 6.99 (s, 1H), 6.85 (s, 1H), 6.70 (d, *J* = 7.9 Hz, 2H), 6.57 (d, *J* = 8.1 Hz, 2H), 5.03 (s, 1H), 3.83 (s, 2H), 3.54 (s, 2H), 3.46 (s, 4H), 3.20 (s, 3H), 2.89 - 2.80 (m, 1H), 2.75 - 2.67 (s, 2H), 2.65 - 2.57 (s, 2H), 2.56 - 2.53 (s, 2H), 1.99 - 1.92 (m, 1H), 1.21 (s, 2H), 0.90 (s, 3H).

MS calculated for 820.28; found 821.3 [M+H]⁺.

### Example 39: Synthesis of 2-(2,6-dioxopiperidin-3-yl)-4-((3-ethyl-1-(4-((6-hydroxy-2-(4-(methylsulfonyl)phenyl)naphthalen-1-yl)oxy)phenoxy)-6,9,12-trioxa-3-azatetradecan-14-yl)amino)isoindoline-1,3-dione (compound D39)

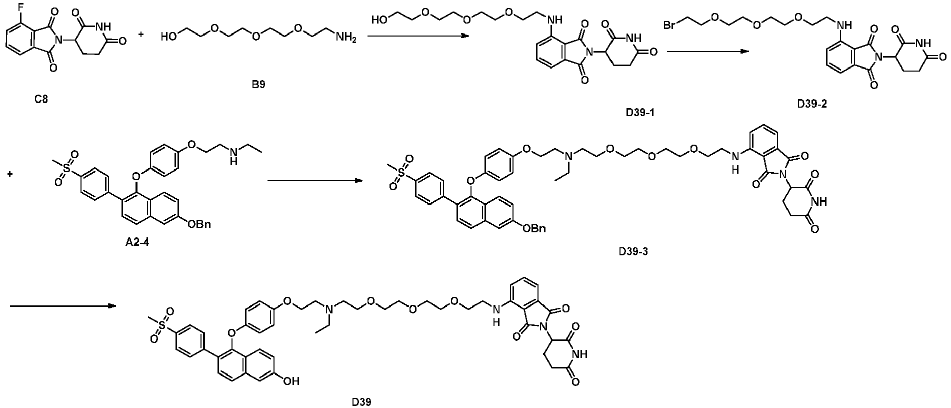

Compound C8 (100.0 mg, 0.36 mmol), 2-(2-(2-aminoethoxy)ethoxy)ethylene glycol (compound B9) (77 mg, 0.4 mmol), and DIPEA (0.13 mL, 0.72 mmol) were sequentially added to DMF (3.0 mL) at room temperature. The reaction mixture was stirred at 90°C for 16 hours. The reaction mixture was cooled to room temperature, concentrated under reduced pressure, added with water (10.0 mL), and extracted twice with dichloromethane (10.0 mL). The organic phases were combined, washed with saturated brine (10.0 mL), dried over anhydrous sodium sulfate, filtered, and concentrated under reduced pressure. The reaction mixture was purified by silica gel column chromatography (dichloromethane/methanol = 10/1) to obtain 2-(2,6-dioxopiperidin-3-yl)-4-((2-(2-(2-hydroxyethoxy)ethoxy)ethyl)amino)isoindoline-1,3-dione (compound D39-1) (105 mg, yield: 48.4%).

Carbon tetrabromide (93 mg, 0.28 mmol) was slowly added to a solution of compound D39-1 (105.0 mg, 0.23 mmol) and triphenylphosphine (72 mg, 0.28 mmol) in dichloromethane (3.0 mL) at 0°C, and the reaction mixture was stirred at room temperature for 16 hours. The reaction mixture was concentrated under reduced pressure and purified by silica gel column chromatography (dichloromethane/methanol = 10/1) to obtain 4-((2-(2-(2-bromoethoxy)ethoxy)ethyl)amino)-2-(2,6-dioxopiperidin-3-yl)isoindoline-1,3-dione (compound D39-2) (141 mg, yield: 100%).

Compound D39-2 (70.0 mg, 0.137 mmol), compound A2-4 (65 mg, 0.11 mmol), DIPEA (0.08 mL, 0.44 mmol), and potassium iodide (2 mg, 0.014 mmol) were sequentially added to DMF (2.0 mL) at room temperature. The reaction mixture was stirred at 85°C for 16 hours. The reaction mixture was cooled to room temperature, concentrated under reduced pressure, added with water (10.0 mL), and extracted twice with dichloromethane (10.0 mL). The organic phases were combined, washed with saturated brine (10.0 mL), dried over anhydrous sodium sulfate, filtered, and concentrated under reduced pressure. The crude product was purified by silica gel column chromatography (dichloromethane/methanol = 10/1) to obtain 4-((1-(4-((6-(benzyloxy)-2-(4-(methylsulfonyl)phenyl)naphthalen-1-yl)oxy)phenoxy)-3-ethyl-6,9,12-trioxa-3-azatetradecan-14-yl)amino)-2-(2,6-dioxopiperidin-3-yl)isoindoline-1,3-dione (compound D39-3) (45 mg, yield: 39.5%).

Compound D39-3 (45.0 mg, 0.06 mmol) and wet palladium on carbon (60 mg, 10 wt%) were sequentially added to a mixed solvent of methanol (5.0 mL) and tetrahydrofuran (2.0 mL) at room temperature. The reaction mixture was stirred at 20°C for 16 hours under hydrogen atmosphere, filtered through diatomite, and the filtrate was concentrated under reduced pressure. The crude product was purified by silica gel column chromatography (dichloromethane/methanol = 10/1) to obtain compound D39 (11 mg, yield: 26.8%).

¹H NMR (400 MHz, DMSO-*d*₆) δ 11.09 (s, 1H), 10.05 (s, 1H), 7.93 (d, *J* = 8.5 Hz, 2H), 7.86 (d, *J* = 8.4 Hz, 2H), 7.81 - 7.72 (m, 2H), 7.64 - 7.54 (m, 2H), 7.27 (t, *J* = 4.8 Hz, 1H), 7.18 - 7.09 (m, 2H), 7.04 (t, *J* = 7.1 Hz, 1H), 6.75 (d, *J* = 8.3 Hz, 2H), 6.62 (d, *J=* 8.8 Hz, 2H), 5.06 (dt, *J* = 12.6, 6.2 Hz, 1H), 3.87 (s, 1H), 3.54 (ddd, *J* = 30.0, 14.1, 5.5 Hz, 16H), 3.24 (s, 3H), 2.97 - 2.83 (m, 1H), 2.83 - 2.54 (m, 8H), 2.10 - 1.98 (m, 1H), 1.01 - 0.82 (m, 3H).

MS calculated for 908.33; found 909.2 [M+H]⁺.

### Example 40: Synthesis of 3-(4-(4-(2-(2-(2-(2-(2-(4-((6-hydroxy-2-(4-(methylsulfonyl)phenyl)naphthalen-1-yl)oxy)phenoxy)ethyl)amino)ethoxy)ethyl)piperazin-1-yl)-1-oxoisoindolin-2-yl)piperidine-2,6-dione (compound D40)

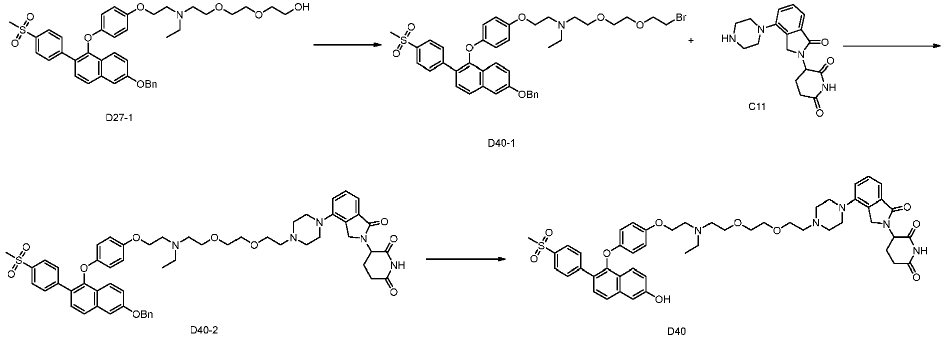

Compound D27-1 (238 mg, 0.34 mmol), triphenylphosphine (160.3 mg, 0.612 mmol), and carbon tetrabromide (203 mg, 0.612 mmol) were sequentially added to dichloromethane (15 mL) at room temperature, and the resulting mixture was stirred at room temperature for 4 hours under nitrogen atmosphere at 107°C. The mixture was concentrated, and the crude product was purified by silica gel column chromatography (dichloromethane/methanol = 10/1) to obtain 2-(4-((6-(benzyloxy)-2-(4-(methylsulfonyl)phenyl)naphthalen-1-yl)oxy)phenoxy)-N-(2-(2-(2-bromoethoxy)ethoxy)ethyl)-N-ethylethan-1-amine (compound D40-1) (400 mg, yield: 52.5%).

Compound D40-1 (400 mg, 0.26 mmol), 3-(1-oxo-4-(piperazin-1-yl)isoindolin-2-yl)piperidine-2,6-dione (compound C11) (94 mg, 0.26 mmol), DIPEA (0.19 mL, 1.04 mmol), and potassium iodide (43 mg, 0.26 mmol) were sequentially added to acetonitrile (20 mL) at room temperature, and the resulting mixture was stirred at 90°C for 16 hours. The reaction mixture was concentrated and purified by silica gel column chromatography (dichloromethane/methanol = 10/1) to obtain 3-(4-(4-(2-(2-(2-((2-(4-((6-(benzyloxy)-2-(4-(methylsulfonyl)phenyl)naphthalen-1-yl)oxy)phenoxy)ethyl)(ethyl)amino)ethoxy)ethyl)piperazin-1-yl)-1-oxoisoindolin-2-yl)piperidine-2,6-dione (compound D40-2) (200 mg, yield: 76%).

Compound D40-2 (200 mg, 0.20 mmol) and wet palladium on carbon (200 mg, 10 wt%) were added to tetrahydrofuran/methanol (15 mL/10 mL) at room temperature. The reaction mixture was stirred at room temperature for 16 hours under hydrogen atmosphere, filtered, and the resulting filtrate was concentrated. The crude product was purified by silica gel column chromatography (dichloromethane/methanol = 10/1) to obtain compound D40 (40 mg, yield: 22%).

¹H NMR (400 MHz, DMSO-*d*₆) δ 11.02 (s, 1H), 10.11 (s, 1H), 7.92 (d, *J* = 8.3 Hz, 2H), 7.86 (d, *J* = 8.2 Hz, 2H), 7.76 (dd, *J* = 18.1, 8.9 Hz, 2H), 7.60 (d, *J* = 8.6 Hz, 1H), 7.44 (t, *J* = 7.7 Hz, 1H), 7.34 (d, *J* = 7.4 Hz, 1H), 7.28 (d, *J* = 2.3 Hz, 1H), 7.18 - 7.08 (m, 2H), 6.77 (d, *J* = 8.6 Hz, 2H), 6.63 (d, *J* = 8.6 Hz, 2H), 5.14 (dd, *J* = 13.3, 5.1 Hz, 1H), 4.46 (d, *J* = 17.3 Hz, 1H), 4.31 (d, *J* = 17.2 Hz, 1H), 3.75 - 3.46 (m, 14H), 3.24 (s, 3H), 3.19 - 2.88 (m, 10H), 2.74 - 2.56 (m, 8H), 2.06 - 1.96 (m, 1H).

MS calculated for 920.1; found 921.0 [M+H]⁺.

### Example 41: Synthesis of 2-(2,6-dioxopiperidin-3-yl)-4-(2-(2-(2-(4-((6-hydroxy-2-(4-(methylsulfonyl)phenyl)naphthalen-1-yl)oxy)phenoxy)ethyl)amino)ethoxy)ethoxy)isoindoline-1,3-dione (compound D41)

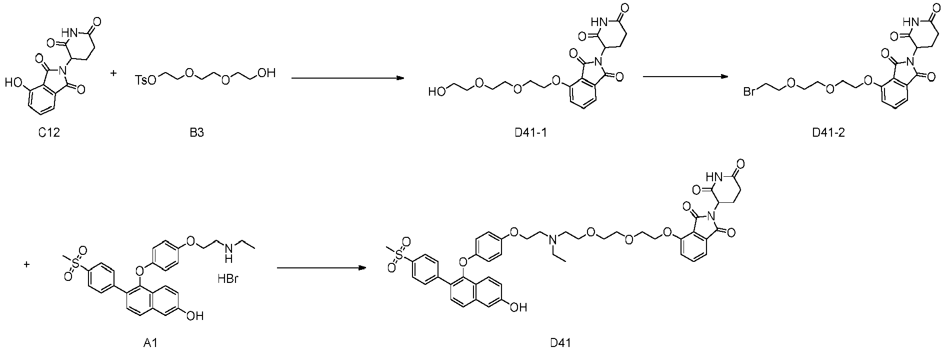

2-(2,6-Dioxopiperidin-3-yl)-4-hydroxyisoindoline-1,3-dione (compound C12) (823 mg, 3.0 mmol), compound B3 (1.83 g, 6.0 mmol), sodium bicarbonate (504 mg, 6.0 mmol), and sodium iodide (450 mg, 3.0 mmol) were sequentially added to DMF (7.5 mL) at room temperature. The mixture was stirred at 70°C for 20 hours. The mixture was cooled, concentrated under vacuum, and purified by silica gel column chromatography (dichloromethane/methanol = 10/1) to obtain 2-(2,6-dioxopiperidin-3-yl)-4-(2-(2-hydroxyethoxy)ethoxy)isoindoline-1,3-dione (compound D41-1) (1030 mg, yield: 84%).

Carbon tetrabromide (249 mg, 0.75 mmol) was added to a solution of compound D41-1 (255 mg, 0.63 mmol) and triphenylphosphine (197 mg, 0.75 mmol) in dichloromethane (6 mL) at 0°C, and the reaction mixture was warmed to room temperature and stirred for 16 hours. The reaction mixture was concentrated and purified by silica gel column chromatography (dichloromethane/methanol = 10/1) to obtain 4-(2-(2-bromoethoxy)ethoxy)-2-(2,6-dioxopiperidin-3-yl)isoindoline-1,3-dione (compound D41-2) (374 mg, > 99%).

Compound A1 (92 mg, 0.19 mmol), compound D41-2 (216 mg, 0.23 mmol), DIPEA (98 mg, 0.76 mmol), and potassium iodide (7 mg, 0.038 mmol) were sequentially added to DMF (4 mL) at room temperature, and the mixture was stirred at 90°C for 18 hours. The reaction mixture was cooled, dissolved in dichloromethane, sequentially washed with water and saturated brine, concentrated and purified by silica gel column chromatography (dichloromethane/methanol = 10/1) to obtain compound D41 (25 mg, yield: 15%) as a white solid.

¹H NMR (400 MHz, DMSO-*d*₆) δ 11.09 (s, 1H), 10.05 (s, 1H), 7.90 (d, *J* = 8.5 Hz, 2H), 7.83 (d, *J* = 8.5 Hz, 2H), 7.79 - 7.67 (m, 3H), 7.57 (d, *J* = 8.6 Hz, 1H), 7.48 (d, *J* = 8.6 Hz, 1H), 7.43 (d, J=7.2 Hz, 1H), 7.24 (d, *J* = 2.4 Hz, 1H), 7.09 (dd, *J* = 9.1, 2.4 Hz, 1H), 6.71 (d, *J* = 8.7 Hz, 2H), 6.58 (d, *J* = 8.7 Hz, 2H), 5.07 (dd, *J* = 12.9, 5.4 Hz, 1H), 4.29 (t, *J* = 4.6 Hz, 2H), 3.92 - 3.70 (m, 3H), 3.67 - 3.56 (m, 2H), 3.54 - 3.34 (m, 5H), 3.21 (s, 3H), 2.94 - 2.80 (m, 2H), 2.79 - 2.69 (m, 1H), 2.63 - 2.51 (m, 6H), 2.06 - 1.91 (m, 1H), 1.03 - 0.79 (m, 3H).

MS calculated for 865.29; found 866.3 [M+H]⁺.

### Example 42: Synthesis of 2-(2,6-dioxopiperidin-3-yl)-4-((2-(2-(2-(4-((6-hydroxy-2-(4-(methylsulfonyl)phenyl)naphthalen-1-yl)oxy)phenoxy)ethyl)amino)ethoxy)ethyl)amino)isoindoline-1,3-dione (compound D42)

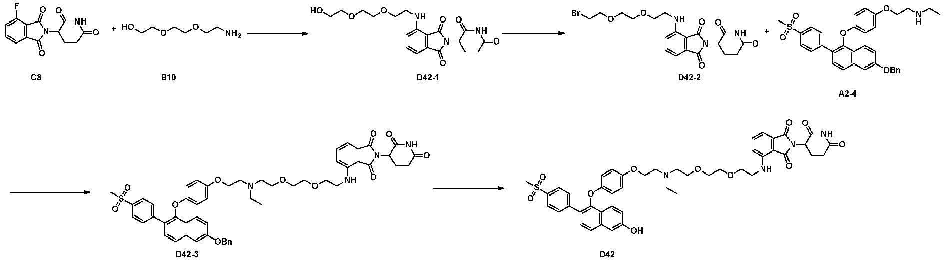

Compound C8 (322.0 mg, 1.16 mmol), compound B10 (191 mg, 1.28 mmol), and DIPEA (0.4 mL, 2.32 mmol) were sequentially added to DMF (6.0 m) at room temperature. The reaction mixture was stirred at 90°C for 13 hours. The reaction mixture was cooled to room temperature, concentrated under reduced pressure, added with water (10.0 mL), and extracted twice with dichloromethane (10.0 mL). The organic phases were combined, washed with saturated brine (10.0 mL), dried over anhydrous sodium sulfate, filtered, and concentrated under reduced pressure. The crude product was purified by silica gel column chromatography (dichloromethane/methanol = 10/1) to obtain 2-(2,6-dioxopiperidin-3-yl)-4-((2-(2-hydroxyethoxy)ethoxy)ethyl)amino)isoindoline-1,3-dione (compound D42-1) (159 mg, yield: 33.7%).

Carbon tetrabromide (234 mg, 0.7 mmol) was slowly added to a solution of compound D42-1 (184 mg, 0.45 mmol) and triphenylphosphine (184 mg, 0.7 mmol) in dichloromethane (6.0 mL) at 0°C, and the reaction mixture was stirred at room temperature for 16 hours. The reaction mixture was concentrated under reduced pressure and purified by silica gel column chromatography (dichloromethane/methanol = 10/1) to obtain 4-((2-(2-(2-bromoethoxy)ethoxy)ethyl)amino)-2-(2,6-dioxopiperidin-3-yl)isoindoline-1,3-dione (compound D42-2) (183 mg, yield: 87%).

Compound D42-2 (183.0 mg, 0.39 mmol), compound A2-4 (170.0 mg, 0.3 mmol), DIPEA (0.21 mL, 1.2 mmol), and potassium iodide (8 mg, 0.05 mmol) were sequentially added to DMF (3.0 mL) at room temperature. The reaction mixture was stirred at 85°C for 16 hours. The reaction mixture was cooled to room temperature, concentrated under reduced pressure, added with water (10.0 mL), and extracted twice with dichloromethane (10.0 mL). The organic phases were combined, washed with saturated brine (10.0 mL), dried over anhydrous sodium sulfate, filtered, and concentrated under reduced pressure. The crude product was purified by silica gel column chromatography (dichloromethane/methanol = 10/1) to obtain 4-((2-(2-(2-((2-(4-((6-(benzyloxy)-2-(4-(methylsulfonyl)phenyl)naphthalen-1-yl)oxy)phenoxy)ethyl)(ethyl)amino)ethoxy)ethoxy)ethyl)amino)-2-(2,6-dioxopiperidin-3-yl)isoindoline-1,3-dione (compound D42-3) (107 mg, yield: 37.4%).

Compound D42-3 (107.0 mg, 0.11 mmol) and wet palladium on carbon (50 mg, 10 wt%) were sequentially added to a mixed solvent of methanol (8.0 mL) and tetrahydrofuran (3.0 mL) at room temperature. The reaction mixture was stirred at 20°C for 16 hours under hydrogen atmosphere, filtered, and concentrated. The crude product was purified by silica gel column chromatography (dichloromethane/methanol = 10/1) to obtain compound D42 (27 mg, yield: 27.9%).

¹H NMR (400 MHz, DMSO-*d*₆) δ: 11.10 (s, 1H), 10.05 (s, 1H), 7.93 (d, *J* = 8.4 Hz, 2H), 7.86 (d, *J* = 8.4 Hz, 2H), 7.81 - 7.72 (m, 2H), 7.63 - 7.54 (m, 2H), 7.27 (t, *J* = 3.9 Hz, 1H), 7.12 (d, *J* = 8.6 Hz, 2H), 7.04 (t, *J* = 6.5 Hz, 1H), 6.81 - 6.71 (m, 2H), 6.61 (d, *J* = 8.3 Hz, 2H), 5.06 (dt, *J* = 15.5, 7.6 Hz, 1H), 3.87 (s, 1H), 3.67- 3.42 (m , 16H), 3.24 (s, 3H), 2.97 - 2.83 (m, 1H), 2.80 - 2.57 (m, 4H), 2.09 - 1.94 (m, 1H), 1.04 - 0.77 (m, 3H).

MS calculated for 864.3; found 865.3 [M+H]⁺.

### Example 43: Synthesis of 3-(6-(4-(2-(2-(4-((6-hydroxy-2-(4-(methylsulfonyl)phenyl)naphthalen-1-yl)oxy)phenoxy)ethoxy)ethyl)piperazin-1-yl)-1-oxoisoindolin-2-yl)piperidine-2,6-dione (compound D43)

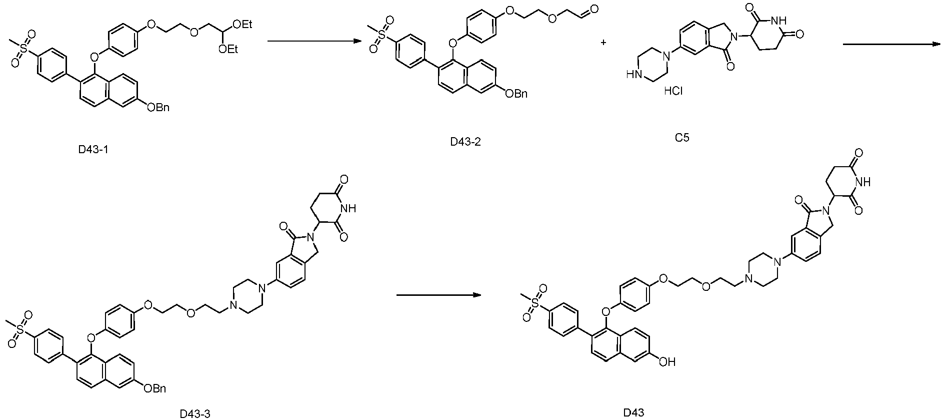

6-(Benzyloxy)-1-(4-(2-(2,2-diethoxyethoxy)ethoxy)phenoxy)-2-(4-(methylsulfonyl)phenyl)naphthalene (compound D43-1) (400 mg, 0.61 mmol) was added to a mixed solvent of hydrochloric acid (4 M, 3 mL) and tetrahydrofuran (6.0 mL) at room temperature, and the reaction mixture was heated to 40°C and stirred for 1 hour. The reaction mixture was concentrated under reduced pressure, added with water (10.0 mL), and extracted twice with dichloromethane (10.0 mL). The organic phases were combined, washed with saturated brine (10.0 mL), dried over anhydrous sodium sulfate, filtered, and concentrated under reduced pressure to obtain a crude product of 2-(2-(4-((6-(benzyloxy)-2-(4-(methylsulfonyl)phenyl)naphthalen-1-yl)oxy)phenoxy)ethoxy)acetaldehyde (compound D43-2) (355 mg, yield: 100%).

Compound D43-2 (355 mg, 0.61 mmol), hydrochloride of compound C5 (219 mg, 0.61 mmol), and potassium acetate (300 mg, 3.05 mmol) were sequentially added to a mixed solvent of methanol (5.0 mL) and dichloromethane (2.0 mL) at room temperature. The reaction mixture was stirred at room temperature for 10 minutes, added with sodium triacetoxyborohydride (393 mg, 3.0 mmol), and stirred at 20°C for 16 hours. The reaction mixture was concentrated under reduced pressure and purified by silica gel column chromatography (dichloromethane/methanol = 10/1) to obtain 3-(6-(4-(2-(2-(4-((6-(benzyloxy)-2-(4-(methylsulfonyl)phenyl)naphthalen-1-yl)oxy)phenoxy)ethoxy)ethyl)piperazin-1-yl)-1-oxoisoindolin-2-yl)piperidine-2,6-dione (compound D43-3) (250 mg, yield: 46%).

Compound D43-3 (100 mg, 0.11 mmol) and wet palladium on carbon (150 mg, 10 wt%) were sequentially added to a mixed solvent of methanol (3.0 mL) and tetrahydrofuran (5.0 mL) at room temperature. The reaction mixture was stirred at 20°C for 16 hours under hydrogen atmosphere, filtered, and the filtrate was concentrated under reduced pressure. The crude product was purified by silica gel column chromatography (dichloromethane/methanol = 10/1) to obtain compound D43 (30 mg, yield: 34%).

¹H NMR (400 MHz, DMSO-*d*₆) δ: 11.0 (s, 1H), 10.10 (s, 1H), 7.92 (d, *J* = 8.0 Hz, 2H), 7.86 (d, *J* = 8.0 Hz, 2H), 7.77 (dd, *J* = 20.0, 10.0 Hz, 2H), 7.60 (d, *J* = 8.0 Hz, 1H), 7.45 (d, *J* = 8.0 Hz, 1H), 7.27 (m, 2H), 7.14 (m, 2H), 6.82- 6.72 (d, *J* = 8.0 Hz, 2H), 6.62 (d, *J* = 8.0 Hz, 2H), 5.12 (dd, *J* = 12.0 , 4.0 Hz, 1H), 4.35 (d, *J* = 16.0 Hz, 1H), 4.23 (d, *J* = 16.0 Hz, 1H), 3.98 - 3.90 (m, 2H), 3.70 - 3.50 (m, 12H), 3.24 (s, 3H), 2.99 - 2.84 (m, 2H), 2.61 (d, *J* = 16.6 Hz, 4H), 2.45 - 2.32 (m, 1H), 1.83 - 1.75 (m, 1H).

MS calculated for 804.28; found 805.2 [M+H]⁺.

### Example 44: Synthesis of 3-(6-(4-(2-(2-(2-(2-(2-(4-((6-hydroxy-2-(4-(methylsulfonyl)phenyl)naphthalen-1-yl)oxy)phenoxy)ethyl)amino)ethoxy)acetyl)piperazin-1-yl)-1-oxoisoindolin-2-yl)piperidine-2,6-dione (compound D44)

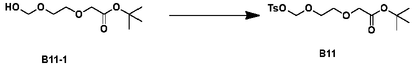

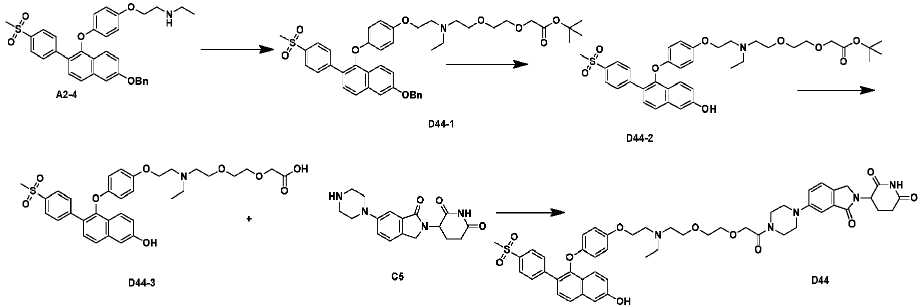

tert-Butyl 2-(2-(hydroxymethoxy)ethoxy)acetate (compound B11-1) (1.37 g, 6.23 mmol) was dissolved in anhydrous dichloromethane (10 mL), and p-toluenesulfonyl chloride (1.78 g, 9.34 mmol) and triethylamine (1.89 g, 18.69 mmol) were added thereto at room temperature. The reaction mixture was stirred at room temperature for 2 hours, poured into water (30 mL), and extracted three times with dichloromethane (30 mL). The organic phases were combined, washed with saturated brine, dried, evaporated to dryness by rotary evaporation, and purified by silica gel column chromatography (dichloromethane/methanol = 10/1) to obtain tert-butyl 2-(2-((tosyloxy)methoxy)ethoxy)acetate (compound B11) (2 g, yield: 85.8%).

Compound A2-4 (1.0 g, 1.76 mmol) was dissolved in DMF (10 mL), and compound B11 (989 mg, 2.64 mmol) and potassium carbonate (728 mg, 5.28 mmol) were added thereto at room temperature. The reaction mixture was heated to 80°C, stirred for 3 hours, diluted with water (30 mL), and extracted three times with ethyl acetate (30 mL). The organic phases were combined, washed with saturated brine, dried over anhydrous sodium sulfate, evaporated to dryness by rotary evaporation, and purified by silica gel column chromatography (dichloromethane/methanol = 10/1) to obtain tert-butyl 2-(2-(2-((2-(4-((6-(benzyloxy)-2-(4-(methylsulfonyl)phenyl)naphthalen-1-yl)oxy)phenoxy)ethyl)(ethyl)amino)ethoxy)acetate (compound D44-1) (1.1 g, yield: 81.5%).

Compound D44-1 (1.1 g, 1.43 mmol) was dissolved in tetrahydrofuran (5 mL)/methanol (5 mL), and wet palladium on carbon (200 mg, 10 wt%) was added thereto at room temperature. The reaction mixture was heated to 50°C and stirred for 18 hours. The reaction mixture was filtered, dried, and evaporated to dryness by rotary evaporation to obtain tert-butyl 2-(2-(2-(ethyl(2-(4-((6-hydroxy-2-(4-(methylsulfonyl)phenyl)naphthalen-1-yl)oxy)phenoxy)ethyl)amino)ethoxy)acetate (compound D44-2) (900 mg, yield: 92.6%).

Compound D44-2 (900 mg, 1.32 mmol) was dissolved in dioxane (1 mL), and hydrogen chloride (4 M in dioxane, 10 mL) was added thereto at room temperature. The reaction mixture was stirred at room temperature for 3 hours. The reaction mixture was evaporated to dryness by rotary evaporation to obtain 2-(2-(2-(2-(4-((6-hydroxy-2-(4-(methylsulfonyl)phenyl)naphthalen-1-yl)oxy)phenoxy)ethyl)amino)ethoxy)acetic acid (compound D44-3) (900 mg, yield > 99%).

Compound D44-3 (200 mg, 0.28 mmol) was dissolved in DMF (4 mL), then 3-(1-oxo-6-(piperazin-1-yl)isoindolin-2-yl)piperidine-2,6-dione (compound C5) (122.3 mg, 0.336 mmol), HATU (128 mg, 0.336 mmol), and DIPEA (108 mg, 0.84 mmol) were sequentially added thereto under ice water, and the reaction mixture was stirred at room temperature for 3 hours. The reaction mixture was diluted with water (5 mL), and extracted three times with ethyl acetate (5 mL). The organic phases were combined, washed with saturated brine, dried, filtered, and the crude product was purified by prep-HPLC to obtain compound D44 (180 mg, yield: 69.2%).

¹H NMR (400 MHz, DMSO-*d*₆) δ 11.00 (s, 1H), 9.36 (s, 1H), 7.93 (d, *J* = 8.5 Hz, 2H), 7.87 (d, *J* = 8.6 Hz, 2H), 7.81 (d, *J* = 8.6 Hz, 1H), 7.75 (d, *J* = 9.1 Hz, 1H), 7.62 (d, *J* = 8.6 Hz, 1H), 7.48 (d, *J* = 8.5 Hz, 1H), 7.30 (d, *J* = 6.4 Hz, 2H), 7.23 (s, 1H), 7.12 (dd, *J* = 9.1, 2.3 Hz, 1H), 6.82 (d, *J* = 9.1 Hz, 2H), 6.67 (d, *J* = 9.1 Hz, 2H), 5.12 (dd, *J* = 13.3, 5.1 Hz, 1H), 4.37 (d, *J* = 17.2 Hz, 4H), 4.24 (t, *J* = 12.7 Hz, 7H), 3.80 (s, 2H), 3.59 (d, *J* = 27.7 Hz, 10H), 3.41 (d, *J* = 10.6 Hz, 2H), 3.18 (s, 2H), 2.97 - 2.91 (m, 1H), 2.63 (d, *J* = 18.0 Hz, 1H), 2.47 - 2.34 (m, 1H), 2.05 - 1.98 (m, 1H), 1.23 (t, *J* = 7.2 Hz, 3H).

MS calculated for 933.36; found 934.3 [M+H]⁺.

### Example 45: Synthesis of 2-(2,6-dioxopiperidin-3-yl)-5-((2-(2-(2-(2-(4-((6-hydroxy-2-(4-(methylsulfonyl)phenyl)naphthalen-1-yl)oxy)phenoxy)ethyl)amino)ethoxy)ethyl)amino)isoindoline-1,3-dione (compound D45)

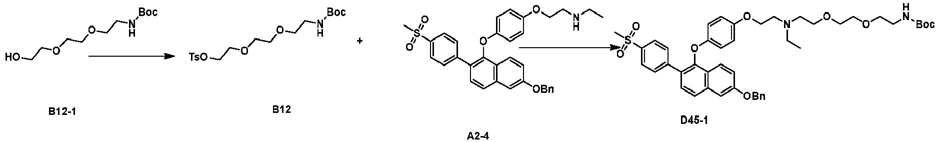

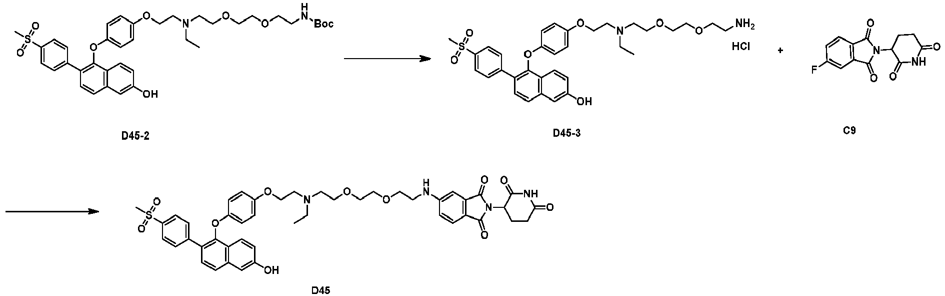

p-Toluenesulfonyl chloride (0.92 g, 4.81 mmol) was added to a solution of tert-butyl (2-(2-(2-hydroxyethoxy)ethoxy)ethyl)carbamate (compound B12-1) (1 g, 4.01 mmol), 4-dimethylaminopyridine (50 mg, 0.40 mmol), and triethylamine (1.22 g, 12.03 mmol) in dichloromethane (80 mL) at room temperature, and the reaction mixture was stirred at room temperature for 16 hours. The reaction mixture was diluted with water, extracted three times with dichloromethane (100 mL), washed with saturated brine, dried over anhydrous sodium sulfate, and concentrated to obtain 2,2-dimethyl-4-oxo-3,8,11-trioxa-5-azatridecan-13-yl 4-methylbenzenesulfonate (compound B12) (1.3 g, yield: 80.3%).

Compound A2-4 (1.0 g, 1.76 mmol), compound B12 (1.07 g, 2.64 mmol), and triethylamine (534 mg, 5.28 mmol) were dissolved in DMF (5 mL), and the reaction mixture was stirred at 80°C for 16 hours. The reaction mixture was concentrated to obtain a crude product, which was purified by silica gel column chromatography (dichloromethane/methanol = 10/1) to obtain tert-butyl (2-(2-(2-((2-(4-((6-(benzyloxy)-2-(4-(methylsulfonyl)phenyl)naphthalen-1-yl)oxy)phenoxy)ethyl)(ethyl)amino)ethoxy)ethoxy)ethyl)carbamate (compound D45-1) (500 mg, yield: 35.7%).

Compound D45-1 (500 mg, 0.63 mmol) was dissolved in methanol (3 mL) and tetrahydrofuran (2 mL), and wet palladium on carbon (100 mg, 10 wt%) was added thereto under nitrogen atmosphere. The reaction mixture was replaced with hydrogen three times, and stirred at room temperature for 2 hours. The reaction mixture was filtered through diatomite, and the filtrate was concentrated to obtain tert-butyl (2-(2-(2-(ethyl(2-(4-((6-hydroxy-2-(4-(methylsulfonyl)phenyl)naphthalen-1-yl)oxy)phenoxy)ethyl)amino)ethoxy)ethyl)carbamate (compound D45-2) (450 mg, yield: 100%).

Compound D45-2 (450 mg, 0.63 mmol) was dissolved in dioxane (5 mL), a solution of hydrochloric acid in dioxane (2.5 mL, 4 M) was slowly added thereto at 0°C, and the reaction mixture was stirred at room temperature for 2 hours. The reaction mixture was concentrated to obtain 5-(4-(2-((2-(2-aminoethoxy)ethoxy)ethyl)(ethyl)amino)ethoxy)phenoxy)-6-(4-(methylsulfonyl)phenyl)naphthalen-2-ol hydrochloride (compound D45-3) (500 mg, yield: 100%).

Compound D45-3 (500 mg, 0.83 mmol) and DIPEA (323 mg, 2.50 mmol) were dissolved in dimethyl sulfoxide (5 mL), then compound C9 (345 mg, 1.25 mmol) was added thereto, and the reaction mixture was stirred at 120°C for 10 hours under nitrogen atmosphere. The reaction mixture was evaporated to dryness by rotary evaporation, and the crude product was purified by prep-HPLC to obtain compound D45 (70 mg, yield: 9.7%).

¹H NMR (400 MHz, DMSO-*d*₆) δ 11.02 (s, 1H), 10.05 (s, 1H), 7.87 (d, *J* = 8.3 Hz, 2H), 7.80 (d, *J* = 8.2 Hz, 2H), 7.70 (dd, *J* = 17.2, 8.9 Hz, 2H), 7.52 (dd, *J* = 14.7, 8.6 Hz, 2H), 7.22 (s, 1H), 7.11 (s, 1H), 7.07 (d, *J* = 8.8 Hz, 1H), 6.96 (s, 1H), 6.84 (d, *J* = 8.1 Hz, 1H), 6.69 (d, *J* = 8.9 Hz, 2H), 6.56 (d, *J=* 9.1 Hz, 2H), 4.99 (dd, *J* = 12.8, 5.0 Hz, 1H), 3.81 (s, 2H), 3.52 (d, *J* = 5.4 Hz, 2H), 3.47 (s, 2H), 3.46 (s, 2H), 3.40 (s, 2H), 3.32 (s, 2H), 3.18 (s, 3H), 2.90-2.78 (m, 1H), 2.77 - 2.66 (m, 2H), 2.65 - 2.54 (m, 4H), 2.01 - 1.90 (m, 1H), 1.21 (s, 2H), 0.89 (t, *J* = 6.5 Hz, 3H).

MS calculated for 864.30; found 865.3 [M+H]⁺.

### Example 46: Synthesis of 3-(5-(4-(2-(2-(2-(2-(2-(4-((6-hydroxy-2-(4-(methylsulfonyl)phenyl)naphthalen-1-yl)oxy)phenoxy)ethyl)amino)ethoxy)ethoxy)acetyl)piperazin-1-yl)-1-oxoisoindolin-2-yl)piperidine-2,6-dione (compound D46)

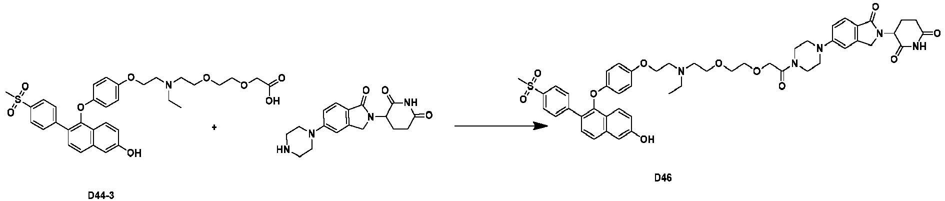

Compound D44-3 (200 mg, 0.28 mmol) was dissolved in DMF (4 mL), then 3-(1-oxo-5-(piperazin-1-yl)isoindolin-2-yl)piperidine-2,6-dione (non-salt substance corresponding to compound C2) (122.3 mg, 0.336 mmol), HATU (128 mg, 0.336 mmol), and DIPEA (108 mg, 0.84 mmol) were sequentially added thereto under ice water, and the reaction mixture was stirred at room temperature for 3 hours. The reaction mixture was diluted with water (10 mL), and extracted three times with ethyl acetate (10 mL). The organic phases were combined, washed with saturated brine, dried, filtered, and the crude product was purified by prep-HPLC to obtain compound D46 (114.2 mg, yield: 43.7%).

¹H NMR (400 MHz, DMSO-*d*₆) δ 10.97 (s, 1H), 9.44 (s, 1H), 7.94 - 7.84 (m, 4H), 7.77 (dd, *J* = 22.7, 8.9 Hz, 2H), 7.59 (dd, *J* = 15.1, 8.5 Hz, 2H), 7.29 (d, *J* = 2.1 Hz, 1H), 7.15 - 7.04 (m, 3H), 6.82 (d, *J* = 9.1 Hz, 2H), 6.66 (d, *J* = 9.1 Hz, 2H), 5.08 (dd, *J* = 13.2, 5.1 Hz, 1H), 4.36 (d, *J* = 16.9 Hz, 1H), 4.26 - 4.19 (m, 5H), 3.80 (s, 2H), 3.58 (d, *J* = 30.8 Hz, 10H), 3.43 - 3.21 (m, 12H), 3.00 - 2.87 (m, 1H), 2.61 (d, *J* = 16.7 Hz, 1H), 2.39 (dd, *J* = 13.5, 4.3 Hz, 1H), 2.06 - 1.93 (m, 1H), 1.23 (t, *J* = 7.2 Hz, 3H).

MS calculated for 933.36; found 934.3 [M+H]⁺.

### Example 47: Synthesis of 3-(4-(4-(2-(2-(2-(2-(2-(4-(4-(6-hydroxy-2-(4-(methylsulfonyl)phenyl)naphthalen-1-yl)oxy)phenoxy)ethyl)amino)ethoxy)acetyl)piperazin-1-yl)-1-oxoisoindolin-2-yl)piperidine-2,6-dione (compound D47)

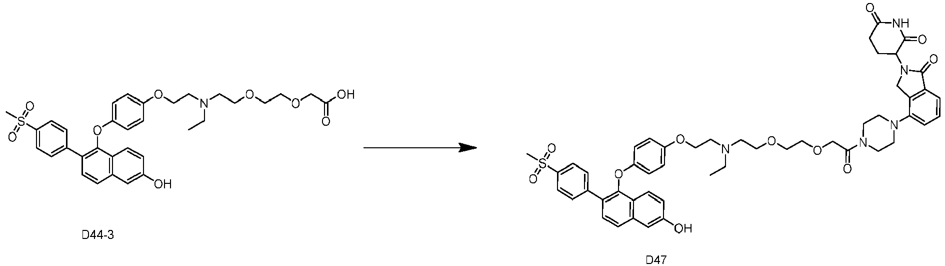

Compound D44-3 (200 mg, 0.28 mmol) was dissolved in DMF (4 mL), then 3-(1-oxo-4-(piperazin-1-yl)isoindolin-2-yl)piperidine-2,6-dione (122.3 mg, 0.336 mmol), HATU (128 mg, 0.336 mmol), and DIPEA (108 mg, 0.84 mmol) were sequentially added thereto under ice water, and the reaction mixture was stirred at room temperature for 3 hours. The reaction mixture was diluted with water (10 mL), and extracted three times with ethyl acetate (10 mL). The organic phases were combined, washed with saturated brine, dried, filtered, and the crude product was purified by prep-HPLC to obtain compound D47 (97.7 mg, yield: 37.4%).

¹H NMR (400 MHz, DMSO-*d*₆) δ 11.02 (s, 1H), 9.31 (s, 1H), 7.93 - 7.84 (m, 4H), 7.77 (dd, *J* = 21.6, 8.8 Hz, 2H), 7.61 (d, *J* = 8.6 Hz, 1H), 7.47 (t, *J* = 7.7 Hz, 1H), 7.38 (d, *J* = 7.1 Hz, 1H), 7.28 (d, *J* = 2.1 Hz, 1H), 7.17 (d, *J* = 7.7 Hz, 1H), 7.11 (dd, *J* = 9.1, 2.3 Hz, 1H), 6.81 (d, *J* = 9.1 Hz, 2H), 6.66 (d, *J* = 9.1 Hz, 2H), 5.16 (dd, *J* = 13.4, 5.2 Hz, 1H), 4.50 (d, *J* = 17.4 Hz, 2H), 4.35 (d, *J* = 17.4 Hz, 2H), 4.21 (d, *J* = 12.7 Hz, 4H), 3.79 (s, 2H), 3.57 (d, *J* = 36.4 Hz, 10H), 3.39 (s, 2H), 3.24 (s, 5H), 3.11 - 2.89 (m, 5H), 2.63 (d, *J=* 17.0 Hz, 1H), 2.50 - 2.40 (m, 1H), 2.07 - 1.96 (m, 1H), 1.23 (t, *J=* 7.2 Hz, 3H).

MS calculated for 933.36; found 934.3 [M+H]⁺.

### Example 48: Synthesis of 2-(2,6-dioxopiperidin-3-yl)-5-((3-ethyl-1-(4-((6-hydroxy-2-(4-(methylsulfonyl)phenyl)naphthalen-1-yl)oxy)phenoxy)-6,9,12-trioxa-3-azatetradecan-14-yl)oxy)isoindoline-1,3-dione (compound D48)

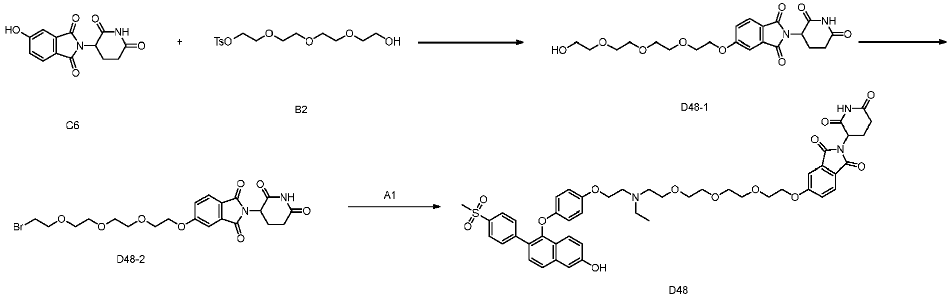

Compound C6 (274 mg, 1.0 mmol), compound B2 (500 mg, 1.44 mmol), sodium bicarbonate (252 mg, 3.0 mmol), and sodium iodide (150 mg, 1.0 mmol) were sequentially added to DMF (2.5 mL) at room temperature. The mixture was stirred at 80°C for 20 hours. The reaction mixture was cooled, concentrated under vacuum, and purified by silica gel column chromatography (eluent: dichloromethane/methanol = 1% to 5%) to obtain 2-(2,6-dioxopiperidin-3-yl)-5-(2-(2-(2-hydroxyethoxy)ethoxy)ethoxy)isoindoline-1,3-dione (compound D48-1) (233 mg, yield: 51%).

Carbon tetrabromide (206 mg, 0.62 mmol) was added to a solution of compound D48-1 (233 mg, 0.52 mmol) and triphenylphosphine (163 mg, 0.62 mmol) in dichloromethane (6 mL) at 0°C, and the reaction mixture was warmed to room temperature and stirred for 16 hours. The reaction mixture was concentrated and purified by silica gel column chromatography (dichloromethane/methanol = 10/1) to obtain 5-(2-(2-(2-(2-bromoethoxy)ethoxy)ethoxy)-2-(2,6-dioxopiperidin-3-yl)isoindoline-1,3-dione (compound D48-2) (451 mg, yield > 99%).

Compound A1 (116 mg, 0.24 mmol), compound D48-2 (271 mg, 0.32 mmol), DIPEA (124 mg, 0.96 mmol), and potassium iodide (8 mg, 0.05 mmol) were sequentially added to DMF (4 mL) at room temperature. The mixture was stirred at 85°C for 30 hours. The reaction mixture was cooled, dissolved in dichloromethane, washed with water and saturated brine, concentrated and purified by prep-HPLC to obtain compound D48 (35 mg, yield: 16%).

¹H NMR (400 MHz, DMSO-*d*₆) δ 11.11 (s, 1H), 10.08 (s, 1H), 9.34 (s, 1H), 7.89 (d, *J* = 8.6 Hz, 2H), 7.86 - 7.80 (m, 3H), 7.77 (d, *J* = 8.6 Hz, 1H), 7.71 (d, *J* = 9.1 Hz, 1H), 7.59 (d, *J* = 8.6 Hz, 1H), 7.42 (d, *J* = 2.3 Hz, 1H), 7.32 (dd, *J* = 8.3, 2.3 Hz, 1H), 7.25 (d, *J* = 2.4 Hz, 1H), 7.09 (dd, *J=* 9.1, 2.4 Hz, 1H), 6.78 (d, *J=* 9.2 Hz, 2H), 6.63 (d, *J=* 9.1 Hz, 2H), 5.12 (dd, *J* = 12.8, 5.4Hz, 1H), 4.26 (dd, *J* = 5.8, 3.1 Hz, 2H), 4.19 - 4.11 (m, 2H), 3.73 (d, *J* = 4.7 Hz, 4H), 3.60 -3.47 (m, 11H), 3.27 - 3.17 (m, 6H), 2.89 (ddd, *J* = 16.9, 13.8, 5.4 Hz, 1H), 2.69 - 2.53 (m, 2H),2.11 - 2.00 (m, 1H), 1.18 (t, *J* = 7.2 Hz, 3H).

MS calculated for 909.31; found 910.3 [M+H]⁺.

### Example 49: Synthesis of 3-(6-(4-(2-(2-(2-((2-(4-((6-(benzyloxy)-2-(4-(methylsulfonyl)phenyl)naphthalen-1-yl)oxy)phenoxy)ethyl)(ethyl)amino)ethoxy)ethyl)piperazin-1-yl)-1-oxoisoindolin-2-yl)piperidine-2,6-dione (compound D49)

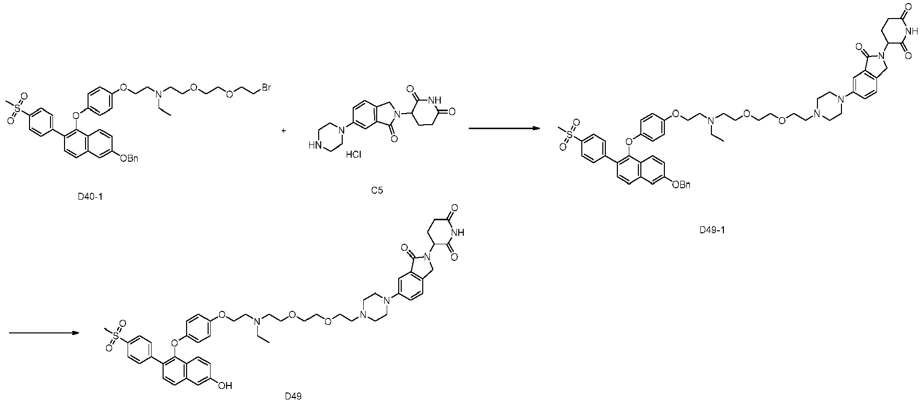

Compound D40-1 (310 mg, 0.407 mmol) and DIPEA (221 mg, 0.65 mmol) were added to DMF (3.0 mL) solvent at room temperature, then hydrochloride of compound C5 (148 mg, 0.407 mmol) was added thereto, and the reaction mixture was heated to 80 to 85°C and stirred for 3 hours. The reaction mixture was concentrated under reduced pressure, added with water (10.0 mL), and extracted twice with dichloromethane (30.0 mL). The organic phases were combined, washed with saturated brine (20.0 mL), dried over anhydrous sodium sulfate, filtered, and concentrated under reduced pressure to obtain a crude product, which was purified by silica gel column chromatography (dichloromethane/methanol = 10/1) to obtain 3-(6-(4-(2-(2-(2-((2-(4-((6-(benzyloxy)-2-(4-(methylsulfonyl)phenyl)naphthalen-1-yl)oxy)phenoxy)ethyl)(ethyl)amino)ethoxy)ethyl)piperazin-1-yl)-1-oxoisoindolin-2-yl)piperidine-2,6-dione (compound D49-1) (260 mg, yield: 64%).

Compound D49-1 (260 mg, 0.26 mmol) and wet palladium on carbon (150 mg, 10 wt%) were sequentially added to a mixed solvent of methanol (3.0 mL) and tetrahydrofuran (6.0 mL) at room temperature. The reaction mixture was stirred at 20°C for 16 hours under hydrogen atmosphere, filtered, and the filtrate was concentrated under reduced pressure. The crude product was purified by silica gel column chromatography (dichloromethane/methanol = 10/1) to obtain compound D49 (60 mg, yield: 25%).

¹H NMR (400 MHz, DMSO-*d*₆) δ: 11.0 (s, 1H), 10.10 (s, 1H), 7.92 (d*, J* = 8.0 Hz*,* 2H), 7.86 (d, *J* = 8.0 Hz, 2H), 7.77 (dd, *J* = 20.0, 10.0 Hz, 2H), 7.60 (d, *J* = 8.0 Hz, 1H), 7.45 (d, *J* = 8.0 Hz, 1H), 7.27 (m, 2H), 7.14 (m, 2H), 6.80- 6.72 (d, *J* = 8.0 Hz, 2H), 6.62 (d, *J* = 8.0 Hz, 2H), 5.14 (dd, *J* = 12.0 , 4.0 Hz, 1H), 4.35 (d, *J* = 16.0 Hz, 1H), 4.23 (d, *J* = 16.0 Hz, 1H), 3.98 - 3.90 (m, 2H), 3.70 - 3.50 (m, 16H), 3.24 (s, 3H), 2.99 - 2.84 (m, 4H), 2.61 (d, *J* = 16.6 Hz, 4H), 2.45 - 2.32 (m, 1H), 1.83 - 1.75 (m, 1H), 1.28 (s, 3H).

MS calculated for 919.4; found 920.3 [M+H]⁺.

### Example 50: Synthesis of 3-(6-(4-(2-(2-(2-(2-(4-((6-hydroxy-2-(4-(methylsulfonyl)phenyl)naphthalen-1-yl)oxy)phenoxy)ethyl)amino)ethoxy)acetyl)piperazin-1-yl)-1-oxoisoindolin-2-yl)piperidine-2,6-dione (compound D50)

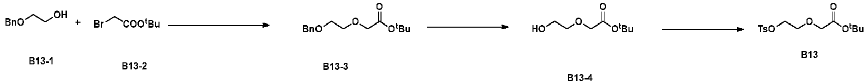

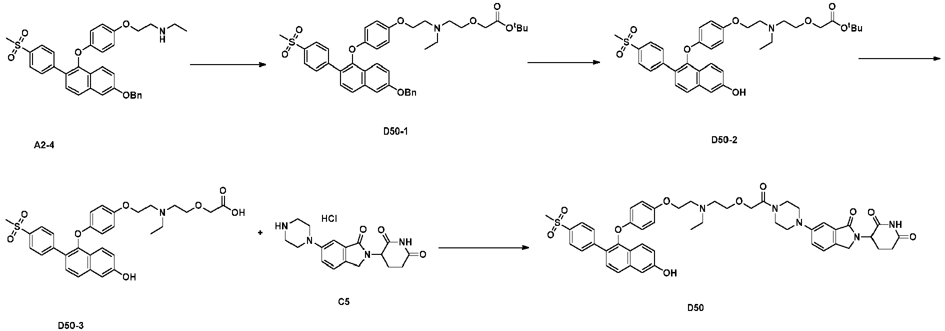

Sodium hydride (1.58 g, 39.4 mmol) was added to a solution of 2-(benzyloxy)ethan-1-ol (compound B13-1) (5 g, 32.8 mmol) in tetrahydrofuran (70 mL) at 0°C, and the reaction mixture was stirred for 0.5 hours. The reaction mixture was added dropwise with tert-butyl bromoacetate (compound B13-2) (7.70 g, 39.4 mmol), warmed to room temperature, and stirred for 15 hours. The reaction mixture was cooled to 0°C, added with saturated ammonium chloride to quench, extracted with ethyl acetate, concentrated under reduced pressure, and purified by silica gel column chromatography (petroleum ether/ethyl acetate = 4:1) to obtain tert-butyl 2-(2-(benzyloxy)ethoxy)acetate (compound B13-3) (2.42 g, yield: 28%).

Wet palladium on carbon (200 mg, 10 wt%) was added to a solution of compound B13-3 (2.42 g, 9.1 mmol) in tetrahydrofuran/methanol (20 mL/5 mL). The mixture was stirred at 30°C for 18 hours under hydrogen balloon atmosphere. The reaction mixture was filtered, washed with tetrahydrofuran/dichloromethane, and concentrated to obtain tert-butyl 2-(2-hydroxyethoxy)acetate (compound B13-4) (1.6 g, yield: 100%).

DIPEA (2.5 mL, 18.2 mmol) and p-toluenesulfonyl chloride (1.91 g, 10.0 mmol) were added to a solution of compound B13-4 (1.6 g, 9.1 mmol) and 4-dimethylaminopyridine (11 mg, 0.09 mmol) in dichloromethane (40 mL) at 0°C, and the reaction mixture was warmed to room temperature and stirred for 10 hours. The reaction mixture was diluted with water, extracted with ethyl acetate, concentrated under reduced pressure, and purified by silica gel column chromatography (petroleum ether/ethyl acetate = 4:1) to obtain tert-butyl 2-(tosyloxy)ethoxy)acetate (compound B13) (2.58 g, yield: 86%).

A mixture of compound A2-4 (500 mg, 0.88 mmol), compound B13 (436 mg, 1.3 mmol), potassium carbonate (365 mg, 2.64 mmol), and potassium iodide (44 mg, 0.26 mmol) in DMF (6 mL) was stirred at 85°C for 40 hours. The reaction mixture was cooled, concentrated and purified by silica gel column chromatography (dichloromethane/methanol = 10/1) to obtain tert-butyl 2-(2-((2-(4-((6-(benzyloxy)-2-(4-(methylsulfonyl)phenyl)naphthalen-1-yl)oxy)phenoxy)ethyl)(ethyl)amino)ethoxy)acetate (compound D50-1) (440 mg, yield: 69%).

Wet palladium on carbon (150 mg, 10 wt%) was added to a solution of compound D50-1 (440 mg, 0.61 mmol) in tetrahydrofuran/methanol (10 mL/4 mL). The mixture was stirred at 30°C for 15 hours under hydrogen balloon atmosphere. The reaction mixture was filtered, washed with methanol/dichloromethane, and concentrated to obtain tert-butyl 2-(2-(ethyl(2-(4-((6-hydroxy-2-(4-(methylsulfonyl)phenyl)naphthalen-1-yl)oxy)phenoxy)ethyl)amino)ethoxy)acetate (compound D50-2) (354 mg, yield: 91%).

Trifluoroacetic acid (2.5 mL) was added to a solution of compound D50-2 (130 mg, 0.20 mmol) in dichloromethane (4 mL), and the reaction mixture was stirred at room temperature for 12 hours. The reaction mixture was concentrated to obtain 2-(2-(ethyl(2-(4-((6-hydroxy-2-(4-(methylsulfonyl)phenyl)naphthalen-1-yl)oxy)phenoxy)ethyl)amino)ethoxy)acetic acid (compound D50-3) (220 mg, yield: 99%).

DIPEA (129 mg, 1.0 mmol) and HATU (76 mg, 0.20 mmol) were added to a solution of compound D50-3 (220 mg, 0.2 mmol) and hydrochloride of compound C5 (82 mg 0.20 mmol) in DMF (3 mL), and the reaction mixture was stirred at room temperature for 2 hours. The reaction mixture was concentrated under reduced pressure, dissolved in dichloromethane/methanol (10/1 v/v, 20 mL), washed with water, concentrated and purified by prep-HPLC to obtain compound D50 (75 mg, yield: 42%).

¹H NMR (400 MHz, DMSO-*d*₆) δ 10.96 (s, 1H), 8.15 (s, 1H), 7.89 (d, *J* = 8.5 Hz, 2H), 7.85 - 7.79 (m, 2H), 7.73 (dd, *J* = 17.3, 8.9 Hz, 2H), 7.57 (d, *J* = 8.6 Hz, 1H), 7.41 (d, *J* = 8.4 Hz, 1H), 7.27 - 7.17 (m, 3H), 7.08 (dd, *J* = 9.1, 2.4 Hz, 1H), 6.72 (d, *J* = 9.1 Hz, 2H), 6.58 (d, *J* = 9.1 Hz, 2H), 5.10 (dd, *J* = 13.3, 5.1 Hz, 1H), 4.33 (d, *J* = 16.7 Hz, 1H), 4.21 (d, *J* = 16.8 Hz, 1H), 4.15 (s, 2H), 3.86 (t, *J* = 6.0 Hz, 2H), 3.60 - 3.53 (m, 4H), 3.50 (t, *J* = 5.9 Hz, 2H), 3.20 (s, 3H), 3.19 - 3.12 (m, 4H), 2.96 - 2.85 (m, 1H), 2.79 (t, *J=* 6.0 Hz, 2H), 2.69 (t, *J* = 6.1 Hz, 2H), 2.63 - 2.55 (m, 3H), 2.42 - 2.30 (m, 1H), 2.03 - 1.92 (m, 1H), 0.94 (t, *J* = 7.0 Hz, 3H).

MS calculated for 889.3; found 890.3 [M+H]⁺.

Example 51: Synthesis of 3-(6-(4-(2-(2-(2-(4-(4-((6-hydroxy-2-(4-(methylsulfonyl)phenyl)naphthalen-1-yl)oxy)phenoxy)ethyl)amino)ethoxy)ethyl)piperazin-1-yl)-1-oxoisoindolin-2-yl)piperidine-2,6-dione (compound D51)

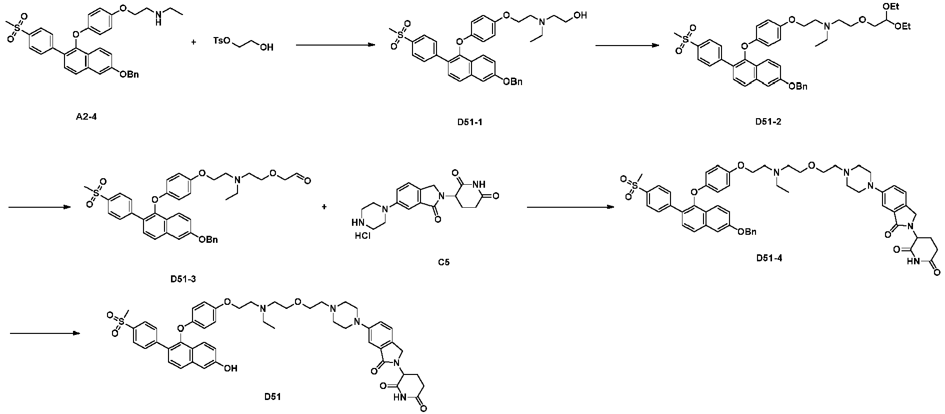

Compound A2-4 (1.13 g, 2 mmol), potassium carbonate (552 mg, 4 mmol), and potassium iodide (66.4 mg, 0.4 mmol) were added to DMF (15.0 mL) solvent at room temperature, then 2-hydroxyethyl 4-methylbenzenesulfonate (650 mg, 3 mmol) was added thereto, and the reaction mixture was heated to 90 to 100°C and stirred for 4 hours. The reaction mixture was concentrated under reduced pressure, added with water (40.0 mL), and extracted twice with dichloromethane (40.0 mL). The organic phases were combined, washed with saturated brine (40.0 mL), dried over anhydrous sodium sulfate, filtered, and concentrated under reduced pressure to obtain a crude product, which was then slurried with tert-butyl methyl ether (15 mL), and filtered to obtain 2-((2-(4-((6-(benzyloxy)-2-(4-(methylsulfonyl)phenyl)naphthalen-1-yl)oxy)phenoxy)ethyl)(ethyl)amino)ethan-1-ol (compound D51-1) (610 mg, yield: 50%).

Sodium hydride (120 mg, 3.0 mmol) was added to anhydrous tetrahydrofuran (5 mL) at room temperature. The reaction mixture was stirred for 10 minutes, added with a solution of compound D51-1 (610 mg, 1 mmol) in DMF (3 mL)/tetrahydrofuran (3 mL) at 0°C, stirred at room temperature for 30 minutes, then added with 2-bromo-1,1-diethoxyethane (600 mg, 3 mmol), heated to 70 to 80°C, stirred and reacted for 4 hours, concentrated under reduced pressure, and purified by silica gel column chromatography (dichloromethane/methanol = 10/1) to obtain 2-(4-((6-(benzyloxy)-2-(4-(methylsulfonyl)phenyl)naphthalen-1-yl)oxy)phenoxy)-N-(2-(2,2-diethoxyethoxy)ethyl)-N-ethylethan-1-amine (compound D51-2) (727 mg, yield: 100%).

Compound D51-2 (727 mg, 1 mmol) was added to a mixed solvent of a solution of hydrochloric acid in ethyl acetate (4 M, 6.0 mL) and tetrahydrofuran (6.0 mL) at room temperature, and the reaction mixture was stirred at room temperature for 30 minutes. The reaction mixture was concentrated under reduced pressure, added with water (20.0 mL), and extracted twice with dichloromethane (20.0 mL). The organic phases were combined, washed with saturated brine (20.0 mL), dried over anhydrous sodium sulfate, filtered, concentrated under reduced pressure to obtain a crude product, which was purified by silica gel column chromatography (dichloromethane/methanol = 10/1) to obtain 2-(2-((2-(4-((6-(benzyloxy)-2-(4-(methylsulfonyl)phenyl)naphthalen-1-yl)oxy)phenoxy)ethyl)(ethyl)amino)ethoxy)acetaldehyde (compound D51-3) (310 mg, yield: 47.4%).

Compound D51-3 (200 mg, 0.61 mmol), hydrochloride of compound C5 (127 mg, 0.67 mmol), and potassium acetate (152 mg, 3 mmol) were sequentially added to a mixed solvent of methanol (4.0 mL) and dichloromethane (8.0 mL) at room temperature. The reaction mixture was stirred at room temperature for 10 minutes, added with sodium triacetoxyborohydride (200 mg, 3.0 mmol), and stirred at 20°C for 16 hours. The reaction mixture was concentrated under reduced pressure and purified by silica gel column chromatography (dichloromethane/methanol = 10/1) to obtain 3-(6-(4-(2-(2-(2-(4-(4-((6-hydroxy-2-(4-(methylsulfonyl)phenyl)naphthalen-1-yl)oxy)phenoxy)ethyl)amino)ethoxy)ethyl)piperazin-1-yl)-1-oxoisoindolin-2-yl)piperidine-2,6-dione (compound D51-4) (240 mg, yield: 45%).

Compound D51-4 (100 mg, 0.114 mmol) and wet palladium on carbon (120 mg, 10 wt%) were sequentially added to a mixed solvent of methanol (2.0 mL) and tetrahydrofuran (4.0 mL) at room temperature. The reaction mixture was stirred at 20°C for 16 hours under hydrogen atmosphere, filtered, and the filtrate was concentrated under reduced pressure. The crude product was purified by silica gel column chromatography (dichloromethane/methanol = 10/1) to obtain compound D51 (20 mg, yield: 20%).

¹H NMR (400 MHz, DMSO-*d*₆) δ: 11.0 (s, 1H), 10.02 (s, 1H), 7.93 (d, *J* = 8.2 Hz, 2H), 7.86 (d, *J* = 8.3 Hz, 2H), 7.77 (dd, *J* = 16.2, 8.8 Hz, 2H), 7.60 (d, *J* = 7.5 Hz, 1H), 7.45 (d, *J* = 8.0 Hz, 1H), 7.27 (t, *J* = 5.7 Hz, 2H), 7.11 (dt, *J* = 8.9, 4.4 Hz, 2H), 6.83 - 6.77 (m, 2H), 6.65 (d, *J* = 9.1 Hz, 2H), 5.14 (dd, *J* = 12.0 , 4.0 Hz, 1H), 4.38 (d, *J* = 16.0 Hz, 1H), 4.25 (d, *J* = 16.0 Hz, 1H), 3.98 - 3.90 (m, 2H), 3.70 - 3.50 (m, 12H), 3.24 (s, 3H), 2.99 - 2.84 (m, 4H), 2.61 (d, *J* = 16.6 Hz, 4H), 2.45 - 2.32 (m, 1H), 1.83 - 1.75 (m, 1H), 1.28 (s, 3H).

MS calculated for 875.36; found 876.3 [M+H]⁺.

Example 52: Synthesis of 3-(4-(4-(2-(2-(2-(2-(4-((6-hydroxy-2-(4-(methylsulfonyl)phenyl)naphthalen-1-yl)oxy)phenoxy)ethyl)amino)ethoxy)acetyl)piperazin-1-yl)-1-oxoisoindolin-2-yl)piperidine-2,6-dione (compound D52)

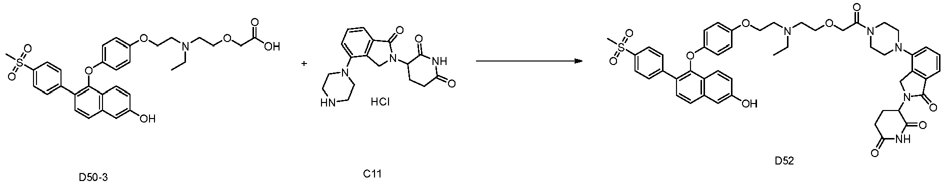

DIPEA (135 mg, 1.05 mmol) and HATU (80 mg, 0.21 mmol) were added to a solution of compound D50-3 (218 mg, 0.21 mmol) and hydrochloride of compound C11 (77 mg 0.21 mmol) in DMF (3 mL), and the reaction mixture was stirred at room temperature for 2 hours. The reaction mixture was concentrated under reduced pressure, dissolved in dichloromethane (20 mL)/methanol (2 mL), washed with water, concentrated and purified by silica gel column chromatography (dichloromethane/methanol = 10/1) to obtain compound D52 (70 mg, yield: 37%).

¹H NMR (400 MHz, DMSO-*d*₆) δ 11.00 (s, 1H), 10.09 (s, 1H), 7.89 (d, *J* = 8.5 Hz, 2H), 7.83 (d, *J=* 8.5 Hz, 2H), 7.73 (dd, *J=* 17.9, 8.9 Hz, 2H), 7.57 (d, *J* = 8.6 Hz, 1H), 7.45 (t, *J* = 7.7 Hz, 1H), 7.36 (d, *J* = 7.4 Hz, 1H), 7.25 (d, *J* = 2.4 Hz, 1H), 7.17 - 7.06 (m, 2H), 6.76 (d, *J* = 9.1 Hz, 2H), 6.62 (d, *J* = 9.1 Hz, 2H), 5.14 (dd, *J* = 13.3, 5.1 Hz, 1H), 4.47 (d, *J* = 17.4 Hz, 1H), 4.32 (d, *J* = 17.4 Hz, 1H), 4.27 - 4.18 (m, 2H), 4.17 - 4.06 (m, 2H), 3.78 - 3.66 (m, 2H), 3.64 - 3.53 (m, 2H), 3.50 - 3.37 (m, 4H), 3.27 - 3.19 (m, 5H), 3.17 - 2.96 (m, 6H), 2.95 - 2.85 (m, 1H), 2.69 - 2.55 (m, 1H), 2.48 - 2.37 (m, 1H), 2.06 - 1.95 (m, 1H), 1.22 - 1.12 (m, 3H).

MS calculated for 889.34; found 890.3 [M+H]⁺.

### Example 53: Synthesis of 3-(4-(4-(2-(4-((6-hydroxy-2-(4-(methylsulfonyl)phenyl)naphthalen-1-yl)oxy)phenoxy)ethyl)piperazin-1-yl)-1-oxoisoindolin-2-yl)piperidine-2,6-dione (compound D53)

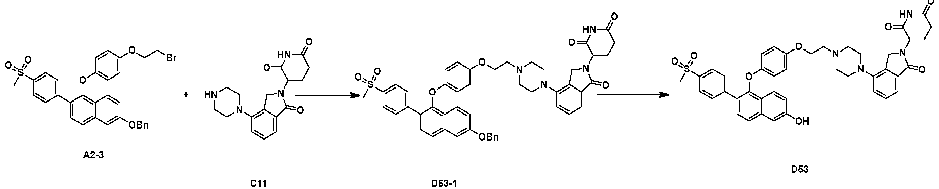

Compound A2-3 (100 mg, 0.166 mmol) was dissolved in DMF (1 mL), and compound C11 (120.9 mg, 0.332 mmol) and DIPEA (64.2 mg, 0.498 mmol) were sequentially added thereto at room temperature. The reaction mixture was heated to 80°C and stirred for 3 hours. The reaction mixture was poured into water (15 mL), and extracted three times with ethyl acetate (15 mL). The organic phases were combined, washed with saturated brine, dried, evaporated to dryness by rotary evaporation, and purified by silica gel column chromatography (dichloromethane/methanol = 10/1) to obtain 3-(4-(4-(2-(4-((6-(benzyloxy)-2-(4-(methylsulfonyl)phenyl)naphthalen-1-yl)oxy)phenoxy)ethyl)piperazin-1-yl)-1-oxoisoindolin-2-yl)piperidine-2,6-dione (compound D53-1) (100 mg, yield: 70.9%).

Compound D53-1 (100 mg, 0.117 mmol) was dissolved in tetrahydrofuran (1 mL) and methanol (1 mL), and wet palladium on carbon (10 mg, 10 wt%) was added thereto at room temperature. The reaction mixture was stirred at room temperature for 18 hours under hydrogen balloon atmosphere, filtered, and evaporated to dryness by rotary evaporation. The crude product was purified by prep-HPLC to obtain compound D53 (45.0 mg, yield: 50.6%).

¹H NMR (400 MHz, DMSO-*d*₆) δ 10.99 (s, 1H), 10.07 (s, 1H), 7.95 - 7.84 (m, 4H), 7.77 (dd, *J* = 15.6, 8.9 Hz, 2H), 7.61 (d, *J* = 8.6 Hz, 1H), 7.45 (t, *J* = 7.6 Hz, 1H), 7.33 (d, *J* = 7.3 Hz, 1H), 7.28 (d, *J* = 2.1 Hz, 1H), 7.19 - 7.10 (m, 2H), 6.79 (d, *J* = 9.1 Hz, 2H), 6.63 (d, *J* = 9.1 Hz, 2H), 5.13 (dd, *J* = 13.3, 5.0 Hz, 1H), 4.46 (d, *J* = 17.3 Hz, 1H), 4.31 (d, *J* = 17.3 Hz, 1H), 4.00 (d, *J* = 5.8 Hz, 2H), 3.24 (s, 3H), 3.07 (d, *J* = 5.3 Hz, 5H), 2.98 - 2.89 (m, 1H), 2.71 (s, 2H), 2.64 (s, 5H), 2.01 (d, *J* = 10.7 Hz, 1H).

MS calculated for 760.26; found 761.3 [M+H]⁺.

### Example 54: Synthesis of 3-(5-(4-(2-(4-((6-hydroxy-2-(4-(methylsulfonyl)phenyl)naphthalen-1-yl)oxy)phenoxy)acetyl)piperazin-1-yl)-1-oxoisoindolin-2-yl)piperidine-2,6-dione (compound D54)

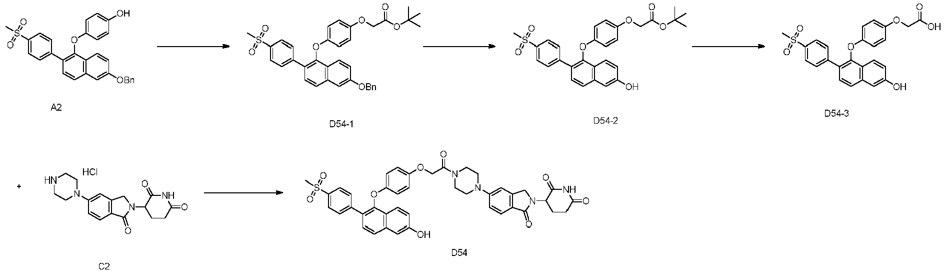

A mixture of compound A2 (300 mg, 0.6 mmol), ethyl bromoacetate (176 mg, 0.91 mmol), and potassium carbonate (248 mg, 1.80 mmol) in DMF (5 mL) was stirred at 60°C for 5 hours. The reaction mixture was diluted with water, extracted with ethyl acetate, concentrated under reduced pressure, and purified by silica gel column chromatography (dichloromethane/methanol = 10/1) to obtain tert-butyl 2-(4-((6-(benzyloxy)-2-(4-(methylsulfonyl)phenyl)naphthalen-1-yl)oxy)phenoxy)acetate (compound D54-1) (340 mg, yield: 93%).

Wet palladium on carbon (250 mg, 10 wt%) was added to a solution of compound D54-1 (340 mg, 0.55 mmol) in tetrahydrofuran/methanol (12 mL/2 mL). The mixture was stirred at 30°C for 15 hours under hydrogen balloon atmosphere. The reaction mixture was filtered, washed with dichloromethane/tetrahydrofuran, and concentrated under reduced pressure to obtain tert-butyl 2-(4-((6-hydroxy-2-(4-(methylsulfonyl)phenyl)naphthalen-1-yl)oxy)phenoxy)acetate (compound D54-2) (340 mg, yield > 100%).

Trifluoroacetic acid (2 mL) was added to a solution of compound D54-2 (216 mg, 0.36 mmol) in dichloromethane (5 mL), and the reaction mixture was stirred at room temperature for 2 hours. The reaction mixture was concentrated to obtain 2-(4-((6-hydroxy-2-(4-(methylsulfonyl)phenyl)naphthalen-1-yl)oxy)phenoxy)acetic acid (compound D54-3) (267 mg, yield > 99%).

DIPEA (139 mg, 1.1 mmol) and HATU (136 mg, 0.36 mmol) were added to a solution of compound D54-3 (267 mg, 0.36 mmol) and compound C2 (130 mg, 0.36 mmol) in DMF (4 mL). The reaction mixture was washed with water, concentrated and purified by silica gel column chromatography (eluent: methanol/dichloromethane = 1% to 20%), concentrated and purified by prep-HPLC, and lyophilized to obtain compound D54 (50 mg, yield: 18%).

¹H NMR (500 MHz, DMSO-*d*₆) δ 10.94 (s, 1H), 10.04 (s, 1H), 7.90 (d, *J* = 8.5 Hz, 2H), 7.84 (d, *J* = 8.5 Hz, 2H), 7.74 (dd, *J* = 21.2, 8.9 Hz, 2H), 7.56 (dd, *J* = 22.8, 8.5 Hz, 2H), 7.25 (d, *J* = 2.4 Hz, 1H), 7.12 - 7.05 (m, 3H), 6.74 (d, *J* = 9.2 Hz, 2H), 6.60 (d, *J* = 9.2 Hz, 2H), 5.05 (dd, *J* = 13.3, 5.1 Hz, 1H), 4.72 (s, 2H), 4.33 (d, *J* = 16.9 Hz, 1H), 4.21 (d, *J* = 16.9 Hz, 1H), 3.62 - 3.51 (m, 4H), 3.33 - 3.25 (m, 4H), 3.22 (s, 3H), 2.96 - 2.84 (m, 1H), 2.66 - 2.54 (m, 1H), 2.44 - 2.31 (m, 1H), 2.03 - 1.91 (m, 1H).

MS calculated for 774.24; found 775.3 [M+H]⁺.

### Example 55: Synthesis of 5-(4-(2-(4-(2-(2,6-dioxopiperidin-3-yl)-1-oxoisoindolin-5-yl)piperazin-1-yl)ethoxy)phenoxy)-6-(4-(methylsulfonyl)phenyl)-2-naphthonitrile (compound D55)

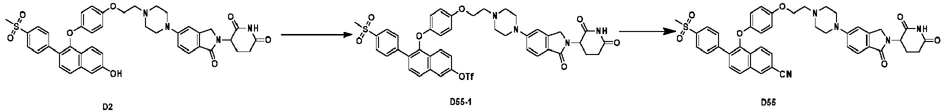

Compound D2 (500 mg, 0.66 mmol) was dissolved in anhydrous DMF (5 mL), and phenyltrifluoromethanesulfonimide (260 mg, 0.725 mmol) and DIPEA (170 mg, 1.32 mmol) were sequentially added thereto at room temperature. The reaction mixture was stirred at room temperature for 2 hours. The reaction mixture was poured into water (15 mL), and extracted three times with ethyl acetate (15 mL). The organic phases were combined, washed with saturated brine, dried, evaporated to dryness by rotary evaporation, and the crude product was purified by silica gel column chromatography (dichloromethane/methanol = 10/1) to obtain 5-(4-(2-(4-(2,6-dioxopiperidin-3-yl)-1-oxoisoindolin-5-yl)piperazin-1-yl)ethoxy)phenoxy)-6-(4-(methylsulfonyl)phenyl)naphthalen-2-yl trifluoromethanesulfonate (compound D55-1) (400 mg, yield: 68.1%).

Compound D55-1 (100 mg, 0.112 mmol) was dissolved in DMF (1 mL), and zinc cyanide (20 mg, 0.168 mmol), bis(dibenzylideneacetone)palladium (10.3 mg, 0.0112 mmol), and 1'-bis(diphenylphosphino)ferrocene (7.5 mg, 0.0134 mmol) were sequentially added thereto at room temperature. The reaction mixture was replaced with nitrogen, heated to 100°C, and stirred for 18 hours. The reaction mixture was poured into water (15 mL), and extracted three times with ethyl acetate (15 mL). The organic phases were combined, washed with saturated brine, dried, evaporated to dryness by rotary evaporation, and the crude product was purified by prep-HPLC to obtain compound D55 (15.0 mg, yield: 17.4%).

¹H NMR (400 MHz, DMSO-*d*₆) δ 10.94 (s, 1H), 8.78 (s, 1H), 8.18 (d, *J* = 8.4 Hz, 1H), 7.98 (dd, *J* = 17.0, 8.7 Hz, 3H), 7.91 (d, *J* = 7.9 Hz, 3H), 7.87 - 7.81 (m, 1H), 7.58 (d, *J* = 8.5 Hz, 1H), 7.18 - 7.10 (m, 2H), 6.83 (d, *J* = 9.1 Hz, 2H), 6.68 (d, *J* = 9.2 Hz, 2H), 5.06 (dd, *J* = 13.2, 4.9 Hz, 1H), 4.35 (d, *J* = 17.1 Hz, 1H), 4.24 (d, *J* = 9.4 Hz, 3H), 3.98 (s, 2H), 3.54 (s, 3H), 3.24 (s, 4H), 3.15 (s, 2H), 2.95 - 2.86 (m, 1H), 2.59 (d, *J* = 19.9 Hz, 1H), 2.37 (dd, *J*= 19.6, 11.0 Hz, 1H), 1.96 (d, *J* = 10.6 Hz, 1H).

MS calculated for 729.26; found 730.3 [M+H]⁺.

### Example 56: Synthesis of 3-(6-(4-(2-(ethyl)(2-(4-((6-hydroxy-2-(4-(methylsulfonyl)phenyl)naphthalen-1-yl)oxy)phenoxy)ethyl)amino)ethyl)piperazin-1-yl)-1-oxoisoindolin-2-yl)piperidine-2,6-dione (compound D56)

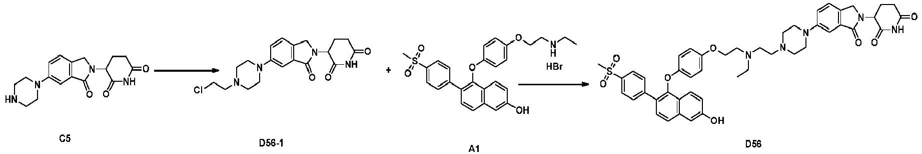

Potassium acetate (356 mg, 3.63 mmol) was added to a solution of compound C5 (500 mg, 1.2 mmol) in methanol (10 mL), and the reaction mixture was stirred for 15 minutes. The reaction mixture was added with acetic acid (14 mg, 0.23 mmol) and chloroacetaldehyde (40% aqueous solution, 472 mg, 2.42 mmol) and stirred for 0.5 hours. The reaction mixture was added with 2-methylpyridine borane complex (259 mg, 2.42 mmol) and stirred at room temperature for 18 hours. The reaction mixture was concentrated under reduced pressure, diluted with water, extracted with ethyl acetate, concentrated and purified by silica gel column chromatography (dichloromethane/methanol = 10/1) to obtain 3-(6-(4-(2-chloroethyl)piperazin-1-yl)-1-oxoisoindolin-2-yl)piperidine-2,6-dione (compound D56-1) (310 mg, yield: 65%).

A mixture of compound D56-1 (100 mg, 0.25 mmol), compound A1 (100 mg, 0.17 mmol), DIPEA (154 mg, 1.19 mmol), and potassium iodide (56 mg, 0.34 mmol) in DMF (3 mL) was stirred at 100°C for 24 hours. The reaction mixture was cooled, dissolved in dichloromethane, washed with water and saturated brine, concentrated and purified by prep-HPLC, and lyophilized to obtain compound D56 (40 mg, yield: 18%).

¹H NMR (400 MHz, DMSO-*d*₆) δ 10.95 (s, 1H), 10.04 (s, 1H), 7.90 (d, *J* = 8.5 Hz, 2H), 7.83 (d, *J=* 8.5 Hz, 2H), 7.74 (dd, *J* = 15.8, 8.9 Hz, 2H), 7.58 (d, *J* = 8.6 Hz, 1H), 7.42 (d, *J* = 8.4 Hz, 1H), 7.29 - 7.20 (m, 2H), 7.15 (d, *J* = 2.3 Hz, 1H), 7.09 (dd, *J* = 9.1, 2.4 Hz, 1H), 6.75 (d, *J* = 9.1 Hz, 2H), 6.60 (d, *J* = 9.1 Hz, 2H), 5.09 (dd, *J* = 13.3, 5.1 Hz, 1H), 4.33 (d*, J* = 16.7 Hz, 1H), 4.21 (d, *J* = 16.7 Hz, 1H), 3.92 (t, *J* = 5.9 Hz, 2H), 3.21 (s, 3H), 3.19 - 3.09 (m, 6H), 2.95 - 2.84 (m, 3H), 2.75 - 2.55 (m, 9H), 2.40 - 2.30 (m, 1H), 2.05 - 1.94 (m, 1H).

MS calculated for 831.33; found 832.3 [M+H]⁺.

### Example 57: Synthesis of 3-(5-(4-(2-(ethyl(2-(4-((6-hydroxy-2-(4-(methylsulfonyl)phenyl)naphthalen-1-yl)oxy)phenoxy)ethyl)amino)ethyl)piperazin-1-yl)-1-oxoisoindolin-2-yl)piperidine-2,6-dione (compound D57)

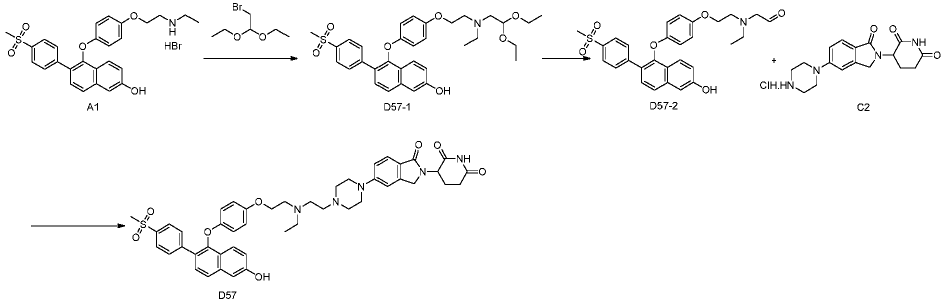

Compound A1 (296 mg, 0.53 mmol) and cesium carbonate (345 mg, 1.06 mmol) were added to DMF (5.0 mL) at room temperature. The resulting mixture was stirred at 70°C for 30 minutes, and then bromoacetaldehyde diethyl acetal (522 mg, 2.65 mmol) was added thereto. The reaction mixture was then heated to 100°C, stirred for 10 hours, cooled to room temperature, and diluted with water (10 mL). The resulting mixture was extracted twice with dichloromethane (20.0 mL). The organic phases were combined, dried over anhydrous sodium sulfate, filtered, and concentrated under reduced pressure. The crude product was purified by silica gel column chromatography (dichloromethane/methanol = 10/1) to obtain compound D57-1 (260 mg, yield: 83%).

Trifluoroacetic acid (0.5 mL) was added dropwise to a solution of compound D57-1 (113 mg, 0.19 mmol) in dichloromethane (5.0 mL) at room temperature. The resulting mixture was stirred at room temperature for 3 hours, diluted with dichloromethane (20.0 mL), and then washed twice with saturated sodium bicarbonate aqueous solution (20.0 mL). The organic phase was separated, dried over anhydrous sodium sulfate, filtered, and concentrated to dryness under reduced pressure to obtain a crude product of compound D57-2 (100 mg, yield: 100%).

Compound D57-2 (100 mg, 0.19 mmol), compound C2 (69 mg, 0.19 mmol), and potassium acetate (56 mg, 0.57 mmol) were sequentially added to a mixed solvent of dichloromethane (5.0 mL) and methanol (5.0 mL) at room temperature. The resulting mixture was stirred at room temperature for 30 minutes, then added with sodium triacetoxyborohydride (81 mg, 0.38 mmol), stirred for another 18 hours, and diluted with water (20.0 mL). The organic phase was separated, and the aqueous phase was extracted twice with dichloromethane (20.0 mL). The organic phases were combined, dried over anhydrous sodium sulfate, filtered, and concentrated under reduced pressure. The crude product was purified by silica gel column chromatography (dichloromethane/methanol = 10/1) and purified by prep-HPLC to obtain compound D57 (4.7 mg, yield: 3%, purity: 95%) as a white solid.

MS calculated for 831.3; found 832.2 [M+H]⁺.

### Example 58: Synthesis of 3-(5-(4-(4-(4-((6-methoxy-2-(4-(methylsulfonyl)phenyl)naphthalen-1-yl)oxy)phenoxy)butyl)piperazin-1-yl)-1-oxoisoindolin-2-yl)piperidine-2,6-dione (compound D58)

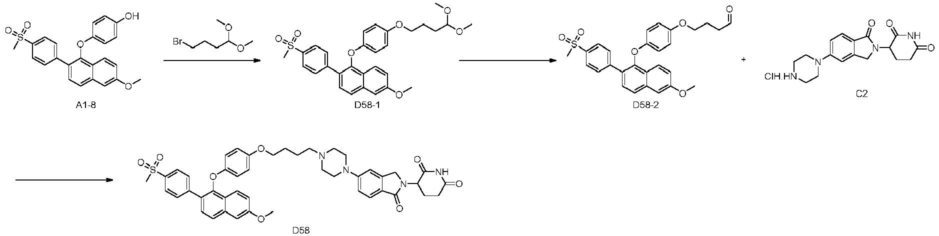

Compound A1-8 (300 mg, 0.71 mmol) and cesium carbonate (465 mg, 1.42 mmol) were added to DMF (8.0 mL) at room temperature, and then 4-bromo-1,1-dimethoxybutane (700 mg, 3.55 mmol) was added thereto. The reaction mixture was then heated to 100°C, stirred for 12 hours, cooled to room temperature, and diluted with water (20 mL). The resulting mixture was extracted twice with dichloromethane (30.0 mL). The organic phases were combined, dried over anhydrous sodium sulfate, filtered, and concentrated under reduced pressure. The crude product was purified by silica gel column chromatography (EtOAc/PE = 10% to 40%, 5% dichloromethane/PE) to obtain compound D58-1 (280 mg, yield: 73%).

Trifluoroacetic acid (1.5 mL) was added dropwise to a solution of compound D58-1 (280 mg, 0.52 mmol) in dichloromethane (5.0 mL) at room temperature. The resulting mixture was stirred at room temperature for 5 hours, diluted with dichloromethane (20.0 mL), and then washed twice with saturated sodium bicarbonate aqueous solution (30.0 mL). The organic phase was separated, dried over anhydrous sodium sulfate, filtered, and concentrated to dryness under reduced pressure to obtain a crude product of compound D58-2 (150 mg, yield: 60%).

Compound D58-2 (50 mg, 0.10 mmol), compound C2 (55 mg, 0.10 mmol), and potassium acetate (30 mg, 0.30 mmol) were sequentially added to a mixed solvent of dichloromethane (5.0 mL) and methanol (5.0 mL) at room temperature. The resulting mixture was stirred at room temperature for 30 minutes, then added with sodium triacetoxyborohydride (42 mg, 0.20 mmol), stirred for another 18 hours, and diluted with water (10.0 mL). The organic phase was separated, and the aqueous phase was extracted twice with dichloromethane (20.0 mL). The organic phases were combined, dried over anhydrous sodium sulfate, filtered, and concentrated under reduced pressure. The crude product was purified by silica gel column chromatography (dichloromethane/methanol = 10/1) and purified by prep-HPLC to obtain compound D58 (12.5 mg, yield: 15%, purity: 96%) as a pale yellow solid.

MS calculated for 802.3; found 803.2 [M+H]⁺.

### Example 59: Synthesis of 3-(5-(4-(4-(4-((6-hydroxy-2-(4-(methylsulfonyl)phenyl)naphthalen-1-yl)oxy)phenoxy)butyl)piperazin-1-yl)-1-oxoisoindolin-2-yl)piperidine-2,6-dione (compound D59)

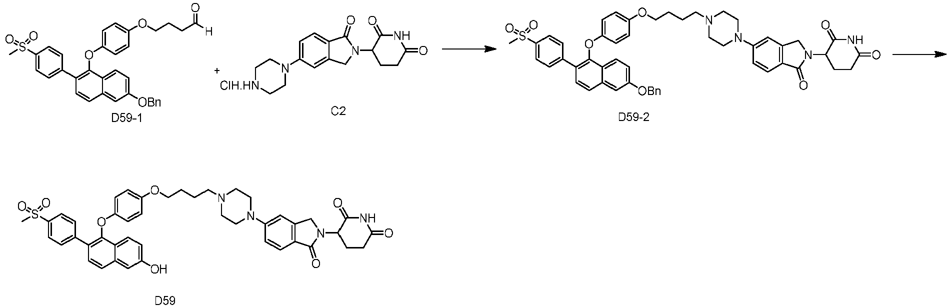

4-(4-((6-(Benzyloxy)-2-(4-(methylsulfonyl)phenyl)naphthalen-1-yl)oxy)phenoxy)butanal (compound D59-1) was synthesized using a preparation method similar to that of compound A2-2 in Example 2, wherein the reactants were compound A2 and 4-bromobutyraldehyde dimethylacetal.

Compound D59-1 (500 mg, 0.88 mmol, 1 eq) and compound C2 (164 mg, 0.44 mmol, 0.5 eq) were placed in dichloromethane (10 mL), and potassium acetate (44.2 mg, 0.44 mmol, 0.5 eq) was added thereto, and the reaction mixture was stirred for 30 minutes. Sodium triacetoxyborohydride (195 mg, 0.88 mmol, 1 eq) was added thereto, and the reaction mixture was stirred at room temperature overnight. Water (30 mL) was added thereto, and the reaction mixture was stirred for 5 minutes, and extracted with dichloromethane (15 mL × 2). The organic phases were combined, dried, filtered, concentrated, and the crude product was purified by flash column chromatography (100% DCM) to obtain 3-(5-(4-(4-(4-((6-(benzyloxy)-2-(4-(methylsulfonyl)phenyl)naphthalen-1-yl)oxy)phenoxy)butyl)piperazin-1-yl)-1-oxoisoindolin-2-yl)piperidine-2,6-dione (compound D59-2) (300 mg, yield: 40%).

Compound D59-2 (300 mg, 0.34 mmol,1 eq) was placed in tetrahydrofuran (15 mL), and 100 mg of wet palladium on carbon (10 wt% Pd, 50 wt% water) was added thereto. The reaction mixture was hydrogenated for 10 hours, filtered, and the filtrate was concentrated to obtain a crude product, which was purified by prep-HPLC to obtain compound D59 (3 mg, yield: 1.2%) as a white solid.

MS calculated for 788.3; found 789.2 [M+H]⁺.

### Example 60: Synthesis of 2-(2,6-dioxopiperidin-3-yl)-4-((5-((2-(4-((6-hydroxy-2-(4-(methylsulfonyl)phenyl)naphthalen-1-yl)oxy)phenoxy)ethyl)amino)pentyl)amino)isoindoline-1,3-dione (compound D60)

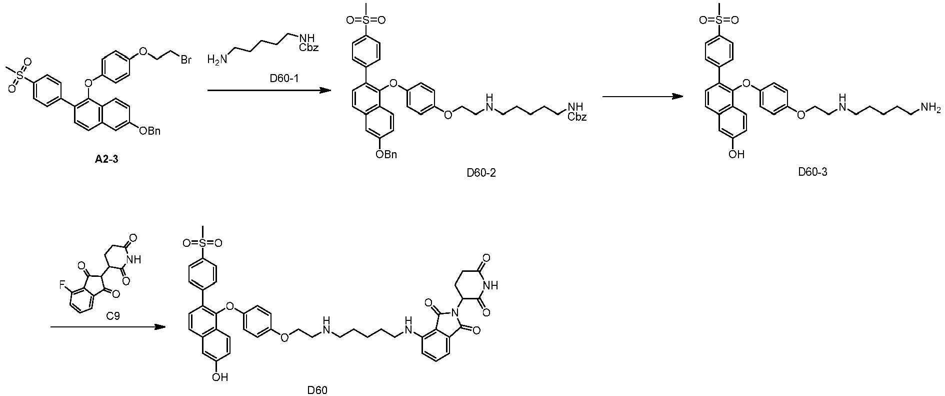

Compound D60-1 (587 mg, 2.48 mmol, 3.0 eq) and tetrahydrofuran (4 mL) were added to compound A2-3 (500 mg, 0.83 mmol, 1.0 eq) and triethylamine (251 mg, 2.48 mmol, 3.0 eq) in DMF (4 mL), and the reaction mixture was stirred at 80°C for 16 hours. The reaction mixture was concentrated with an oil pump to obtain a crude product, which was purified by column chromatography (MeOH/DCM = 0% to 10%) to obtain compound D60-2 (300 mg, yield: 47.8%) as a yellow solid.

Compound D60-2 (300 mg, 0.39 mmol, 1.0 eq) was added to methanol (5 mL), and palladium on carbon (100 mg, containing 10 wt% palladium and 50 wt% water) was added thereto under nitrogen atmosphere. The reaction mixture was replaced with hydrogen, and stirred at room temperature for 16 hours. The reaction mixture was filtered through diatomite, and the filtrate was concentrated to obtain compound D60-3 (200 mg, yield: 71.1%) as a yellow solid.

Compound D60-3 (50 mg, 0.094 mmol, 1.0 eq), compound C9 (25.8 mg, 0.094 mmol, 1.0 eq), and N,N-diisopropylethylamine (36.3 mg, 0.28 mmol, 3.0 eq) were dissolved in dimethyl sulfoxide (1 mL), and the reaction mixture was stirred at 120°C for 10 hours under nitrogen atmosphere. The reaction mixture was purified by C18 column chromatography (ACN/H₂O = 5% to 85%, 0.4 mmol/L HCl aqueous solution) to obtain compound D60 (5 mg, yield: 6.3%, purity: 90.15%) as a yellow solid.

MS calculated for 789.27; found 790.2 [M+H]⁺.

### Example 61: Synthesis of 3-(6-(4-(2-((2R,5S)-4-(2-(4-((6-hydroxy-2-(4-(methylsulfonyl)phenyl)naphthalen-1-yl)oxy)phenoxy)ethyl)-2,5-dimethylpiperazin-1-yl)ethyl)piperazin-1-yl)-1-oxoisoindolin-2-yl)piperidine-2,6-dione (compound D61)

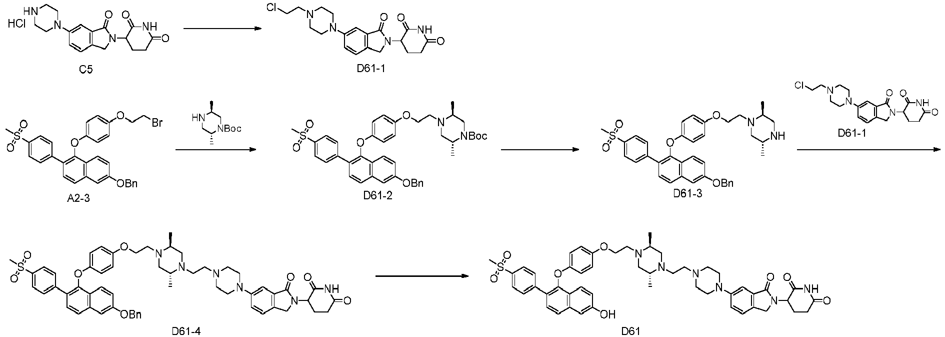

Hydrochloride of compound C5 (500 mg, 1 eq) was dissolved in methanol (10 mL), and chloroacetaldehyde (534 mg, 2 eq), KOAc (403 mg, 3 eq), and HOAc (12.33 mg, 0.15 eq) were sequentially added thereto. The reaction mixture was stirred at room temperature for 1 hour, added with 2-methylpyridine borane complex (293 mg, 2 eq), stirred at room temperature for 18 hours, and LCMS showed that the reaction was completed. The reaction mixture was poured into water (15 mL), and extracted three times with EA (15 mL). The organic phases were combined, washed with saturated brine, dried, and subjected to column chromatography (DCM: MeOH = 1: 1, UV) to obtain compound D61-1 (360 mg, yield: 67.4%) as a white solid.

Compound A2-3 (1 g, 1 eq) was dissolved in DMF (10 mL), and tert-butyl (2R,5S)-2,5-dimethylpiperazine-1-carboxylate (426 mg, 1.2 eq), K₂CO₃ (687 mg, 2 eq), and KI (82.7 mg, 0.3 eq) were sequentially added thereto at room temperature. The reaction mixture was stirred at 80°C for 18 hours. LCMS showed that the reaction was completed. The reaction mixture was poured into water (15 mL), and extracted three times with EA (15 mL). The organic phases were combined, washed with saturated brine, dried, and subjected to column chromatography (PE: EA = 1:1, UV) to obtain compound D61-2 (1.0 g, yield > 99.0%) as a white solid.

Compound D61-2 (800 mg, 1 eq) was dissolved in dioxane (10 mL), and a solution of HCl in ethyl acetate (4 M, 10 mL) was added thereto. The reaction mixture was stirred at room temperature for 2 hours, and evaporated to dryness by rotary evaporation to obtain compound D61-3 (1.0 g, yield > 99.0%) as a white solid.

Compound D61-3 (100 mg, 1 eq) was dissolved in DMF (1 mL), and compound D61-1 (92 mg, 1.5 eq), DIPEA (61 mg, 3 eq), and KI (7.8 mg, 0.3 eq) were sequentially added thereto at room temperature. The reaction mixture was heated to 80°C and stirred for 18 hours. LCMS showed that the reaction was completed. The reaction mixture was poured into water, and extracted three times with EA. The organic phases were combined, washed with saturated brine, dried, and subjected to column chromatography (DCM: MeOH = 1: 1, UV) to obtain compound D61-4 (50 mg, yield: 32.3%) as a white solid.

Compound D61-4 (50 mg, 1 eq) was dissolved in THF (1 mL) and MeOH (1 mL), and Pd/C (wet, 10%, 10 mg) was added thereto. The reaction mixture was stirred at 50°C for 18 hours under hydrogen balloon atmosphere, and LCMS showed that the reaction was completed. The reaction mixture was filtered and evaporated to dryness by rotary evaporation. The crude product was purified by prep-HPLC to obtain compound D61 (3.8 mg, yield: 8.3%) as a white solid.

MS calculated for 900.39; found 451.4 [M/2+H]⁺.

### Example 62: Synthesis of 2-(2,6-dioxopiperidin-3-yl)-5-((3-ethyl-1-(4-((6-hydroxy-2-(4-(methylsulfonyl)phenyl)naphthalen-1-yl)oxy)phenoxy)-6,9,12-trioxa-3-azatetradecan-14-yl)amino)isoindoline-1,3-dione (compound D62)

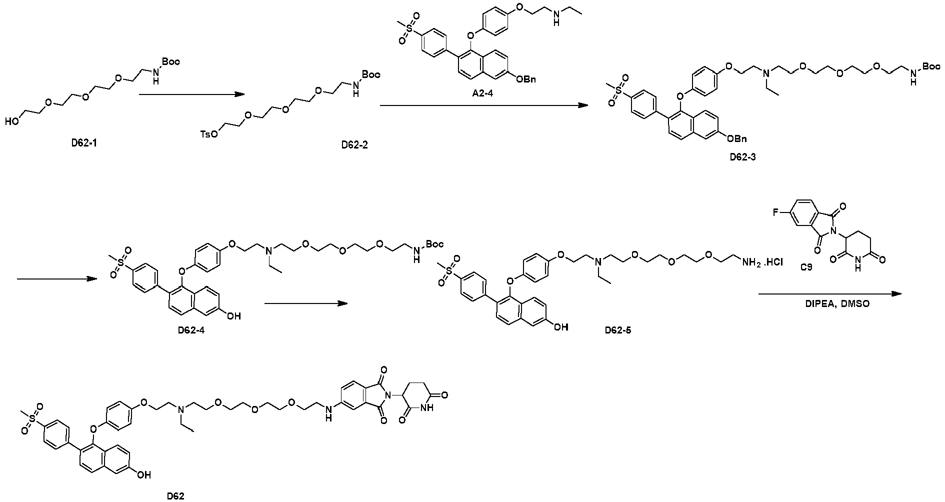

p-Toluenesulfonyl chloride (0.78 g, 4.09 mmol, 1.2 eq) was added to a solution of compound D62-1 (1 g, 3.41 mmol, 1.0 eq), DMAP (42 mg, 0.34 mmol, 0.1 eq), and triethylamine (1.03 g, 10.23 mmol, 3.0 eq) in dichloromethane (80 mL), and the reaction mixture was stirred at room temperature for 16 hours. The reaction mixture was washed with water (100 mL × 3), washed with saturated brine (100 mL), dried over anhydrous sodium sulfate, and concentrated to obtain compound D62-2 (1.2 g, yield: 78.4%) as a yellow oil.

Compound A2-4 (1.0 g, 1.76 mmol, 1.0 eq), compound D62-2 (1.18 g, 2.64 mmol, 1.5 eq), and triethylamine (534 mg, 5.28 mmol, 3.0 eq) were dissolved in DMF (5 mL), and the reaction mixture was stirred at 90°C for 16 hours. The reaction mixture was concentrated to obtain a crude product, which was purified by column chromatography (MeOH/DCM = 0% to 10%, MeOH with 5% NH₃·H₂O) to obtain compound D62-3 (600 mg, yield: 40.5%) as a yellow oil.

Compound D62-3 (600 mg, 0.71 mmol, 1.0 eq) was dissolved in methanol (5 mL) and tetrahydrofuran (5 mL), and palladium on carbon (100 mg, containing 10 wt% Pd and 50 wt% water) was added thereto under nitrogen atmosphere. The reaction mixture was replaced with hydrogen three times, and stirred at room temperature for 16 hours. The reaction mixture was filtered, and the filtrate was concentrated to obtain compound D62-4 (600 mg, yield: 100%) as a yellow oil.

Compound D62-4 (600 mg, 0.71 mmol, 1.0 eq) was dissolved in dioxane (5 mL), and a solution of hydrochloric acid in dioxane (2.0 mL, 4 M) was slowly added thereto at 0°C, and the reaction mixture was stirred at room temperature for 16 hours. The reaction mixture was concentrated to obtain compound D62-5 (500 mg, yield: 100%) as a white solid.

Compound D62-5 (300 mg, 0.44 mmol, 1.0 eq) and N,N-diisopropylethylamine (168 mg, 1.31 mmol, 3.0 eq) were dissolved in dimethyl sulfoxide (5 mL), and compound C9 (144 mg, 0.52 mmol, 1.0 eq) was added thereto, and the reaction mixture was stirred at 120°C for 10 hours under nitrogen atmosphere. The reaction mixture was purified by a silica gel plate (MeOH/DCM = 0% to 9%), and then purified by prep-HPLC (ACN/water = 5% to 70%) to obtain compound D62 (5 mg, purity: 93%, yield: 1.3%) as a green solid.

MS calculated for 908.3; found 909.3 [M+H]⁺.

### Example 63: Synthesis of 2-(2,6-dioxopiperidin-3-yl)-5-(9-(ethyl(2-(4-((6-hydroxy-2-(4-(methylsulfonyl)phenyl)naphthalen-1-yl)oxy)phenoxy)ethyl)amino)nonyl)isoindoline-1,3-dione (compound D63)

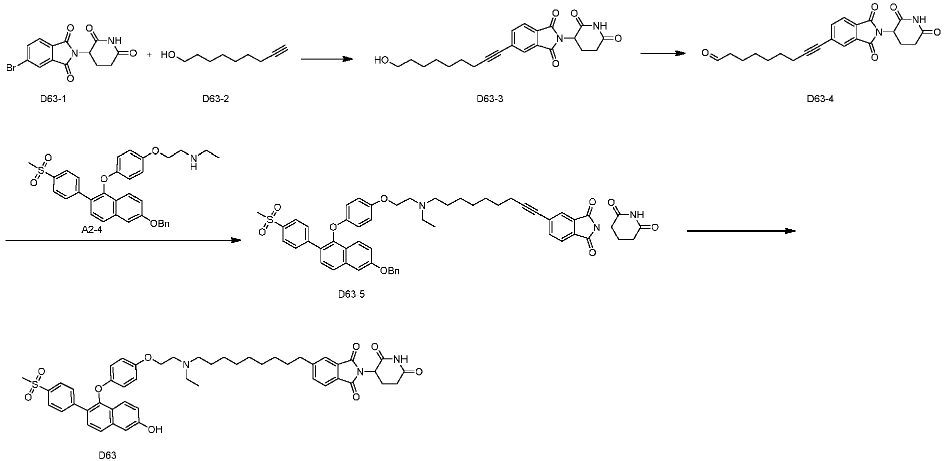

5-Bromo-2-(2,6-dioxopiperidin-3-yl)isoindoline-1,3-dione (compound D63-1) (1 g, 2.97 mmol, 1 eq) and 9-hydroxy-non-1-yne (compound D63-2) were placed in DMF (10 mL), and tetrakis(triphenylphosphine)palladium (104 mg, 0.15 mmol, 0.05 eq), copper iodide (28 mg, 0.15 mmol, 0.05 eq), and diisopropylethylamine (764 mg, 5.92 mmol, 2 eq) were added thereto. The reaction mixture was replaced with nitrogen, reacted at 90°C for 8 hours, concentrated, and purified to obtain compound D63-3 (600 mg, purity: 95%, yield: 51%).

Compound D63-3 (600 mg, 1.5 mmol, 1 eq) was placed in dichloromethane (20 mL), and Dess-Martin periodinane (637 mg, 1.5 mmol, 1 eq) was added thereto. The reaction mixture was reacted at room temperature for 3 hours, washed with saturated sodium carbonate aqueous solution, and the phases were separated. The organic phase was dried and then concentrated to obtain compound D63-4 (500 mg, crude product, purity: 40%, yield: 83%).

Compound D63-4 (500 mg, 1.27 mmol, 1 eq) and compound A2-4 (361 mg, 1.27 mmol, 1 eq) were placed in dichloromethane (20 mL), and sodium triacetoxyborohydride (402 mg, 1.9 mmol, 1.5 eq) was added thereto. The reaction mixture was reacted at room temperature for 3 hours, concentrated, and the crude product was purified by column chromatography to obtain compound D63-5 (100 mg, purity: 91%, yield: 8.3%).

Compound D63-5 (100 mg, 0.11 mmol, 1 eq) was placed in THF (10 mL), and wet palladium on carbon (100 mg) was added thereto. The reaction mixture was reacted for 10 hours, filtered, and the filtrate was concentrated to obtain a product, which was purified by prep-HPLC to obtain compound D63 (1.1 mg, purity: 95%, yield: 1.2%) as a white solid.

MS calculated for 859.5; found 860.4 [M+H]⁺.

### Example 64: Synthesis of 4-(1-(4-(2-(4-(2-(2,6-dioxopiperidin-3-yl)-1-oxoisoindolin-5-yl)piperazin-1-yl)ethoxy)phenoxy)-6-hydroxynaphthalen-2-yl)-N-methylbenzamide (compound D64)

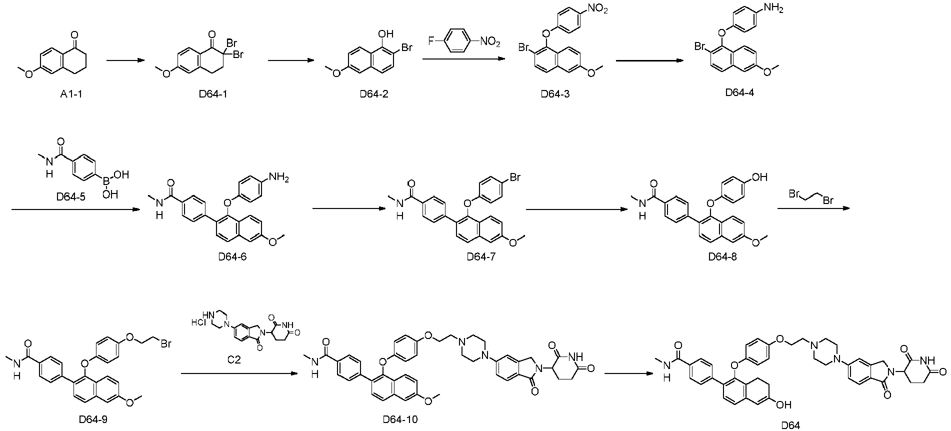

A solution of Br₂ (27.3 g, 2.01 eq.) in CHCl₃ (150 mL) was added dropwise to a solution of compound A1-1 (15 g, 85.1 mmol, 1.0 eq.) in CHCl₃ (400 mL) at room temperature of 20°C (about 2.5 hours). The internal temperature was maintained at 20 to 25°C. After the dropwise addition was completed, the mixture was stirred at 25°C for 1 hour. LCMS showed that the starting material was consumed and > 90% of the product was generated (235 nm). The reaction mixture was concentrated to obtain a crude product of compound D64-1 (28.9 g, yield: 100%, purity: 86%) as a yellow oil.

MS calculated for 331.9; found 333.0 [M+H]⁺.

DBU (19 mL, 19.4 g, 1.5 eq.) was added dropwise to a solution of compound D64-1 (28.9 g, 85.1 mmol, 1.0 eq.) in MeCN (400 mL) at 0°C. After the dropwise addition was completed, the mixture was slowly warmed to room temperature (20°C) and stirred for 1.5 hours. LCMS showed that the main peak was the product. The reaction mixture was concentrated, diluted with DCM (400 mL), and washed with 1 N HCl (336 mL) and saturated brine (100 mL). The organic phase was concentrated and purified by flash column chromatography (80 g, EtOAc/PE = 0 to 10%) to obtain compound D64-2 (20.48 g, yield: 95%, purity: 95%) as a yellow oil.

MS calculated for 252.0; found 253.0 [M+H]⁺.

1H NMR (400 MHz, DMSO-*d*₆) δ 9.78 (s, 1H), 8.12 (d, *J* = 9.2 Hz, 1H), 7.48 (d, *J* = 8.8 Hz, 1H),7.32 - 7.24 (m, 2H), 7.16 (dd, *J* = 9.2, 2.6 Hz, 1H), 3.86 (s, 3H).

Compound D64-2 (5 g, 19.76 mmol, 1.0 eq.), 4-fluoronitrobenzene (3.34 g, 1.2 eq.), and Cs₂CO₃ (12.8 g, 2.0 eq.) in DMF (50 mL) was heated to 100°C and stirred for 3 hours. LCMS showed that the reaction was completed. The reaction mixture was cooled and concentrated. The reaction mixture was diluted with water (50 mL), extracted with EtOAc (100 mL × 2), and washed with saturated brine (50 mL). The organic phase was concentrated and purified by flash column chromatography (80 g, EtOAc/PE = 1% to 20%) to obtain compound D64-3 (6.03 g, yield: 81%, purity: 98%) as a yellow solid.

Pt/C (1.4 g) was added to a solution of compound D64-3 (6.03 g, 16.1 mmol, 1.0 eq.) in EtOAc (150 mL). The mixture was stirred at 30°C for 24 hours under hydrogen balloon atmosphere. LCMS showed that the reaction was completed. The reaction mixture was filtered, washed with DCM/MeOH, and concentrated under vacuum to obtain compound D64-4 (5.46 g, yield: 98.5%, purity: 97%) as a yellow solid.

A solution of compound D64-4 (500 mg, 1.45 mmol, 1.0 eq.), compound D64-5 (390 mg, 1.5 eq.), Pd(dppf)Cl₂·DCM (118 mg, 0.1 eq.), and K₃PO₄ (923 mg, 3.0 eq.) in 1,4-dioxane/water (10 mL/1 mL) was replaced with nitrogen, heated to 100°C, and stirred for 18 hours. LCMS showed that the reaction was completed. The reaction mixture was concentrated and purified by flash column chromatography (12 g, EtOAc/PE = 10% to 100%, 20% DCM in PE) to obtain compound D64-6 (620 mg, yield: 89%, purity: 91%) as a white solid.

MS calculated for 398.1; found 399.2 [M+H]⁺.

t-BuONO (150 mg, 1.31 mmol, 1.0 eq., purity: 90%) was added dropwise to a solution of compound D64-6 (520 mg, 1.31 mmol, 1.0 eq.) and CuBr₂ (351 mg, 1.2 eq.) in MeCN (16 mL) at room temperature, and the reaction mixture was stirred for 2 hours. TLC showed that the starting material was consumed. The reaction mixture was concentrated, diluted with water, and extracted with EtOAc. The reaction mixture was filtered, concentrated and purified by flash column chromatography (12 g, EtOAc/PE = 4% to 70%) to obtain compound D64-7 (530 mg, yield: 88%, purity: 88%) as a yellow gel.

MS calculated for 461.1; found 462.1 [M+H]⁺.

A solution of compound D64-7 (530 mg, 1.15 mmol, 1.0 eq.), KOH (193 mg, 3.0 eq.), Pd₂(dba)₃ (105 mg, 0.1 eq.), and tBuXPhos (98 mg, 0.2 eq.) in 1,4-dioxane/water (10 mL/1 mL) was replaced with a gas, heated to 90°C, and stirred for 15 hours. LCMS showed that the product was generated. The reaction mixture was concentrated, diluted with DCM/water (10 mL/10 mL), added with 1 M HCl aqueous solution to adjust the pH to 7, and extracted with DCM. The organic phase was concentrated and purified by flash column chromatography (12 g, EtOAc/PE = 10% to 100%) to obtain compound D64-8 (320 mg, yield: 60%, purity: 90%) as a white solid.

MS calculated for 399.1; found 400.2 [M+H]⁺.

A solution of compound D64-8 (185 mg, 0.46 mmol, 1.0 eq.), 1,2-dibromoethane (870 mg, 10 eq.), and Cs₂CO₃ (453 mg, 3.0 eq.) in MeCN (6 mL) was heated to 80°C and stirred for 15 hours. The reaction mixture was added with 1,2-dibromoethane (435 mg, 5 eq.), heated to 80°C, and stirred for another 24 hours. LCMS showed that 40% of the product was generated. The reaction mixture was concentrated, diluted with DCM and water, added with 1 M HCl to adjust the pH to 7, extracted with DCM, concentrated and purified by flash column chromatography (12 g, EtOAc/PE = 4% to 70%) to obtain compound D64-9 (95 mg, yield: 41%, purity: 88%) as a yellow gel.

A mixture of compound D64-9 (95 mg, 0.19 mmol, 1.0 eq.), compound C2 (137 mg, 0.37 mmol, 2.0 eq.), DIEA (98 mg, 4.0 eq.), and KI (32 mg, 1.0 eq.) in DMF (3 mL) was stirred at 100°C for 40 hours. The reaction mixture was added with compound C2 (35 mg, 0.5 eq.) and KI (16 mg, 0.5 eq.), heated to 110°C, and reacted for another 20 hours. LCMS showed that 33% of the product was generated. The reaction mixture was cooled, concentrated, dissolved in DCM, neutralized with solid NaHCO₃, concentrated and purified by flash column chromatography (12 g, MeOH/DCM = 0 to 6%) to obtain compound D64-10 (75 mg, yield: 52%, purity: 92%) as a yellow gel.

2 MBBr₃/DCM (0.25 mL, 5.0 eq.) was added to compound D64-10 (75 mg, 0.1 mmol, 1.0 eq.) in DCM (3 mL) at 0°C, and the reaction mixture was slowly warmed to room temperature and stirred for 1 hour. LCMS showed that most of the starting material was consumed. The reaction mixture was diluted with DCM, added with water to quench, neutralized with saturated NaHCO₃ to adjust the pH to neutrality, extracted with DCM, and dried over Na₂SO₄. The reaction mixture was filtered, concentrated and purified by flash column chromatography (12 g, MeOH/DCM = 0% to 8%), and lyophilized to obtain compound D64 (21 mg, yield: 28%, purity: 92.12%) as a white solid.

MS calculated for 739.3; found 740.3 [M+H]⁺.

1H NMR (400 MHz, DMSO-*d*₆) δ 10.91 (s, 1H), 9.97 (s, 1H), 8.39 (d, *J* = 4.8 Hz, 1H),8.13 (s, 1H), 7.80 (d, *J* = 8.2 Hz, 2H), 7.72 (t, *J* = 8.2 Hz, 2H), 7.65 (d, *J* = 8.0 Hz, 2H),7.54 (dd, *J* = 23.7, 8.5 Hz, 2H), 7.24 (d, *J* = 2.5 Hz, 1H), 7.07 (dt, *J* = 13.3, 5.6 Hz, 3H),6.75 (d, *J* = 9.0 Hz, 2H), 6.58 (d, *J* = 9.0 Hz, 2H), 5.03 (dd, *J* = 13.3, 5.1 Hz, 1H), 4.32(d, *J* = 16.9 Hz, 1H), 4.20 (d, *J* = 16.9 Hz, 1H), 3.98 (t, *J* = 5.7 Hz, 2H), 2.88 (td, J=13.6, 13.2, 6.7 Hz, 2H), 2.81 - 2.55 (m, 10H), 2.45 - 2.27 (m, 1H), 2.07 - 1.88 (m, 1H).

### Example 65: Synthesis of 3-(5-(4-(2-(4-((6-hydroxy-2-(4-(pyrrolidine-1-carbonyl)phenyl)naphthalen-1-yl)oxy)phenoxy)ethyl)piperazin-1-yl)-1-oxoisoindolin-2-yl)piperidine-2,6-dione (compound D65)

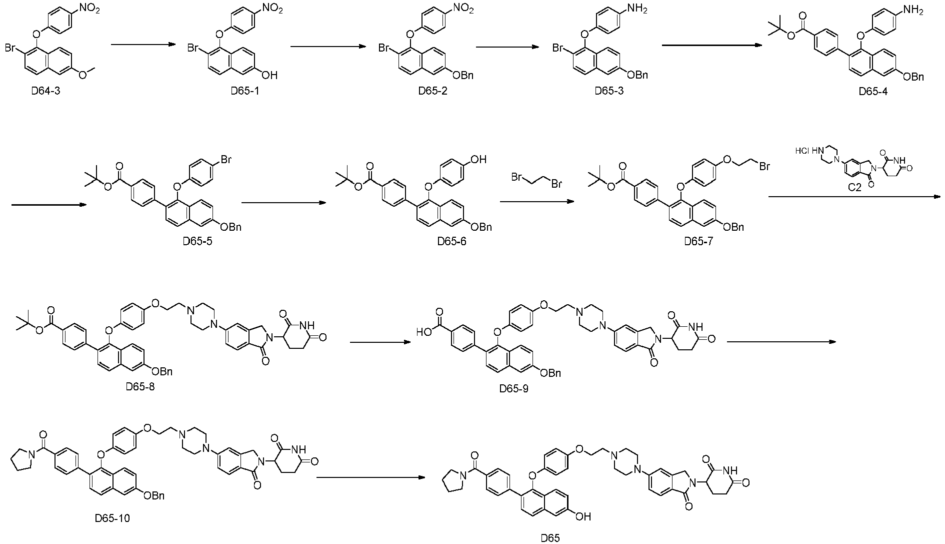

BBr₃ (24 g, 2.0 eq.) was added to a solution of compound D64-3 (18 g, 48.1 mmol, 1.0 eq.) in DCM (180 mL) at 0°C. The reaction mixture was reacted at room temperature for 2 hours. LCMS showed that the reaction was completed. The reaction mixture was added with NaHCO₃ aqueous solution at 0°C to quench and adjust the pH to 7, diluted with water (250 mL), extracted with DCM (300 mL × 2), and washed with saturated brine (250 mL). The organic phase was concentrated and purified by flash column chromatography (80 g, EtOAc/PE = 1% to 20%) to obtain compound D65-1 (16 g, yield: 92%) as a brown oil.

Compound D65-1 (16 g, 50.1 mmol, 1.0 eq.) was dissolved in DMF (150 mL), then cesium carbonate (32.6 g, 2.0 eq.) and benzyl bromide (10.3 g, 1.2 eq.) were added thereto, and the mixture was stirred at 25°C for 2 hours. LCMS showed that the reaction was completed. The reaction mixture was diluted with water (250 mL), extracted with EtOAc (300 mL × 2), and washed with saturated brine (250 mL). The organic phase was concentrated and purified by flash column chromatography (80 g, EtOAc/PE = 1% to 10%) to obtain compound D65-2 (16 g, yield: 81%) as a white solid.

A mixture of compound D65-2 (16 g, 40.1 mmol, 1.0 eq.), iron powder (11.2 g, 200 mmol, 5 eq.), and ammonium chloride (21.4 g, 401 mmol, 10.0 eq.) in ethanol (300 mL) and water (150 mL) was heated to 90°C, and stirred for 2 hours under nitrogen atmosphere. TLC showed that the starting material disappeared, and LCMS also showed that the product was generated. The reaction mixture was thermally filtered through diatomite, and the diatomite was washed repeatedly with ethanol and dichloromethane until there was no residual product in the diatomite. The reaction mixture was concentrated, diluted with water (200 mL), extracted with EA (300 mL × 2), and washed with saturated brine (200 mL). The organic phase was concentrated and precipitated by recrystallization in a mixed solvent (PE/EA= 5/1), and filtered to obtain a portion of the product. The remaining unprecipitated portion was purified by flash column chromatography (80 g, EtOAc/PE = 1% to 50%) and combined to obtain compound D65-3 (13 g, yield: 88%) as a white solid.

Compound D65-3 (12 g, 28.5 mmol, 1.0 eq.), 4-tert-butoxycarbonylbenzeneboronic acid pinacol ester (10.4 g, 34.28 mmol, 1.2 eq.), Pd(dppf)Cl₂·DCM (1.8 g, 2.28 mmol, 0.05 eq.), and K₃PO₄ (18.17 g, 85.71 mmol, 3.0 eq.) were dissolved in 1,4-dioxane/water (120 mL/15 mL) solution. The reaction mixture was replaced with nitrogen, heated to 90°C, and stirred for 16 hours. The reaction was processed with three identical batches together. LCMS showed that the reaction was completed. The reaction mixture was diluted with water (400 mL), extracted with EA (400 mL × 2), and washed with saturated brine (400 mL). The organic phase was concentrated and purified by silica gel column chromatography (PE/DCM = 50% to 70%) to obtain compound D65-4 (32 g, yield: 73%) as a white solid.

t-BuONO (3.34 g, 1.05 eq.) was added dropwise to a solution of compound D65-4 (16 g, 30.9 mmol, 1.0 eq.) and CuBr₂ (7.2 g, 1.05 eq.) in MeCN (200 mL) at 0°C, and the reaction mixture was stirred for 20 minutes. The reaction mixture was warmed to room temperature and stirred for 1 hour. The reaction was processed with two identical batches together. LCMS showed that most of the starting material was consumed. The reaction mixture was concentrated, diluted with water, extracted with EA, and washed with saturated brine (200 mL). The organic phase was concentrated and purified by silica gel column chromatography (PE/EA = 10/1) to obtain compound D65-5 (28 g, yield: 78%) as a yellow gel.

A solution of compound D65-5 (14 g, 24.1 mmol, 1.0 eq.), KOH (4.05 g, 72.3 mmol, 3.0 eq.), Pd₂(dba)₃ (2.2 g, 2.41 mmol, 0.1 eq.), and t-BuXPhos (2.0 g, 4.8 mmol, 0.2 eq.) in 1,4-dioxane/water (150 mL/15 mL) was replaced with nitrogen, heated to 90°C, and stirred for 4 hours. The reaction was processed with two identical batches together. LCMS showed that the product was generated. The reaction mixture was diluted with water (200 mL), extracted with EA (300 mL), and washed with saturated brine (200 mL). The organic phase was concentrated and purified by silica gel column chromatography (PE/DCM = 50% to 100% DCM) to obtain compound D65-6 (17 g, yield: 68%) as a brown solid.

A solution of compound D65-6 (17 g, 32.8 mmol, 1.0 eq.), 1,2-dibromoethane (185 g, 984 mmol, 30 eq.), and Cs₂CO₃ (85.6 g, 262.5 mmol, 8 eq.) in MeCN (200 mL) and DMF (100 mL) was heated to 85°C and stirred for 18 hours. LCMS showed that the product was generated. The reaction mixture was concentrated, diluted with water (300 mL), extracted with EA (400 mL), and washed with saturated brine (300 mL). The organic phase was concentrated and purified by silica gel column chromatography (PE/EA = 10/1) to obtain compound D65-7 (12 g, yield: 60%) as a yellow gel.

A solution of compound D65-7 (12 g, 19.2 mmol, 1.0 eq.), compound C2 (9.2 g, 1.2 eq.), DIEA (12.38 g, 5.0 eq.), and KI (12.7 g, 4.0 eq.) in DMF (150 mL) was stirred at 80°C overnight, and LCMS showed that the starting material disappeared and that the product was generated. The reaction mixture was cooled, diluted with DCM (300 mL), washed with water (200 mL × 2), washed with saturated brine (200 mL × 1), and dried over Na₂SO₄. The organic phase was concentrated and purified by silica gel column chromatography (DCM/MeOH = 60/1 to 20/1) to obtain compound D65-8 (12 g, yield: 71%) as a brown solid.

TFA(70 mL) was added dropwise to a solution of compound D65-8 (12 g, 13.7 mmol, 1.0 eq.) in DCM (70 mL), and the reaction mixture was stirred at room temperature for 1 hour. LCMS showed that the product was generated and that the starting material disappeared. The reaction mixture was concentrated, added with water (40 mL), and filtered to obtain a white solid. The solid was dissolved in a mixed solvent of acetonitrile and water, and lyophilized to obtain compound D65-9 (9.6 g, yield: 86%) as a white solid.

DIPEA (100 mg, 0.73 mmol, 3.0 eq.), HATU (111 mg, 0.29 mmol, 1.2 eq.), and pyrrolidine (21 mg, 0.29 mmol, 1.2 eq.) were added to a solution of compound D65-9 (200 mg, 0.25 mmol, 1.0 eq.) in DMF (5 mL), and the mixture was stirred at room temperature for 2 hours. LCMS showed that the product was generated. The reaction mixture was extracted with EA, concentrated and purified by flash column chromatography (4 g silica gel, MeOH/DCM = 0% to 10%) to obtain compound D65-10 (190 mg, yield: 91%, purity: 90% as measured by LCMS) as a white solid.

MS calculated for 869.4; found 870.3 [M+H]⁺.

Pd/C (100 mg) and Pd(OH)₂/C (100 mg) were added to a solution of compound D65-10 (190 mg) in THF (5 mL). The mixture was stirred at 40°C for 16 hours under hydrogen balloon atmosphere, and LCMS showed that the starting material was consumed. The reaction mixture was filtered, washed with DCM/MeOH, concentrated under vacuum and purified by flash column chromatography, and prepared by prep-HPLC to obtain compound D65 (96 mg, yield: 56%, purity: 100%) as a white solid. MS calculated for 779.3; found 780.3 [M+H]⁺.

1HNMR (400 MHz, DMSO) δ 10.96 (s, 1H), 10.00 (s, 1H), 7.82 - 7.44 (m, 9H), 7.27 (d, *J=* 2.0 Hz, 1H), 7.11 (dd, *J* = 9.0, 2.2 Hz, 3H), 6.80 (d, *J* = 8.4 Hz, 2H), 6.63 (d, *J* = 8.6 Hz, 2H), 5.07 (dd, *J* = 13.3, 5.0 Hz, 1H), 4.29 (dd, *J* = 48.6, 17.0 Hz, 2H), 4.08 (s, 2H), 3.47 (t, *J* = 6.5 Hz, 3H), 3.36 (s, 6H), 3.26 (s, 3H), 2.91 (dd, *J* = 21.8, 9.2 Hz, 2H), 2.61 (d, *J* = 16.6 Hz, 2H), 2.46 - 2.31 (m, 1H), 2.03 - 1.94 (m, 1H), 1.92 - 1.77 (m, 4H).

### Example 66: Synthesis of 4-((2-(4-(methylsulfonyl)phenyl)naphthalen-1-yl)oxy)phenyl 4-(2-(2,6-dioxopiperidin-3-yl)-1-oxoisoindolin-5-yl)piperazine-1-carboxylate (compound D66)

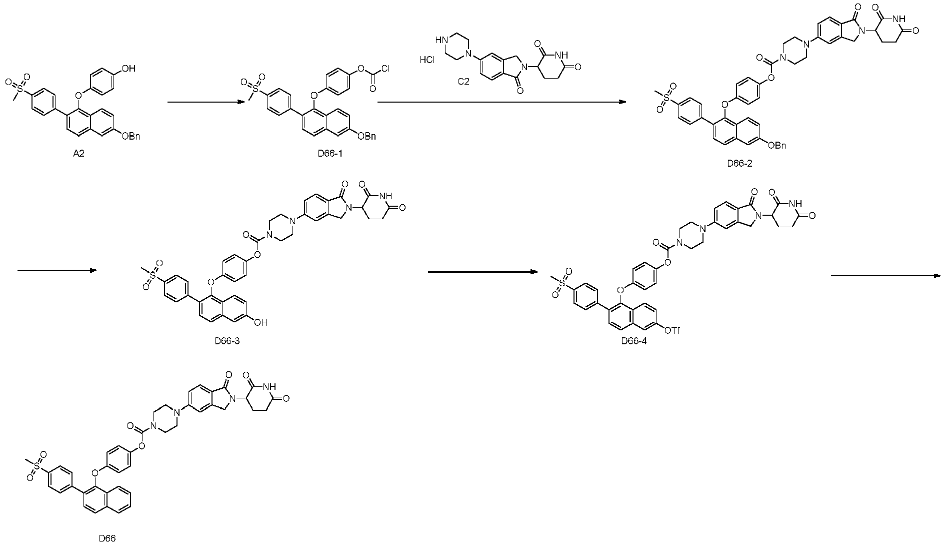

Compound A2 (15 g, 30.2 mmol) was dissolved in dichloromethane (100 mL) at 0°C, and a solution of triphosgene (6.0 g, 20.2 mmol) dissolved in dichloromethane (50 mL) was slowly added to the reaction system. The reaction mixture was stirred for 5 minutes, then added dropwise with N,N-diisopropylethylamine (5.26 mL, 30.2 mmol) and reacted at 0°C for 15 minutes, and then reacted at room temperature for 3 hours. The reaction mixture was diluted with dichloromethane (500 mL), added with ice water (100 mL) to quench, and extracted with water (200 mL × 2) to obtain a crude product of 4-((6-(benzyloxy)-2-(4-(methylsulfonyl)phenyl)naphthalen-1-yl)oxy)phenyl carbonochloridate (compound D66-1) (17.8 g, crude yield > 100%).

Compound C2 (20.58 g, 51.3 mmol) was dissolved in N,N-dimethylformamide (100 mL) at 0°C, and N,N-diisopropylethylamine (34.4 mL, 197.8 mmol) was added thereto. The reaction mixture was stirred at room temperature for 15 minutes, then cooled to 0°C, and added dropwise with a mixture of compound D66-1 (17.8 g, crude product) dissolved in dichloromethane (100 mL). The reaction system was left at room temperature overnight. The reaction mixture was directly filtered, and the filtrate was extracted with dichloromethane (200 mL) and saturated brine (300 mL × 3). The organic phase and the filter cake were concentrated together, and then purified by silica gel column chromatography (gradient elution, eluent: dichloromethane/methanol = 100% to 96%/4%) to obtain 4-((6-(benzyloxy)-2-(4-(methylsulfonyl)phenyl)naphthalen-1-yl)oxy)phenyl 4-(2-(2,6-dioxopiperidin-3-yl)-1-oxoisoindolin-5-yl)piperazine-1-carboxylate (compound D66-2) (18.28 g, 21.5 mmol, yield: 71%).

Compound D66-2 (18.28 g, 21.5 mmol) was dissolved in tetrahydrofuran (180 mL) at room temperature, palladium hydroxide on carbon (containing about 50% water) (9.1 g) was added thereto, and the reaction mixture was stirred at room temperature overnight under hydrogen (15 psi) atmosphere. The reaction mixture was filtered, and the filtrate was concentrated and purified by silica gel column chromatography (gradient elution, eluent: dichloromethane/methanol = 100% to 95%/5%) to obtain compound D66-3 (10.48 g, yield: 64%).

¹H NMR (400 MHz, DMSO-*d*₆) δ 10.96 (s, 1H), 10.11 (s, 1H), 7.94 (d, *J* = 8.6 Hz, 2H), 7.87 (d, *J* = 8.6 Hz, 2H), 7.80 (dd, *J* = 15.2, 8.9 Hz, 2H), 7.62 (d, *J* = 8.8 Hz, 1H), 7.58 (d, *J* = 8.4 Hz, 1H), 7.31 (d, *J* = 2.4 Hz, 1H), 7.16 (dd, *J* = 9.2, 2.4 Hz, 1H), 7.11 (s, 2H), 7.03-6.94 (m, 2H), 6.75 - 6.66 (m, 2H), 5.77 (s, 1H), 5.08 (dd, *J* = 13.2, 5.1 Hz, 1H), 4.36 (d, *J* = 17.2 Hz, 1H), 4.25 (d, *J=* 17.2 Hz, 1H), 4.12 (s, 1H), 3.69 (s, 2H), 3.57 (s, 3H), 3.20 (s, 4H), 2.99 - 2.85 (m, 1H), 2.66 - 2.56 (m, 1H), 2.46 - 2.31 (m, 1H), 2.04 - 1.94 (m, 1H).

MS calculated for 760.22; found 761.2 [M+H]⁺.

PhNTf₂ (1.04 g, 2.9 mmol, 1.1 eq.) and DIPEA (680 mg, 5.3 mmol, 2.0 eq.) were added to a solution of compound D66-3 (2 g, 2.6 mmol, 1.0 eq.) in DMF (15 mL), and the mixture was stirred at room temperature for 2 hours. LCMS showed that the product was generated. The reaction mixture was extracted with ethyl acetate, concentrated and purified by flash column chromatography (25 g silica gel, MeOH/DCM = 0% to 10%) to obtain compound D66-4 (2.3 g, yield: 98%, purity: 95%) as a white solid.

MS calculated for 892.2; found 893.3 [M+H]⁺.

Pd/C (470 mg) and Pd(OH)₂/C (470 mg) were added to a solution of compound D66-4 (2.3 g, 2.6 mmol, 1.0 eq) in THF/MeOH (1:1, 50 mL/50 mL). The mixture was stirred at room temperature for 2 hours under hydrogen balloon atmosphere, and LCMS showed that the starting material was consumed. The reaction mixture was filtered, washed with DCM/MeOH, concentrated under vacuum and purified by flash column chromatography, and then purified by prep-HPLC to obtain compound D66 (522 mg, yield: 28%, purity: 96%) as a white solid.

MS calculated for 744.2; found 745.3 [M+H]⁺.

¹H NMR (400 MHz, DMSO-*d*₆) δ 10.95 (s, 1H), 8.13 - 8.02 (m, 2H), 7.98 - 7.91 (m, 2H), 7.88 (d, *J* = 8.2 Hz, 3H), 7.73 (d, *J* = 8.5 Hz, 1H), 7.67 - 7.51 (m, 3H), 7.13 - 7.05 (m, 2H), 6.96 (d, *J* = 9.1 Hz, 2H), 6.69 (d, *J* = 9.1 Hz, 2H), 5.05 (dd, *J* = 13.3, 5.1 Hz, 1H), 4.33 (d, *J* = 16.9 Hz, 1H), 4.21 (d, *J* = 17.0 Hz, 1H), 3.76 - 3.45 (m, 4H), 3.40 - 3.35 (m, 4H), 3.24 (s, 3H), 2.95 - 2.84 (m, 1H), 2.62 - 2.53 (m, 1H), 2.42 - 2.32 (m, 1H), 2.01 - 1.89 (m, 1H).

### Example 67: Synthesis of 4-((2-(4-(dimethylcarbamoyl)phenyl)naphthalen-1-yl)oxy)phenyl 4-(2-(2,6-dioxopiperidin-3-yl)-1-oxoisoindolin-5-yl)piperazine-1-carboxylate (compound D67)

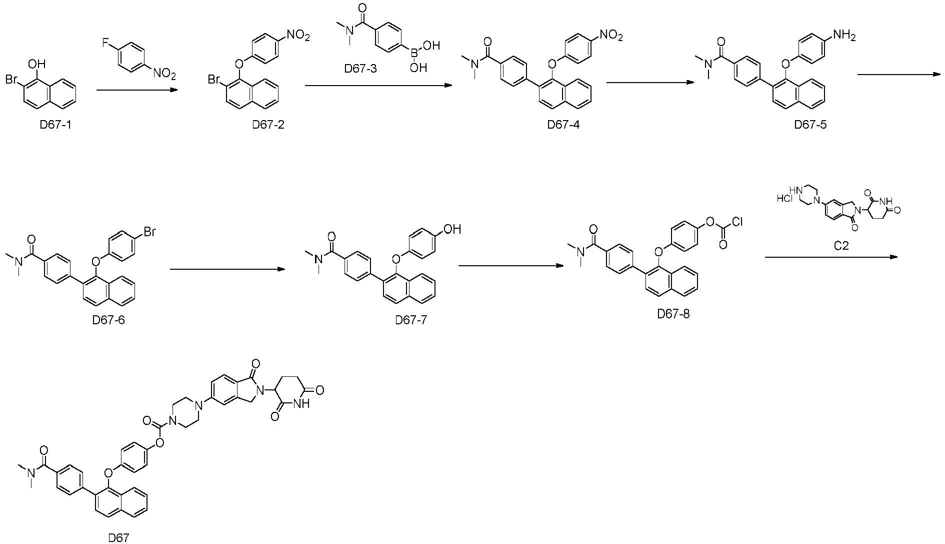

Compound D67-1 (3 g, 13.5 mmol, 1.0 eq.), 4-fluoronitrobenzene (2.28 g, 1.2 eq.), and Cs₂CO₃ (8.76 g, 2.0 eq.) in DMF (35 mL) was heated to 100°C and stirred for 19 hours. LCMS showed that most of the starting material was completely reacted. The reaction mixture was cooled, diluted with EtOAc/water, extracted with EtOAc (50 mL × 2), and washed with saturated brine (50 mL) and water (50 mL). The organic phase was concentrated and purified by flash column chromatography (40 g, EtOAc/PE = 0% to 10%) to obtain compound D67-2 (4.14 g, yield: 89%, purity: 95%) as a yellow gel.

A solution of compound D67-2 (2 g, 5.8 mmol, 1.0 eq.), compound D67-3 (1.3 g, 1.2 eq.), Pd(dppf)Cl₂·DCM (470 mg, 0.1 eq.), and K₃PO₄ (3.7 g, 3.0 eq.) in 1,4-dioxane/water (40 mL/4 mL) was replaced with nitrogen, heated to 100°C, and stirred for 18 hours. LCMS showed that the reaction was completed. The reaction mixture was concentrated and purified by flash column chromatography (20 g, EtOAc/PE = 10% to 100%, 10% DCM in PE) to obtain compound D67-4 (1.72 g, yield: 72%, purity: 99%) as a yellow solid.

MS calculated for 412.1; found 413.2 [M+H]⁺.

Pt/C (200 mg) was added to a solution of compound D67-4 (940 mg, 2.28 mmol, 1.0 eq.) in EtOAc (20 mL). The mixture was stirred at room temperature for 18 hours under hydrogen balloon atmosphere. LCMS showed that the reaction was completed. The reaction mixture was filtered, washed with MeOH, and concentrated under vacuum to obtain compound D67-5 (774 mg, yield: 89%, purity: 90%) as a yellow-red solid.

MS calculated for 382.1; found 383.2 [M+H]⁺.

t-BuONO (460 mg, 1.2 eq., purity: 90%) was added dropwise to a solution of compound D67-5 (1.28 g, 3.35 mmol, 1.0 eq.) and CuBr₂ (898 mg, 1.2 eq.) in MeCN (35 mL) at room temperature, and the reaction mixture was stirred for 1.5 hours. LCMS showed that the starting material was consumed. The reaction mixture was concentrated and purified by flash column chromatography (20 g, EtOAc/PE = 5% to 50%) to obtain compound D67-6 (1.34 g, yield: 89%, purity: 78%) as a yellow gel.

A solution of compound D67-6 (860 mg, 1.93 mmol, 1.0 eq.), KOH (324 mg, 3.0 eq.), Pd₂(dba)₃ (174 mg, 0.1 eq.), and *t*-BuXPhos (164 mg, 0.2 eq.) in 1,4-dioxane/water (20 mL/2 mL) was replaced with nitrogen, heated to 90°C, and stirred for 18 hours. LCMS showed that the product was generated. The reaction mixture was concentrated, diluted with DCM/water, added with 1 M HCl solution to adjust the pH to 7, and extracted with DCM. The organic phase was concentrated and purified by flash column chromatography (12 g, EtOAc/PE = 4% to 80%) to obtain compound D67-7 (670 mg, yield: 76%, purity: 82%) as a yellow solid.

MS calculated for 383.1; found 384.2 [M+H]⁺.

A solution of triphosgene (132 mg, 0.67 eq.) in dichloromethane (1.0 mL) was added to a solution of compound D67-7 (255 mg, 0.66 mmol, 1.0 eq.) in dichloromethane (2.0 mL) at 0°C, and the reaction mixture was stirred. The reaction mixture was added with DIEA (86 mg, 1.0 eq.) and stirred at room temperature for 1.5 hours. LCMS showed that most of the starting material was completely reacted and consumed. The reaction mixture was added with ice water to quench and diluted with dichloromethane. The reaction mixture was extracted with DCM and dried over anhydrous Na₂SO₄. The reaction mixture was filtered, and the filtrate was concentrated to obtain a solution of compound D67-8 in dichloromethane (about 3 mL, yield: 100%).

DIEA (554 mg, 6.5 eq.) was added to a solution of compound C2 (361 mg, 0.99 mmol, 1.5 eq.) in DMF (3.5 mL) at 0°C, then a solution of compound D67-8 in dichloromethane (3.0 mL) was added thereto, and the reaction mixture was stirred at room temperature for 2 hours. LCMS showed that 73% of the product was generated. The reaction mixture was diluted with DCM. The organic phases were combined and washed with brine, dried over Na₂SO₄, concentrated and purified by flash column chromatography (12 g, MeOH/DCM = 0 to 5%), then purified by prep-HPLC (0.1% FA), and lyophilized to obtain compound D67 (256 mg, yield: 53%, purity: 95.5%).

MS calculated for 737.3; found 738.3 [M+H]⁺.

1H NMR (400 MHz, DMSO-*d*₆) δ 10.92 (s, 1H), 8.07 (d, *J* = 8.0 Hz, 1H), 8.01 (d, *J* = 8.5 Hz,1H), 7.90 (d, *J* = 8.3 Hz, 1H), 7.71 (d, *J* = 8.5 Hz, 1H), 7.65 - 7.52 (m, 5H), 7.40 (d, *J* = 8.0 Hz,2H), 7.08 (d, *J* = 9.4 Hz, 2H), 6.94 (d, *J* = 9.0 Hz, 2H), 6.67 (d, *J* = 9.1 Hz, 2H), 5.04 (dd, *J* =13.3, 5.1 Hz, 1H), 4.34 (d, *J* = 16.9 Hz, 1H), 4.22 (d, *J =* 16.9 Hz, 1H), 3.78 - 3.44 (m, 4H), 3.40 - 3.34 (m, 4H), 3.01 - 2.83 (m, 7H), 2.70 - 2.54 (m, 1H), 2.44 - 2.29 (m, 1H), 2.04 - 1.89 (m,1H).

### Example 68: Synthesis of 4-(1-(4-(2-(4-(2-(2,6-dioxopiperidin-3-yl)-1-oxoisoindolin-5-yl)piperazin-1-yl)ethoxy)phenoxy)naphthalen-2-yl)-N,N-dimethylbenzamide (compound D68)

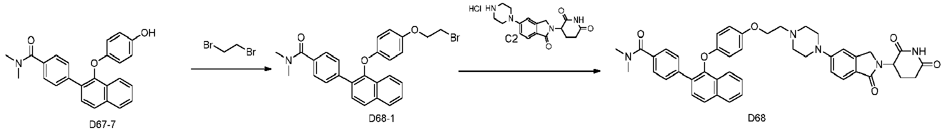

A solution of compound D67-7 (1.2 g, 3.1 mmol, 1.0 eq.), 1,2-dibromoethane (23 g, 124 mmol, 40 eq.), and Cs₂CO₃ (10 g, 31 mmol, 10 eq.) in MeCN (30 mL) and DMF (15 mL) was heated to 85°C and stirred for 12 hours. LCMS showed that the product was generated. The reaction mixture was concentrated, diluted with EtOAc and water, extracted with EtOAc, concentrated and purified by silica gel column chromatography (DCM/MeOH = 100/1 to 20/1) to obtain compound D68-1 (1.4 g, yield: 93%, purity: 82%) as a yellow solid.

MS calculated for 489.1; found 489.9 [M+H]⁺.

A solution of compound D68-1 (560 mg, 1.14 mmol, 1.0 eq.), compound C2 (500 mg, 1.37 mmol, 1.2 eq.), DIEA (590 mg, 4.56 mmol, 4.0 eq.), and KI (570 mg, 3.42 mmol, 3.0 eq.) in DMF (30 mL) was stirred at 90°C for 18 hours, and LCMS showed that the starting material disappeared and that the product was generated. The reaction mixture was cooled, diluted with DCM (100 mL) and water (100 mL), extracted with DCM (100 mL), washed with saturated sodium chloride aqueous solution, concentrated and purified by silica gel column chromatography (DCM/MeOH = 100/1 to 20/1) to obtain a yellow solid. The solid was dissolved in DMF (5 mL), added with water (10 mL) to precipitate a solid, and filtered. The solid was washed with water, dissolved in acetonitrile and water, and lyophilized to obtain compound D68 (303.3 mg, yield: 36%, purity: 94%) as a yellow solid.

MS calculated for 737.3; found 738.1 [M+H]⁺.

¹H NMR (400 MHz, DMSO) δ 10.94 (s, 1H), 8.05 (d, *J* = 7.9 Hz, 1H), 7.98 (d, *J* = 8.6 Hz, 1H), 7.88 (d, *J* = 8.3 Hz, 1H), 7.71 - 7.48 (m, 6H), 7.40 (d, *J* = 8.3 Hz, 2H), 7.08 - 7.01 (m, 2H), 6.75 (d, *J* = 9.1 Hz, 2H), 6.59 (d, *J* = 9.1 Hz, 2H), 5.04 (dd, *J* = 13.2, 5.0 Hz, 1H), 4.26 (dd, *J* = 49.3, 16.9 Hz, 2H), 3.96 (t, *J* = 5.6 Hz, 2H), 3.30 - 3.20 (m, 4H), 3.02 - 2.91 (m, 4H), 2.91 - 2.81 (m, 4H), 2.67 (t, *J* = 5.5 Hz, 2H), 2.63 - 2.54 (m, 5H), 2.36 (qd, *J* = 13.4, 4.6 Hz, 1H), 2.02 - 1.88 (m, 1H).

### Example 69: Synthesis of 4-((2-(4-(methylcarbamoyl)phenyl)naphthalen-1-yl)oxy)phenyl 4-(2-(2,6-dioxopiperidin-3-yl)-1-oxoisoindolin-5-yl)piperazine-1-carboxylate (compound D69)

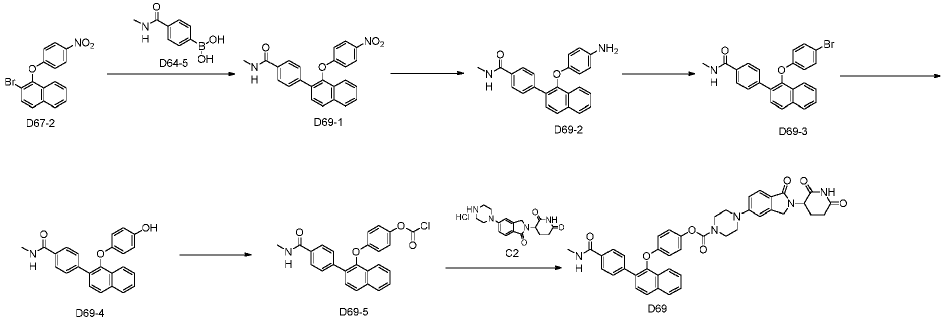

A solution of compound D67-2 (1.8 g, 5.2 mmol, 1.0 eq.), compound D64-5 (1.12 g, 1.2 eq.), Pd(dppf)Cl₂·DCM (428 mg, 0.1 eq.), and K₃PO₄ (3.3 g, 3.0 eq.) in 1,4-dioxane/water (40 mL/4 mL) was replaced with nitrogen, heated to 90°C, and stirred for 2 hours. LCMS showed that the reaction was completed. The reaction mixture was cooled to room temperature, added with water (30 mL), and extracted with DCM (50 mL × 3). The organic phase was dried over anhydrous sodium sulfate, concentrated and purified by flash column chromatography (EtOAc/PE = 20% to 80%) to obtain compound D69-1 (2 g, yield: 96%, purity: 98%) as a yellow solid.

Pd/C (500 mg) was added to a solution of compound D69-1 (1 g, 2.5 mmol, 1.0 eq.) in EtOH (25 mL), and the reaction mixture was stirred for 2 hours. LCMS showed that the reaction was completed. The reaction mixture was filtered through diatomite, washed with ethanol, concentrated, and purified by flash column chromatography (EtOAc/PE = 4% to 80%) to obtain compound D69-2 (850 mg, yield: 92%, purity: 95%) as a brown solid.

Compound D69-2 (850 mg, 2.3 mmol, 1.0 eq.) was dissolved in ACN (30 mL), and t-BuONO (285 mg, 2.7 mmol, 1.2 eq.) and CuBr₂ (618 mg, 2.7 mmol, 1.2 eq) were added thereto. The reaction mixture was stirred at room temperature for 2 hours, and LCMS showed that the reaction was completed. The reaction mixture was added with water (50 mL), extracted with EtOAc (50 mL × 3), washed with saturated brine, and the organic phase was dried over anhydrous sodium sulfate. The organic phase was filtered and concentrated, and then purified by flash column chromatography (EtOAc/PE = 5% to 100%) to obtain compound D69-3 (235 mg, yield: 60%, purity: 93%) as a white solid.

A solution of compound D69-3 (560 mg, 1.29 mmol, 1.0 eq.), KOH (270 mg, 3.8 mmol, 3.0 eq.), Pd₂(dba)₃ (118 mg, 0.13 mmol, 0.1 eq.), and t-BuXPhos (110 mg, 0.2 eq.) in 1,4-dioxane/water (20 mL/2 mL) was replaced with nitrogen, heated to 90°C, and stirred for 2 hours. LCMS showed that the reaction was completed. The reaction mixture was added with water (20 mL) and EtOAc (50 mL), and extracted. The organic phase was washed with saturated brine, dried over anhydrous sodium sulfate, concentrated and purified by flash column chromatography (EtOAc/PE = 5% to 100%) to obtain compound D69-4 (380 mg, yield: 79%, purity: 95%) as a white solid.

Compound D69-4 (230 mg, 0.62 mmol, 1.0 eq.) and triphosgene (92.7 mg, 0.311 mmol, 0.5 eq) were dissolved in THF (15 mL), and DIPEA (320 mg, 2.48 mmol, 4.0 eq) was added dropwise thereto under an ice-water bath, and the mixture was stirred for 30 minutes to obtain a reaction mixture of compound D69-5. The reaction mixture was added with compound C2 (345 mg, 0.94 mmol, 1.5 eq), and stirred for another 1 hour. LCMS showed that the reaction was completed. The reaction mixture was added with water (20 mL), and extracted with DCM (50 mL × 3). The organic phase was dried over sodium sulfate, concentrated, purified by prep-HPLC, and lyophilized to obtain compound D69 (96 mg, yield: 21%, purity: 95%) as a white solid.

MS calculated for 723.3; found 362.7 [1/2M+H]⁺.

¹H NMR (400 MHz, DMSO) δ 10.92 (s, 1H), 8.42 (d, *J* = 4.5 Hz, 1H), 8.04 (dd, *J* = 24.8, 8.4 Hz, 2H), 7.86 (dd, *J* = 17.2, 8.3 Hz, 3H), 7.71 (t, *J* = 8.4 Hz, 3H), 7.66 - 7.50 (m, 3H), 7.08 (d, *J* = 9.4 Hz, 2H), 6.95 (d, *J =* 9.0 Hz, 2H), 6.67 (d, *J* = 9.0 Hz, 2H), 5.04 (dd, *J* = 13.2, 5.0 Hz, 1H), 4.27 (dd, *J* = 48.1, 17.0 Hz, 2H), 3.60 (d, *J* = 48.9 Hz, 4H), 3.36 (s, 3H), 3.00-2.81 (m, 1H), 2.77 (d, *J* = 4.4 Hz, 4H), 2.58 (d, *J* = 16.7 Hz, 1H), 2.46 - 2.19 (m, 1H), 2.02-1.84 (m, 1H).

### Effect example 1

1. Cell line
   Breast cancer cell line and its culture medium:
   MCF-7 (ATCC; Cat. No.: HTB-22), a Luminal A breast cancer cell; culture medium EMEM.
2. Reagents
   Culture medium EMEM (Gibco, 1964507)
   Fetal bovine serum (Gibco, 10091148)
   Penicillin-streptomycin double antibody (Gibco, S110JV)
   DPBS (Gibco, 14190-144)
   CellTiter-Glo^{®} assay kit (Promega, G7573)
   Trypsin (0.25%) (Gibco, 25200-056)
   DMSO (Sigma, D2650)
3. Instruments
   Biosafety cabinet (AIRTECH, BSC-1300A II)
   Automated cell counter (Life Technologies, countess II)
   Multifunctional microplate reader (Biotek, H1FM)
   Orbital shaker (Hangzhou Allsheng Instruments Co., Ltd., OS-100)
   XI,FIT 5.3 (Inforstack Shanghai Co., Ltd.)
4. Test method
   For this test method, the following experiments were performed in a biosafety cabinet, with the specific steps as follows:
   (1) On the first day, cells were inoculated in a 96-well plate, the previous culture medium was poured off, 5 mL of DPBS was added to wash the cells, and then DPBS was pipetted and discarded. 1 mL of trypsin (0.25%) was then added. The plate was digested in a 37°C, 5% CO₂ cell incubator for about 2 to 5 minutes and then taken out. A new corresponding culture medium was added, and pipetted up and down to resuspend the cells evenly. The cells were then counted using an automated cell counter.
   (2) Each cell was inoculated in columns 1 to 12 of a 96-well plate (Corning, Cat. No.: 356690) at a density of 30,000 cells per well in 100 µL of culture medium. The plate was then placed in a 37°C, 5% CO₂ incubator for 24 hours.
   (3) On the second day, the compound of the present disclosure was 4-fold serially diluted to 10 dose points starting at 1 µM. 10 µL of the serially diluted compound was pipetted and added to 100 µL of cells in columns 1 to 10, and 10 µL of the corresponding culture medium containing 0.33 v% DMSO was added to both columns 11 and 12. The plate was incubated in a 37°C, 5% CO₂ incubator for 4 hours (Note: column 11 was used as a MIN well without compound and primary antibody but with secondary antibody; column 12 was used as a MAX well without compound but with primary antibody and secondary antibody).
   (4) The culture medium was then removed. The cells were washed once with 100 µL of DPBS, and fixed with 100 µL of 1 v% formaldehyde (CST; Cat. No.: 12606) fixative at room temperature for 30 minutes.
   (5) The fixative was removed. The cells were washed once with 100 µL of DPBS, and permeabilized with 100 µL of 80 v% pre-cooled methanol (Jiazu-chemical; Cat. No.: 67-56-1) at 4°C for 15 minutes.
   (6) Methanol was removed, and the cells were washed once with 100 µL of DPBS. The plate was added with 100 µL of primary antibody (1:5000, a volume ratio of primary antibody to BSA; manufacturer of primary antibody: Invitrogen, Cat. No.: MA5-14501), and incubated at 4°C overnight.
   (7) Primary antibody was removed, and the cells were washed three times with 150 µL of DPBS. The plate was added with 100 µL of secondary antibody (1:2500, a volume ratio of secondary antibody to BSA; manufacturer of secondary antibody: Univ, Cat. No.: abs20002), and incubated at room temperature for 1 hour.
   (8) Secondary antibody was removed, and the cells were washed three times with 150 µL of DPBS. The plate was added with 100 µL of tetramethylbenzidine (TMB) (CST; Cat. No.: 7004), and incubated at room temperature for 1 hour.
   (9) The plate was added with 100 µL of stop buffer (CST; Cat. No.: 7002), and incubated at room temperature for 10 minutes. The OD450 signal value was detected with a multifunctional microplate reader, and finally, the degradation effect of the compound of the present disclosure on ER was plotted by XLFIT software, and the DC₅₀ value of the compound of the present disclosure (drug concentration corresponding to 50% protein degradation) was calculated; Dmax = [(the highest level of ERα - the lowest level of ERα) / (the highest level of ERα)]. The results are shown in Table 1.

### Effect example 2

1. Cell line
   (1) Breast cancer cell lines and their culture media:
      MCF-7 (ATCC; Cat. No.: HTB-22), a Luminal A breast cancer cell;
      TamR-MCF-7 (ATCC; Cat. No.: CRL-3435), a breast cancer cell;
      MCF-7 ER^{D538G}: a Luminal A breast cancer cell obtained by constructing MCF-7 (ATCC; Cat. No.: HTB-22) using CRISPR-Cas9 gene editing technology;
      MCF-7 ER^{Y537S}: a Luminal A breast cancer cell obtained by constructing MCF-7 (ATCC; Cat. No.: HTB-22) using CRISPR-Cas9 gene editing technology;
      MDA-MB-231: (manufacturer: Nanjing CoBioer Biosciences Co., Ltd.), a triple-negative breast cancer cell;
      MDA-MB-468: (manufacturer: Nanjing CoBioer Biosciences Co., Ltd.), a triple-negative breast cancer cell.
      Herein, the culture medium of MCF-7, TamR-MCF-7, MCF-7 ER^{D538G}, and MCF-7 ER^{Y537S} was EMEM;
      the culture medium of MDA-MB-231 and MDA-MB-468 was DMEM.
2. Reagents
   Culture medium EMEM (Gibco, 1964507)
   Culture medium DMEM (Gibco, 12430-054)
   Fetal bovine serum (Gibco, 10091148)
   Penicillin-streptomycin double antibody (Gibco, S110JV)
   DPBS (Gibco, 14190-144)
   CellTiter-Glo^{®} assay kit (Promega, G7573)
   Trypsin (0.25%) (Gibco, 25200-056)
   DMSO (Sigma, D2650)
3. Instruments
   Biosafety cabinet (AIRTECH, BSC-1300A II)
   Automated cell counter (Life Technologies, countess II)
   Multifunctional microplate reader (Biotek, H1FM)
   Orbital shaker (Hangzhou Allsheng Instruments Co., Ltd., OS-100)
   XI,FIT 5.3 (Inforstack Shanghai Co., Ltd.)
4. Test method
   For this test method, the following experiments were performed in a biosafety cabinet, with the specific steps as follows:
   (1) Cells were inoculated in a 96-well plate. One day later, the previous culture medium was poured off, 5 mL of DPBS was added to wash the cells, and then DPBS was pipetted and discarded. An appropriate amount of trypsin was then added. The plate was digested in a 37°C, 5% CO₂ cell incubator for about 2 to 5 minutes and then taken out. A new corresponding culture medium was added, and pipetted up and down to resuspend the cells evenly. The cells were then counted using an automated cell counter.
   (2) Each cell was inoculated in columns 1 to 11 of a 96-well plate (Corning, Cat. No.: 3610) at a density of 1,000 or 2,000 cells per well in 100 µL of culture medium, and 100 µL of the corresponding culture medium without cells was added to the wells in column 12. The plate was then placed in a 37°C, 5% CO₂ incubator for 24 hours.
   (3) The compound of the present disclosure and the control drug fulvestrant (Selleck; Cat. No.: S1191) were then 4-fold serially diluted to 10 dose points starting at 10 µM. 10 µL of the serially diluted compound was pipetted and added to 100 µL of cells in columns 1 to 10, and 10 µL of the corresponding culture medium containing 0.33 v% DMSO was added to both columns 11 and 12. The plate was incubated in a 37°C, 5% CO₂ incubator for 6 days or 72 hours (Note: column 11 was used as a MAX well with cells but without compound; column 12 was used as a MIN well without cells but with compound).
   (4) The cell viability detection reagent in the CellTiter-Glo^{®} assay kit was then added to the 96-well plate at 50 µL per well according to the instructions, and shaken on an orbital shaker in the dark for 5 to 10 minutes. The cell viability was then measured using a multifunctional microplate reader. Finally, the inhibition of cell proliferation by the compound of the present disclosure was plotted by XLFIT software, and the IC₅₀ value (half inhibitory concentration) and the IH%max (maximum inhibition percentage) of the compound of the present disclosure were calculated. The results are shown in Table 1.

**Table 1: Viability results**

| Compound No. | MCF-7 | | MCF-7 (CTG) | | TamR-MCF-7 (CTG) | | MCF-7 ER^{D538G} (CTG) | | MCF-7 ER^{Y537S} (CTG) | | MDA-MB-231 (CTG) | | MDA-MB-468 (CTG) | |
|---|---|---|---|---|---|---|---|---|---|---|---|---|---|---|
| | iELISA (processing time: 4 hours, starting concentration: 1 µM) | | 6 days (starting concentration: 10 µM) | | 6 days (starting concentration : 10 µM) | | 6 days (starting concentration : 10 µM) | | 6 days (starting concentration: 10 µM) | | 6 days (starting concentration: 10 µM) | | 6 days (starting concentration: 10 µM) | |
| | DC₅₀ (nM) | Dmax % | IC₅₀ (nM) | IH% max | IC₅₀ (nM) | IH% max | IC₅₀ (nM) | IH% max | IC₅₀ (nM) | IH% max | IC₅₀ (nM) | IH% max | IC₅₀ (nM) | IH% max |
| D1 | / | | / | 30% | / | 33% | | 79% | | 73% | / | / | / | / |
| D2 | / | | 27.7 | 100% | 37.4 | 94% | 88.4 | 93% | 967.6 | 80% | 5.1 | 100% | 5.8 | 99% |
| D3 | / | | | 30% | | | | | | | | | 18.6 | 73% |
| D4 | / | | | | | 32% | 502.5 | 80% | | 63% | | | | 53% |
| D6 | / | | 133.0 | 100% | 331.0 | 90% | 427.8 | 91% | | 78% | 101.9 | 100% | 260.3 | 98% |
| D7 | / | | / | 49% | | 47% | | 40% | | 60% | | | | 63% |
| D8 | / | 43% | | 56% | | 35% | | 44% | | 33% | | | | 68% |
| D9 | / | 48% | 238.2 | 60% | | 31% | 959.9 | 65% | | 34% | | | | 60% |
| D10 | / | / | / | 40% | | 36% | | 67% | | 54% | | 38% | | 68% |
| D11 | / | 40% | / | 45% | | | 788.3 | 54% | | 52% | | | | |
| D12 | / | | / | 45% | | | 761.0 | 68% | | | | 37% | | |
| D13 | / | | / | 34% | | / | | | | | | | | |
| D14 | / | | | 42% | | | | 40% | | | | | | 32% |
| D15 | 7.6 | 60% | 486.5 | 51% | | | / | 48% | | 46% | | | | |
| D16 | / | | | 51% | | 33% | | 47% | | | | | | |
| D17 | / | / | | 58% | | 39% | | 86% | | 77% | | 87% | | |
| D18 | / | / | / | 39% | | | | 81% | | 67% | | 41% | | |
| D19 | / | 47% | / | 44% | | 39% | | 73% | | | | | | |
| D20 | / | | | 38% | | | | | | | | | | |
| D21 | 7.5 | 69% | / | 44% | | | | 55% | | | | | | |
| D22 | 4.9 | 54% | / | 38% | | 33% | | 61% | | | | 36% | | |
| D23 | / | | | 73% | | 64% | 230.0 | 94% | | 96% | | 100% | | 79% |
| D24 | / | | | 74% | | 60% | 448.0 | 93% | | 94% | | 100% | | 62% |
| D25 | / | | | 62% | | | | 86% | | 59% | | 53% | | 96% |
| D26 | / | | | 55% | | 35% | | 75% | | 43% | | | | 37% |
| D27 | / | | 222.4 | 60% | | 40% | | 69% | | | | | | |
| D28 | / | 34% | 215.5 | 53% | | 42% | 567.6 | 81% | | 49% | | 68% | | |
| D29 | / | | | 47% | | | | 47% | | | | | | |
| D30 | / | 35% | 540.7 | 55% | | 30% | | 65% | | | | | | |
| D31 | / | 35% | | 32% | | | | 90% | | 73% | | 56% | | 100% |
| D32 | / | | | 39% | | | | 38% | | 33% | | | | |
| D33 | / | | | 45% | | 45% | 287.7 | 92% | | 76% | | 57% | | 94% |
| D34 | / | 45% | 69.8 | 52% | | 41% | 181.6 | 82% | | 60% | | 37% | | |
| D35 | / | 32% | | 45% | | | | 39% | | | | | | |
| D36 | / | | | | | | | 62% | | 51% | | | | 37% |
| D37 | / | | | 49% | | | | 52% | | | | | | |
| D38 | / | | / | / | | | | 54% | | 47% | | | | |
| D39 | / | / | / | 35% | | 32% | 157.8 | 73% | | 48% | | | | 51% |
| D40 | / | | / | 34% | | 31% | | 72% | | 61% | | | | 71% |
| D41 | / | | / | | | | | 65% | | 50% | | | | 31% |
| D42 | / | / | / | 37% | | | 282.6 | 77% | | 46% | | | | 39% |
| D43 | / | | / | 36% | | | | 36% | | | | | | |
| D44 | / | | / | 33% | / | | | 52% | | | | | | |
| D45 | / | | / | 35% | | 33% | 198.4 | 75% | | 44% | | | | |
| D46 | / | | / | 34% | | | | 46% | | | | / | / | / |
| D47 | / | | / | 31% | | | 830.3 | 89% | | 73% | | 83% | | 94% |
| D48 | / | / | / | 32% | | | | 66% | | 41% | | | | |
| D49 | / | / | / | 42% | | 42% | 201.7 | 96% | | 86% | | 97% | | 99% |
| D50 | | | / | 48.40 % | / | | | | | | | | | |
| D51 | | | 143.2 | 52.90 % | / | | | | | | | | | |
| D52 | | | | 52.60 % | | 39.40 % | | | | | | | | |
| D53 | | | | 49.70 % | | | | | | | | | | 99.20 % |
| D54 | | | 13.2 | 96.50 % | | | | | | | | | | |
| D55 | | | | | | | | | | | | | | |
| D56 | | | | 55.60 % | | | | | | | | | | |
| D57 | / | | | | | | | 51% | | 46% | | | | / |
| D58 | / | | | 39% | | | | | | | | | | |
| D59 | 0.5 | 67% | / | 37% | | / | | | | | | | | |
| D60 | / | | 953.7 | 82% | | 50% | 977.0 | 96% | | 92% | | 96% | | 51% |
| D61 | | | | 45.60 % | | 40.20 % | | | | | | | | 32.70 % |
| D62 | | | | 45.10 % | | | | | | | | | | 98.50 % |
| D63 | | | 153.1 | 98.90 % | | | | | | | | | | |
| D64 | | | | | | | | | | | 9.8 | 79.8 % | 19.3 | 87.0% |
| D65 | | | | | | | | | | | 3.6 | 83.60 % | 29 | 83.60 % |
| D66 | | | | | | | | | | | 1.4 | 85.40 % | 2.2 | 90.60 % |
| D67 | | | | | | | | | | | 3.7 | 79.20 % | 4.4 | 83.60 % |
| D68 | | | | | | | | | | | 44.2 | 71.50 % | 35.4 | 59.30 % |
| D69 | | | | | | | | | | | 2.2 | 91.70 % | 2.7 | 92.80 % |
| Fulvestrant | | | 1.6 | 80.70 % | 115 | 71.80 % | | | | | | | D | 39.40 % |

Compounds D64 to D69 were tested for MDA-MB-231 (CTG) and MDA-MB-468 (CTG) with an incubation time of 72 hours in the incubator.

## Claims

1. A naphthalene ring-containing compound of formula **III,** a pharmaceutically acceptable salt thereof, a solvate thereof, or a solvate of the pharmaceutically acceptable salt thereof; 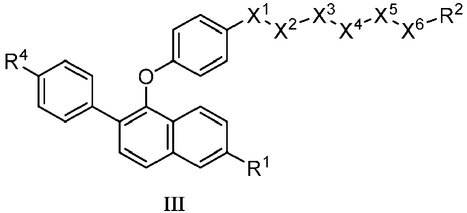 ; wherein R¹ is hydrogen, cyano, hydroxyl, or C₁-C₄ alkoxy;
X¹ is -O-, 5- to 6-membered heterocycloalkylene, or 5- to 6-membered heterocycloalkylene substituted by R^{x1-1}, in the 5- to 6-membered heterocycloalkylene and the 5- to 6-membered heterocycloalkylene substituted by R^{x1-1}, the heteroatom is one or more than one of O, S, and N, and the number of heteroatoms is 1, 2, or 3;
R^{x1-1} is independently C₁-C₄ alkyl;
X² is absent, -C(=O)-, C₁-C₆ alkylene, C₂-C₁₅ heteroalkylene, or C₂-C₁₅ heteroalkylene substituted by R^{x2-1}; in the C₂-C₁₅ heteroalkylene, the heteroatom is one or more than one of O, S, and N, and the number of heteroatoms is 1, 2, 3, 4, 5, 6, 7, 8, or 9;
R^{x2-1} is independently C₁-C₄ alkyl; X³ is absent, -C(=O)-Ph-, 5- to 6-membered heterocycloalkylene, 5- to 6-membered heteroarylene, 5- to 6-membered heterocycloalkylene substituted by R^{X3-1}, or 5- to 6-membered heteroarylene substituted by R^{x3-2}; in the 5- to 6-membered heterocycloalkylene and the 5- to 6-membered heterocycloalkylene substituted by R^{X3-1}, the heteroatom is one or more than one of O, S, and N, and the number of heteroatoms is 1, 2, or 3; in the 5- to 6-membered heteroarylene and the 5- to 6-membered heteroarylene substituted by R^{x3-2}, the heteroatom is one or more than one of O, S, and N, and the number of heteroatoms is 1, 2, or 3;
R^{x3-1} and R^{x3-2} are independently C₁-C₄ alkyl;
X⁴ is absent, C₁-C₁₀ alkylene, or C₂-C₁₅ heteroalkylene; in the C₂-C₁₅ heteroalkylene, the heteroatom is one or more than one of O, S, and N, and the number of heteroatoms is 1, 2, 3, 4, 5, 6, 7, 8, or 9;
X⁵ is absent, 5- to 6-membered heterocycloalkylene, or 5- to 6-membered heterocycloalkylene substituted by R^{x5-1}; in the 5- to 6-membered heterocycloalkylene and the 5- to 6-membered heterocycloalkylene substituted by R^{x5-1}, the heteroatom is one or more than one of O, S, and N, and the number of heteroatoms is 1, 2, or 3;
R^{x5-1} is independently C₁-C₄ alkyl;
X⁶ is absent, -O-, -NH-, -C(=O)-NH-, 5- to 6-membered heterocycloalkylene, -C(=O)-5-to 6-membered heterocycloalkylene, or 5- to 6-membered heterocycloalkylene substituted by R^{x6-1}; in the 5- to 6-membered heterocycloalkylene, the -C(=O)-5- to 6-membered heterocycloalkylene, and the 5- to 6-membered heterocycloalkylene substituted by R^{x1-1}, the heteroatom is one or more than one of O, S, and N, and the number of heteroatoms is 1, 2, or 3;
R^{x6-1} is independently C₁-C₄ alkyl;
R² is 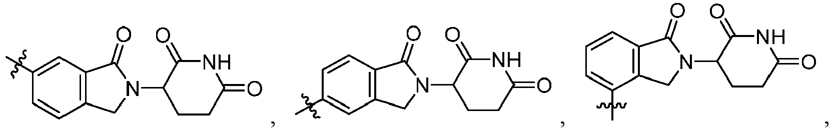 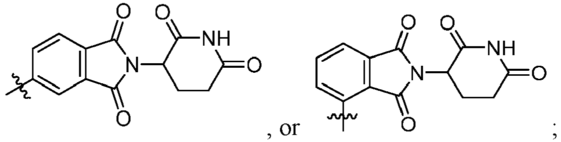
R³ is 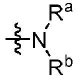 or 3- to 6-membered heterocycloalkyl, R^{a} and R^{b} are independently hydrogen or C₁-C₄ alkyl; in the 3- to 6-membered heterocycloalkyl, the heteroatom is N, and the number of heteroatoms is 1 or 2.

2. The naphthalene ring-containing compound of formula **III,** the pharmaceutically acceptable salt thereof, the solvate thereof, or the solvate of the pharmaceutically acceptable salt thereof according to claim 1, wherein the naphthalene ring-containing compound of formula **III** is a naphthalene ring-containing compound of formula **I** or **II:**

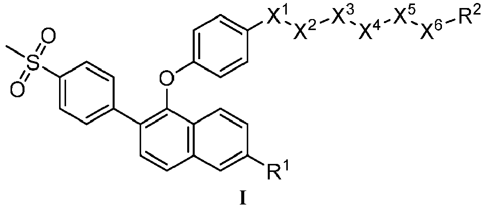

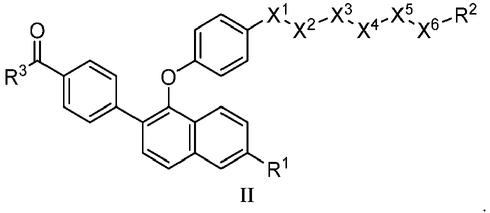

3. The naphthalene ring-containing compound of formula **III,** the pharmaceutically acceptable salt thereof, the solvate thereof, or the solvate of the pharmaceutically acceptable salt thereof according to claim 2, wherein the naphthalene ring-containing compound of formula **III** satisfies one or more than one of the following conditions:
(1) in R¹, the C₁-C₄ alkoxy is methoxy, ethoxy, n-propoxy, or isopropoxy;
(2) in X¹, in the 5- to 6-membered heterocycloalkylene and the 5- to 6-membered heterocycloalkylene substituted by R^{x1-1}, the heteroatom is N, and the number of heteroatoms is 1 or 2;
(3) in X¹, the number of R^{x1-1} is 1, 2, or 3;
(4) in R^{x1-1}, the C₁-C₄ alkyl is methyl or ethyl;
(5) in X², the C₁-C₆ alkylene is methylene, 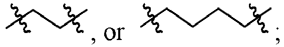
(6) in X², the C₂-C₁₅ heteroalkylene is C₄-C₁₂ heteroalkylene;
(7) in X², in the C₂-C₁₅ heteroalkylene, the heteroatom is O and/or N, and the number of heteroatoms is 1, 2, 3, 4, or 5;
(8) in X², the C₂-C₁₅ heteroalkylene is linked to the other group through the carbon atom;
(9) in X³, in the 5- to 6-membered heterocycloalkylene and the 5- to 6-membered heterocycloalkylene substituted by R^{X3-1}, the heteroatom is N, and the number of heteroatoms is 1 or 2;
(10) in X³, the number of R^{x3-1} is 1, 2, or 3;
(11) in R^{x3-1}, the C₁-C₄ alkyl is methyl or ethyl;
(12) in R^{x3-2}, the C₁-C₄ alkyl is methyl or ethyl;
(13) in X⁴, the C₁-C₁₀ alkylene is C₁-C₄ alkylene;
(14) in X⁴, the C₂-C₁₅ heteroalkylene is C₂-C₄ heteroalkylene;
(15) in X⁴, in the C₂-C₁₅ heteroalkylene, the heteroatom is O and/or N, and the number of heteroatoms is 1 or 2;
(16) in X⁵, in the 5- to 6-membered heterocycloalkylene and the 5- to 6-membered heterocycloalkylene substituted by R^{x5-1}, the heteroatom is N, and the number of heteroatoms is 1 or 2;
(17) in X⁵, the number of R^{x5-1} is 1, 2, or 3;
(18) in R^{x5-1}, the C₁-C₄ alkyl is methyl or ethyl;
(19) in X⁶, in the 5- to 6-membered heterocycloalkylene and the 5- to 6-membered heterocycloalkylene substituted by R^{x6-1}, the heteroatom is N, and the number of heteroatoms is 1 or 2;
(20) in X⁶, the number of R^{x6-1} is 1, 2, or 3;
(21) in R^{x6-1}, the C₁-C₄ alkyl is methyl or ethyl;
(22) in X⁶, in the -C(=O)-5- to 6-membered heterocycloalkylene, the heteroatom is N, and the number of heteroatoms is 1 or 2;
(23) in X⁶, in the -C(=O)-5- to 6-membered heterocycloalkylene, the 5- to 6-membered heterocycloalkylene is piperidinylene or piperazinylene;
(24) X⁶ is linked to R² through O or N;
(25) in R^{x2-1}, the C₁-C₄ alkyl is methyl or ethyl;
(26) in R^{a}, the C₁-C₄ alkyl is methyl or ethyl;
(27) in R^{b}, the C₁-C₄ alkyl is methyl or ethyl; and
(28) in R⁴, in the 3- to 6-membered heterocycloalkyl, the heteroatom is N, and the number of heteroatoms is 1.

4. The naphthalene ring-containing compound of formula **III,** the pharmaceutically acceptable salt thereof, the solvate thereof, or the solvate of the pharmaceutically acceptable salt thereof according to claim 2, wherein the naphthalene ring-containing compound of formula **III** satisfies one or more than one of the following conditions:
(1) in X¹, the 5- to 6-membered heterocycloalkylene is piperidinylene or piperazinylene;
(2) in X¹, in the 5- to 6-membered heterocycloalkylene substituted by R^{x1-1}, the 5- to 6-membered heterocycloalkylene is piperidinylene or piperazinylene; preferably, the 5- to 6-membered heterocycloalkylene substituted by R^{x1-1} is 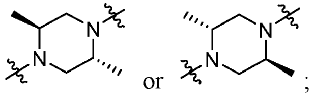
(3) in X³, the 5- to 6-membered heterocycloalkylene is piperidinylene or piperazinylene;
(4) in X³, in the 5- to 6-membered heterocycloalkylene substituted by R^{X3-1}, the 5- to 6-membered heterocycloalkylene is piperidinylene or piperazinylene;
(5) in X³, the 5- to 6-membered heteroarylene is triazolylene;
(6) in X⁴, the C₂-C₁₅ heteroalkylene is C₂-C₄ heteroalkylene in which the heteroatom is O and/or N and the number of heteroatoms is 1 or 2;
(7) in X³, in the 5- to 6-membered heteroarylene substituted by R^{x3-2}, the 5- to 6-membered heteroarylene is triazolylene;
(8) in X⁵, the 5- to 6-membered heterocycloalkylene is piperidinylene or piperazinylene;
(9) in X⁵, in the 5- to 6-membered heterocycloalkylene substituted by R^{x5-1}, the 5- to 6-membered heterocycloalkylene is piperidinylene or piperazinylene;
(10) in X⁵, the 5- to 6-membered heterocycloalkylene substituted by R^{x5-1} is 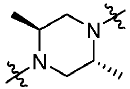 or 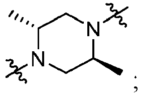
(11) in X⁶, the 5- to 6-membered heterocycloalkylene is piperidinylene or piperazinylene;
(12) in X⁶, in the 5- to 6-membered heterocycloalkylene substituted by R^{x6-1}, the 5- to 6-membered heterocycloalkylene is piperidinylene or piperazinylene;
(13) in X⁶, the 5- to 6-membered heterocycloalkylene substituted by R^{x6-1} is 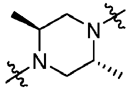 or 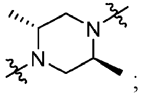
(14) in X⁶, the -C(=O)-5- to 6-membered heterocycloalkylene is 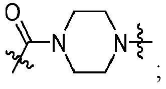 and
(15) in R³, the 3- to 6-membered heterocycloalkyl is

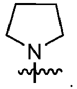

5. The naphthalene ring-containing compound of formula **III,** the pharmaceutically acceptable salt thereof, the solvate thereof, or the solvate of the pharmaceutically acceptable salt thereof according to claim 2, wherein the naphthalene ring-containing compound of formula **III** satisfies one or more than one of the following conditions:
(1) in X¹, the 5- to 6-membered heterocycloalkylene is 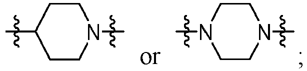
(2) in X¹, in the 5- to 6-membered heterocycloalkylene substituted by R^{x1-1}, the 5- to 6-membered heterocycloalkylene is 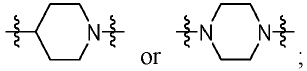
(3) in X³, the 5- to 6-membered heterocycloalkylene is 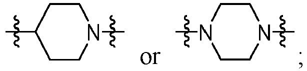
(4) in X³, in the 5- to 6-membered heterocycloalkylene substituted by R^{X3-1}, the 5- to 6-membered heterocycloalkylene is 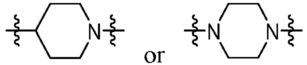 ; preferably, the 5- to 6-membered heterocycloalkylene substituted by R^{x3-1} is 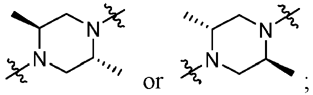
(5) in X³, the 5- to 6-membered heteroarylene is 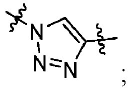
(6) in X³, in the 5- to 6-membered heteroarylene substituted by R^{x3-2}, the 5- to 6-membered heteroarylene is 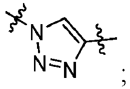
(7) in X⁴, the C₁-C₁₀ alkylene is methylene or 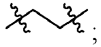
(8) in X⁴, the C₂-C₁₅ heteroalkylene is 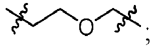
(9) in X⁵, the 5- to 6-membered heterocycloalkylene is 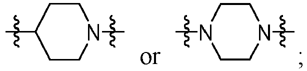
(10) in X⁵, in the 5- to 6-membered heterocycloalkylene substituted by R^{x5-1}, the 5- to 6-membered heterocycloalkylene is 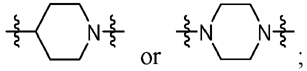
(11) in X⁶, the 5- to 6-membered heterocycloalkylene is 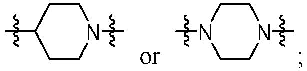
(12) in X⁶, in the 5- to 6-membered heterocycloalkylene substituted by R^{x6-1}, the 5- to 6-membered heterocycloalkylene is 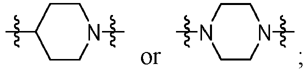
(13) in X⁶, in the -C(=O)-5- to 6-membered heterocycloalkylene, the 5- to 6-membered heterocycloalkylene is 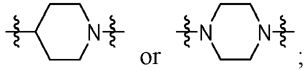
(14) in X², the C₂-C₁₅ heteroalkylene is any one of the following structures: 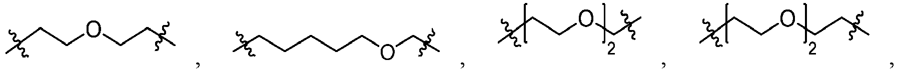 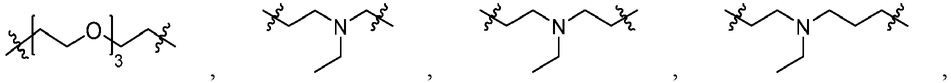 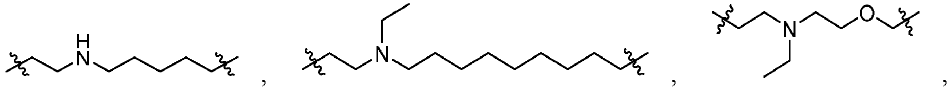 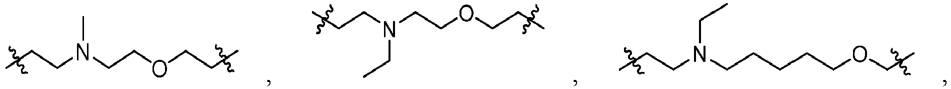 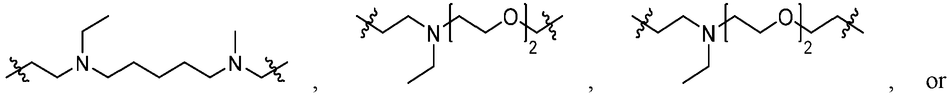 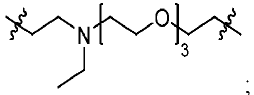
(15) the chiral carbon atom in R² is in S configuration and R configuration, for example, the ratio of S configuration to R configuration is 1: 1; and
(16) the chiral carbon atom in R¹, X¹, X², X³, X⁴, X⁵, and X⁶ is in S configuration and R configuration, for example, the ratio of S configuration to R configuration is 1: 1.

6. The naphthalene ring-containing compound of formula **III,** the pharmaceutically acceptable salt thereof, the solvate thereof, or the solvate of the pharmaceutically acceptable salt thereof according to claim 5, wherein the naphthalene ring-containing compound of formula **III** or the pharmaceutically acceptable salt thereof satisfies one or more than one of the following conditions:
(1) X² is -C(=O)- or C₁-C₆ alkylene;
(2) X⁶ is 5- to 6-membered heterocycloalkylene, -C(=O)-5- to 6-membered heterocycloalkylene, or 5- to 6-membered heterocycloalkylene substituted by R^{x6-1},
(3) R² is 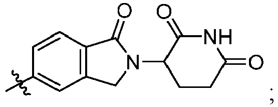
(4) X¹ is -O-; and
(5) X³, X⁴, and X⁵ are absent.

7. The naphthalene ring-containing compound of formula **III,** the pharmaceutically acceptable salt thereof, the solvate thereof, or the solvate of the pharmaceutically acceptable salt thereof according to claim 5, wherein the naphthalene ring-containing compound of formula **III** or the pharmaceutically acceptable salt thereof satisfies one or more than one of the following conditions:
(1) X¹ is -O- or 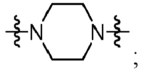
(2) X² is absent, -C(=O)-, methylene, 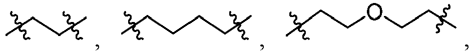 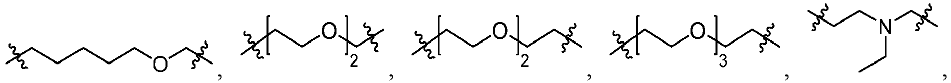 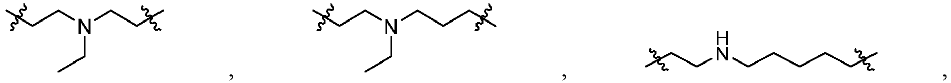 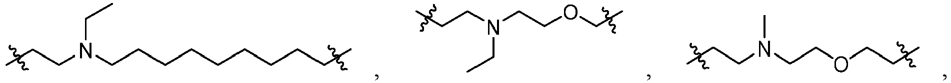 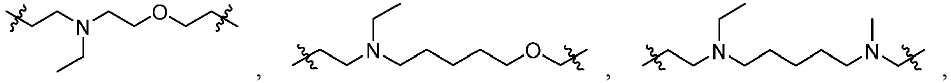 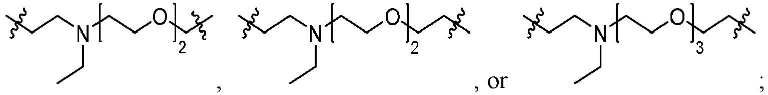
(3) X³ is absent, 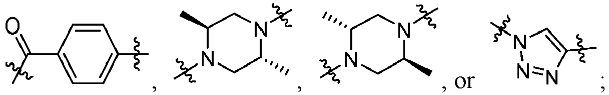
(4) X⁴ is absent, methylene, 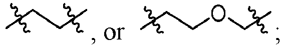
(5) X⁵ is absent or 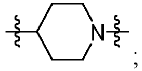
(6) X⁶ is absent, O, NH, -C(=O)-NH-, 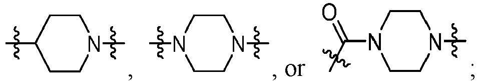
(7) R² is 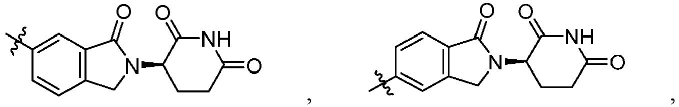 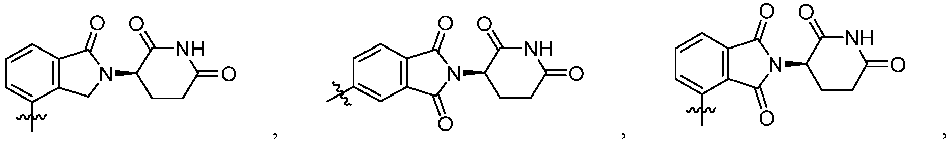 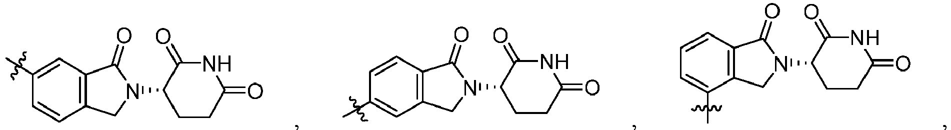 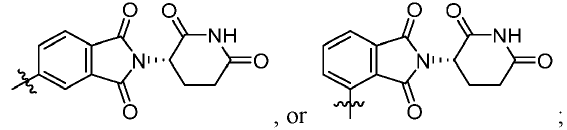
(8) the pharmaceutically acceptable salt is hydrochloride; and
(9) R³ is

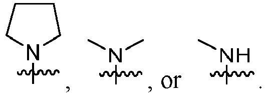

8. The naphthalene ring-containing compound of formula **III,** the pharmaceutically acceptable salt thereof, the solvate thereof, or the solvate of the pharmaceutically acceptable salt thereof according to claim 2, wherein the naphthalene ring-containing compound of formula **III** is defined as any one of the following schemes:
scheme 1: R¹ is hydroxyl, X³, X⁴, and X⁵ are absent, X¹ is -O-, X² is C₁-C₆ alkylene, X⁶ is 5- to 6-membered heterocycloalkylene, -C(=O)-5- to 6-membered heterocycloalkylene, or 5- to 6-membered heterocycloalkylene substituted by R^{x6-1}, and R² is 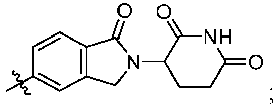
scheme 2: R¹ is hydroxyl;
X¹ is -O-;
X² is C₁-C₆ alkylene, C₂-C₁₅ heteroalkylene, or C₂-C₁₅ heteroalkylene substituted by R^{x2-1};
R^{x2-1} is independently C₁-C₄ alkyl;
X³ is absent, 5- to 6-membered heterocycloalkylene, or 5- to 6-membered heterocycloalkylene substituted by R^{X3-1}, in the 5- to 6-membered heterocycloalkylene and the 5- to 6-membered heterocycloalkylene substituted by R^{X3-1}, the heteroatom is one or more than one of O, S, and N, and the number of heteroatoms is 1, 2, or 3;
X⁴ is absent or C₂-C₁₅ heteroalkylene; in the C₂-C₁₅ heteroalkylene, the heteroatom is one or more than one of O, S, and N, and the number of heteroatoms is 1, 2, 3, 4, 5, 6, 7, 8, or 9;
X⁵ is absent;
X⁶ is 5- to 6-membered heterocycloalkylene, -C(=O)-5- to 6-membered heterocycloalkylene, or 5- to 6-membered heterocycloalkylene substituted by R^{x6-1}, in the 5- to 6-membered heterocycloalkylene, the -C(=O)-5- to 6-membered heterocycloalkylene, and the 5- to 6-membered heterocycloalkylene substituted by R^{x1-1}, the heteroatom is one or more than one of O, S, and N, and the number of heteroatoms is 1, 2, or 3;
R² is 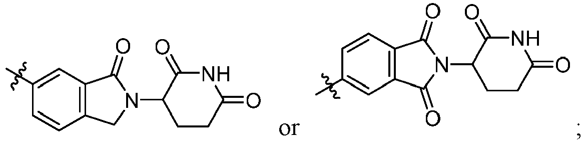
scheme 3: R¹ is hydroxyl;
X¹ is -O-;
X² is C₁-C₆ alkylene, C₂-C₁₅ heteroalkylene, or C₂-C₁₅ heteroalkylene substituted by R^{x2-1}; in the C₂-C₁₅ heteroalkylene, the heteroatom is one or more than one of O, S, and N, and the number of heteroatoms is 1, 2, 3, 4, 5, 6, 7, 8, or 9;
R^{x2-1} is independently C₁-C₄ alkyl;
X³ is absent, 5- to 6-membered heterocycloalkylene, or 5- to 6-membered heterocycloalkylene substituted by R^{X3-1}, in the 5- to 6-membered heterocycloalkylene and the 5- to 6-membered heterocycloalkylene substituted by R^{x3-1}, the heteroatom is one or more than one of O, S, and N, and the number of heteroatoms is 1, 2, or 3;
X⁴ is absent, -C(=O)-Ph-, or C₂-C₁₅ heteroalkylene; in the C₂-C₁₅ heteroalkylene, the heteroatom is one or more than one of O, S, and N, and the number of heteroatoms is 1, 2, 3, 4, 5, 6, 7, 8, or 9;
X⁵ is absent, 5- to 6-membered heterocycloalkylene, or 5- to 6-membered heterocycloalkylene substituted by R^{x5-1}; in the 5- to 6-membered heterocycloalkylene and the 5- to 6-membered heterocycloalkylene substituted by R^{x5-1}, the heteroatom is one or more than one of O, S, and N, and the number of heteroatoms is 1, 2, or 3;
X⁶ is absent, 5- to 6-membered heterocycloalkylene, -C(=O)-5- to 6-membered heterocycloalkylene, or 5- to 6-membered heterocycloalkylene substituted by R^{x6-1}, in the 5- to 6-membered heterocycloalkylene, the -C(=O)-5- to 6-membered heterocycloalkylene, and the 5- to 6-membered heterocycloalkylene substituted by R^{x1-1}, the heteroatom is one or more than one of O, S, and N, and the number of heteroatoms is 1, 2, or 3;
R² is 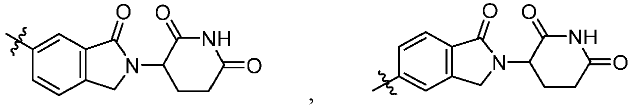 , or 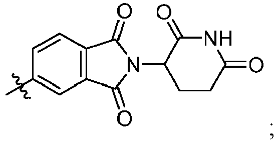
scheme 4: R¹ is hydroxyl;
X¹ is -O-;
X² is C₁-C₆ alkylene;
X³ is absent; X⁴ is absent; X⁵ is absent;
X⁶ is 5- to 6-membered heterocycloalkylene, -C(=O)-5- to 6-membered heterocycloalkylene, or 5- to 6-membered heterocycloalkylene substituted by R^{x6-1}, in the 5- to 6-membered heterocycloalkylene, the -C(=O)-5- to 6-membered heterocycloalkylene, and the 5- to 6-membered heterocycloalkylene substituted by R^{x1-1}, the heteroatom is one or more than one of O, S, and N, and the number of heteroatoms is 1, 2, or 3;
R² is 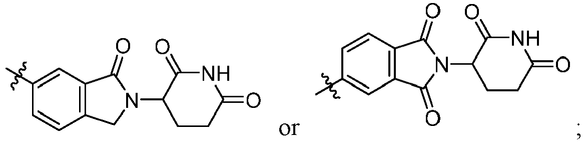
scheme 5: X¹ is -O-;
X² is C₁-C₆ alkylene, C₂-C₁₅ heteroalkylene, or C₂-C₁₅ heteroalkylene substituted by R^{x2-1}; in the C₂-C₁₅ heteroalkylene, the heteroatom is one or more than one of O, S, and N, and the number of heteroatoms is 1, 2, 3, 4, 5, 6, 7, 8, or 9;
R^{x2-1} is independently C₁-C₄ alkyl;
X³ is absent or -C(=O)-Ph-;
X⁴ is absent or C₁-C₁₀ alkylene;
X⁵ is absent, 5- to 6-membered heterocycloalkylene, or 5- to 6-membered heterocycloalkylene substituted by R^{x5-1}; in the 5- to 6-membered heterocycloalkylene and the 5- to 6-membered heterocycloalkylene substituted by R^{x5-1}, the heteroatom is one or more than one of O, S, and N, and the number of heteroatoms is 1, 2, or 3;
X⁶ is -O-, -NH-, 5- to 6-membered heterocycloalkylene, -C(=O)-5- to 6-membered heterocycloalkylene, or 5- to 6-membered heterocycloalkylene substituted by R^{x6-1}, in the 5- to 6-membered heterocycloalkylene, the -C(=O)-5- to 6-membered heterocycloalkylene, and the 5- to 6-membered heterocycloalkylene substituted by R^{x1-1}, the heteroatom is one or more than one of O, S, and N, and the number of heteroatoms is 1, 2, or 3;
R² is 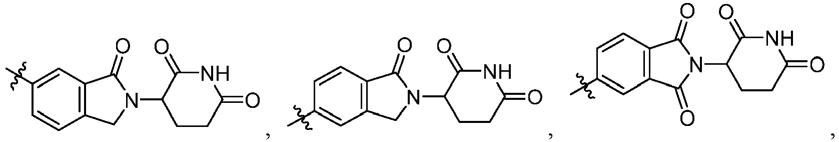 or 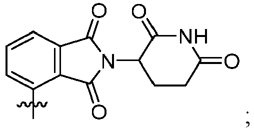
scheme 6: R¹ is hydroxyl or C₁-C₄ alkoxy;
X¹ is -O-;
X² is C₁-C₆ alkylene;
X³ is absent; X⁴ is absent; X⁵ is absent;
X⁶ is 5- to 6-membered heterocycloalkylene, -C(=O)-5- to 6-membered heterocycloalkylene, or 5- to 6-membered heterocycloalkylene substituted by R^{x6-1}, in the 5- to 6-membered heterocycloalkylene, the -C(=O)-5- to 6-membered heterocycloalkylene, and the 5- to 6-membered heterocycloalkylene substituted by R^{x1-1}, the heteroatom is one or more than one of O, S, and N, and the number of heteroatoms is 1, 2, or 3;
R² is 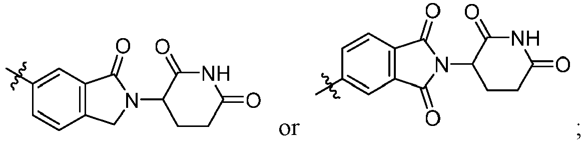
scheme 7: R¹ is hydroxyl;
X¹ is -O-;
X² is C₁-C₆ alkylene or C₂-C₁₅ heteroalkylene;
X³ is absent, 5- to 6-membered heterocycloalkylene, or 5- to 6-membered heterocycloalkylene substituted by R^{X3-1}, in the 5- to 6-membered heterocycloalkylene and the 5- to 6-membered heterocycloalkylene substituted by R^{x3-1}, the heteroatom is one or more than one of O, S, and N, and the number of heteroatoms is 1, 2, or 3;
X⁴ is absent or C₂-C₁₅ heteroalkylene; in the C₂-C₁₅ heteroalkylene, the heteroatom is one or more than one of O, S, and N, and the number of heteroatoms is 1, 2, 3, 4, 5, 6, 7, 8, or 9;
X⁵ is absent;
X⁶ is 5- to 6-membered heterocycloalkylene, -C(=O)-5- to 6-membered heterocycloalkylene, or 5- to 6-membered heterocycloalkylene substituted by R^{x6-1}, in the 5- to 6-membered heterocycloalkylene, the -C(=O)-5- to 6-membered heterocycloalkylene, and the 5- to 6-membered heterocycloalkylene substituted by R^{x1-1}, the heteroatom is one or more than one of O, S, and N, and the number of heteroatoms is 1, 2, or 3;
R² is 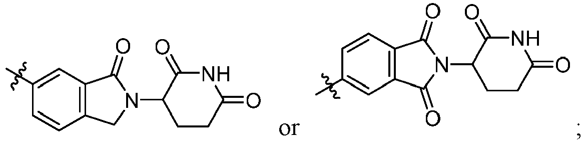
scheme 8: R¹ may be hydroxyl;
X¹ is -O-;
X² is C₁-C₆ alkylene, C₂-C₁₅ heteroalkylene, or C₂-C₁₅ heteroalkylene substituted by R^{x2-1}; in the C₂-C₁₅ heteroalkylene, the heteroatom is one or more than one of O, S, and N, and the number of heteroatoms is 1, 2, 3, 4, 5, 6, 7, 8, or 9;
R^{x2-1} is independently C₁-C₄ alkyl;
X³ is absent, 5- to 6-membered heterocycloalkylene, or 5- to 6-membered heterocycloalkylene substituted by R^{X3-1}, in the 5- to 6-membered heterocycloalkylene and the 5- to 6-membered heterocycloalkylene substituted by R^{X3-1}, the heteroatom is one or more than one of O, S, and N, and the number of heteroatoms is 1, 2, or 3;
X⁴ is absent, -C(=O)-Ph-, or C₂-C₁₅ heteroalkylene; in the C₂-C₁₅ heteroalkylene, the heteroatom is one or more than one of O, S, and N, and the number of heteroatoms is 1, 2, 3, 4, 5, 6, 7, 8, or 9;
X⁵ is absent, 5- to 6-membered heterocycloalkylene, or 5- to 6-membered heterocycloalkylene substituted by R^{x5-1}; in the 5- to 6-membered heterocycloalkylene and the 5- to 6-membered heterocycloalkylene substituted by R^{x5-1}, the heteroatom is one or more than one of O, S, and N, and the number of heteroatoms is 1, 2, or 3;
X⁶ is absent, 5- to 6-membered heterocycloalkylene, -C(=O)-5- to 6-membered heterocycloalkylene, or 5- to 6-membered heterocycloalkylene substituted by R^{x6-1}, in the 5- to 6-membered heterocycloalkylene, the -C(=O)-5- to 6-membered heterocycloalkylene, and the 5- to 6-membered heterocycloalkylene substituted by R^{x1-1}, the heteroatom is one or more than one of O, S, and N, and the number of heteroatoms is 1, 2, or 3;
R² is 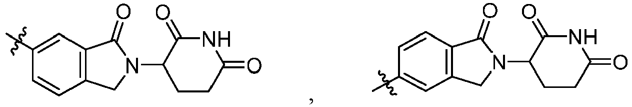 , or 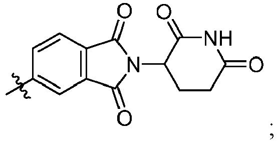
scheme 9: R¹ is cyano, hydroxyl, or C₁-C₄ alkoxy;
X¹ is -O-, 5- to 6-membered heterocycloalkylene, or 5- to 6-membered heterocycloalkylene substituted by R^{x1-1}, in the 5- to 6-membered heterocycloalkylene and the 5- to 6-membered heterocycloalkylene substituted by R^{x1-1}, the heteroatom is one or more than one of O, S, and N, and the number of heteroatoms is 1, 2, or 3;
R^{x1-1} is independently C₁-C₄ alkyl;
X² is C₁-C₆ alkylene or C₂-C₁₅ heteroalkylene; in the C₂-C₁₅ heteroalkylene, the heteroatom is one or more than one of O, S, and N, and the number of heteroatoms is 1, 2, 3, 4, 5, 6, 7, 8, or 9;
X³ is absent, -C(=O)-Ph-, 5- to 6-membered heterocycloalkylene, 5- to 6-membered heteroarylene, 5- to 6-membered heterocycloalkylene substituted by R^{x3-1}, or 5- to 6-membered heteroarylene substituted by R^{x3-2}; in the 5- to 6-membered heterocycloalkylene and the 5- to 6-membered heterocycloalkylene substituted by R^{X3-1}, the heteroatom is one or more than one of O, S, and N, and the number of heteroatoms is 1, 2, or 3; in the 5- to 6-membered heteroarylene and the 5- to 6-membered heteroarylene substituted by R^{x3-2}, the heteroatom is one or more than one of O, S, and N, and the number of heteroatoms is 1, 2, or 3;
R^{x3-1} and R^{x3-2} are independently C₁-C₄ alkyl;
X⁴ is absent, C₁-C₁₀ alkylene, or C₂-C₁₅ heteroalkylene; in the C₂-C₁₅ heteroalkylene, the heteroatom is one or more than one of O, S, and N, and the number of heteroatoms is 1, 2, 3, 4, 5, 6, 7, 8, or 9;
X⁵ is absent, 5- to 6-membered heterocycloalkylene, or 5- to 6-membered heterocycloalkylene substituted by R^{x5-1}; in the 5- to 6-membered heterocycloalkylene and the 5- to 6-membered heterocycloalkylene substituted by R^{x5-1}, the heteroatom is one or more than one of O, S, and N, and the number of heteroatoms is 1, 2, or 3;
R^{x5-1} is independently C₁-C₄ alkyl;
X⁶ is absent, -O-, -NH-, -C(=O)-NH-, 5- to 6-membered heterocycloalkylene, -C(=O)-5-to 6-membered heterocycloalkylene, or 5- to 6-membered heterocycloalkylene substituted by R^{x6-1}; in the 5- to 6-membered heterocycloalkylene, the -C(=O)-5- to 6-membered heterocycloalkylene, and the 5- to 6-membered heterocycloalkylene substituted by R^{x1-1}, the heteroatom is one or more than one of O, S, and N, and the number of heteroatoms is 1, 2, or 3;
R^{x6-1} is independently C₁-C₄ alkyl;
R² is 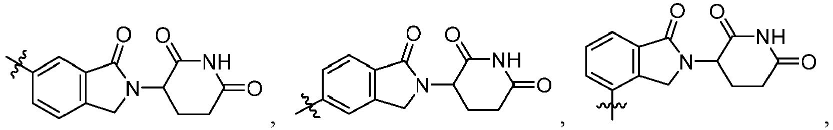 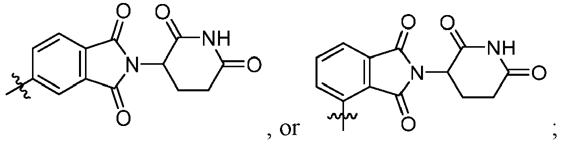
scheme 10: in the naphthalene ring-containing compound of formula **I,**
wherein R¹ is hydrogen, cyano, hydroxyl, or C₁-C₄ alkoxy;
X¹ is -O-, 5- to 6-membered heterocycloalkylene, or 5- to 6-membered heterocycloalkylene substituted by R^{x1-1}, in the 5- to 6-membered heterocycloalkylene and the 5- to 6-membered heterocycloalkylene substituted by R^{x1-1}, the heteroatom is one or more than one of O, S, and N, and the number of heteroatoms is 1, 2, or 3;
R^{x1-1} is independently C₁-C₄ alkyl;
X² is C₁-C₆ alkylene or C₂-C₁₅ heteroalkylene; in the C₂-C₁₅ heteroalkylene, the heteroatom is one or more than one of O, S, and N, and the number of heteroatoms is 1, 2, 3, 4, 5, 6, 7, 8, or 9;
X³ is absent, -C(=O)-Ph-, 5- to 6-membered heterocycloalkylene, 5- to 6-membered heteroarylene, 5- to 6-membered heterocycloalkylene substituted by R^{x3-1}, or 5- to 6-membered heteroarylene substituted by R^{x3-2}; in the 5- to 6-membered heterocycloalkylene and the 5- to 6-membered heterocycloalkylene substituted by R^{X3-1}, the heteroatom is one or more than one of O, S, and N, and the number of heteroatoms is 1, 2, or 3; in the 5- to 6-membered heteroarylene and the 5- to 6-membered heteroarylene substituted by R^{x3-2}, the heteroatom is one or more than one of O, S, and N, and the number of heteroatoms is 1, 2, or 3;
R^{x3-1} and R^{x3-2} are independently C₁-C₄ alkyl;
X⁴ is absent, C₁-C₁₀ alkylene, or C₂-C₁₅ heteroalkylene; in the C₂-C₁₅ heteroalkylene, the heteroatom is one or more than one of O, S, and N, and the number of heteroatoms is 1, 2, 3, 4, 5, 6, 7, 8, or 9;
X⁵ is absent, 5- to 6-membered heterocycloalkylene, or 5- to 6-membered heterocycloalkylene substituted by R^{x5-1}; in the 5- to 6-membered heterocycloalkylene and the 5- to 6-membered heterocycloalkylene substituted by R^{x5-1}, the heteroatom is one or more than one of O, S, and N, and the number of heteroatoms is 1, 2, or 3;
R^{x5-1} is independently C₁-C₄ alkyl;
X⁶ is absent, -O-, -NH-, -C(=O)-NH-, 5- to 6-membered heterocycloalkylene, -C(=O)-5-to 6-membered heterocycloalkylene, or 5- to 6-membered heterocycloalkylene substituted by R^{x6-1}; in the 5- to 6-membered heterocycloalkylene, the -C(=O)-5- to 6-membered heterocycloalkylene, and the 5- to 6-membered heterocycloalkylene substituted by R^{x1-1}, the heteroatom is one or more than one of O, S, and N, and the number of heteroatoms is 1, 2, or 3;
R^{x6-1} is independently C₁-C₄ alkyl;
R² is 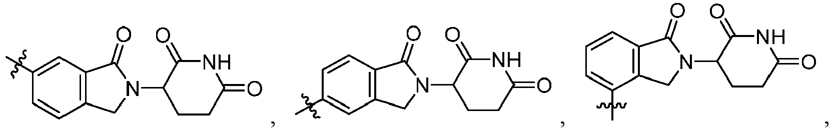 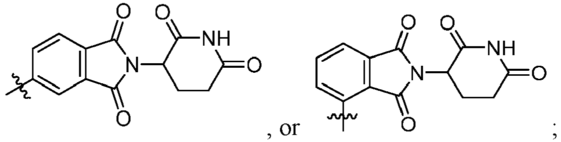
scheme 11:
in the naphthalene ring-containing compound of **II,**
R¹ is hydrogen or hydroxyl;
X¹ is -O-;
X² is -C(=O)- or C₁-C₆ alkylene;
X³ is absent;
X⁴ is absent;
X⁵ is absent;
X⁶ is 5- to 6-membered heterocycloalkylene; in the 5- to 6-membered heterocycloalkylene, the heteroatom is N, and the number of heteroatoms is 1, 2, or 3;
R² is 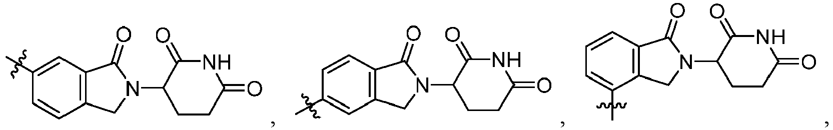 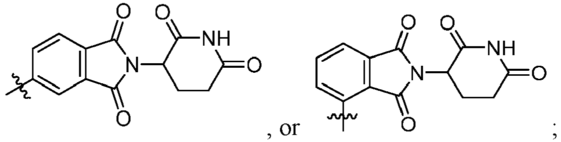
R³ is 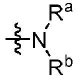 or 3- to 6-membered heterocycloalkyl, R^{a} and R^{b} are independently hydrogen or C₁-C₄ alkyl; the 3- to 6-membered heterocycloalkyl is linked to carbonyl through N; in the 3- to 6-membered heterocycloalkyl, the heteroatom is N, and the number of heteroatoms is 1 or 2.

9. The naphthalene ring-containing compound of formula **III,** the pharmaceutically acceptable salt thereof, the solvate thereof, or the solvate of the pharmaceutically acceptable salt thereof according to claim 1, wherein
-X¹-X²-X³-X⁴-X⁵-X⁶- is 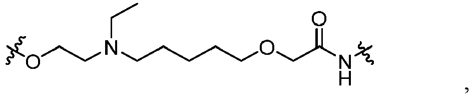 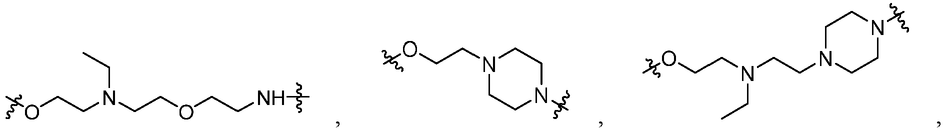 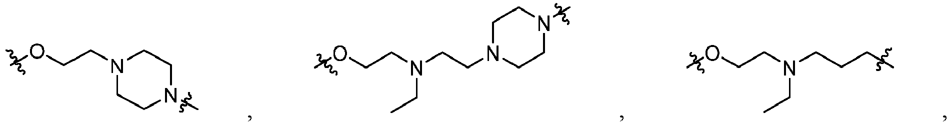 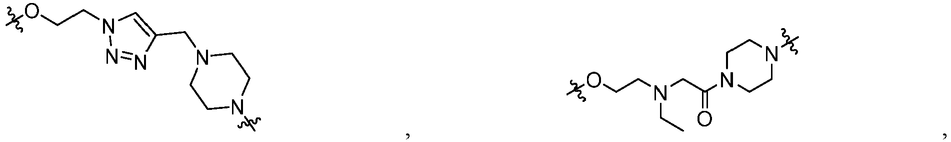 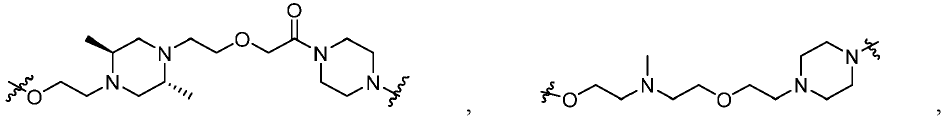 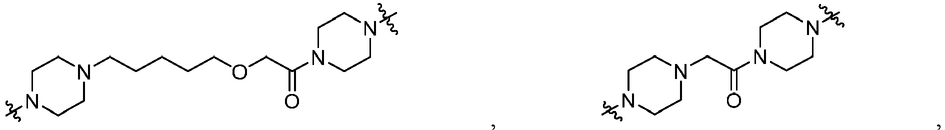 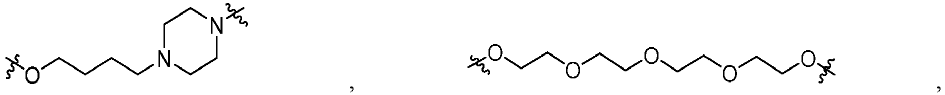 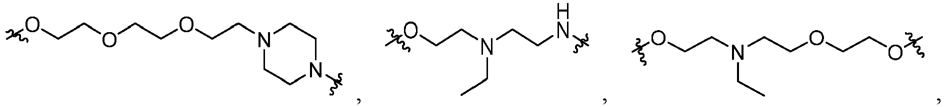 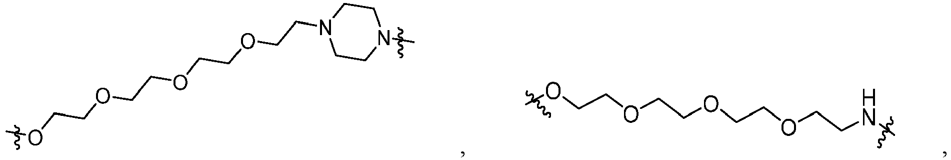 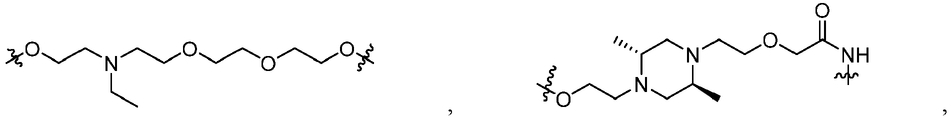 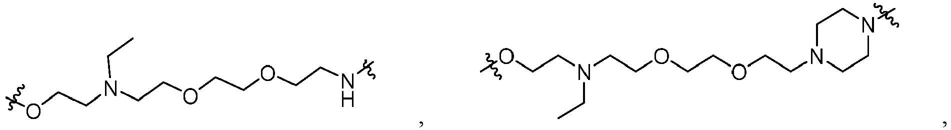 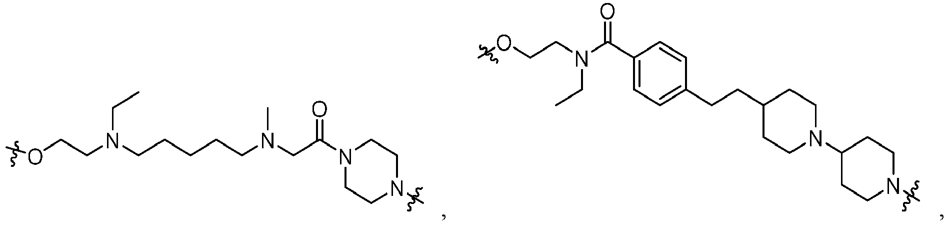 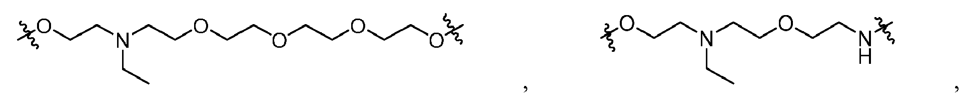 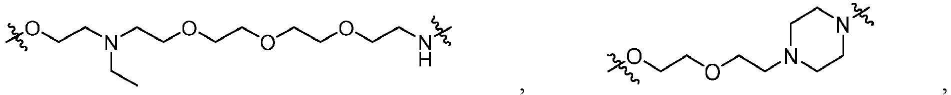 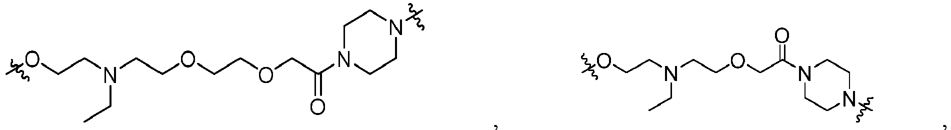 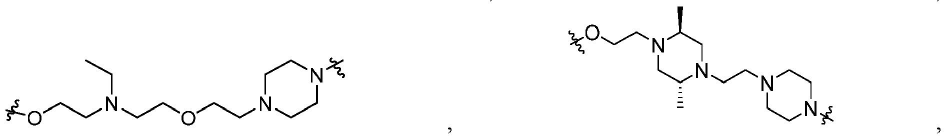 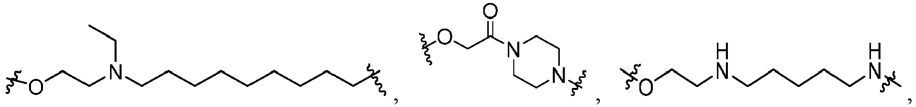 or

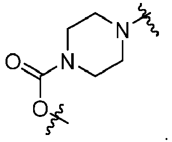

10. The naphthalene ring-containing compound of formula **III,** the pharmaceutically acceptable salt thereof, the solvate thereof, or the solvate of the pharmaceutically acceptable salt thereof according to claim 1, wherein in the naphthalene ring-containing compound of formula **III,** the pharmaceutically acceptable salt thereof, the solvate thereof, or the solvate of the pharmaceutically acceptable salt thereof, the naphthalene ring-containing compound of formula **III** is as represented by any one of the following structures:
| Compound No. | Structural formula |
|---|---|
| **D1** | 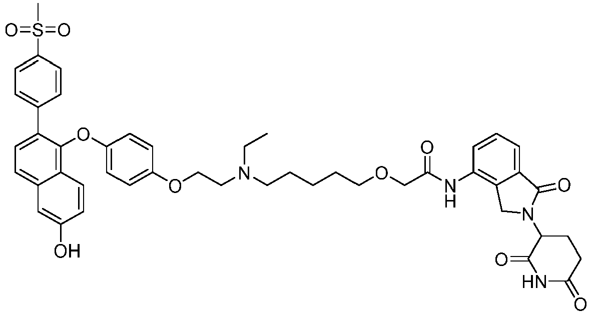 |
| **D2** | 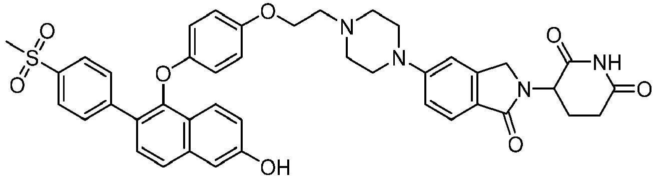 |
| **D57** | 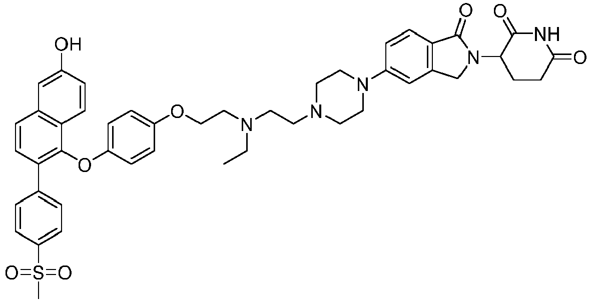 |
| **D3** | 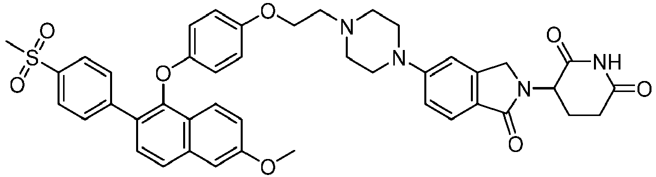 |
| **D4** | 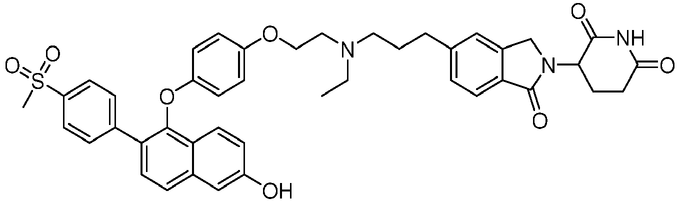 |
| **D5** | 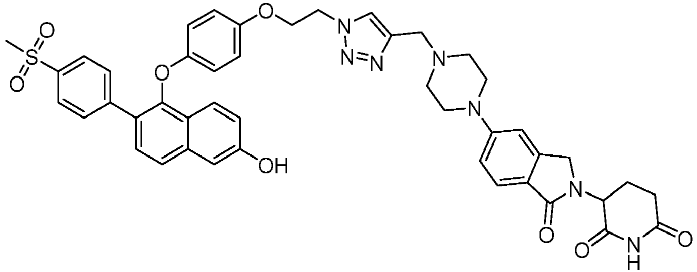 |
| **D58** | 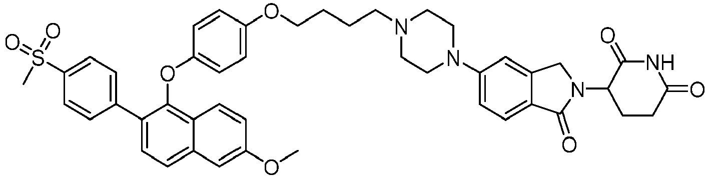 |
| **D6** | 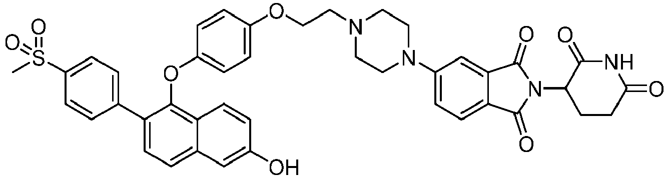 |
| **D7** | 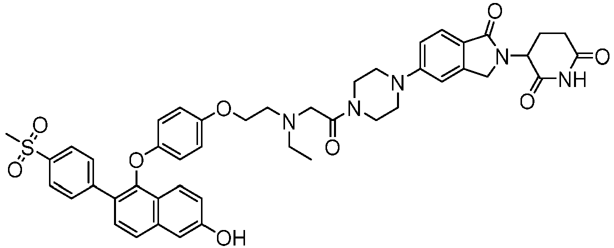 |
| **D8** | 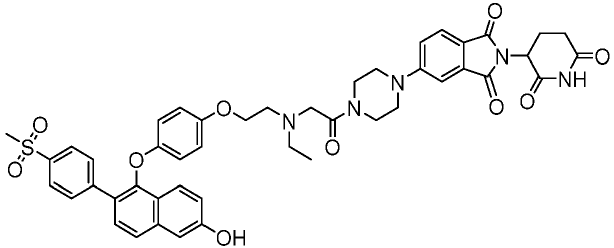 |
| **D9** | 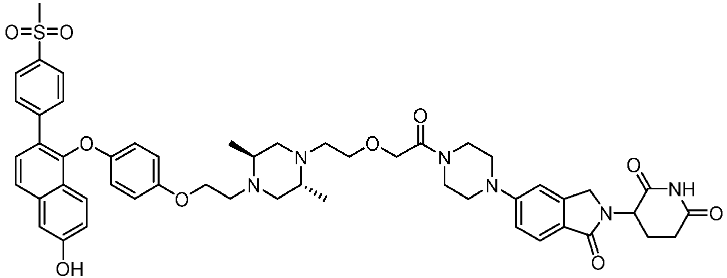 |
| **D10** | 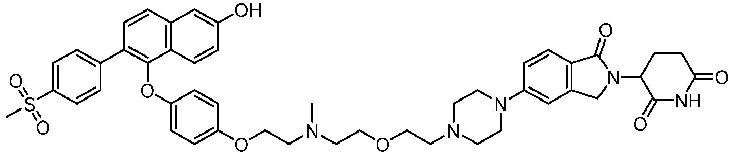 |
| **D11** | 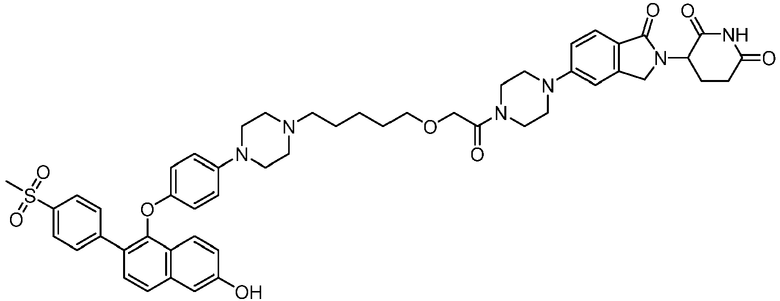 |
| **D12** | 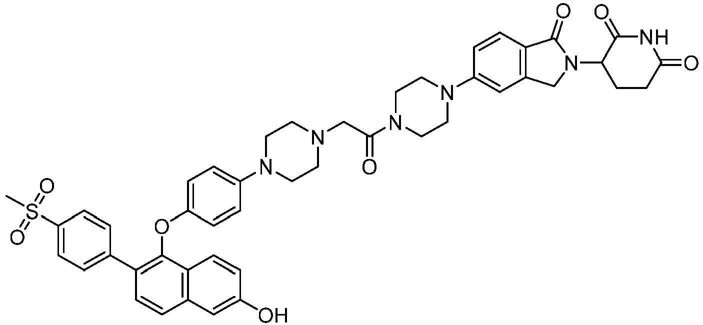 |
| **D13** | 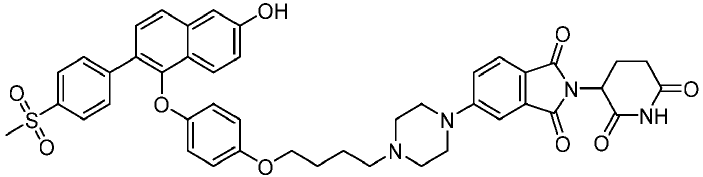 |
| **D59** | 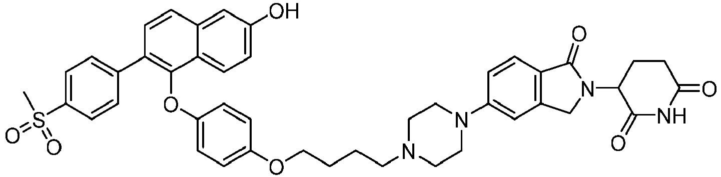 |
| **D14** | 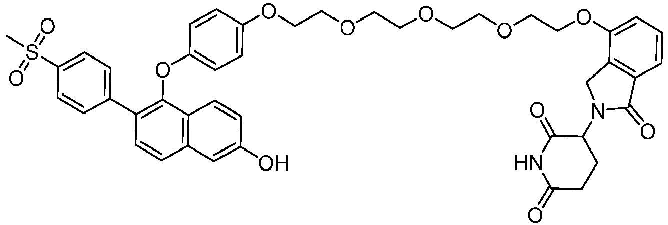 |
| **D15** | 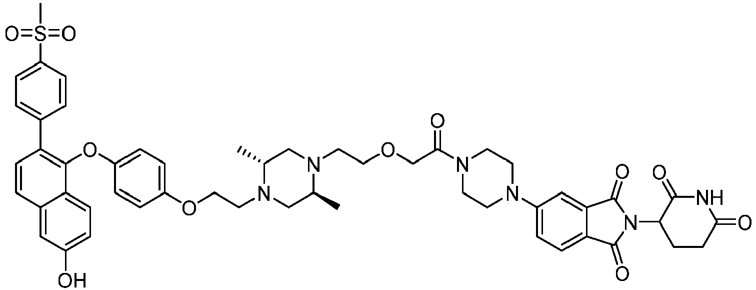 |
| **D16** | 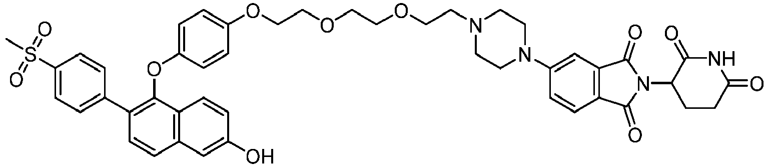 |
| **D17** | 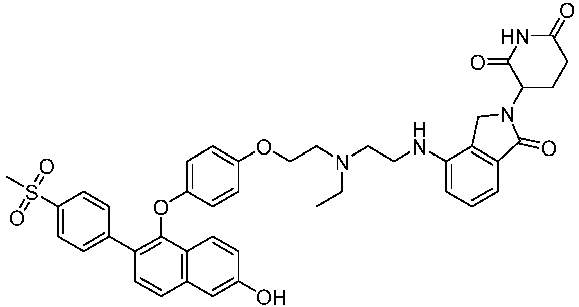 |
| **D18** | 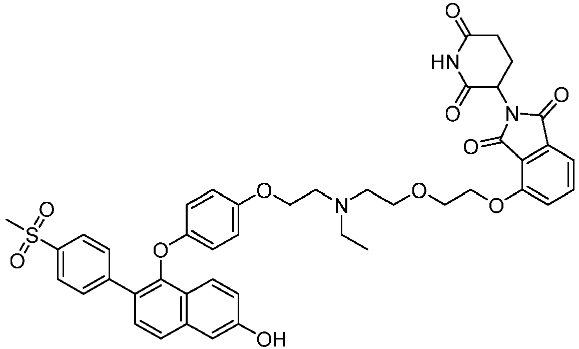 |
| **D19** | 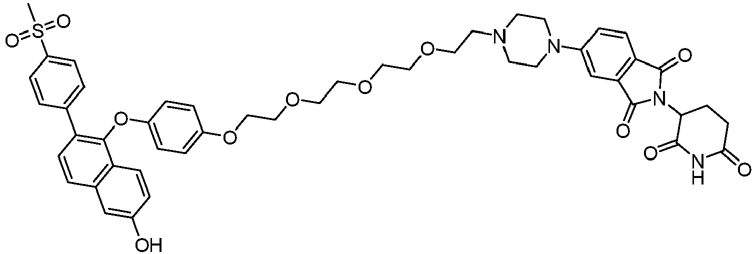 |
| **D20** | 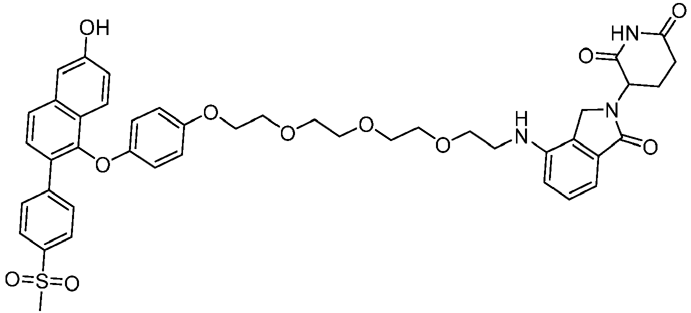 |
| **D21** | 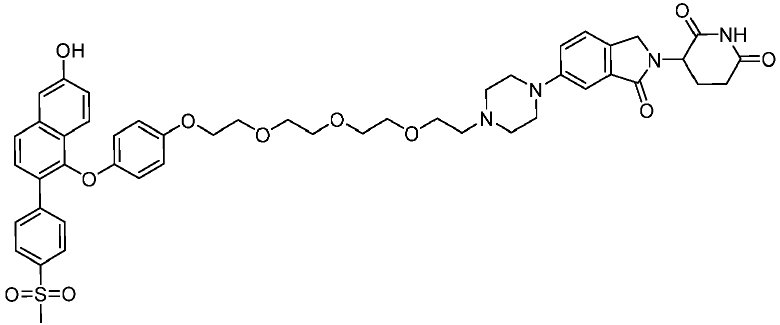 |
| **D22** | 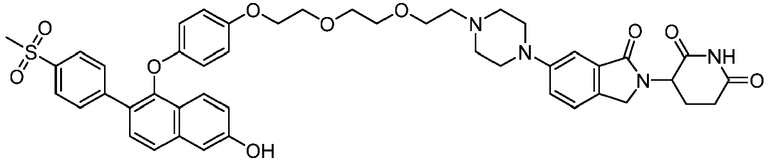 |
| **D23** | 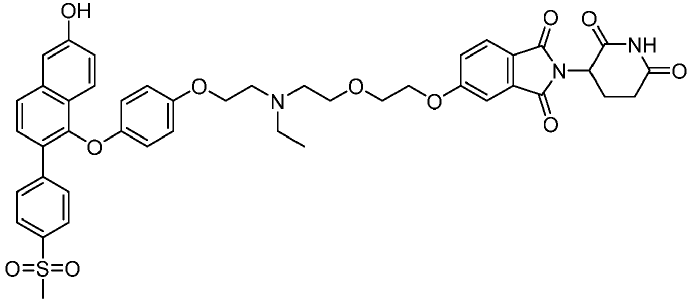 |
| **D24** | 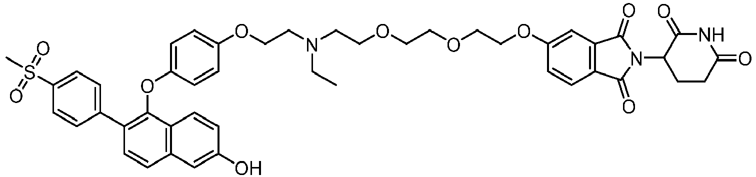 |
| **D25** | 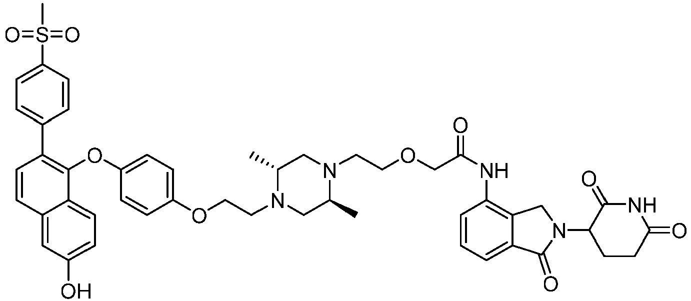 |
| **D26** | 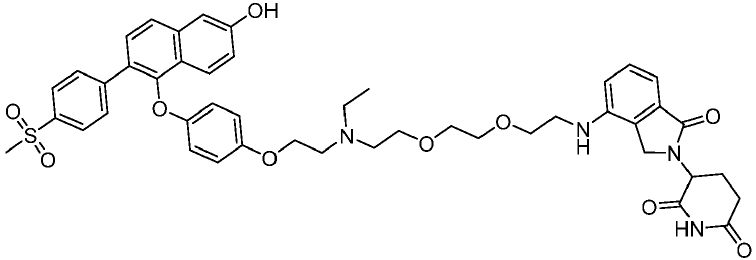 |
| **D27** | 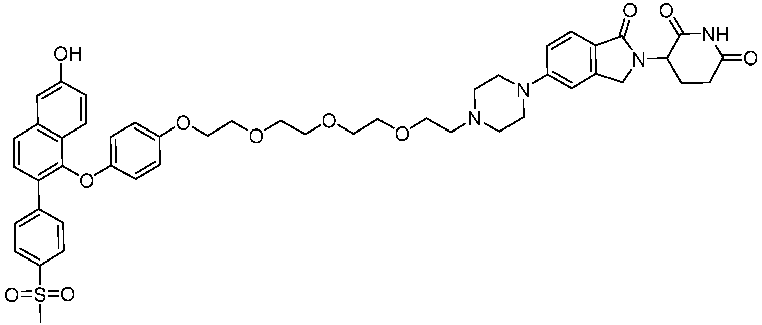 |
| **D28** | 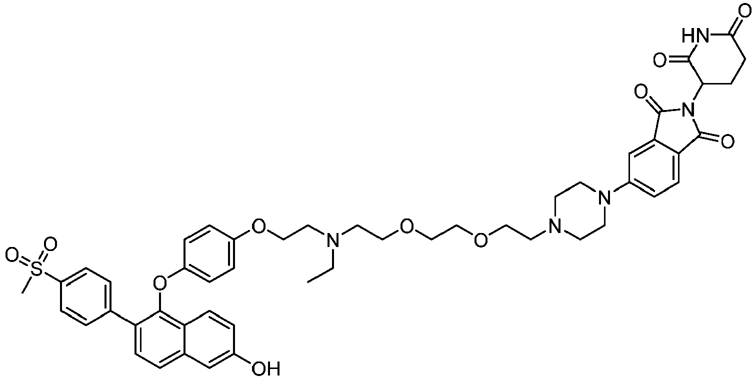 |
| **D29** | 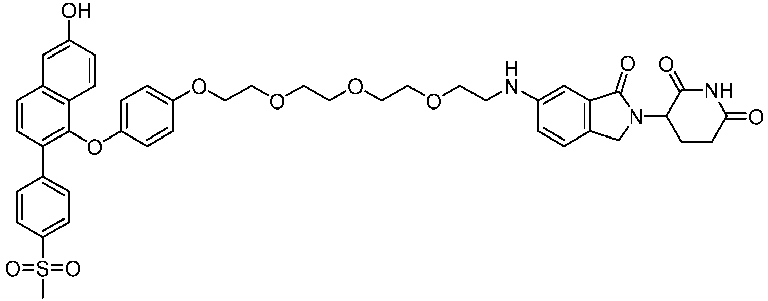 |
| **D30** | 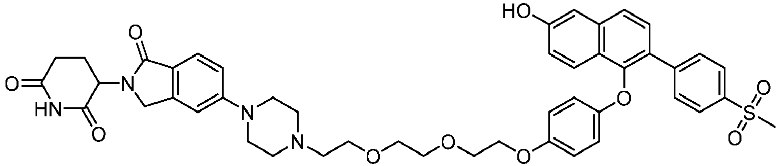 |
| **D60** | 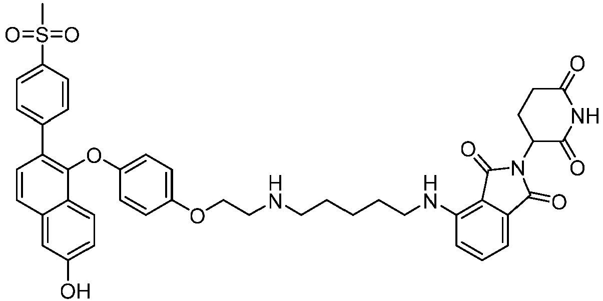 |
| **D31** | 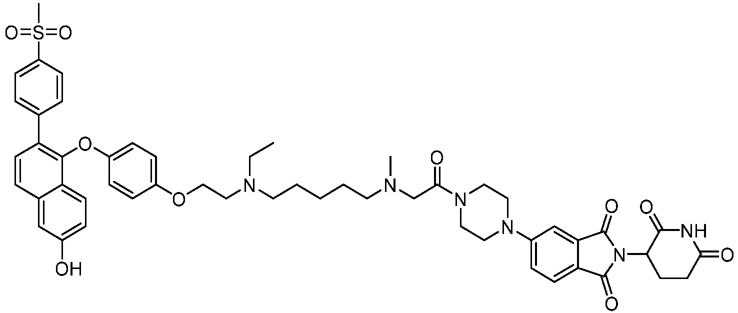 |
| **D32** | 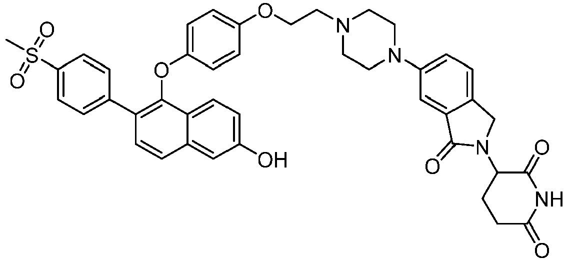 |
| **D33** | 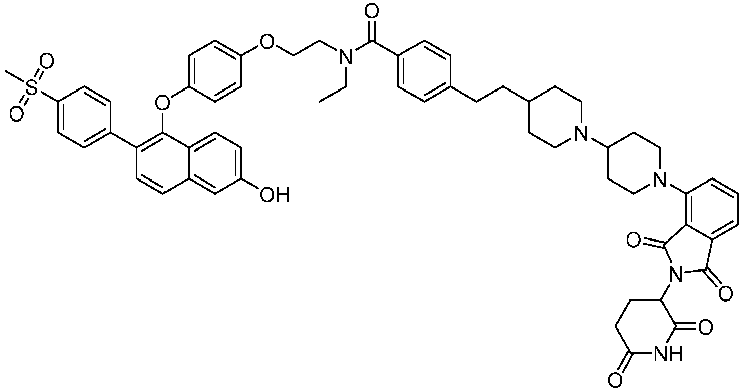 |
| **D34** | 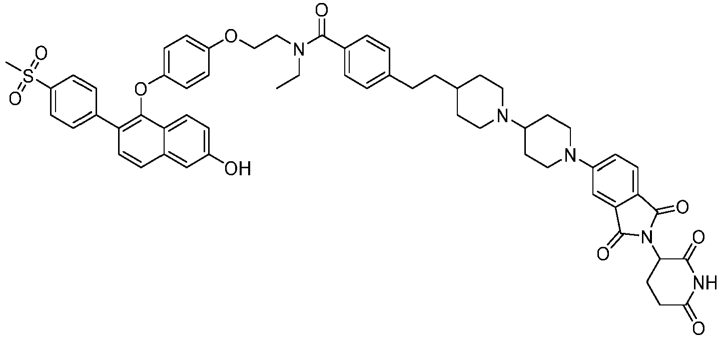 |
| **D35** | 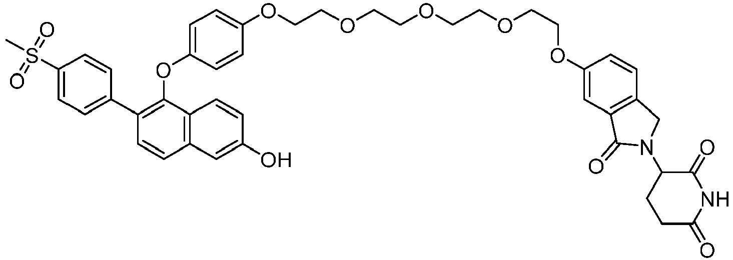 |
| **D36** | 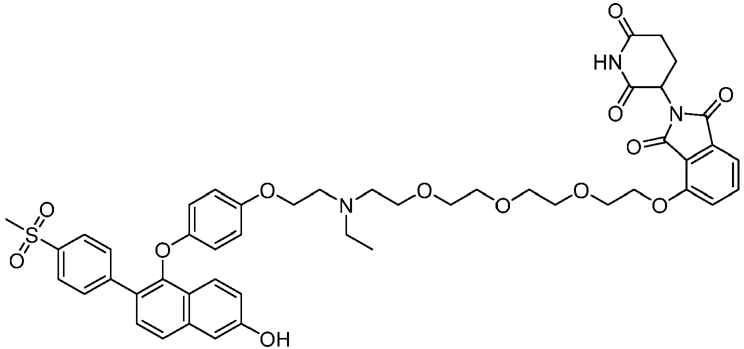 |
| **D37** | 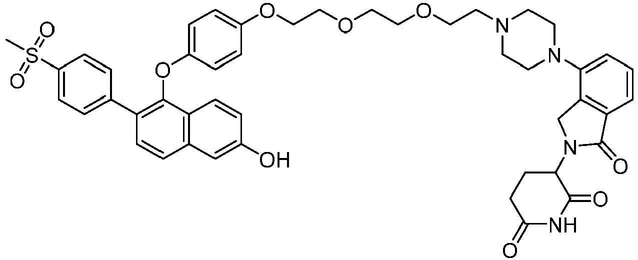 |
| **D38** | 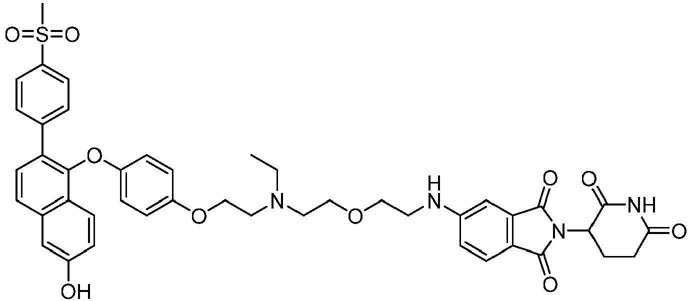 |
| **D39** | 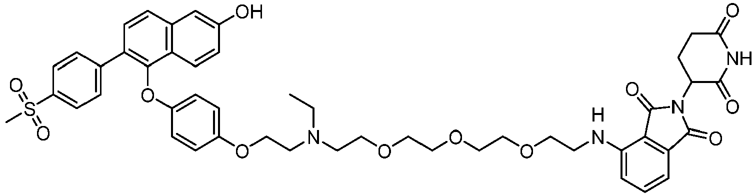 |
| **D40** | 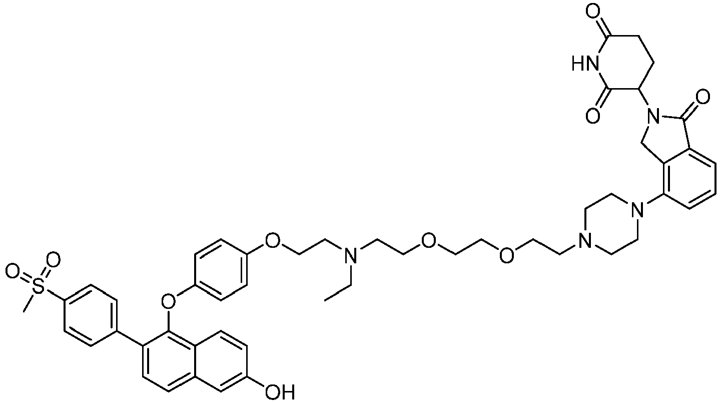 |
| **D41** | 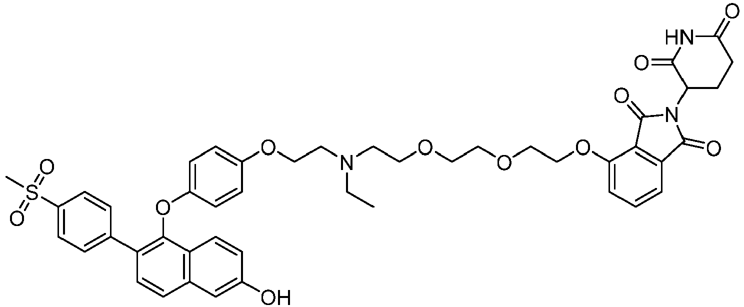 |
| **D42** | 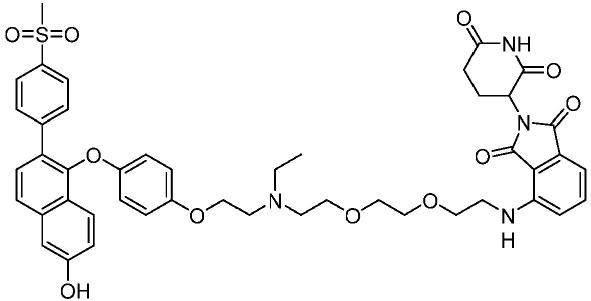 |
| **D43** | 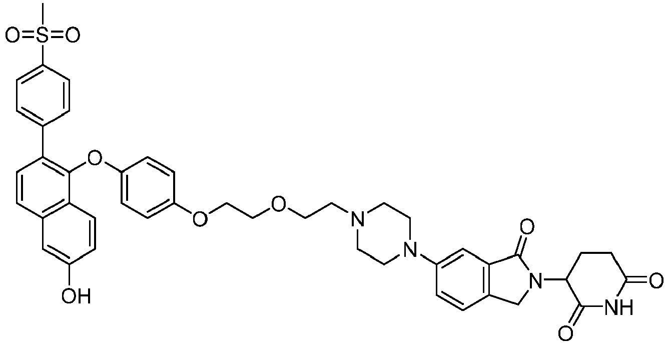 |
| **D44** | 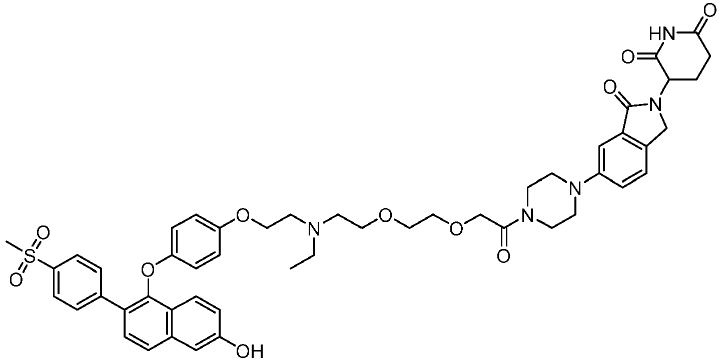 |
| **D45** | 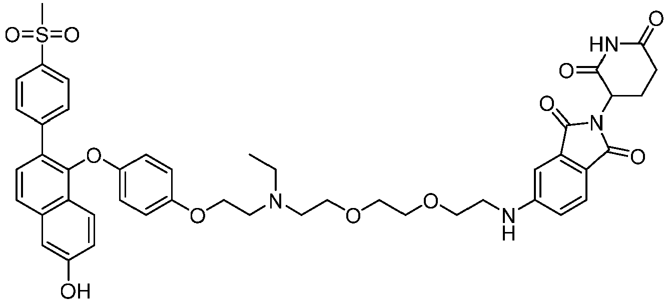 |
| **D46** | 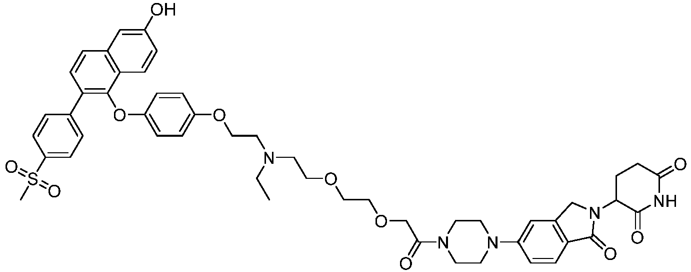 |
| **D47** | 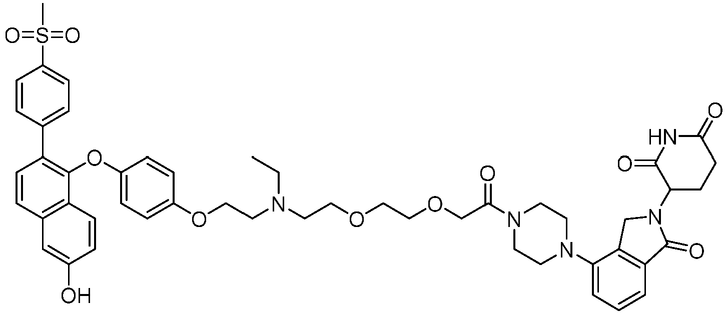 |
| **D48** | 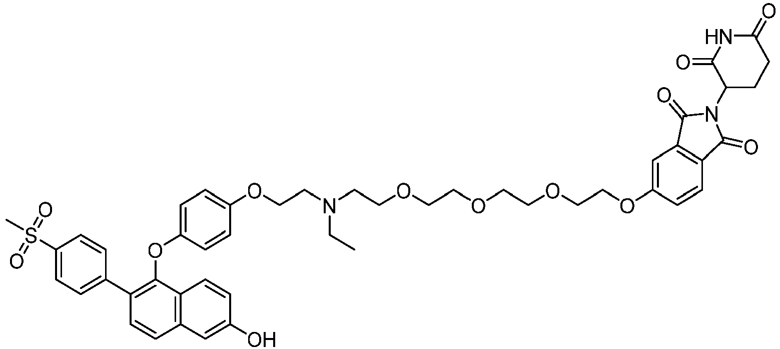 |
| **D49** | 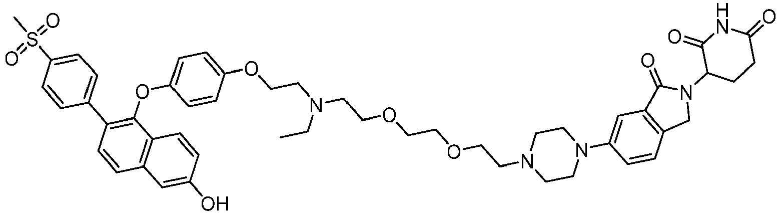 |
| **D50** | 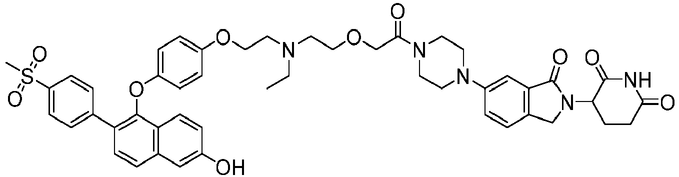 |
| **D51** | 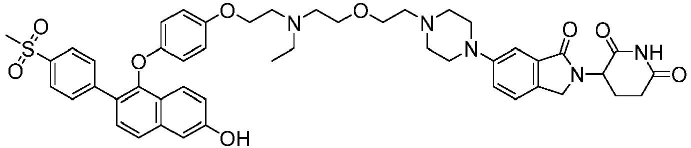 |
| **D52** | 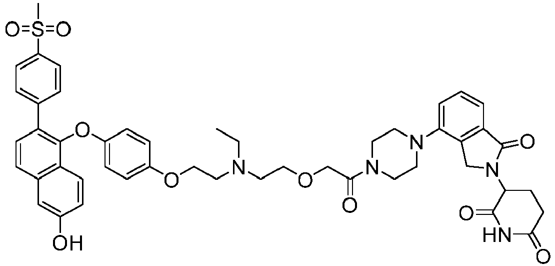 |
| **D61** | 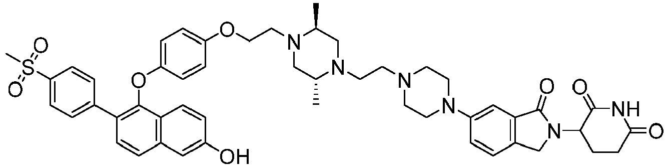 |
| **D53** | 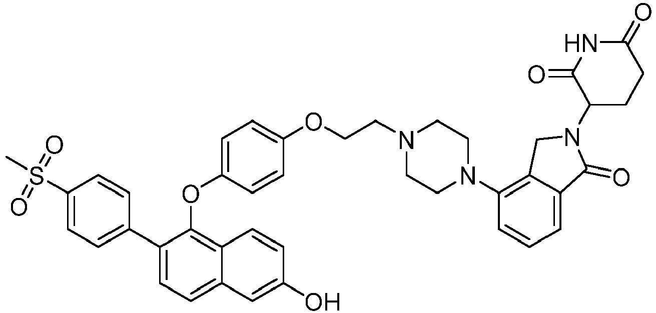 |
| **D62** | 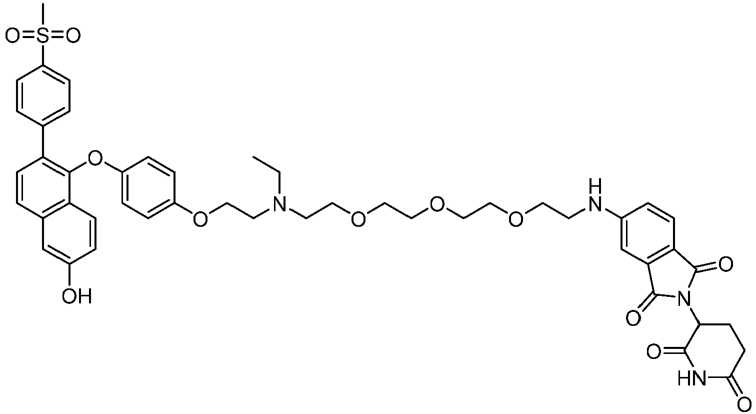 |
| **D63** | 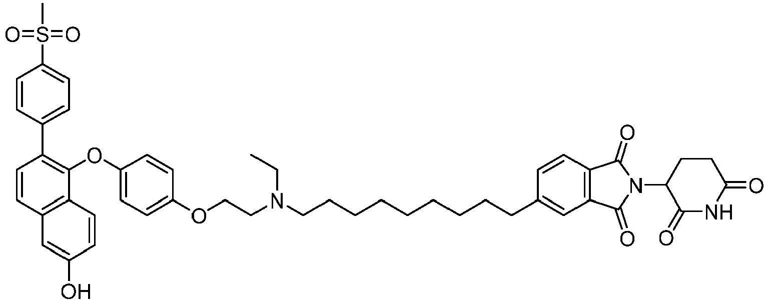 |
| **D54** | 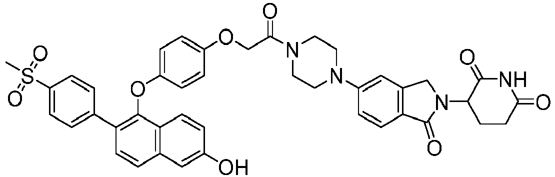 |
| **D55** | 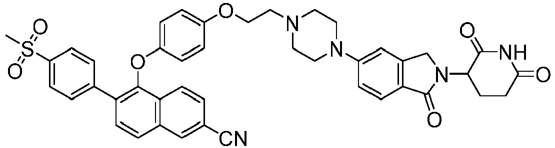 |
| **D56** | 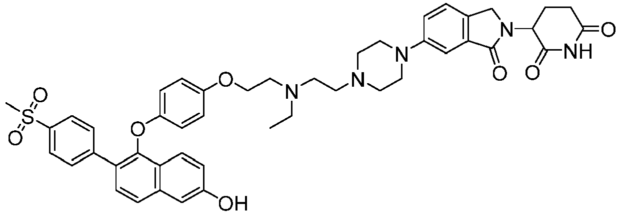 |
| **D64** | 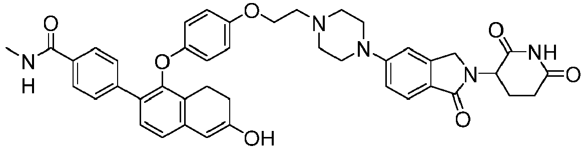 |
| **D65** | 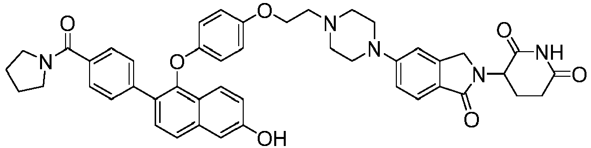 |
| **D66** | 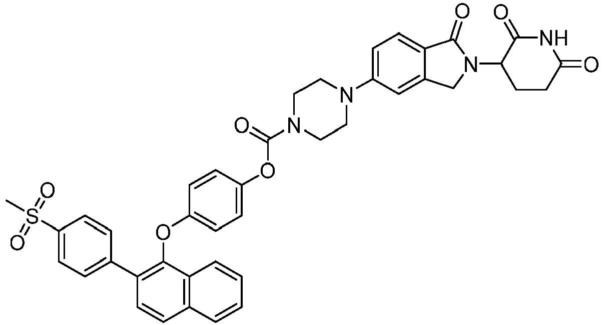 |
| **D67** | 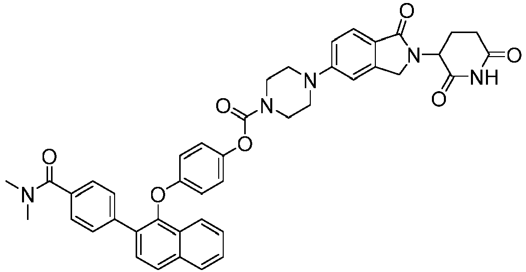 |
| **D68** | 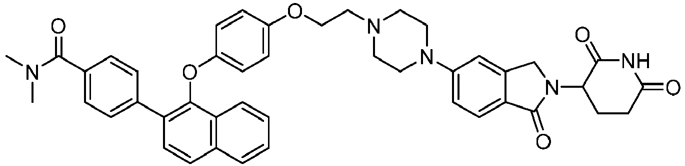 |
| **D69** | 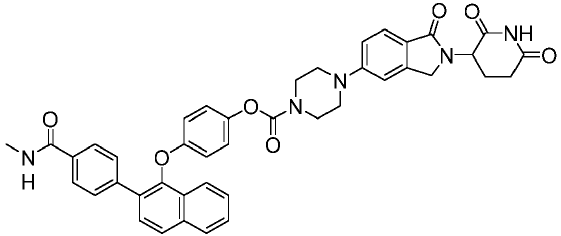 |
or, the pharmaceutically acceptable salt of the naphthalene ring-containing compound of formula **III** is

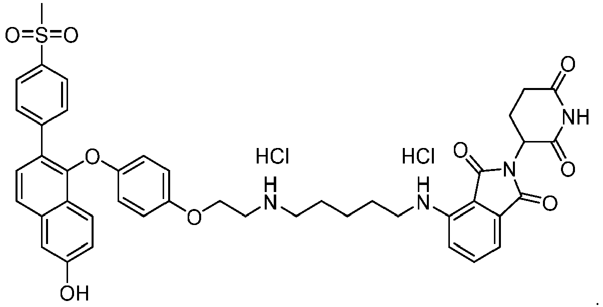

11. A pharmaceutical composition consisting of substance **B** and a pharmaceutical excipient;
the substance **B** is the naphthalene ring-containing compound of formula **III,** the pharmaceutically acceptable salt thereof, the solvate thereof, or the solvate of the pharmaceutically acceptable salt according to any one of claims 1 to 10;
in the pharmaceutical composition, the substance **B** may be a therapeutically effective amount of substance **B.**

12. A use of substance **B** or the pharmaceutical composition according to claim 11 in the manufacture of a medicament;
the substance **B** is the naphthalene ring-containing compound of formula III, the pharmaceutically acceptable salt thereof, the solvate thereof, or the solvate of the pharmaceutically acceptable salt thereof according to any one of claims 1 to 10; and the medicament is a medicament for the treatment or prevention of cancer;
the cancer may be breast cancer;
the breast cancer may be Luminal A breast cancer, Luminal B breast cancer, HER2-positive breast cancer, or triple-negative breast cancer;
the Luminal Abreast cancer may be non-drug-resistant and non-mutated Luminal Abreast cancer, drug-resistant and non-mutated Luminal A breast cancer, or drug-resistant and mutated Luminal A breast cancer;
the drug-resistant and non-mutated Luminal A breast cancer may be tamoxifen-resistant and non-mutated Luminal A breast cancer;
the drug-resistant and mutated Luminal Abreast cancer may be ER^{D538G}-mutated Luminal A breast cancer, and may also be tamoxifen- and/or fulvestrant-resistant and ER^{D538G}-mutated Luminal A breast cancer, and may also be tamoxifen- and fulvestrant-resistant and ER^{D538G}-mutated Luminal A breast cancer;
the drug-resistant and mutated Luminal A breast cancer may be ER^{Y537S}-mutated Luminal A breast cancer, and may also be tamoxifen- and/or fulvestrant-resistant and ER^{Y537S}-mutated Luminal A breast cancer, and may also be tamoxifen- and fulvestrant-resistant and ER^{Y537S}-mutated Luminal A breast cancer.

13. A naphthalene ring-containing compound of formula **IV** or a salt thereof; 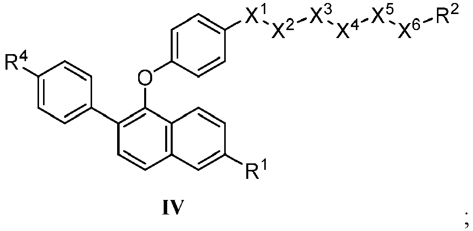
wherein R⁴ is -SO₂-C₁-C₄ alkyl or 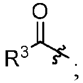 R³ is 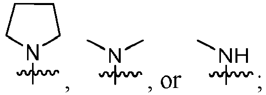
X¹ is -O-;
X², X³, X⁴, X⁵, and X⁶ are as described in any one of claims 1 to 10; X², X³, X⁴, X⁵, and X⁶ are not simultaneously absent;
R² is hydrogen, 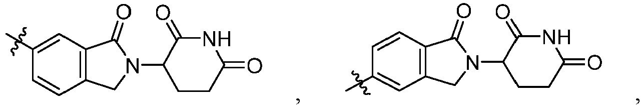 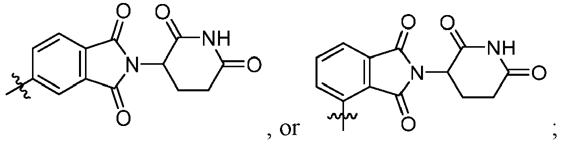
R¹ is hydrogen, benzyl, hydroxyl, or C₁-C₄ alkoxy.

14. A compound or a salt thereof, which has any one of the following structures: 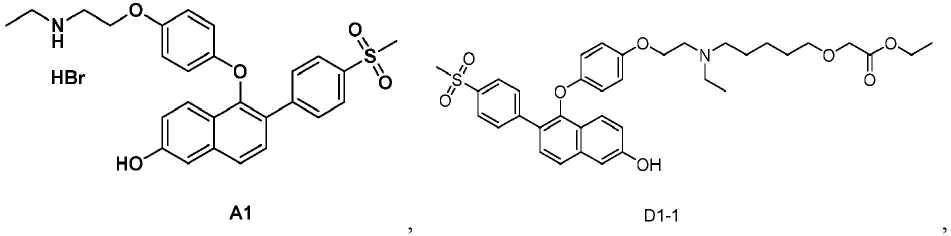 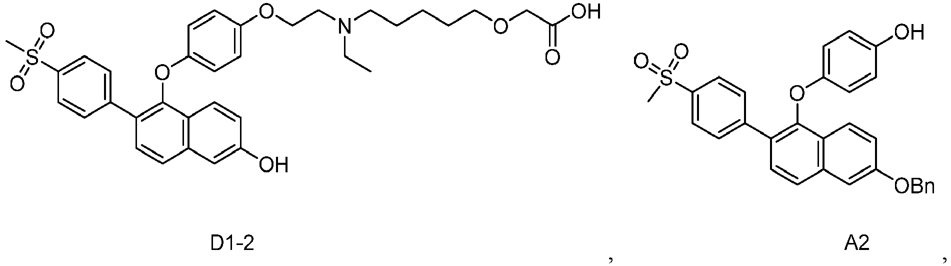 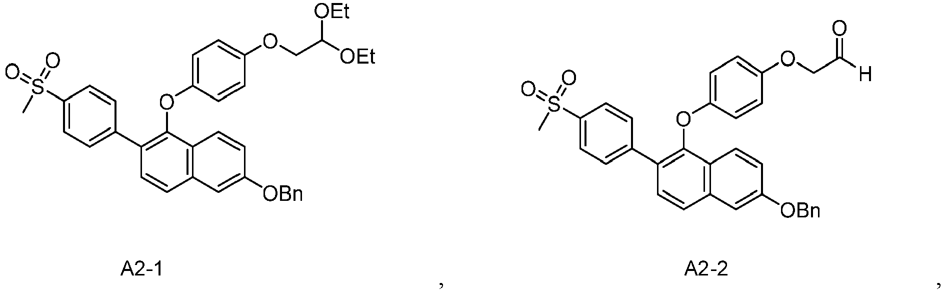 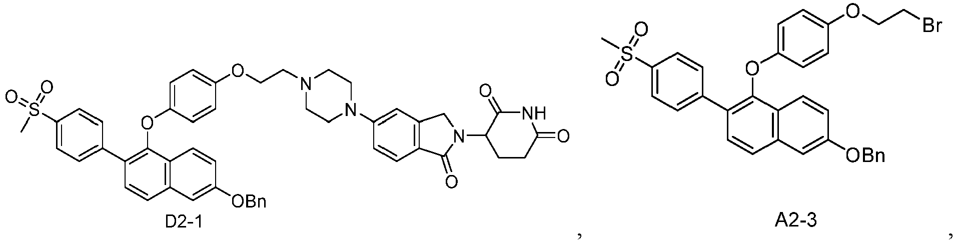 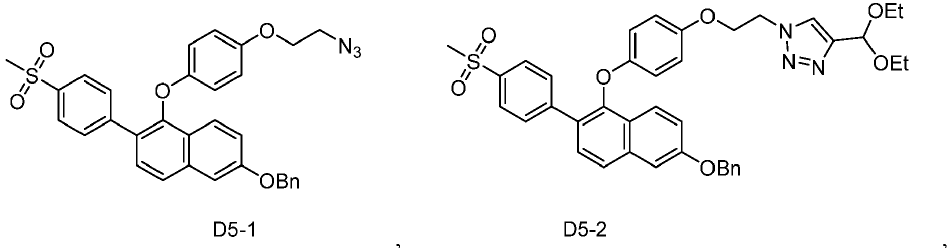 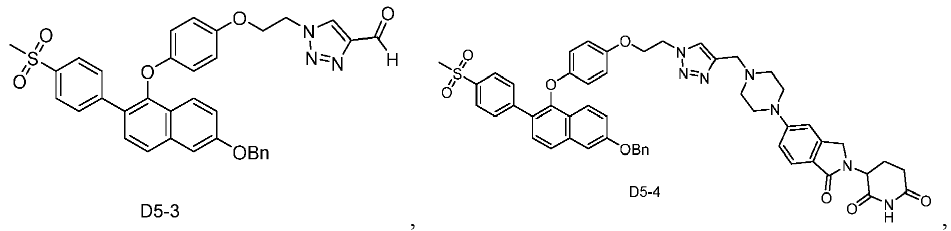 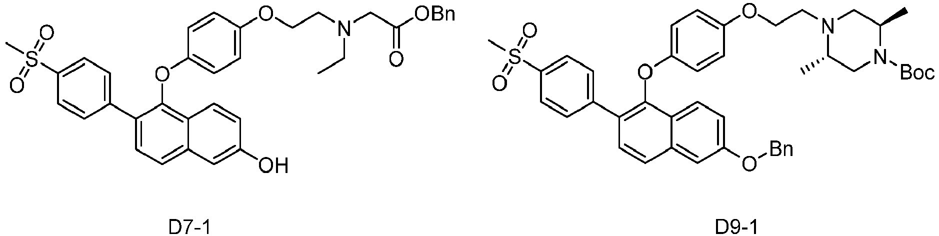 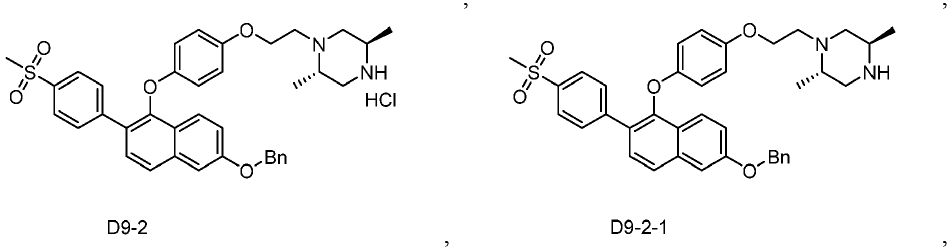 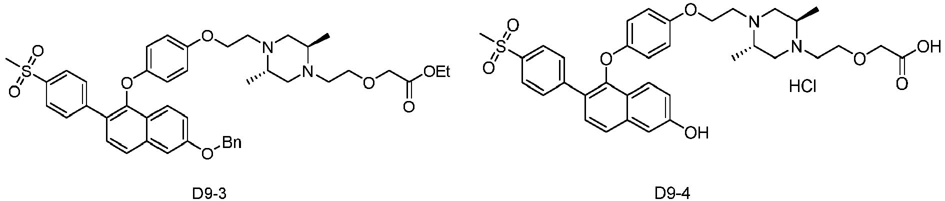 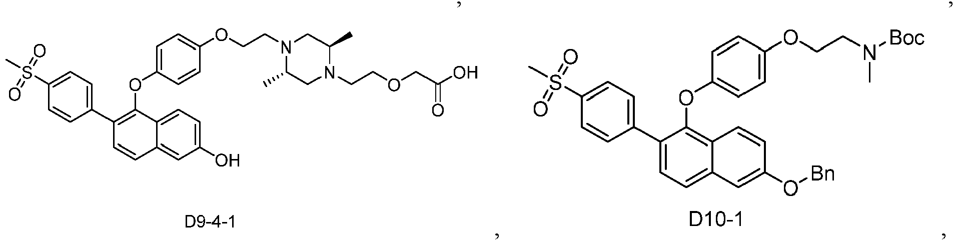 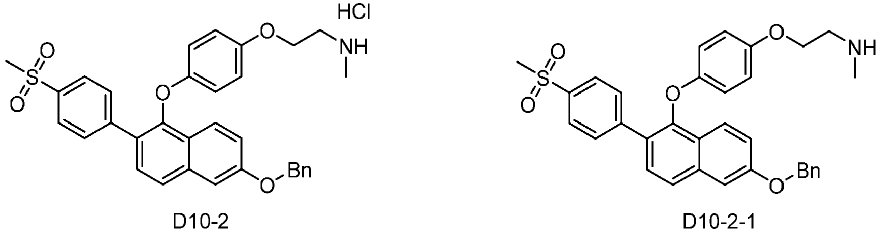 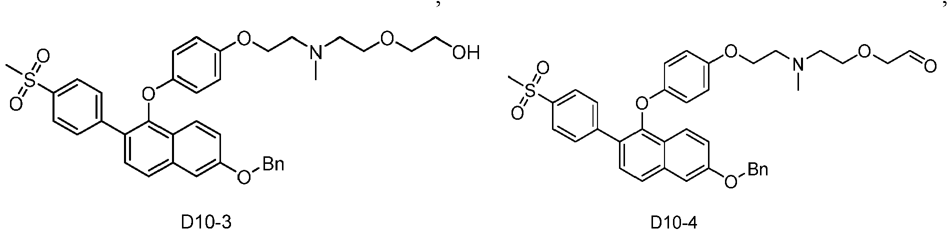 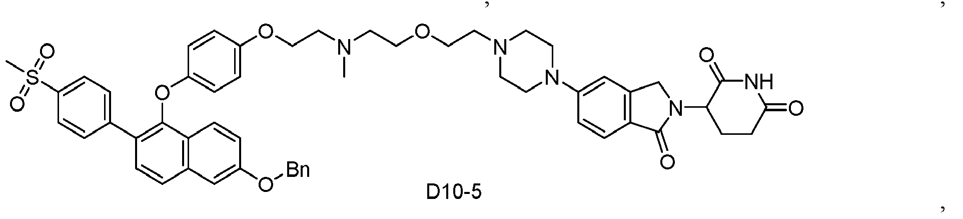 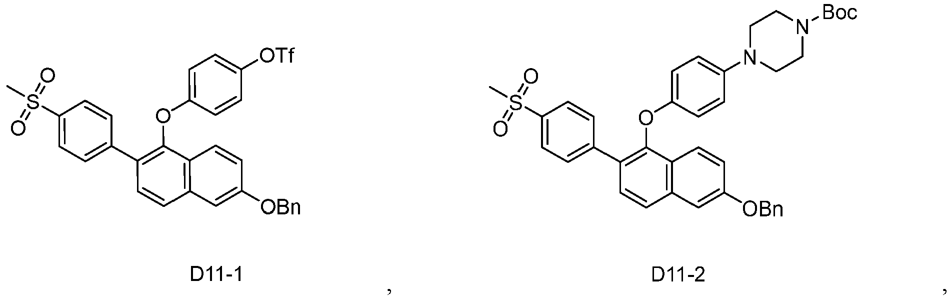 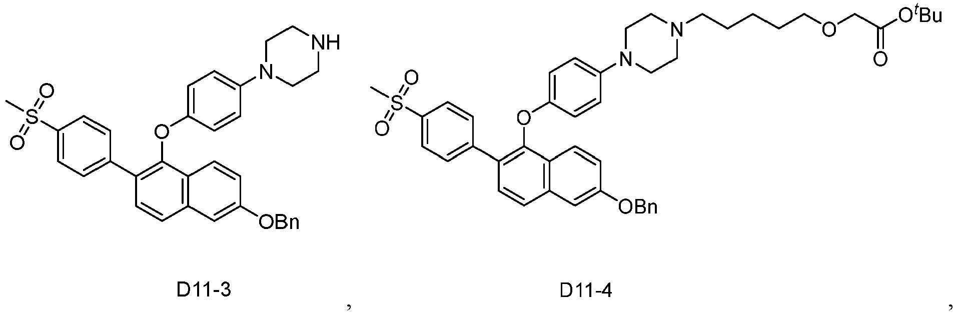 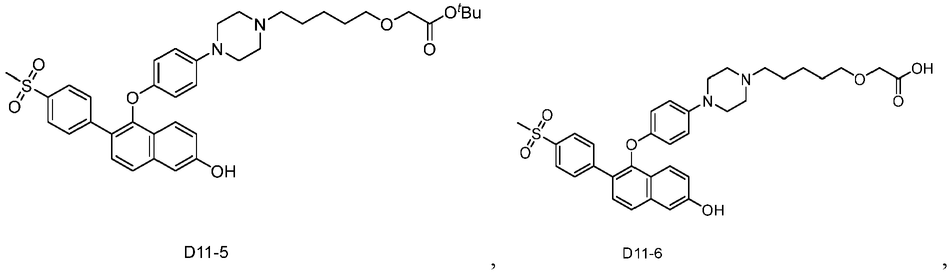 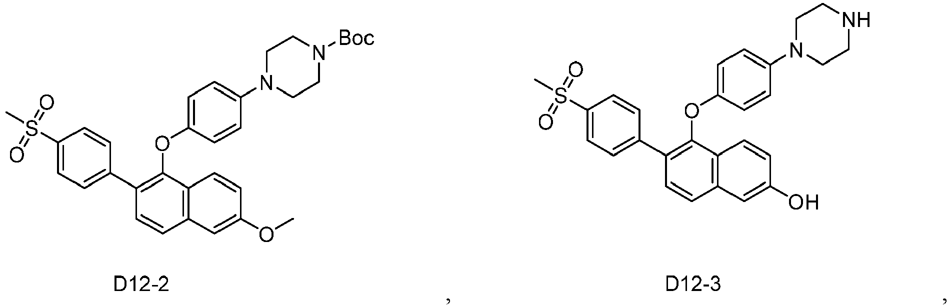 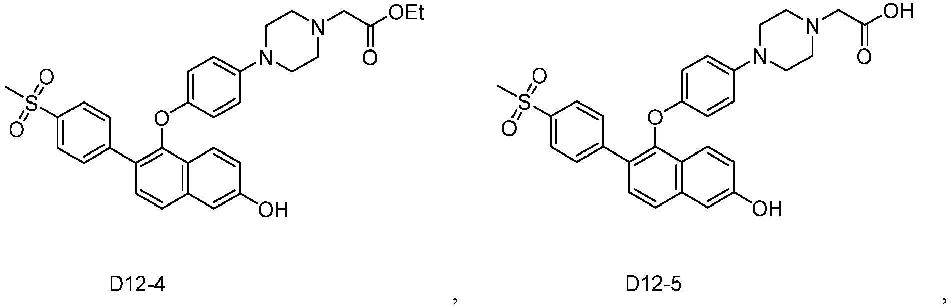 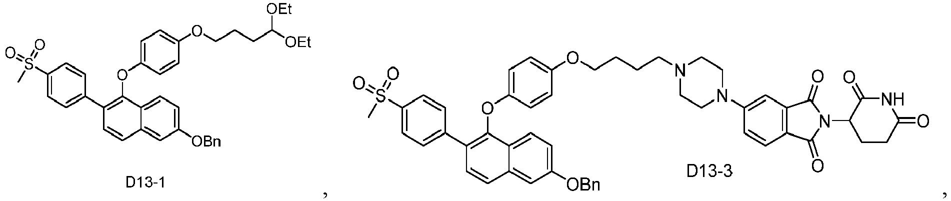 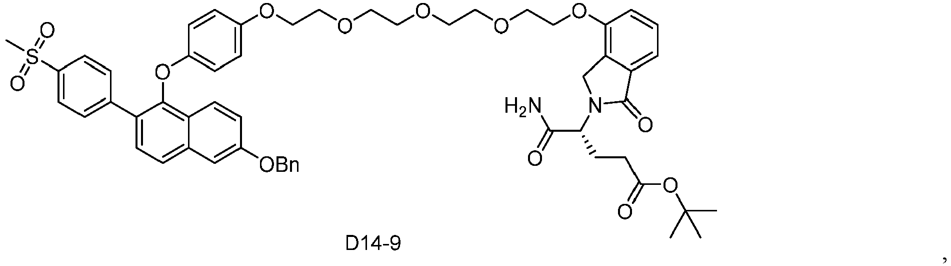 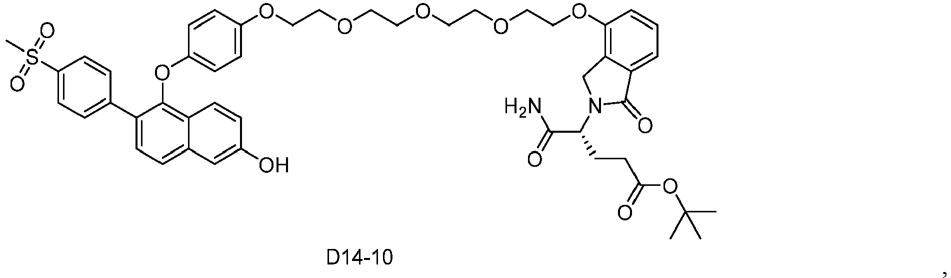 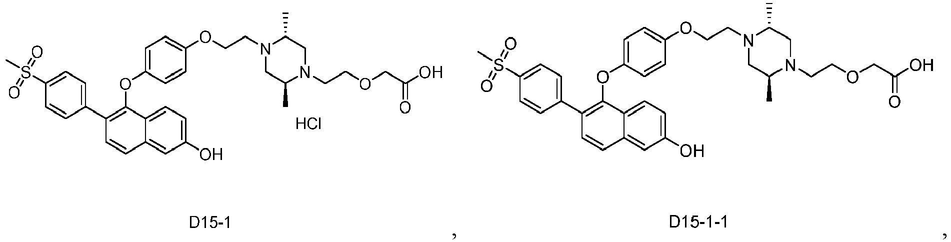 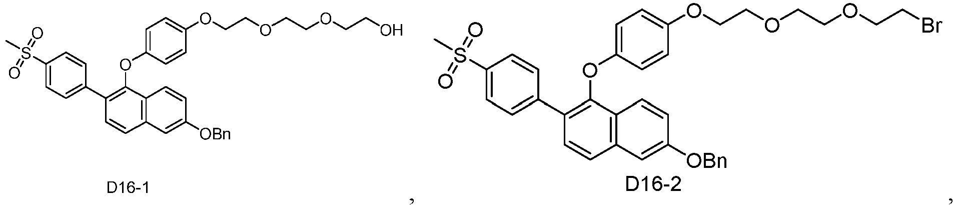 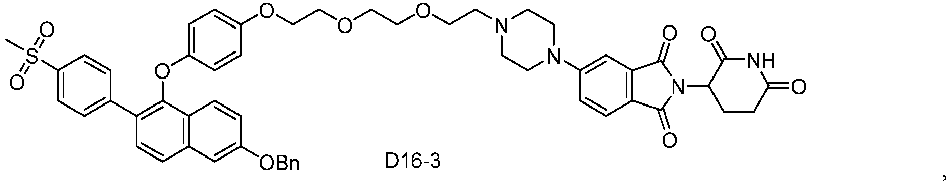 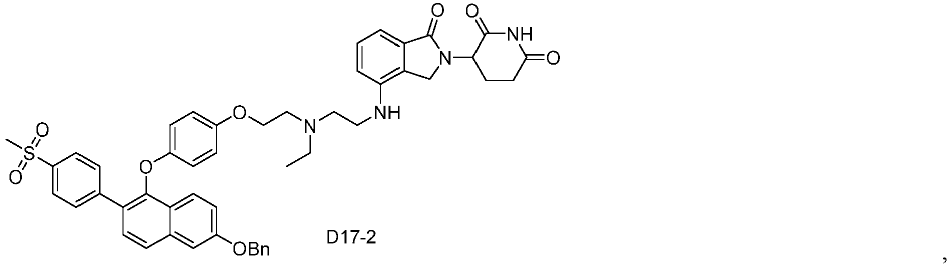 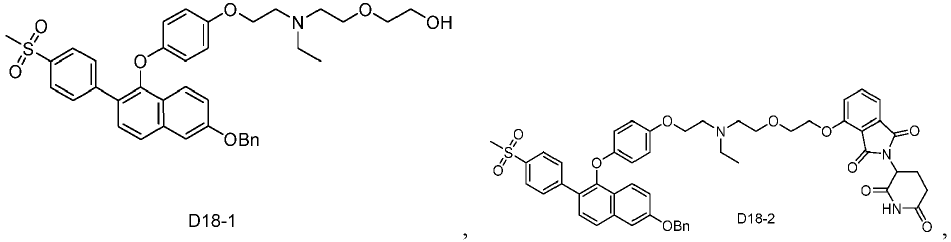 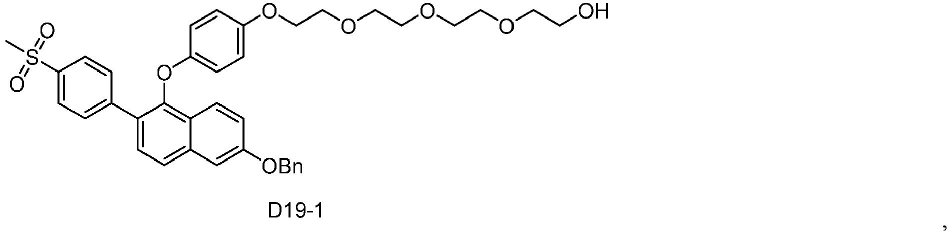 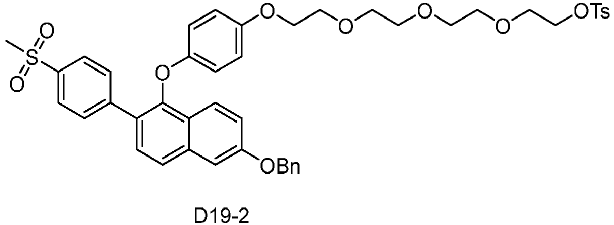 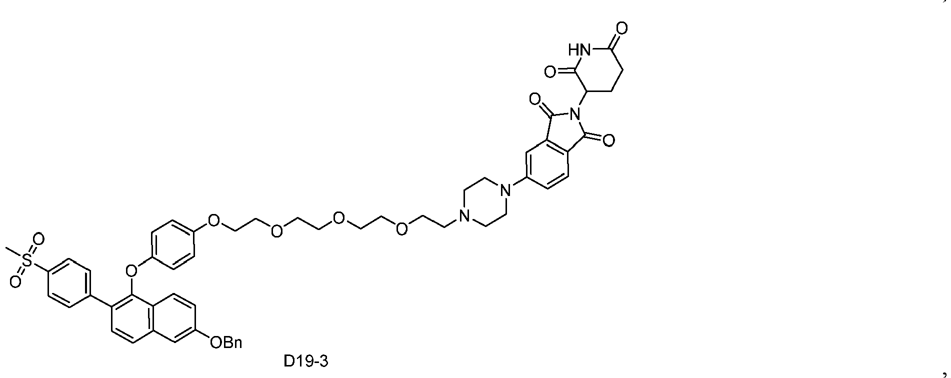 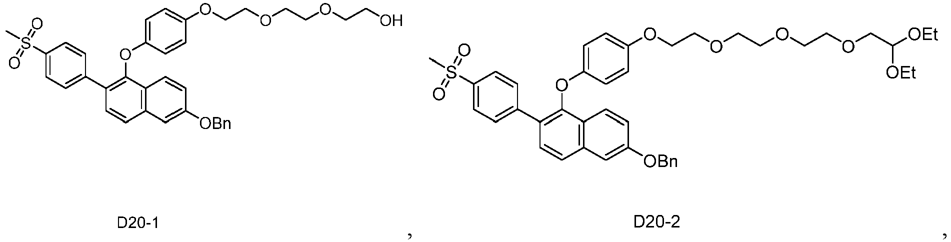 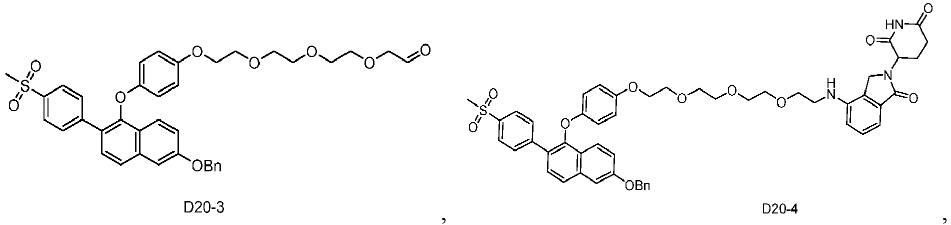 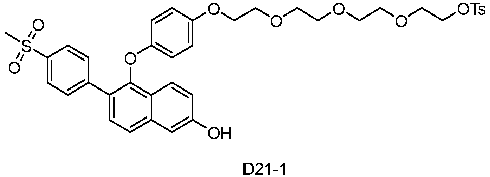 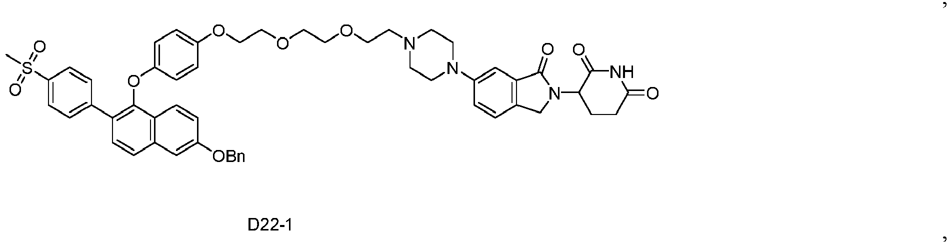 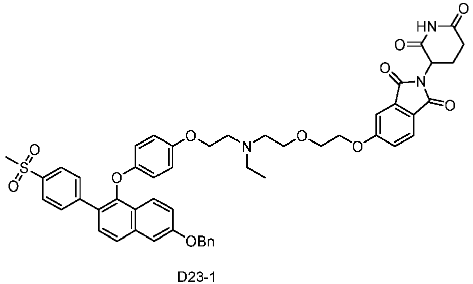 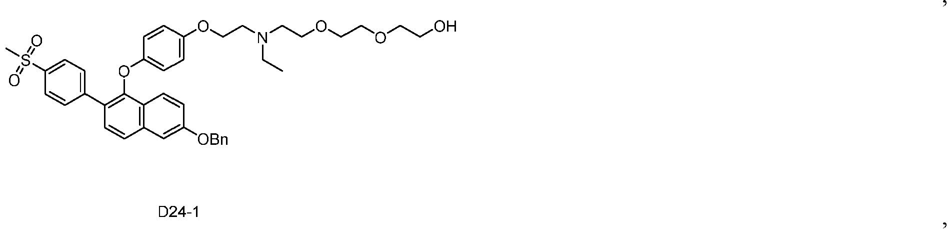 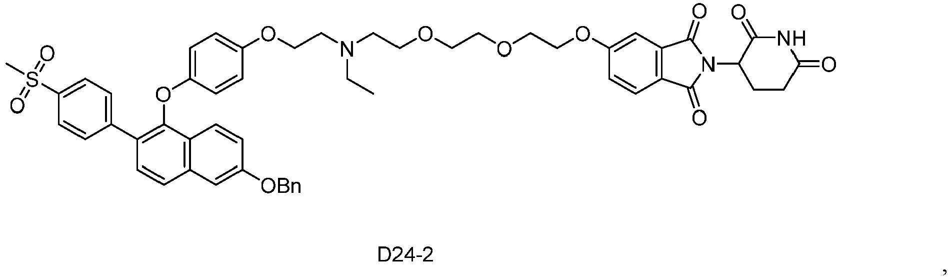 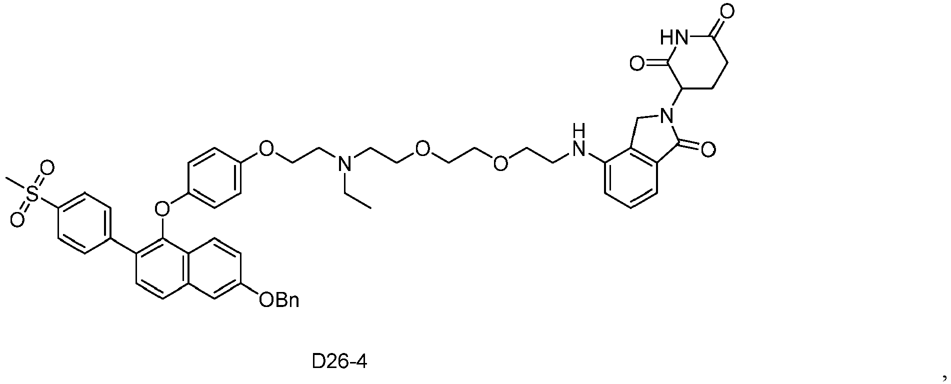 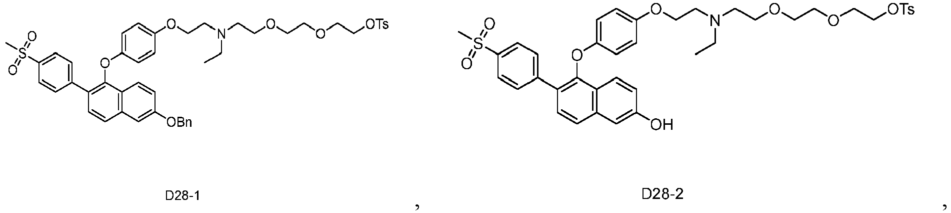 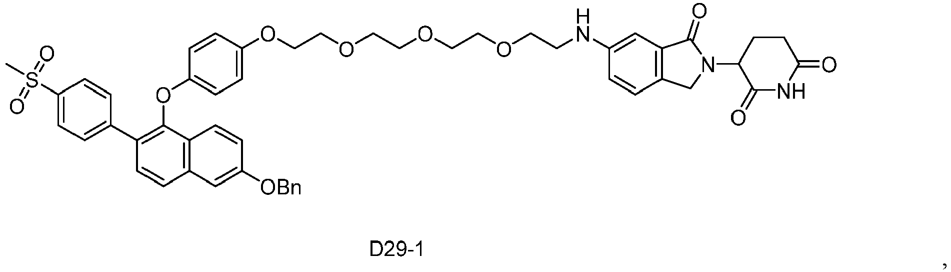 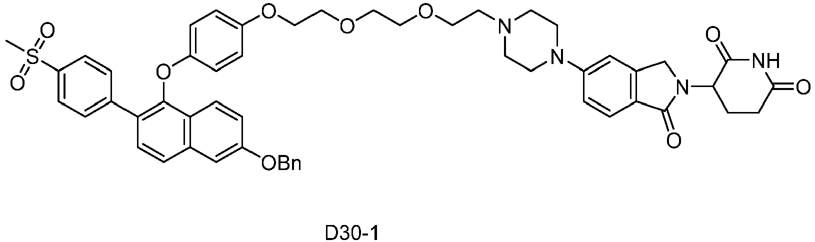 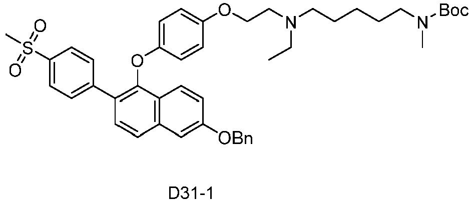 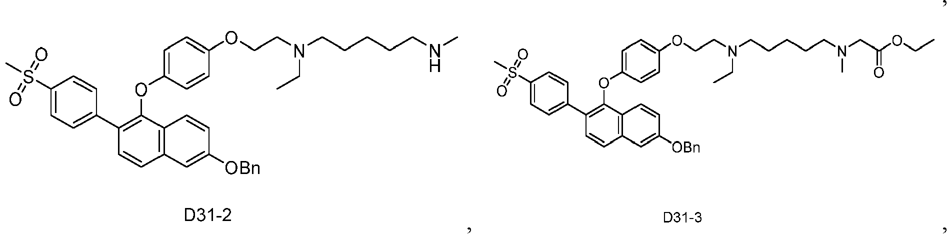 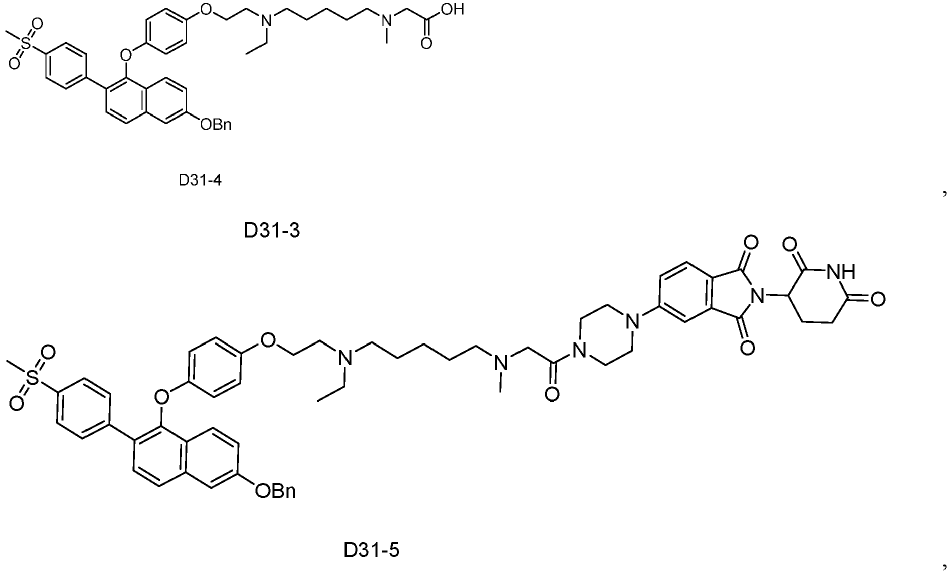 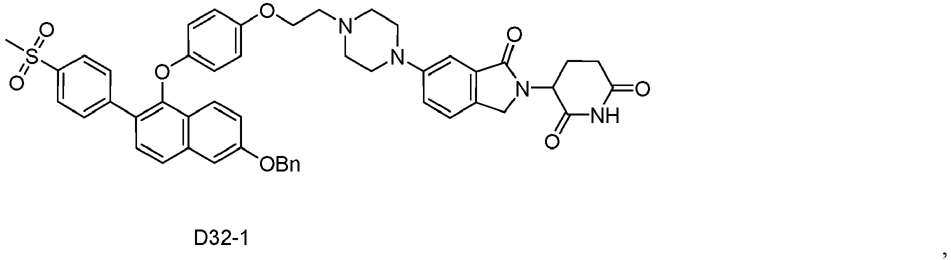 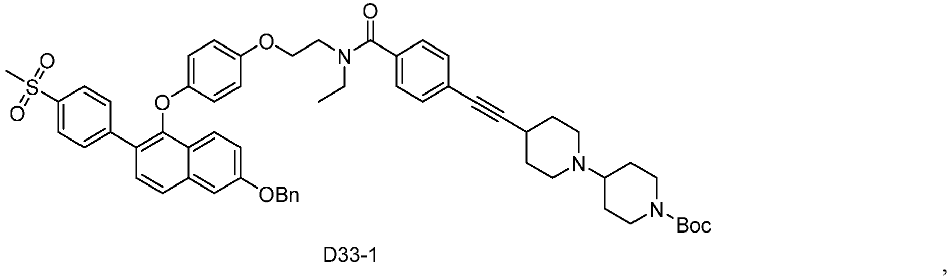 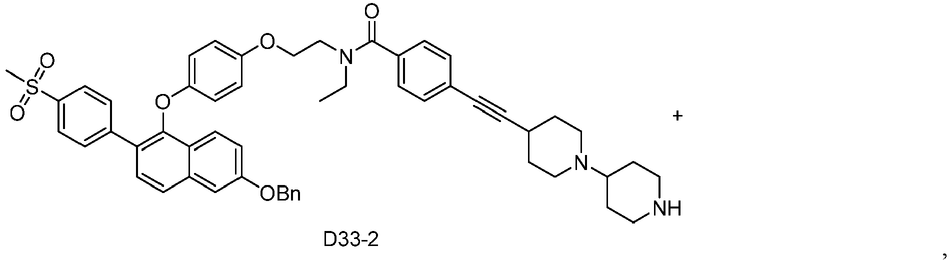 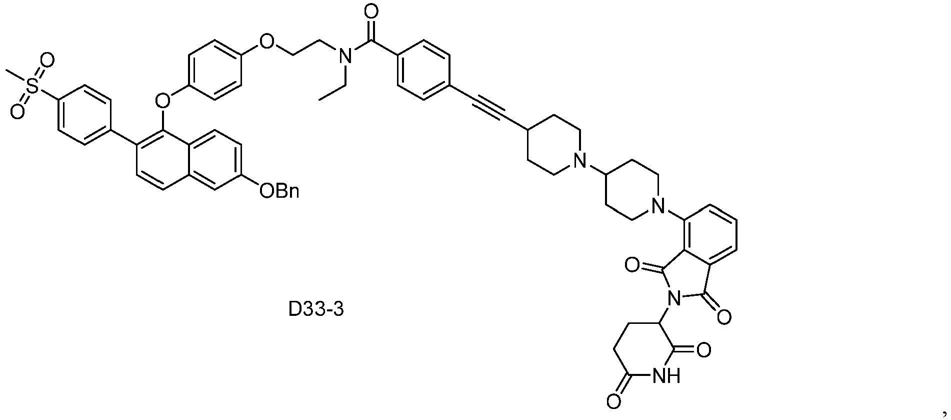 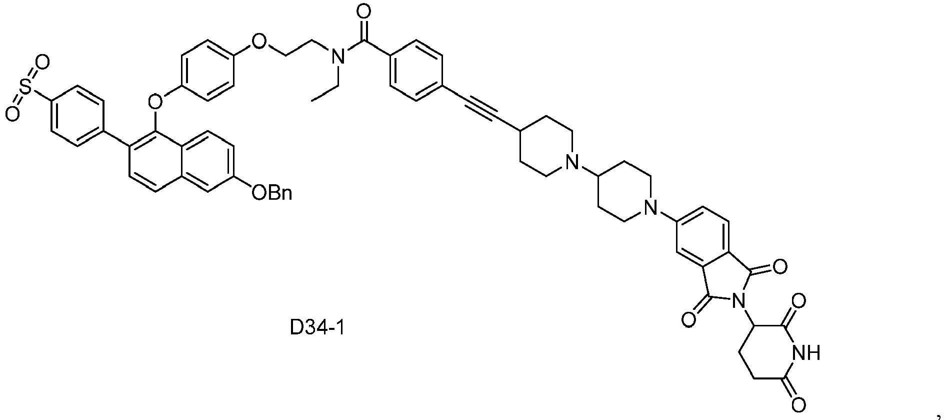 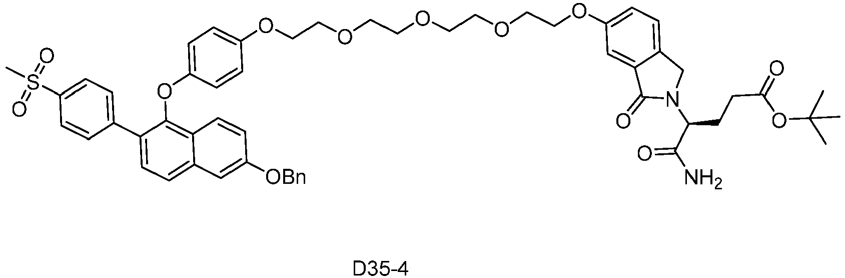 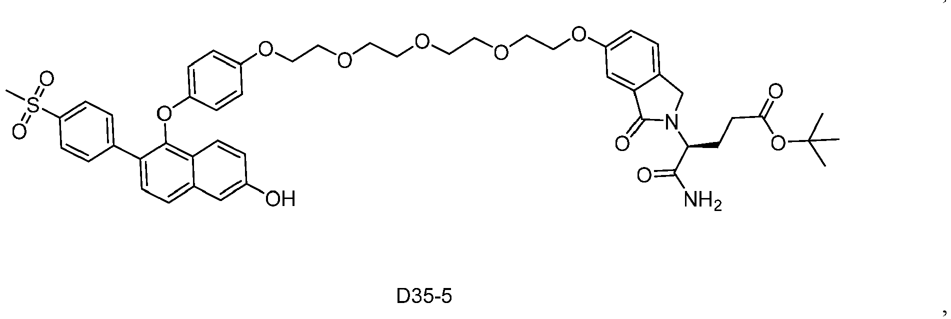 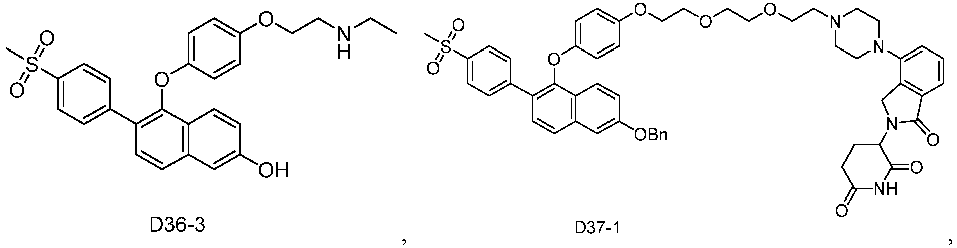 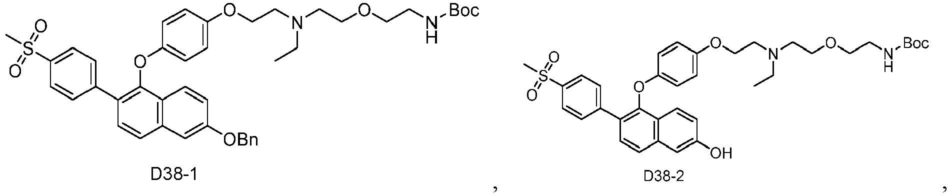 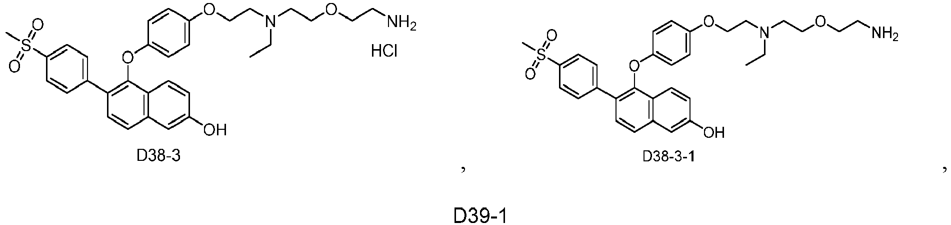 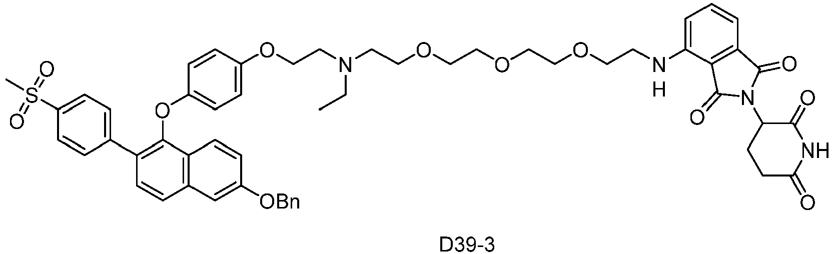 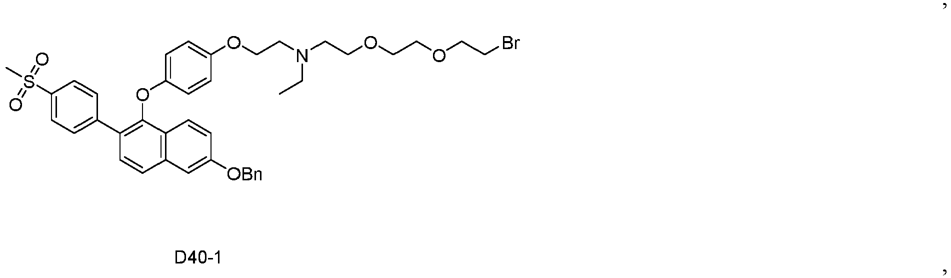 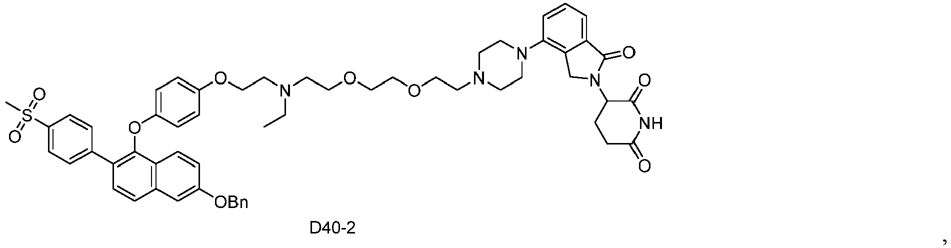 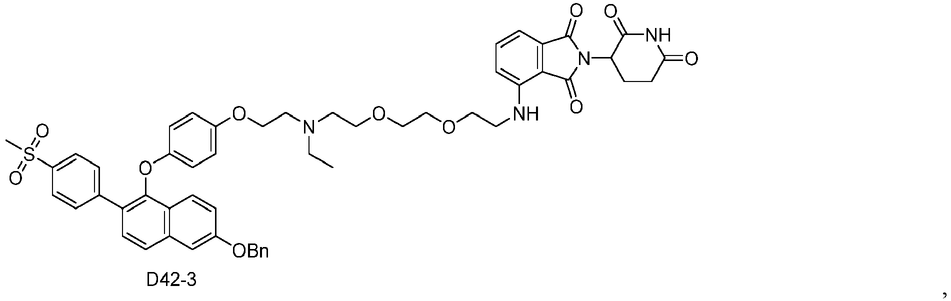 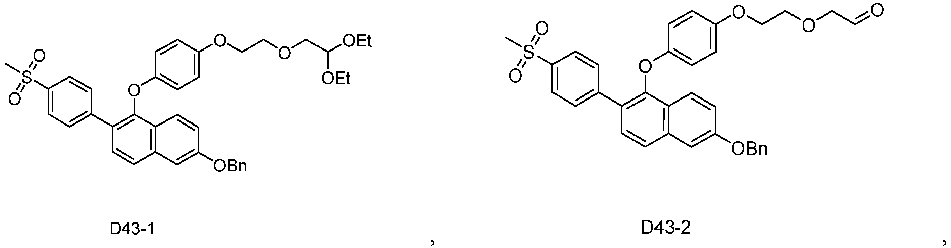 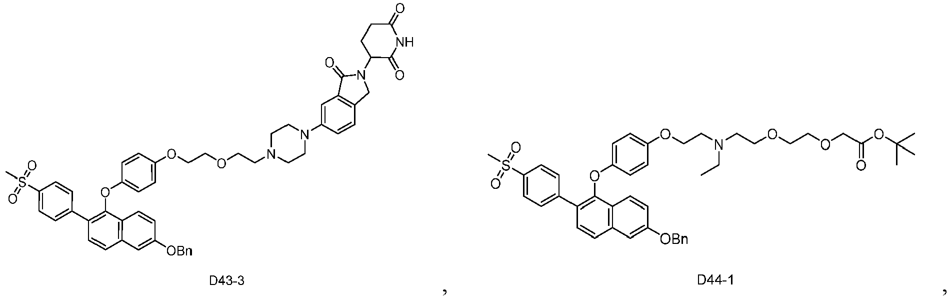 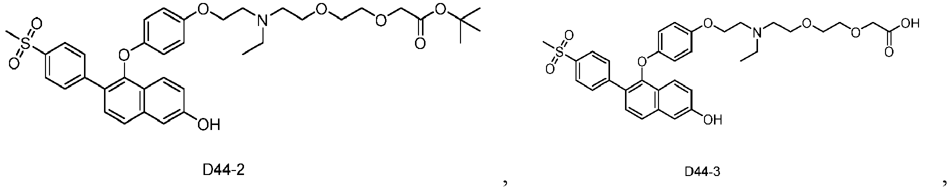 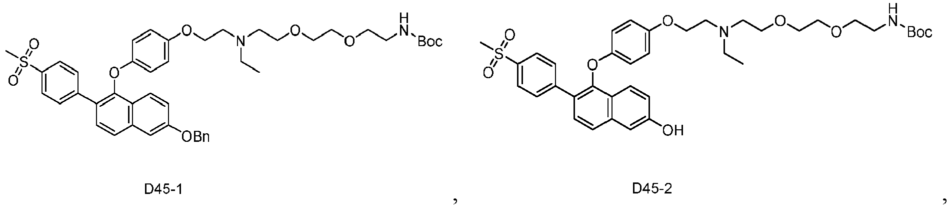 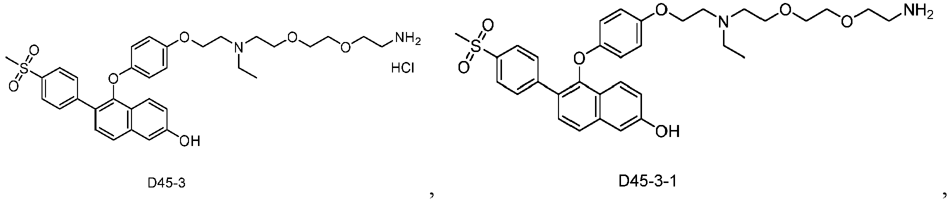 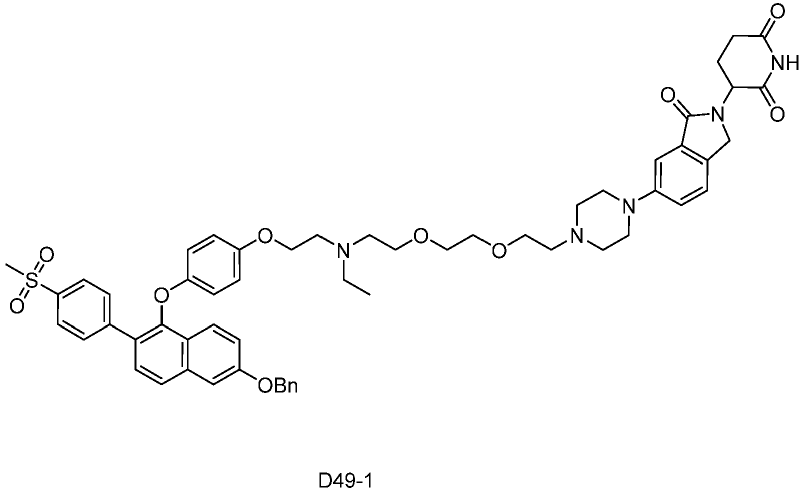 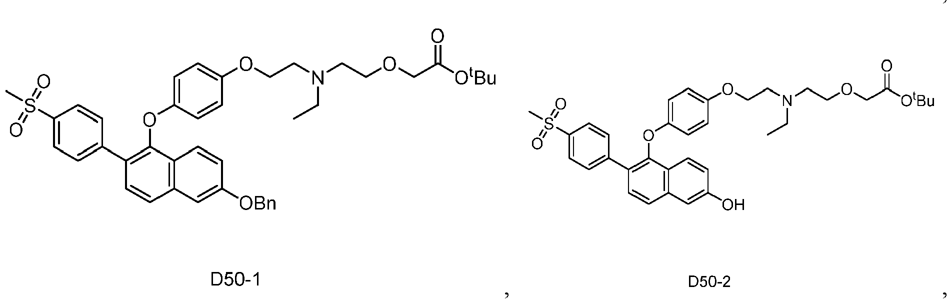 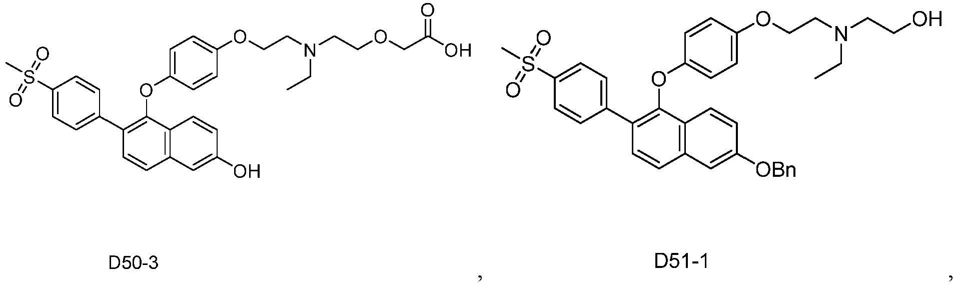 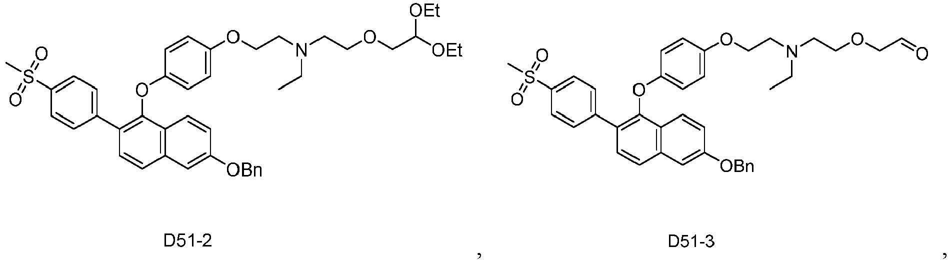 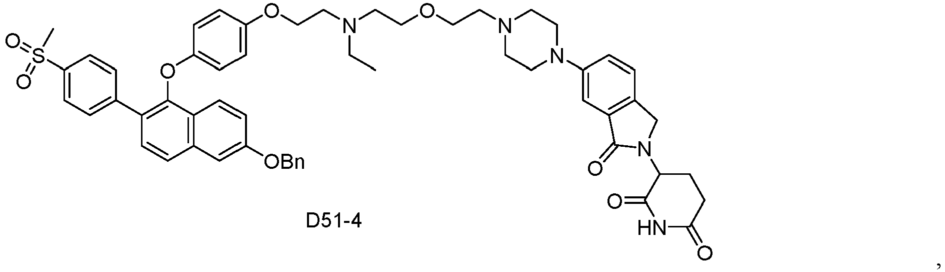 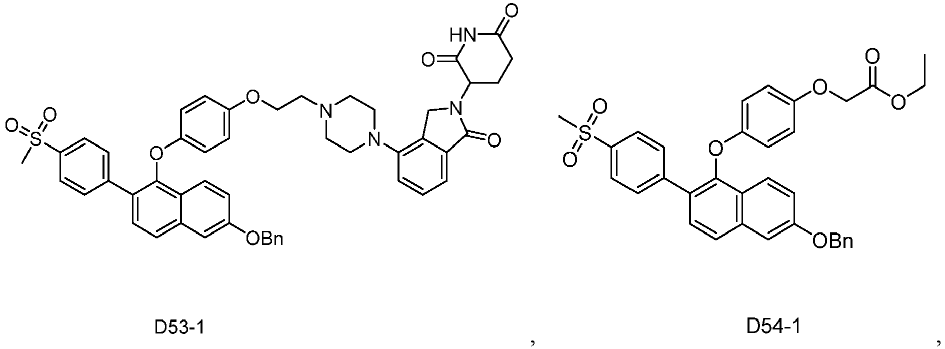 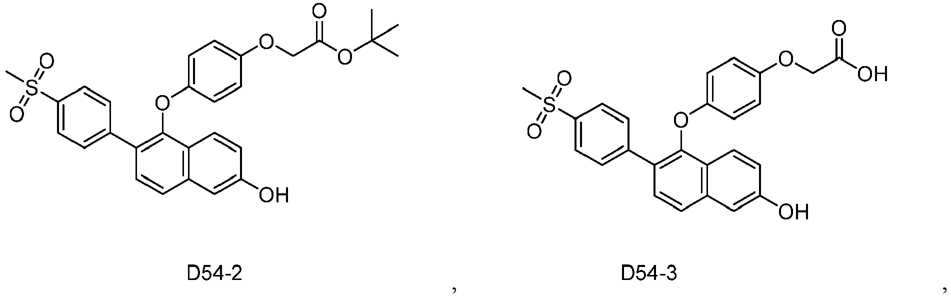 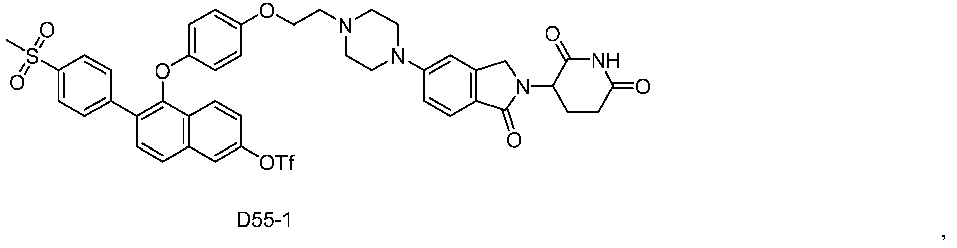 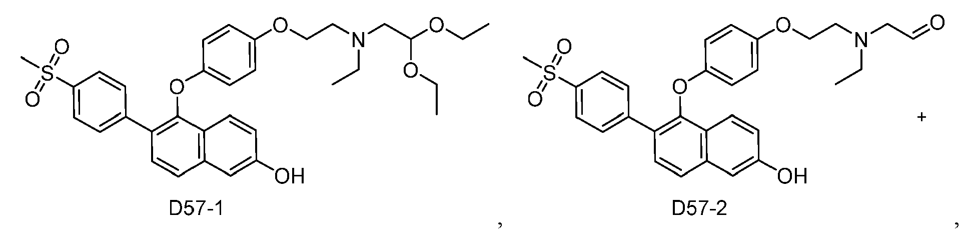 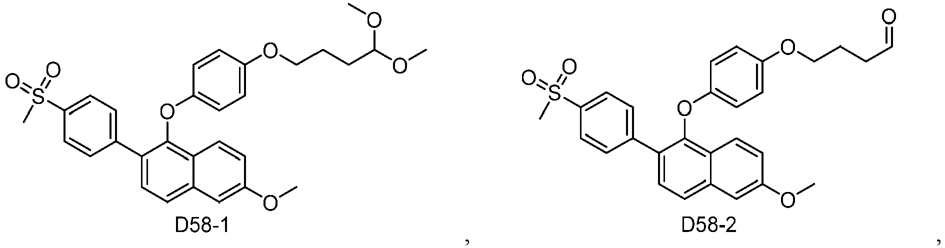 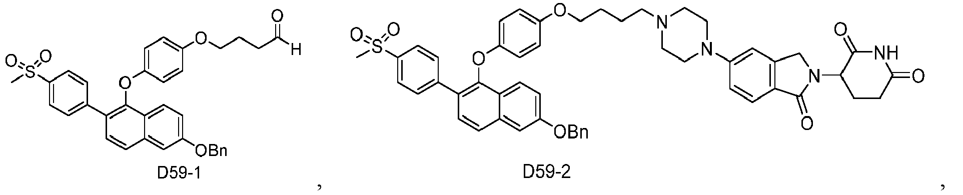 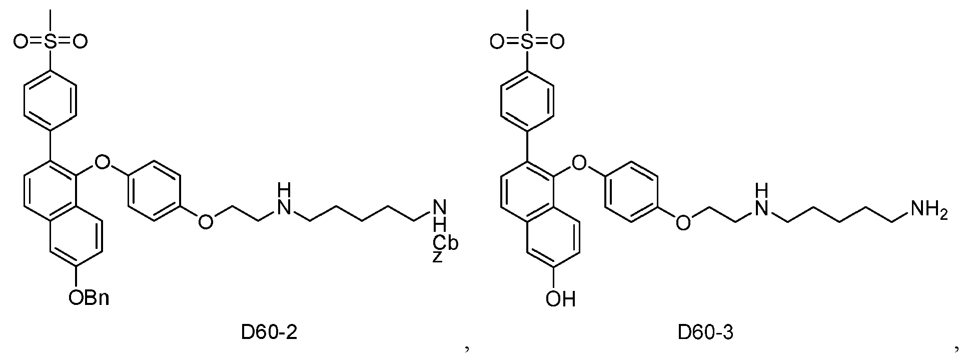 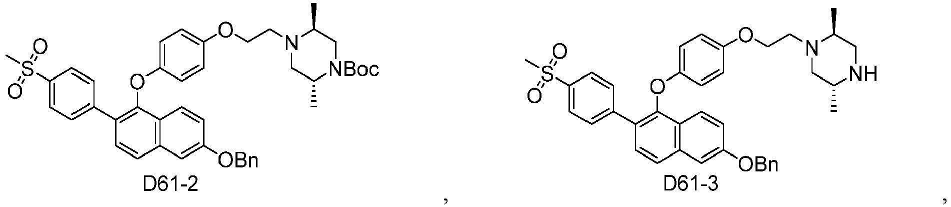 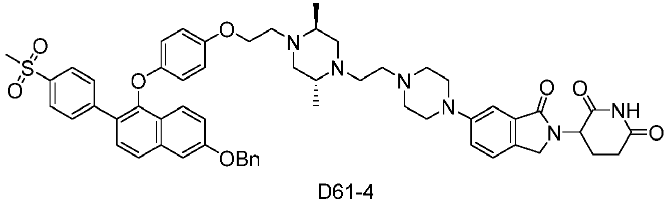 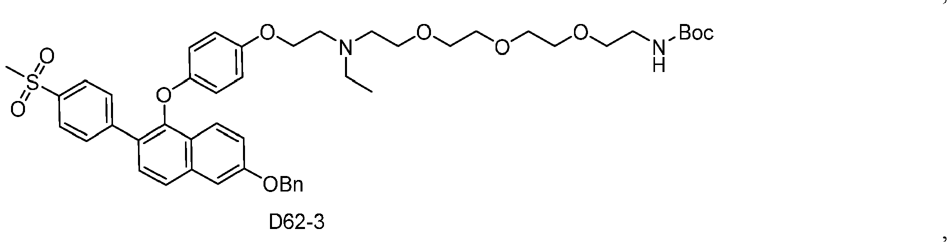 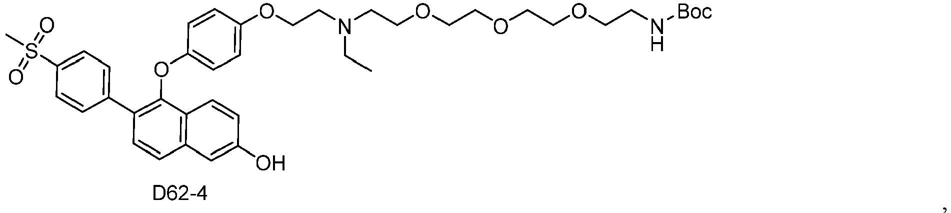 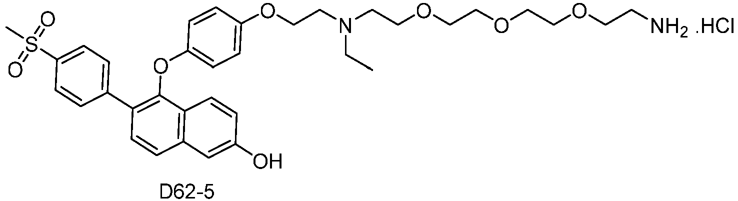 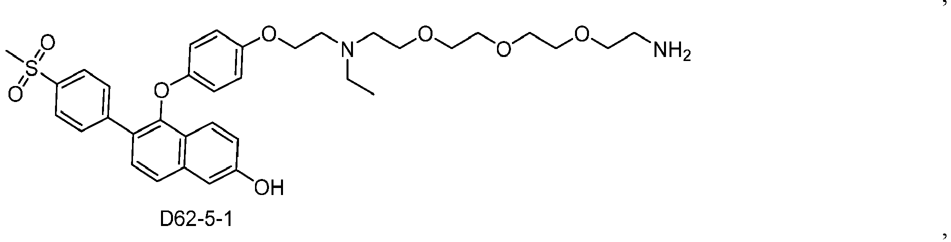 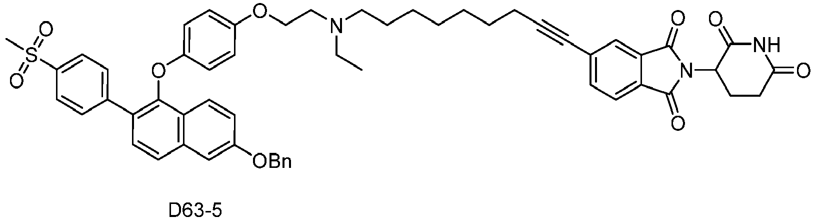 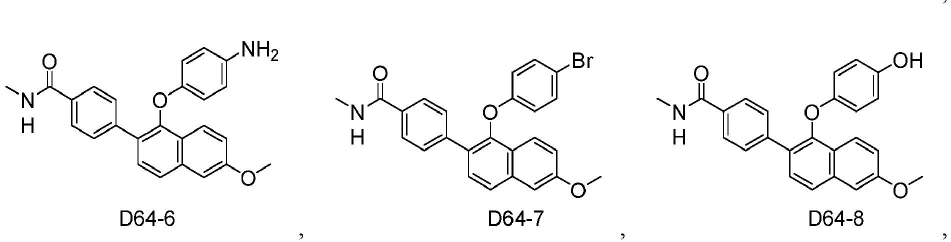 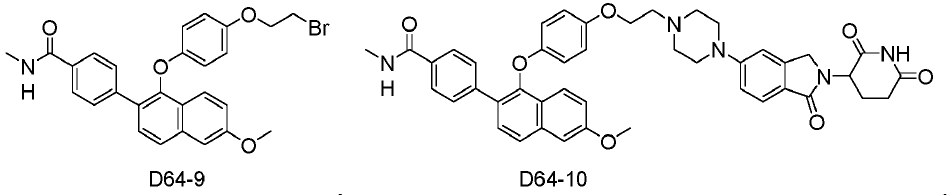 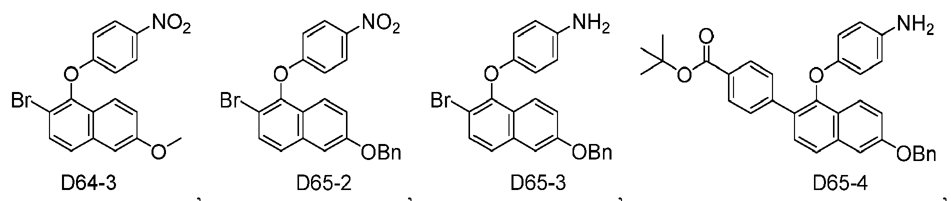 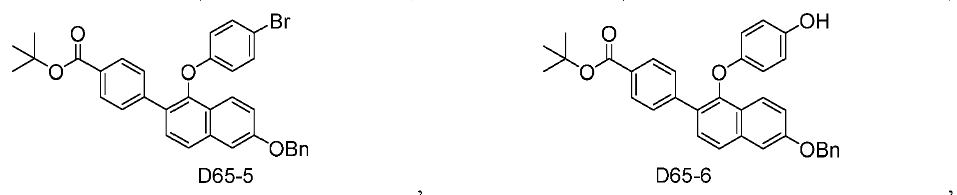 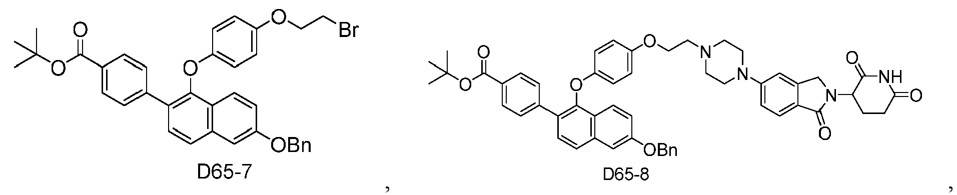 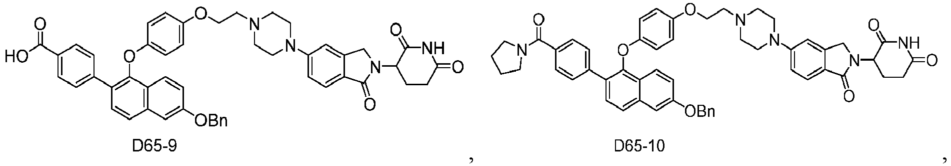 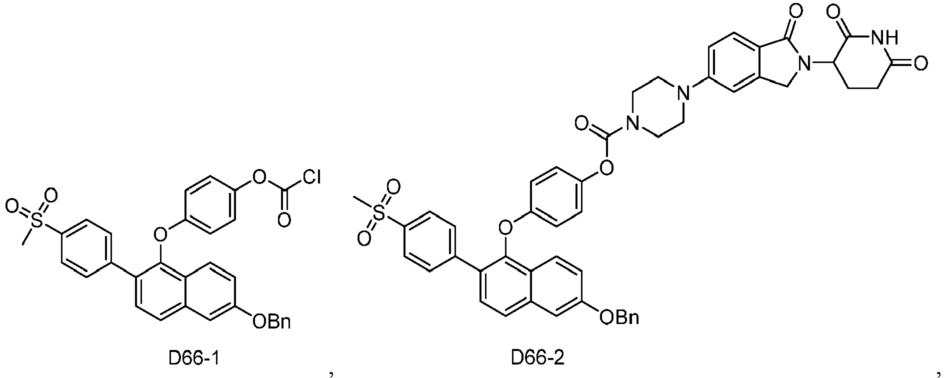 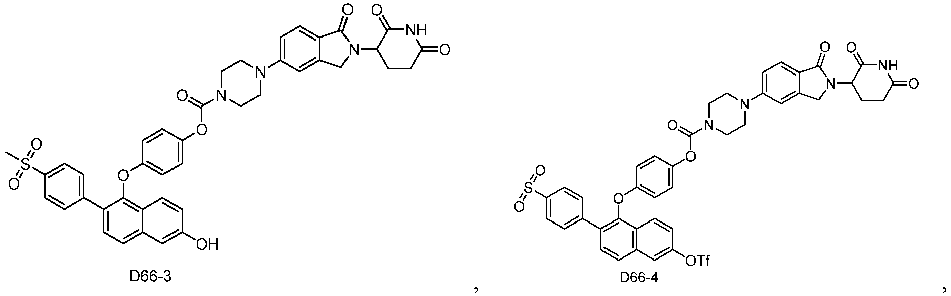 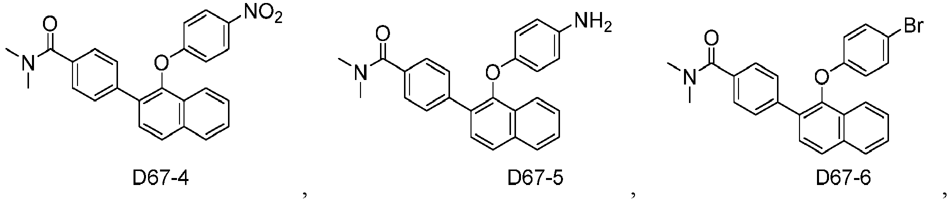 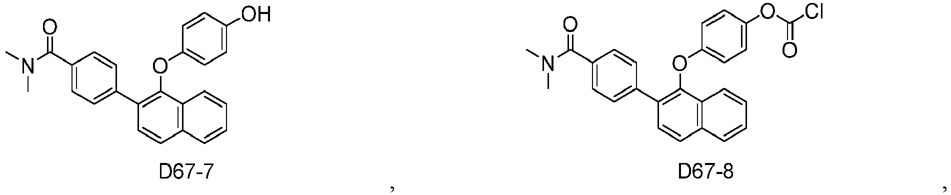 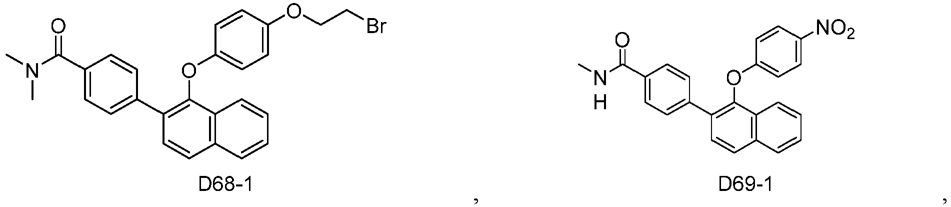 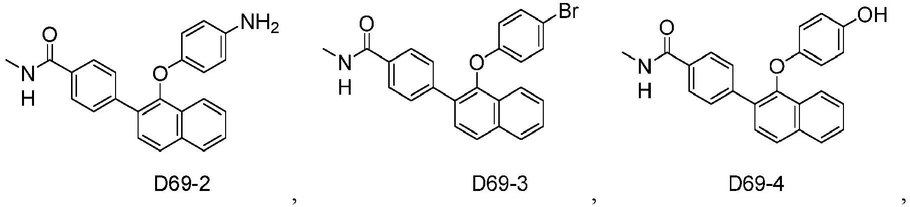 or
